# EUROPEAN PATENT APPLICATION

(11) **EP 4 360 654 A1**
(43) Date of publication of application: **01.05.2024**
(21) Application number: 22827694.5
(22) Date of filing: 24.06.2022
(51) Int. Cl.: A61K 47/54, A61K 47/64, A61K 47/65, A61P 35/00

(54) **LIGAND-DRUG CONJUGATE AND USE THEREOF**

(30) Priority: 25.06.2021 WO PCT/CN2021/102377
(71) Applicant: Coherent Biopharma (Suzhou), Limited, Industrial Park Suzhou Jiangsu 215123 (CN)
(72) Inventor: HUANG, Baohua Robert, Suzhou, Jiangsu 215123 (CN); TAN, Wei, Suzhou, Jiangsu 215123 (CN); WANG, Guitao, Suzhou, Jiangsu 215123 (CN); SHAO, Jun, Suzhou, Jiangsu 215123 (CN); MAO, Shengfei, Suzhou, Jiangsu 215123 (CN); WANG, Zhongbo, Suzhou, Jiangsu 215123 (CN); GU, Longjun, Suzhou, Jiangsu 215123 (CN); QIAN, Gang, Suzhou, Jiangsu 215123 (CN); BU, Tingting, Suzhou, Jiangsu 215123 (CN)
(74) Representative: Simmons & Simmons LLP (Munich)
(86) International application number: PCT/CN2022/101108
(87) International publication number: WO 2022/268202

(57) **Abstract**

A ligand-drug conjugate and the use of the ligand-drug conjugate.

## Description

### Technical Field

The present application relates to the field of biomedical chemistry. More specifically, the present application relates to a ligand-drug conjugate and the use of the ligand-drug conjugate.

### Background Art

Antibody-drug conjugates (ADCs) couple an antibody that can target a cell surface molecule with a biologically active molecule through chemical linkage and transport the biologically active molecule to a cell of interest by virtue of the targeting effect of the antibody, thereby improving the efficacy and reducing the toxicity of cytotoxins to normal tissues and cells. However, due to the complexity and relatively large molecular weight of the antibody in ADC, the development thereof faces many difficulties, including lack of suitable targets, difficulty in production and poor drug stability. Currently, ADCs are mainly used in the field of tumor treatment. In some instances, the affinity of a targeting antibody to a cancer cell surface antigen can reach 10⁻⁹-10⁻¹² (Kd, mole/liter). Therefore, in addition to having a high specificity to target cells, ADCs also have a high specificity to normal cells with the same targeting receptor as the target cells. Moreover, it may take a long time (1 to 3 weeks) to metabolize ADCs in vivo, during which ADCs continuously kill normal cells, leading to significantly increased toxic and side effects. Therefore, ADCs are more applicable to diseases characterized by a very large difference in the amount of cell surface antigens between tumor cells and normal cells. However, very few diseases known in the art can meet such strict requirement. In addition, in the preparation of the antibody-drug conjugate, the ratio of the drug to the antibody is usually uncertain, which affects the efficacy and toxicity evaluation of ADCs.

A drug conjugate compound that delivers a biologically active molecule to a cell of interest can also be a ligand-drug conjugate (LDC), where the ligand can be a peptide or a small molecule. Compared with an antibody-drug conjugate that use the antibody for targeting, the ligand-drug conjugate adopts a peptide or a small molecule for targeting, and the ligand that have a targeting effect and the drug in the ligand-drug conjugate usually both have exact values. However, in terms of bioavailability, stability, efficacy, toxicity, *etc*., there are various challenges in the application of LDC. For example, due to large molecular weights, lipophilicity or other properties, many ligands are incapable of entering cells, which limits their therapeutic applications. In addition, timely and precise release of biologically active molecules by means of linkers is also very important, for example, premature release of drugs may not achieve the desired therapeutic effect and may even lead to animal poisoning and death. As for the selection of biologically active molecules, ligands usually have low efficacy when conjugated with conventional chemotherapeutics (such as doxorubicin and paclitaxel), and when conjugated with highly effective drug molecules (such as MMAE, DM1, *etc*.), ligands may produce a high toxicity, thereby poisoning and even killing animals before a therapeutically effective amount for treating a tumor is achieved. Therefore, the selection of a ligand, a linker, and a bioactive molecule is a key element in the design of a LDC.

Targeting ligand design requires consideration of binding affinity, target selectivity, and compound size. Good ligand binding affinity and selectivity can reasonably reduce the drug dose required to achieve high-efficiency therapy and reduce toxic side effects. The size of the ligand also affects the delivery, including permeation, of a bioactive molecule to a cell of interest (e.g., a toxic agent to a solid tumor) through different mechanisms. A low molecular weight therapeutic drug is easier to release and diffuse, and at the same time, is more easily metabolized. Therefore, even if the drug is off-target, it can usually be excreted from the body in time, thereby reducing unwanted toxicity in normal cells. The choice of ligands is wider, but is not as direct as that of antibodies, and screening for suitable ligands often requires a lot of effort. As the linkage between the targeting ligand and the bioactive molecule, the linker needs to be carefully designed in order to retain the affinity and selectivity of the targeting ligand and the efficacy of the bioactive molecule to achieve functions such as optimal drug release, pharmacokinetics and pharmacodynamics. For the selection of a cytotoxic molecule, the release rate, cell viability, intracellular stability, *etc.* need to be considered.

Therefore, there is an urgent need in the art to obtain an improved LDC. In particular, well-designed linkers enable LDC to obtain the desired effect, and the ligand moiety and the biologically active molecule retain the desired properties and functions, thereby enhancing drug efficacy or reducing drug side effects.

### Summary of the Invention

One aspect of the present application provides a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 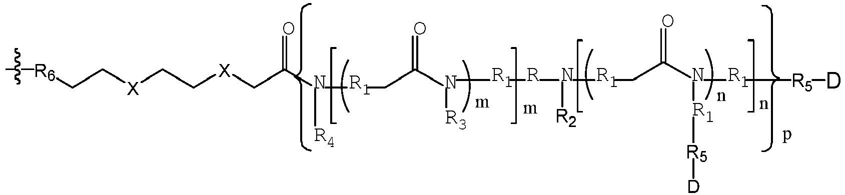 wherein:
   R is selected from 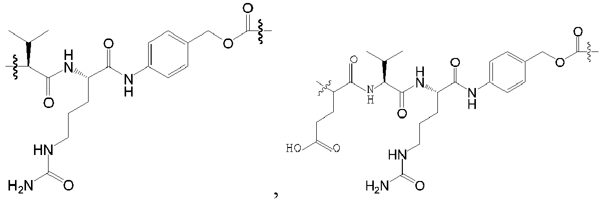 and 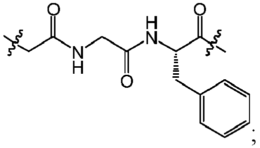
   each R₁ is independently selected from a bond and -CH₂CH₂C(O)-NH-;
   each R₂ is independently selected from hydrogen and 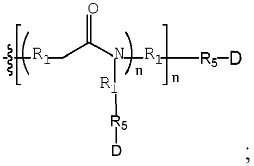
   each R₃ is independently selected from hydrogen and 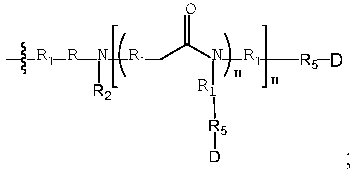
   each R₄ is independently selected from hydrogen and 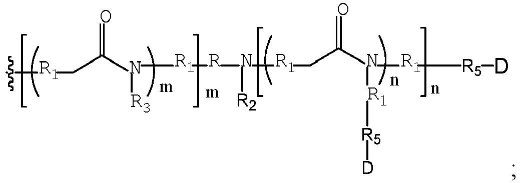
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 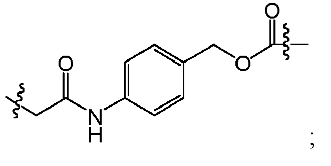
   each R₆ is independently selected from a bond, 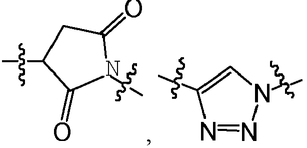 and 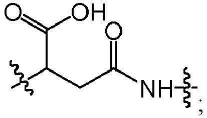
   each X is independently selected from a bond, C and O;
   each m is independently 0 or 1;
   each n is independently 0 or 1;
   p is 0 or 1;
   q is an integer from 1 to 4;
   and D is the payload.

Another aspect of the present application provides a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 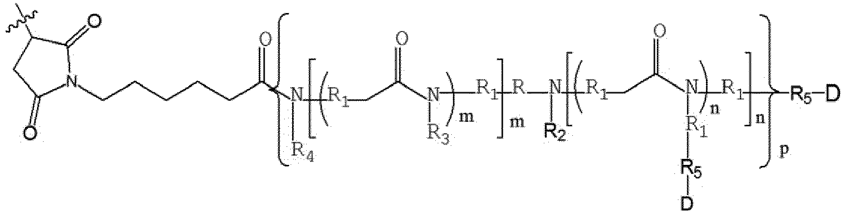 wherein:
   R is selected from 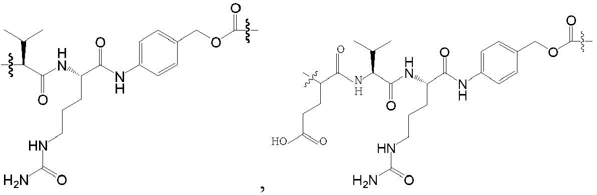 and 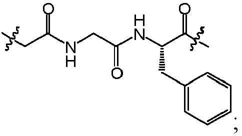
   each R₁ is independently selected from a bond and -CH₂CH₂C(O)-NH-;
   each R₂ is independently selected from hydrogen and 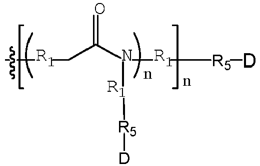 ;
   each R₃ is independently selected from hydrogen and 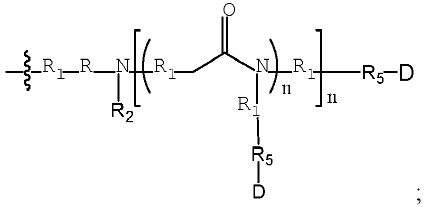
   each R₄ is independently selected from hydrogen and 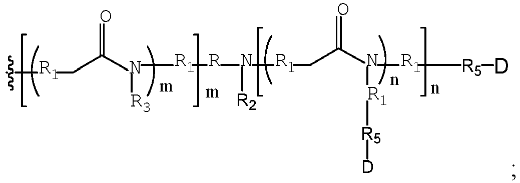
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 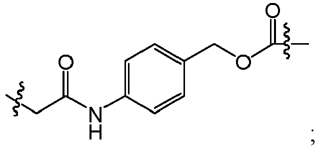
   each m is independently 0 or 1;
   each n is independently 0 or 1;
   p is 0 or 1;
   q is an integer from 1 to 4;
   and D is the payload.

Another aspect of the present application provides a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 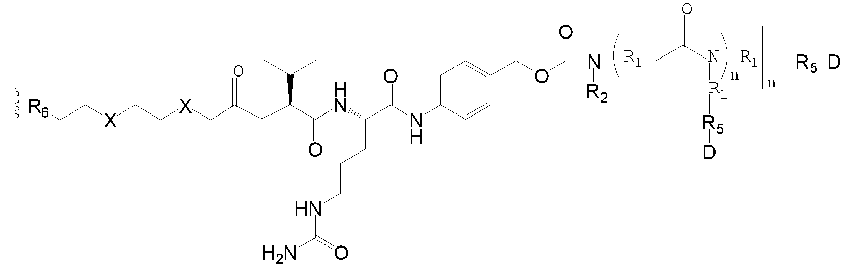 wherein:
   each R₁ is independently selected from a bond and -CH₂CH₂C(O)-NH-; ;
   each R₂ is independently selected from hydrogen and 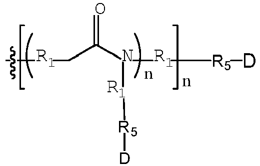
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 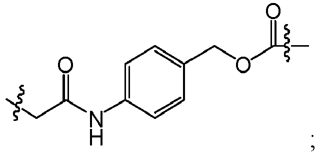
   each R₆ is independently selected from a bond, 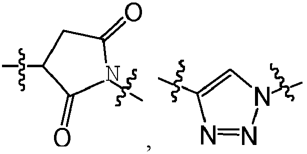 and 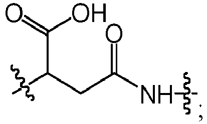
   each X is independently selected from a bond, C and O;
   each n is independently 0 or 1;
   q is an integer from 1 to 4;
   and D is the payload.

Another aspect of the present application provides a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 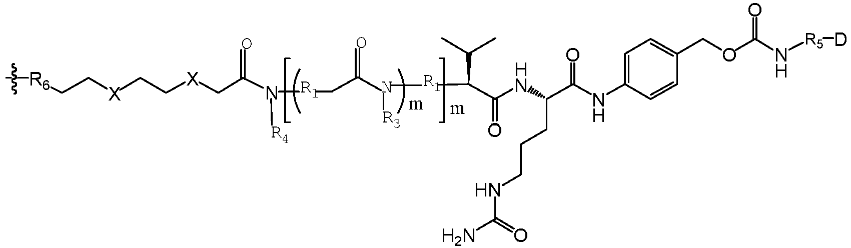 wherein:
   each R₁ is independently selected from a bond and -CH₂CH₂C(O)-NH-;
   each R₃ is independently selected from hydrogen and -R₁-R-NH-R₅-D;
   R₄ is hydrogen or 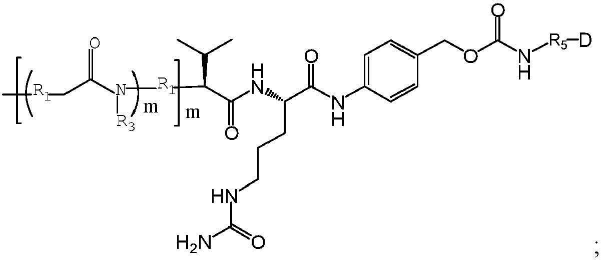
   R is selected from 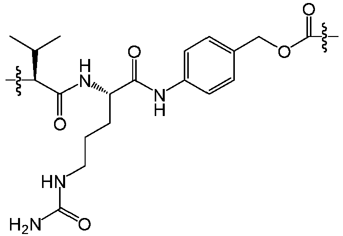 and 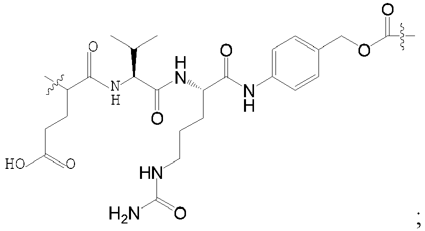
   each R₅ is independently selected from a bond, -(CH₂CH₂O)₃-CH₂CH₂-, - CH₂C(O)-NH-CH₂-, -CH₂C(O)-NH-CH₂C(O)- and -CHzC(O)-;
   each R₆ is independently selected from a bond, 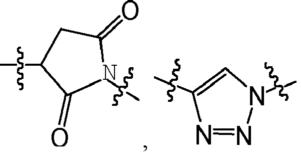 and 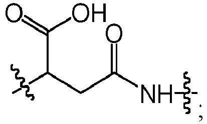
   each X is independently selected from a bond, C and O;
   each m is independently 0 or 1;
   q is an integer from 1 to 4;
   and D is the payload.

Another aspect of the present application provides a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 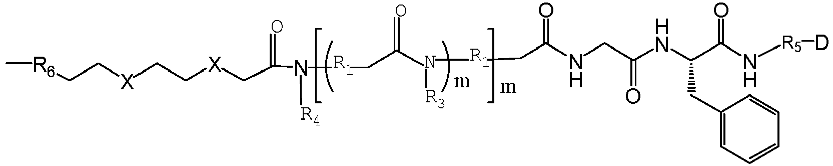 wherein:
   each R₁ is independently selected from a bond and -CH₂CH₂C(O)-NH-;
   each R₃ is independently selected from hydrogen and -R₁-R-NH-R₅-D;
   R₄ is hydrogen or 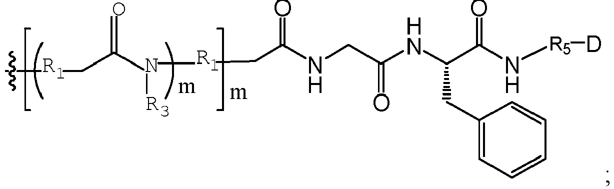
   R is 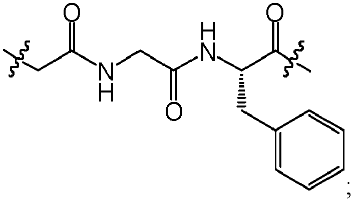
   each R₅ is independently selected from a bond, -(CH₂CH₂O)₃-CH₂CH₂-, - CH₂C(O)-NH-CH₂-, -CH₂C(O)-NH-CH₂C(O)-, -CH₂C(O)- and 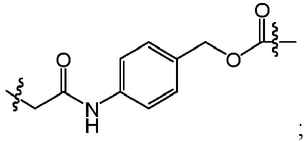
   each R₆ is independently selected from a bond, 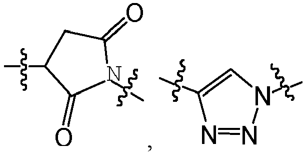 and 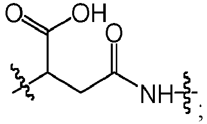
   each X is independently selected from a bond, C and O;
   each m is independently 0 or 1;
   q is an integer from 1 to 4;
   and D is the payload.

In some embodiments, R is

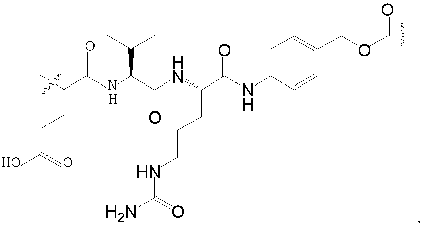

In some embodiments, R₁ is -CH₂CH₂C(O)-NH-.

In some embodiments, R₅ is -(CH₂CH₂O)₃-CH₂CH₂-. In other embodiments, R₅ is -CH₂C(O)-NH-CH₂C(O)-. In still other embodiments, R₅ is -CH₂C(O)-. In some embodiments, R₅ is 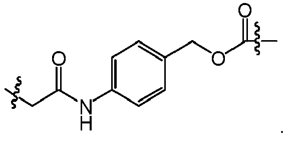 In other embodiments, R₅ is - CH₂C(O)-NH-CH₂-. In still other embodiments, R₅ is not -CH₂C(O)-NH-CH₂.

In some embodiments, each m is independently 0 or 1, and n is 0. In some embodiments, m is 0, and each n is independently 0 or 1. In some embodiments, m is 0 and n is 0. In still other embodiments, m is 0, n is 0, and R₅ is not -CH₂C(O)-NH-CH₂-.

In some embodiments, R₆ is 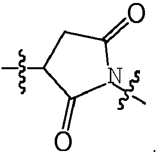 In some embodiments, R₆ is 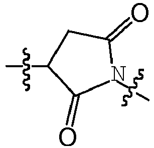 and X is a bond. In some embodiments, R₆ is 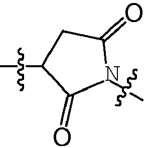 and X is O. In some embodiments, R₆ is 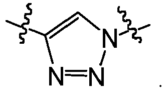 In some embodiments, R₆ is 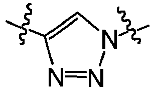 and X is a bond. In still other embodiments, R₆ is 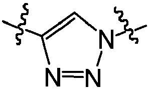 and X is O. In some embodiments, R₆ is 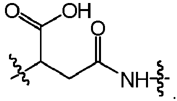 In some embodiments, R₆ is 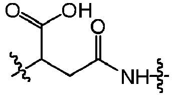 and X is a bond. In still other embodiments, R₆ is 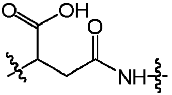 and X is O.

In some embodiments, p is 0.

In some embodiments, the linker has a structure selected from the group of:

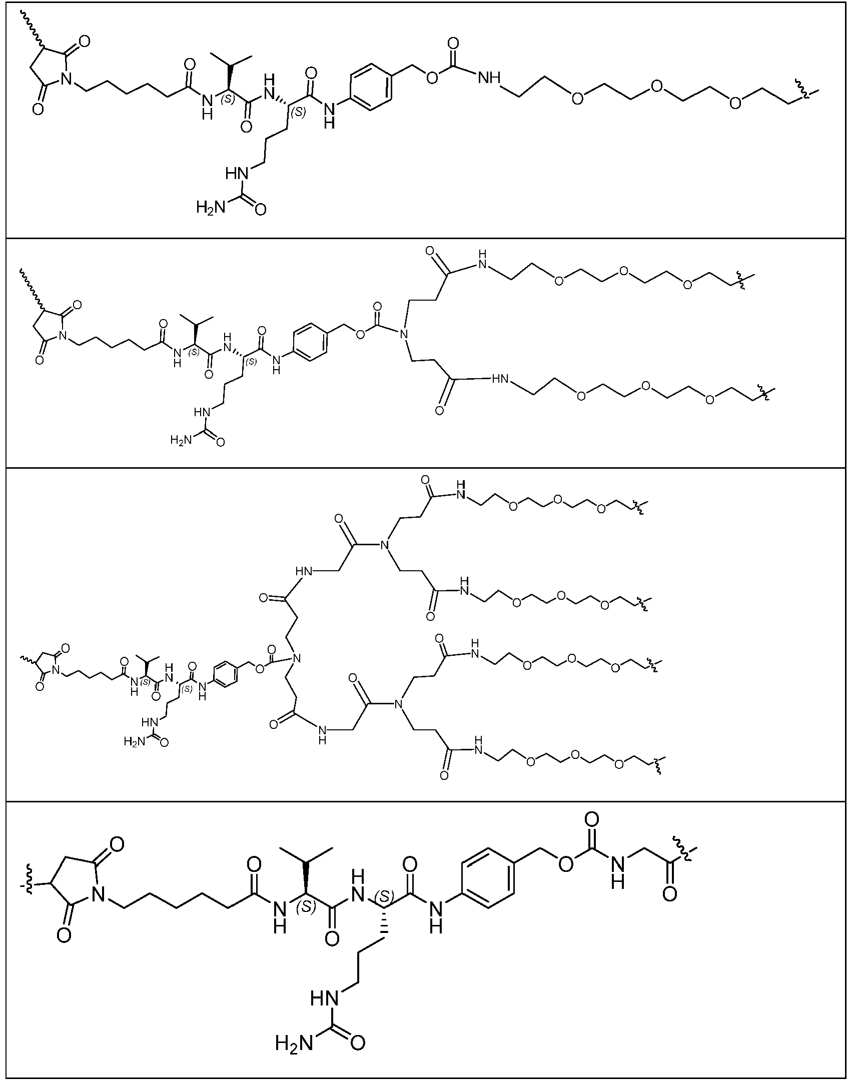

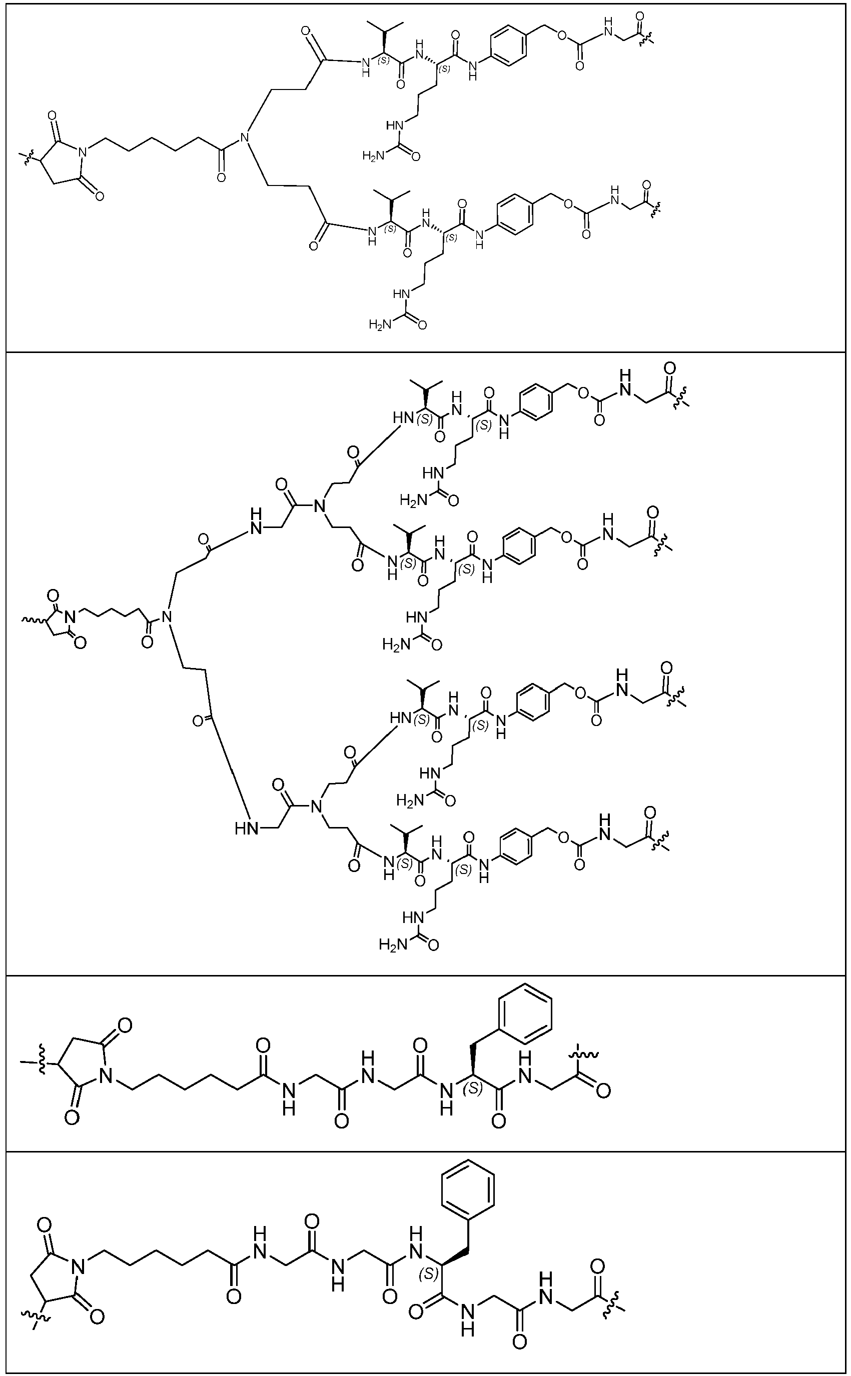

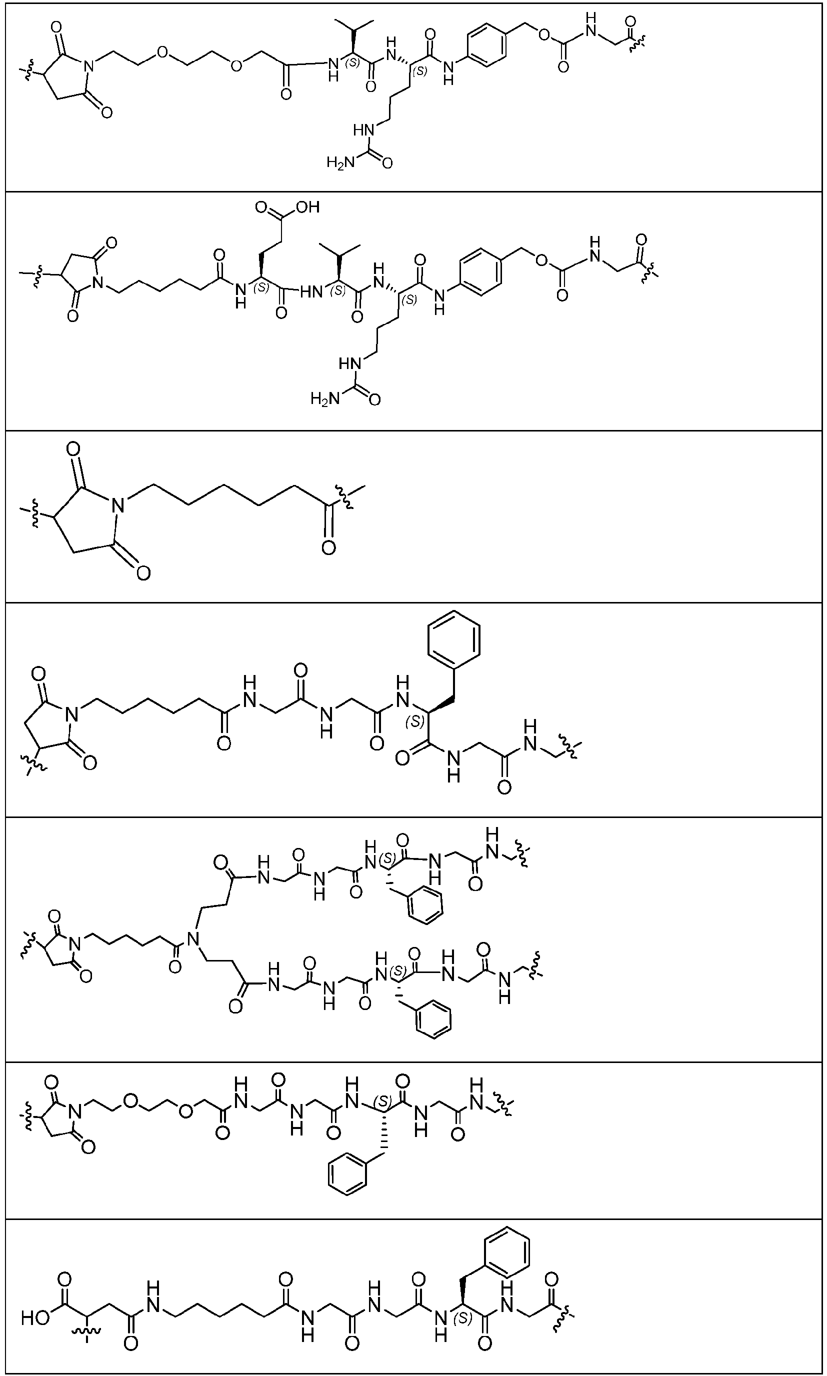

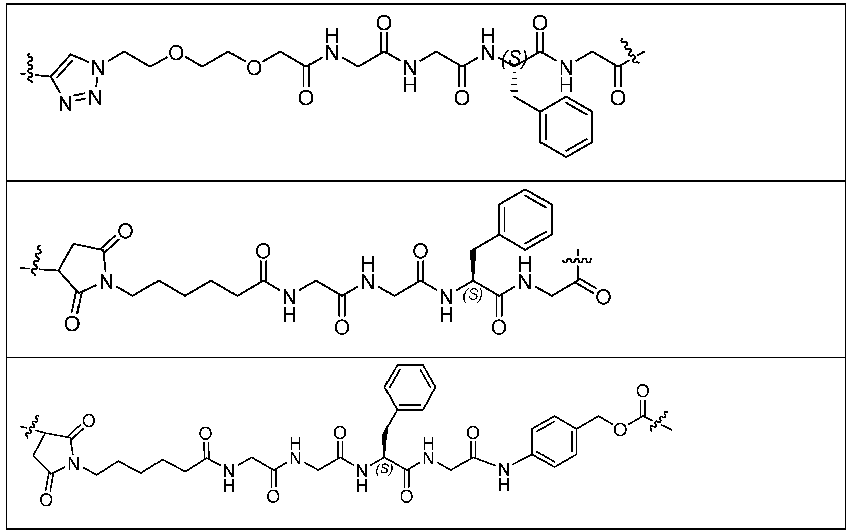

In some embodiments, the payload is selected from the group of: a small molecule compound, a nucleotide and a peptide, preferably, the payload is a small molecule compound.

In some embodiments, the small molecule compound is selected from the group of: camptothecin and any derivative thereof, maytansine and any derivative thereof, a radioactive metal complex, a cyclooxygenase-2 inhibitor and any derivative thereof, paclitaxel and any derivative thereof, epothilone and any derivative thereof, bleomycin and any derivative thereof, dactinomycin and any derivative thereof, plicamycin and any derivative thereof, and mitomycin C and any derivative thereof. In some preferred embodiments, the small molecule compound is camptothecin and any derivative thereof. In some preferred embodiments, the camptothecin is exatecan.

In some embodiments, each D is independently selected from

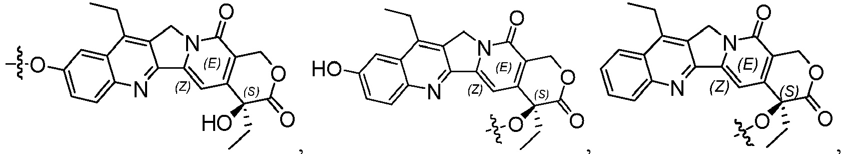

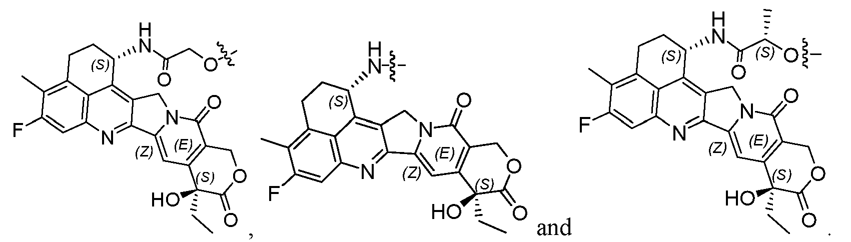

In some embodiments, the cell surface molecule is selected from TRPV6, FOLR1, PSMA and SSTR2.

In some embodiments, the cell surface molecule is TRPV6 and FOLR1, PSMA and FOLR1, or SSTR2 and FOLR1, respectively. In some preferred embodiments, the cell surface molecule is TRPV6 and FOLR1, respectively. In some embodiments, the ligand targeting FOLR1 is selected from folic acid or an analogue thereof. In some preferred embodiments, the folic acid analogue is selected from 5-methyltetrahydrofolate, 5-formyltetrahydrofolate, methotrexate , and 5,10-methylenetetrahydrofolate. In some embodiments, the ligand targeting SSTR2 is selected from octreotide and any analogue thereof.

In some embodiments, the ligand moiety comprises more than three ligands that target cell surface molecules. In some preferred embodiments, the cell surface molecule is selected from TRPV6, FOLR1, PSMA and SSTR2.

In some embodiments, the ligands targeting different cell surface molecules are linked to each other directly, or via a spacer. In some embodiments, the spacer consists of amino acids. In some preferred embodiments, the spacer consists of natural amino acids or unnatural amino acids. In still other preferred embodiments, the spacer is glycine. In some embodiments, the spacer is cleavable under a certain physiological environment by protease cleavage or reduction.

In some embodiments, the ligand moiety has the following structure:

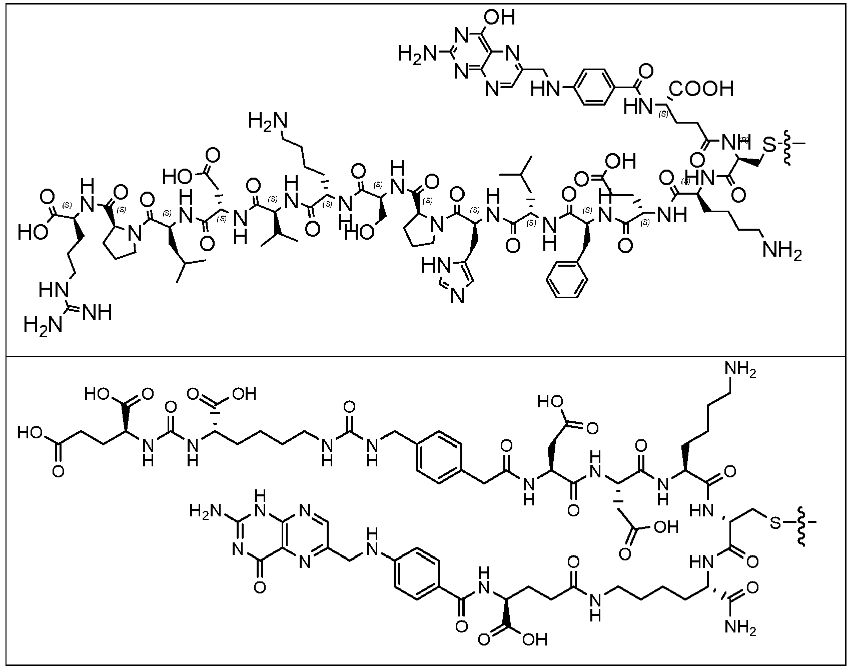

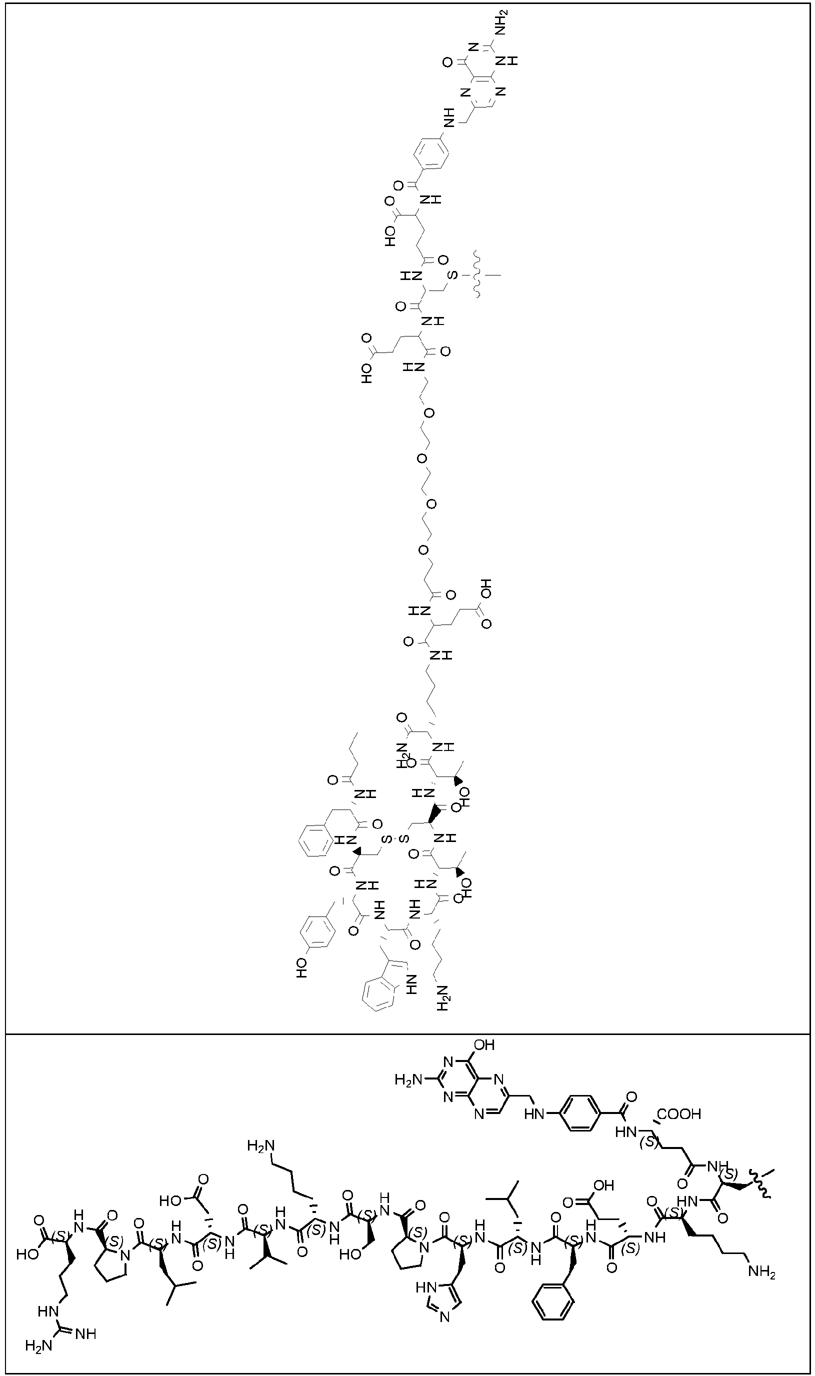

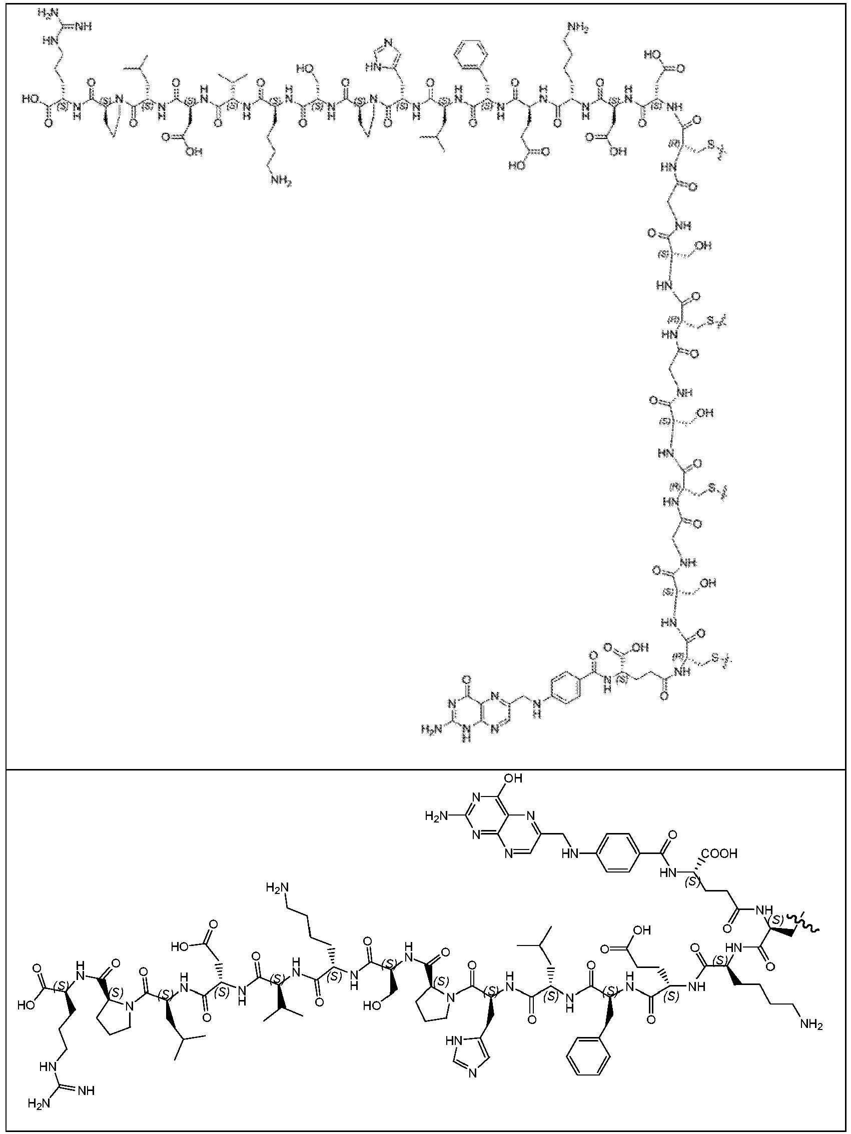

In some embodiments, q is 1. In some embodiments, q is 2. In some embodiments, q is 3. In some embodiments, q is 4.

One aspect of the present application provides a conjugate compound or a pharmaceutically acceptable salt thereof, the conjugate compound is selected from:

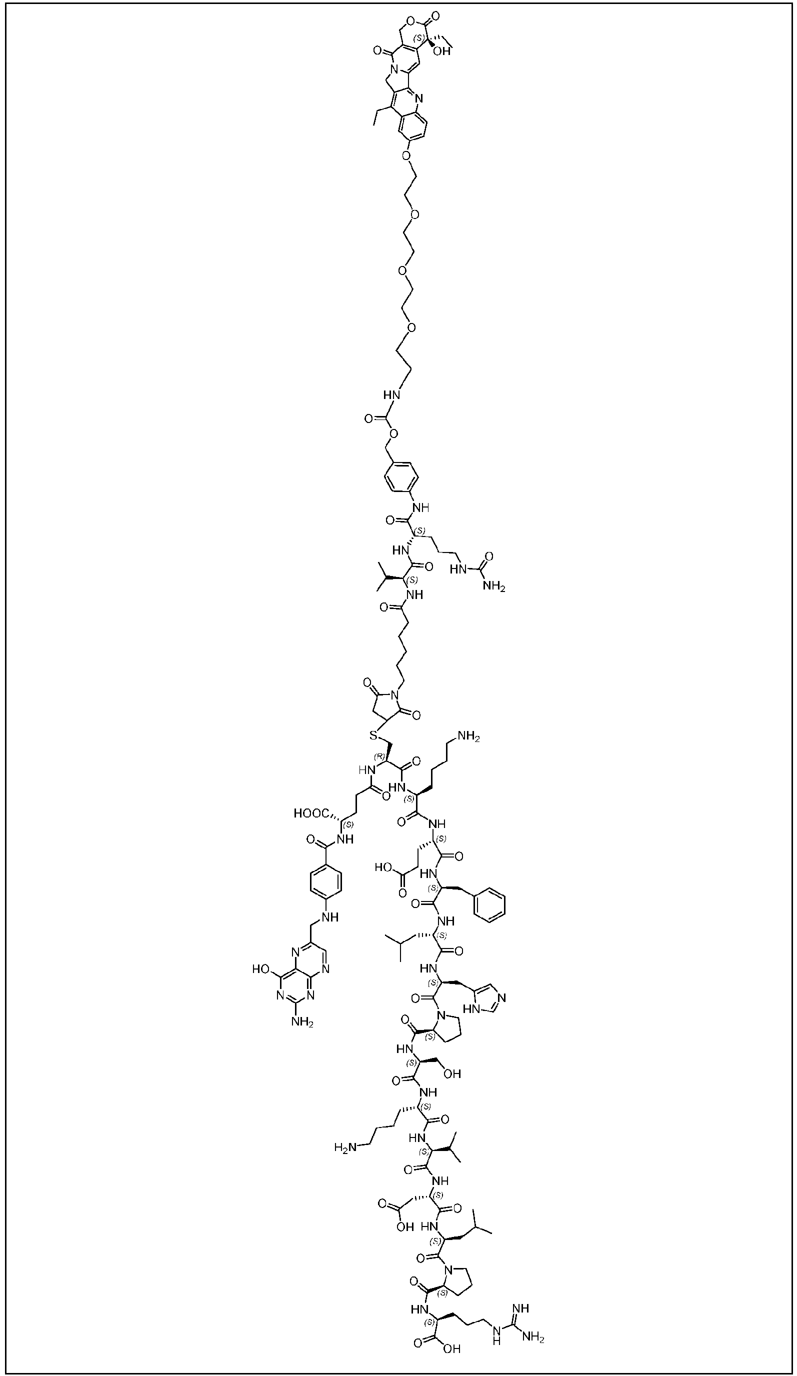

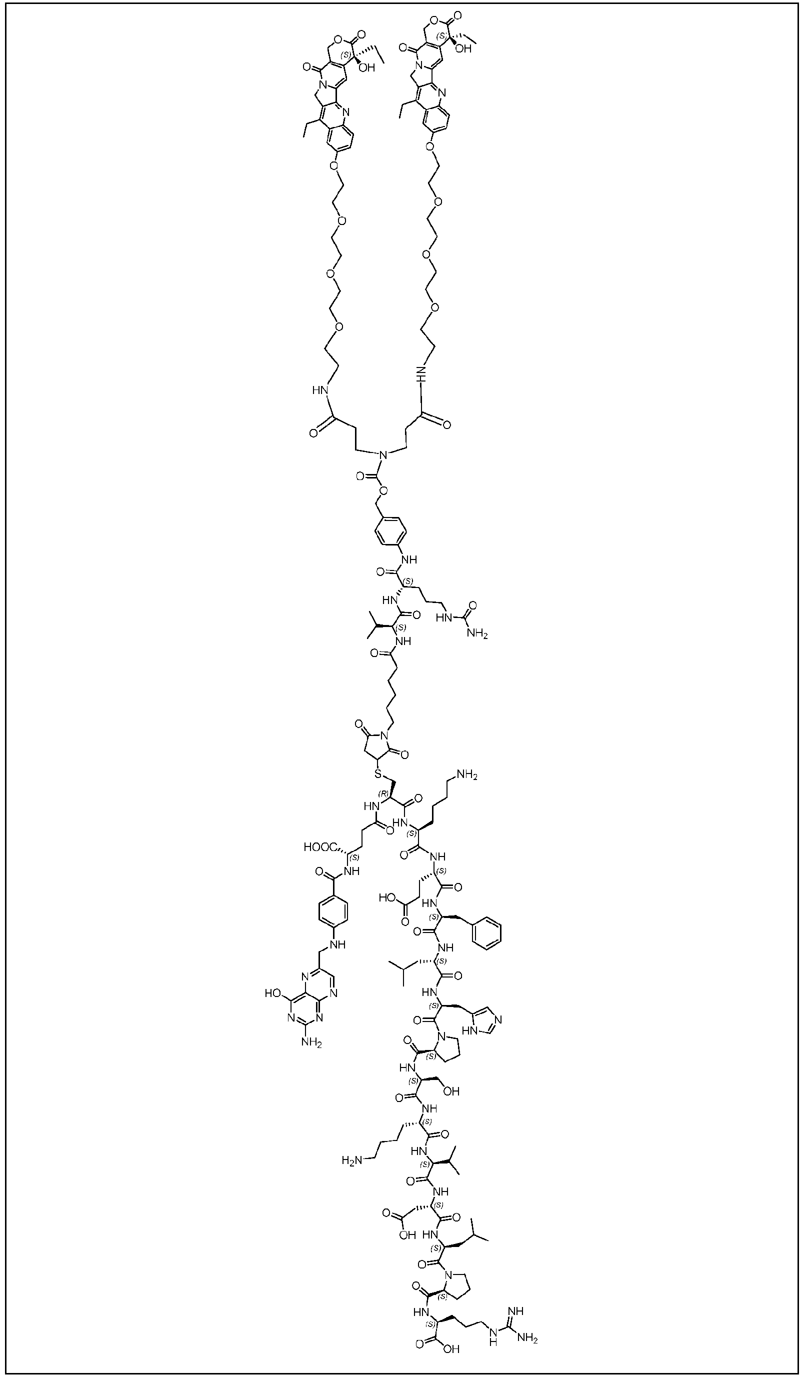

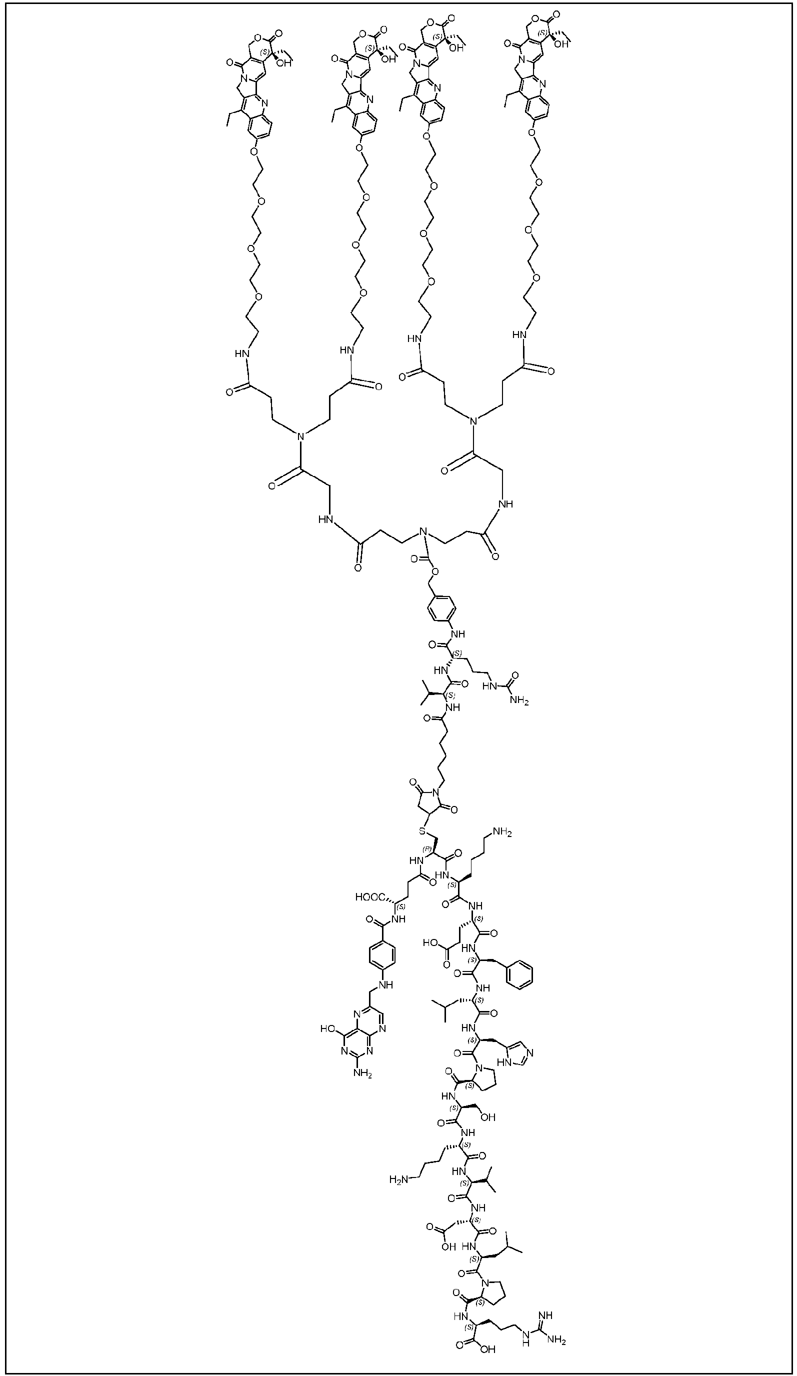

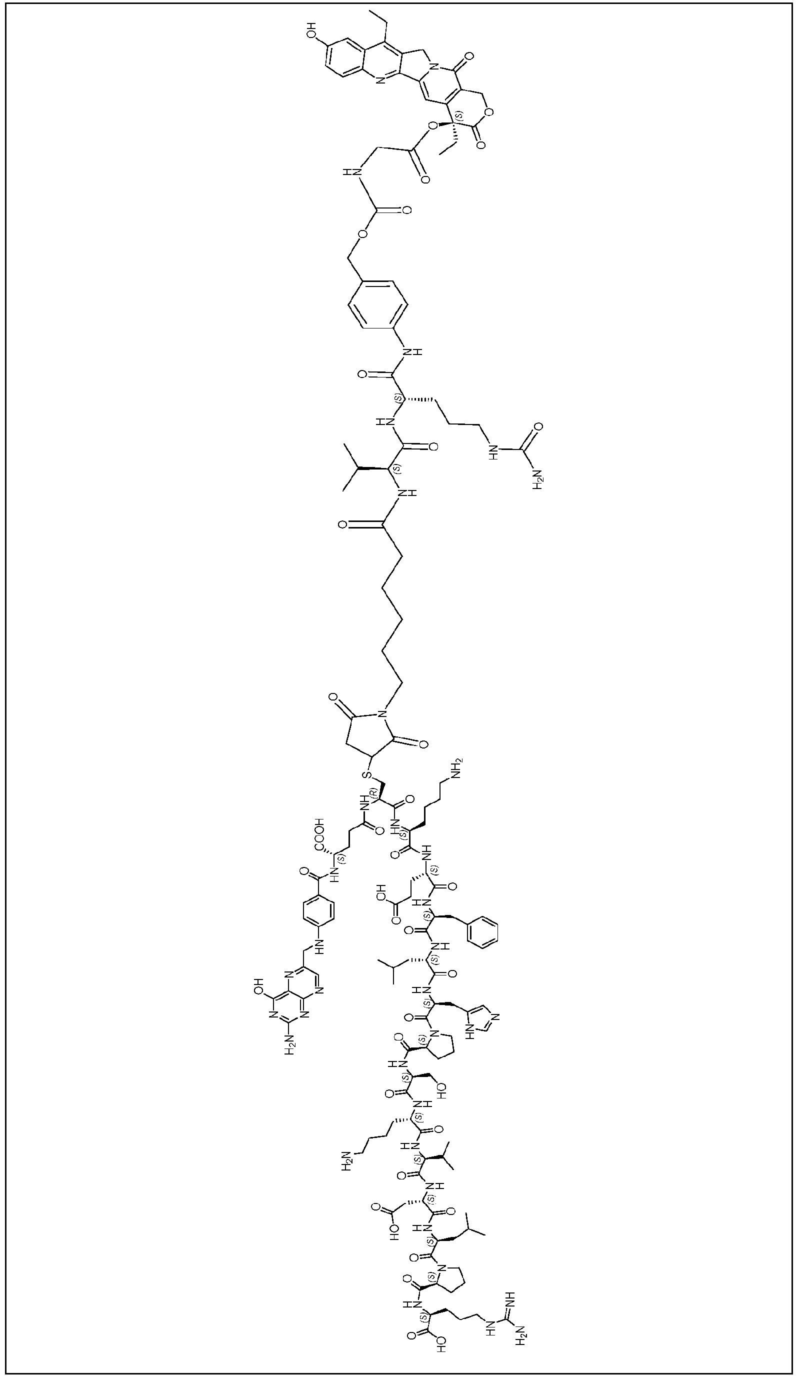

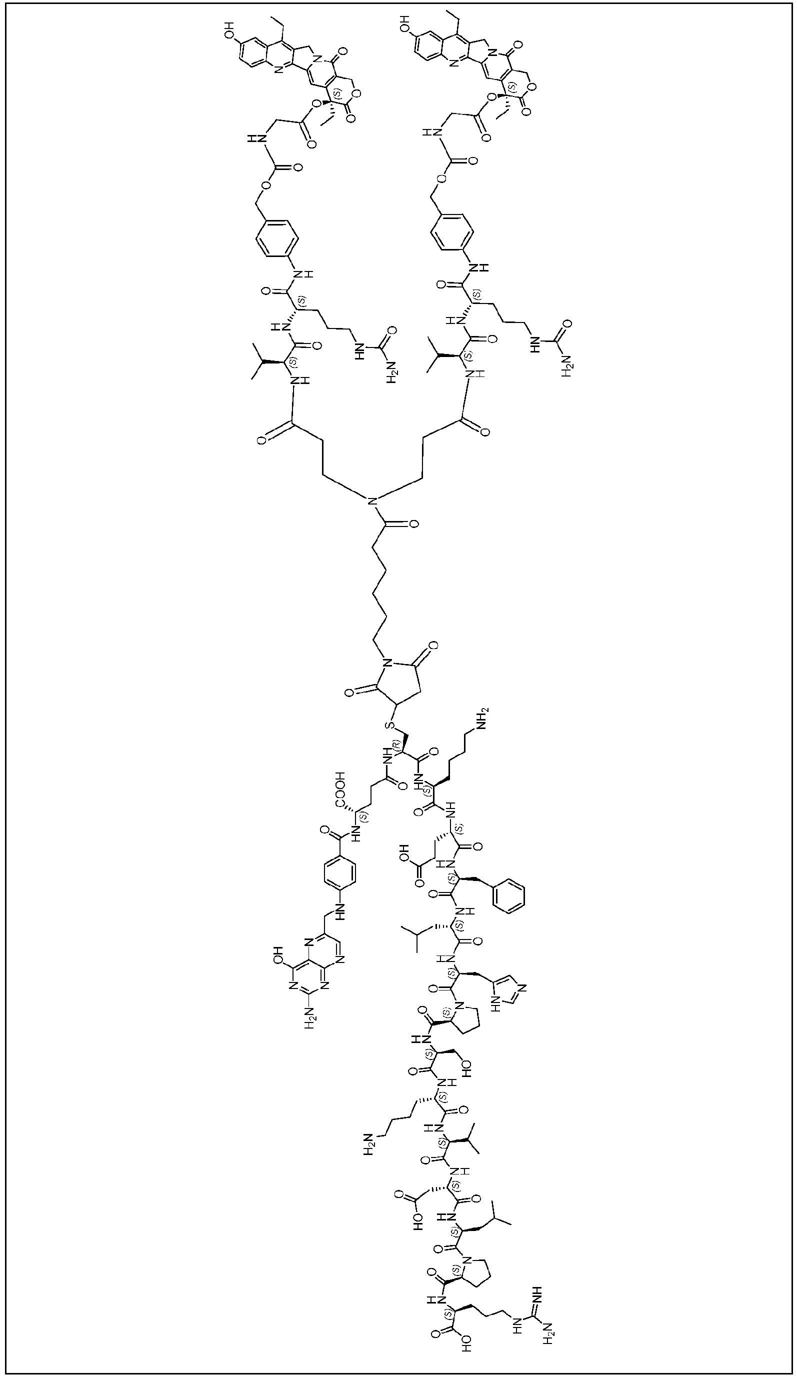

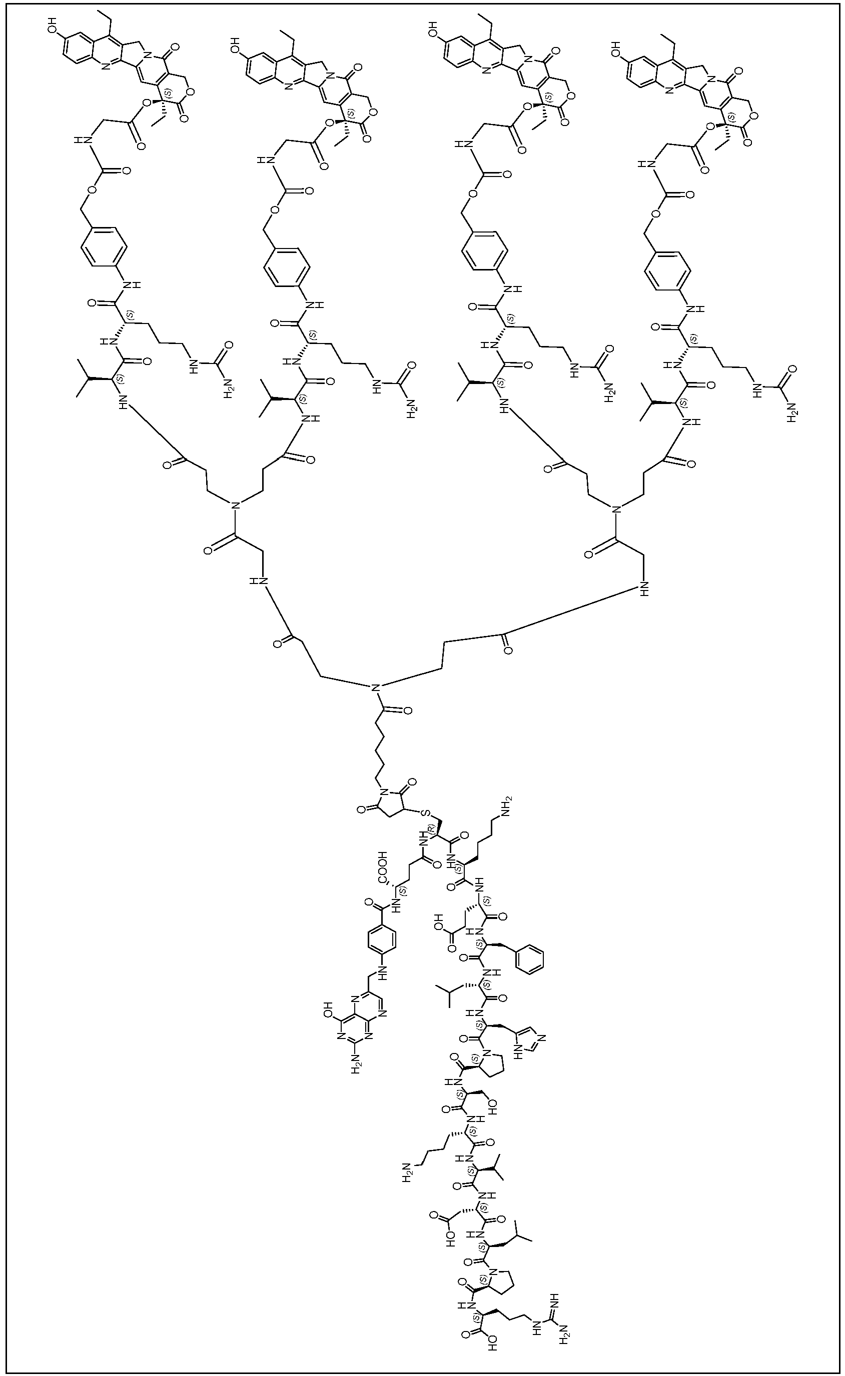

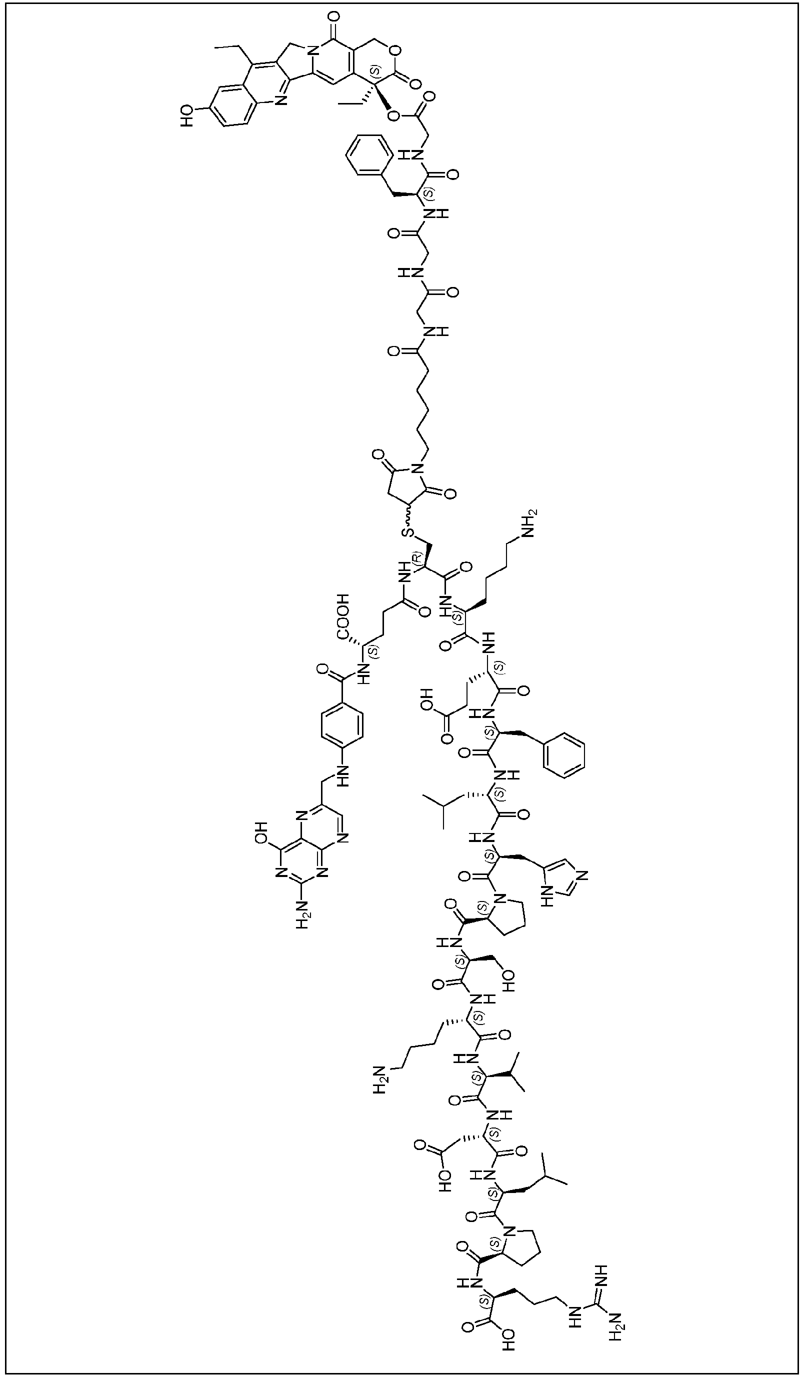

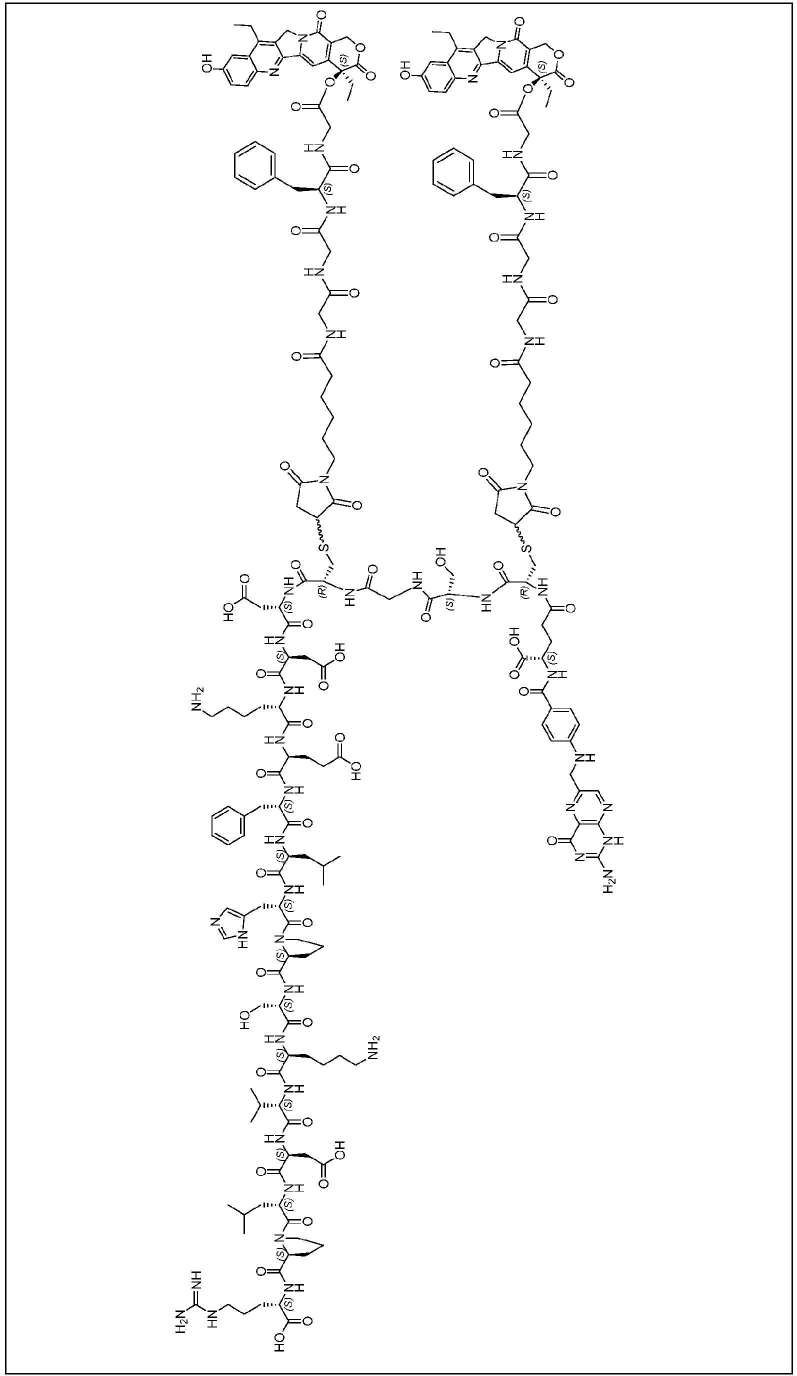

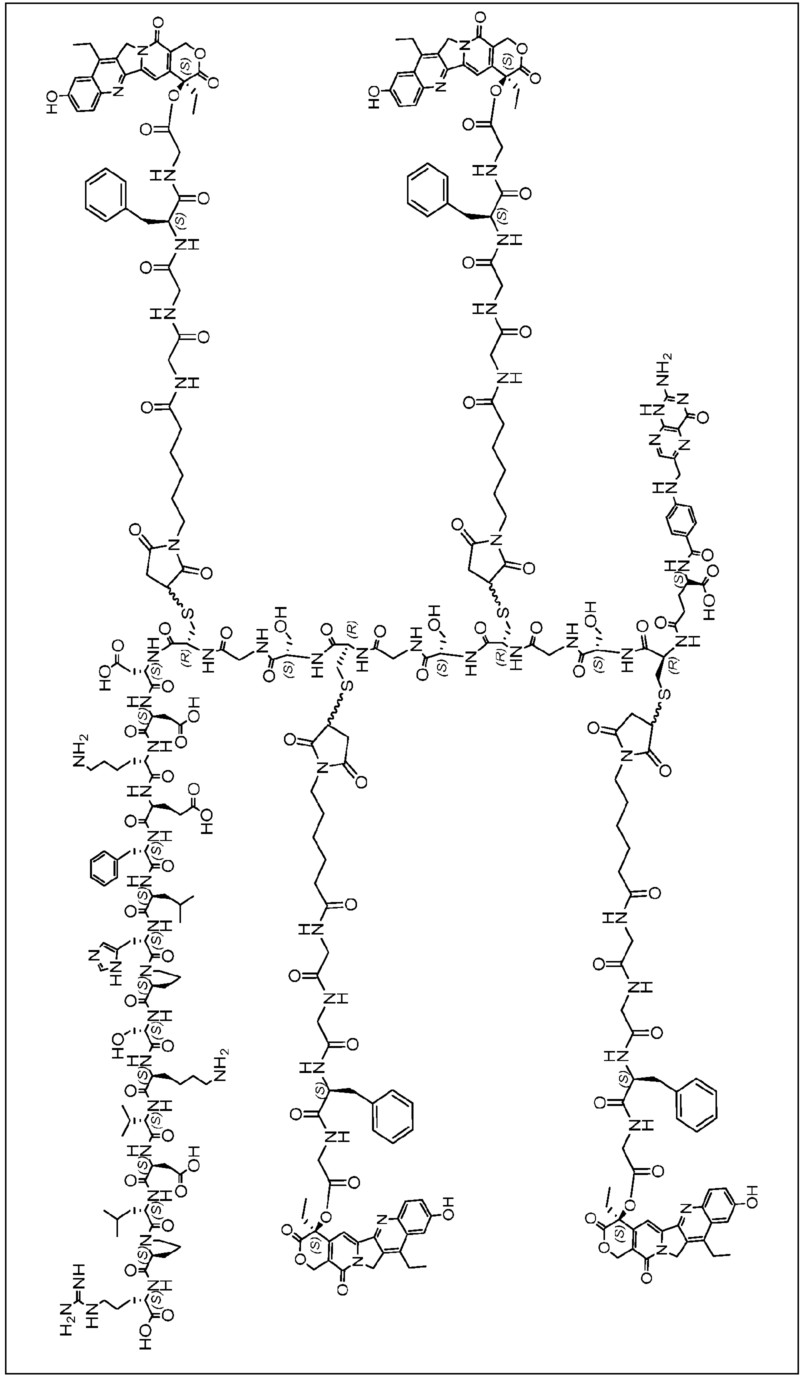

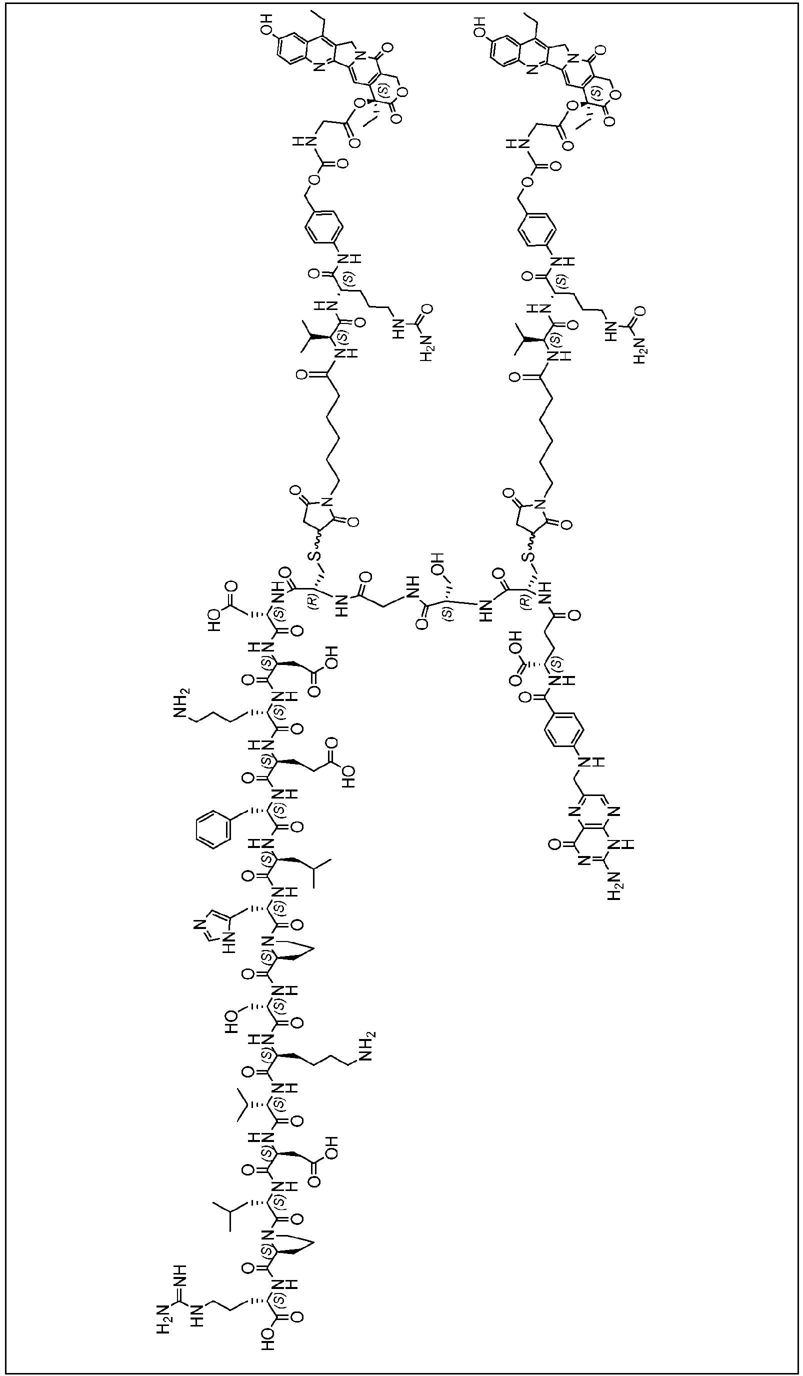

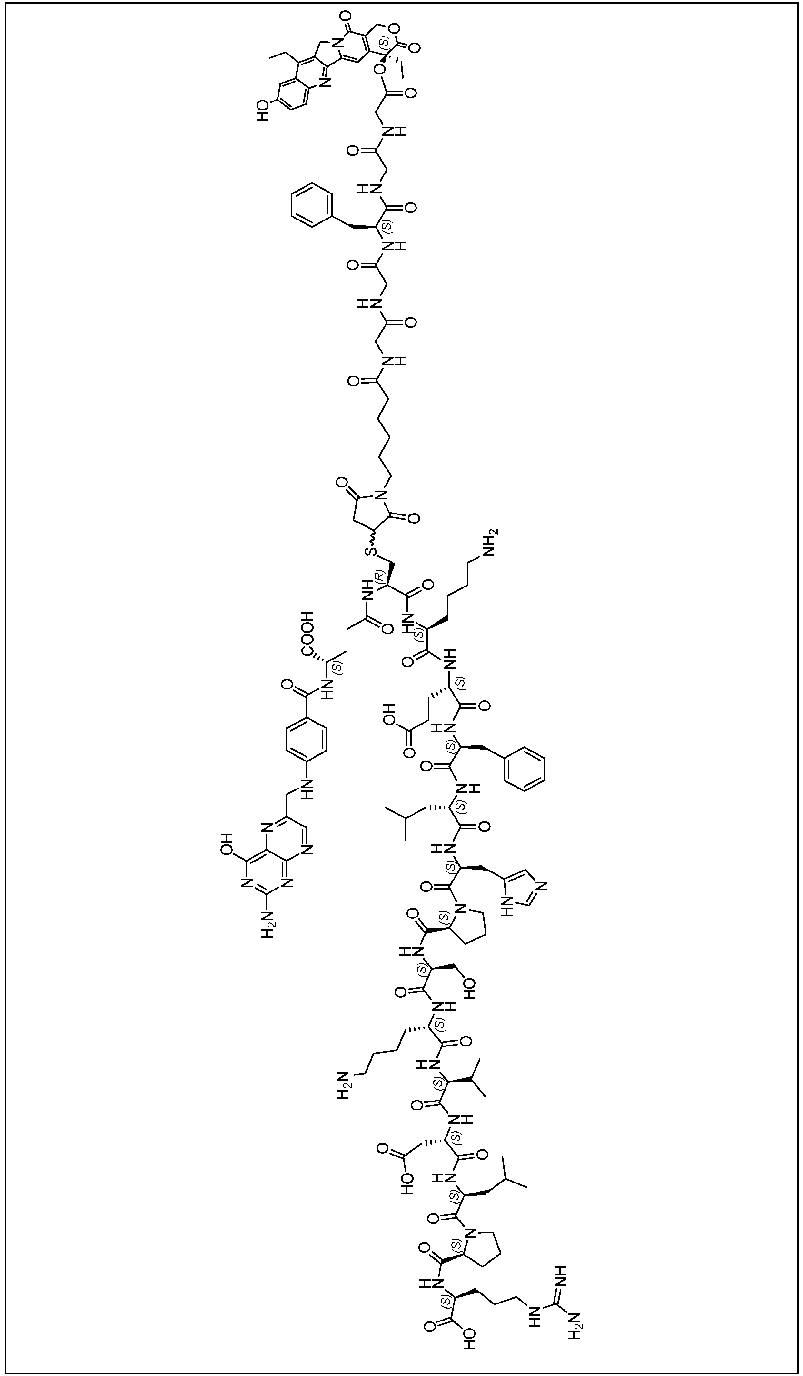

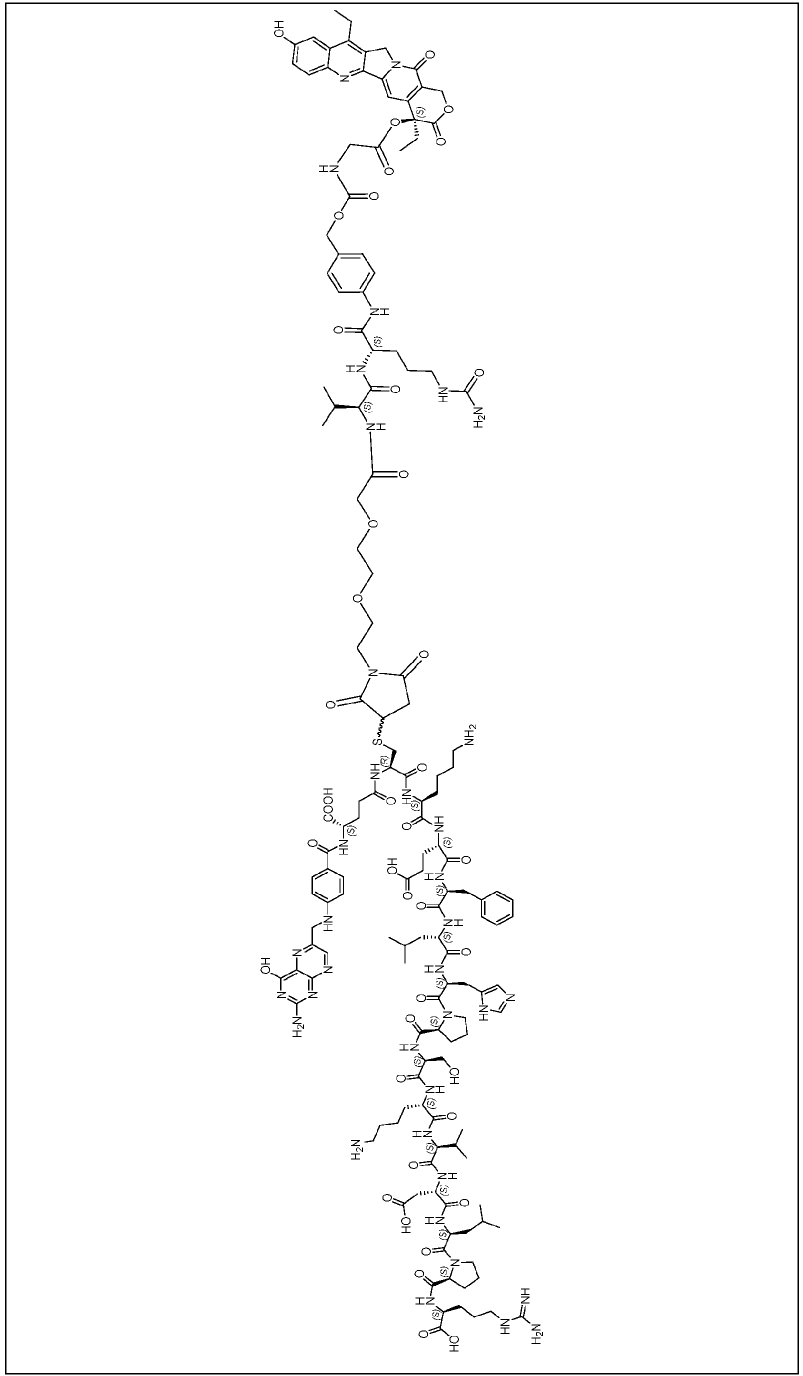

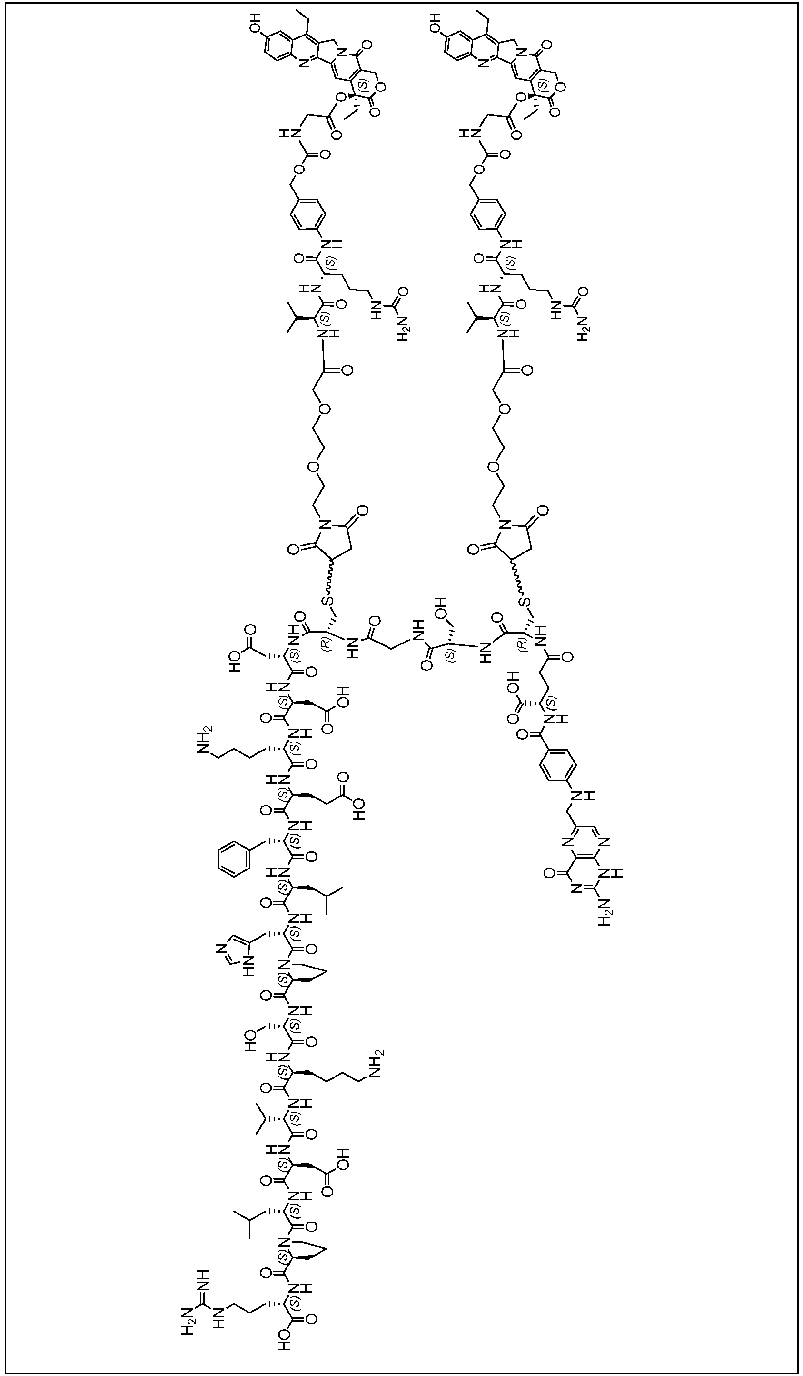

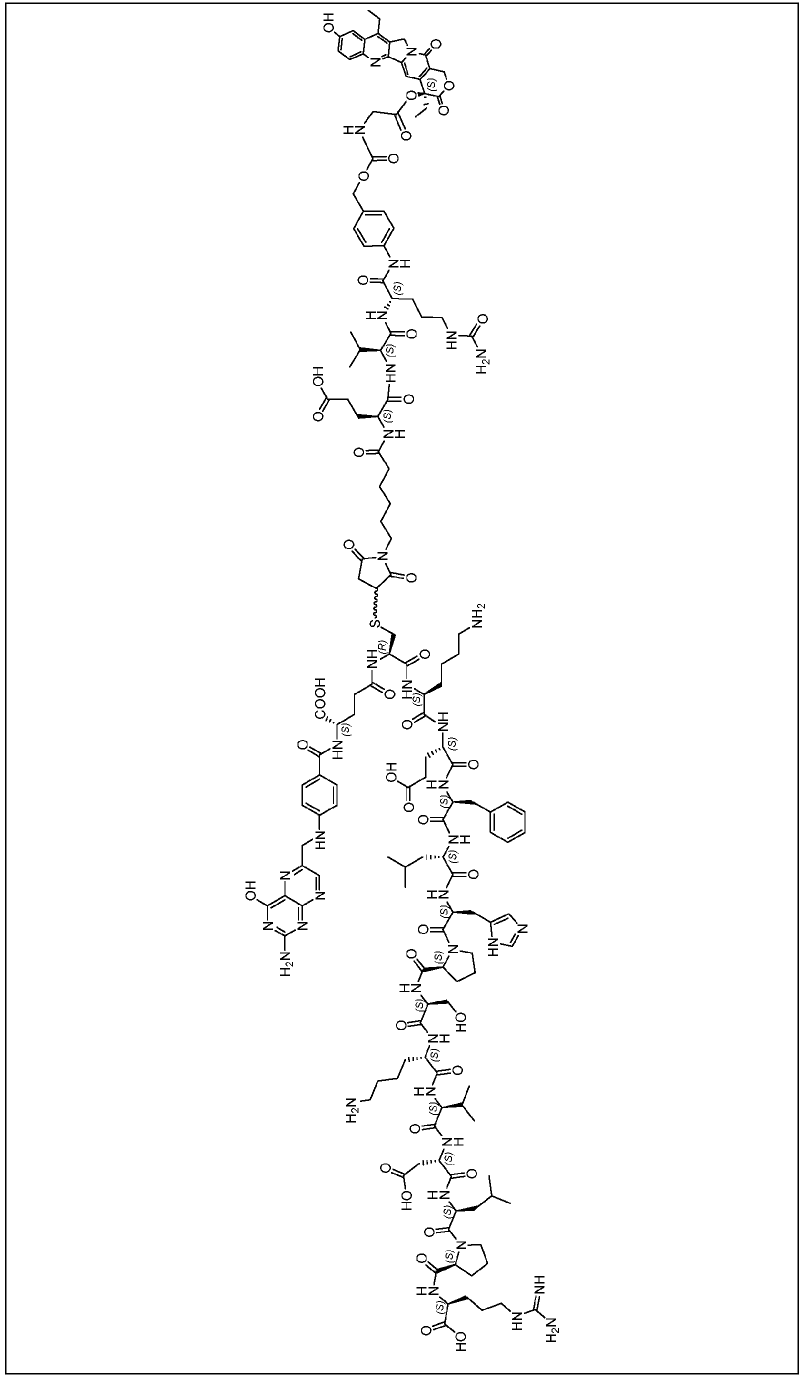

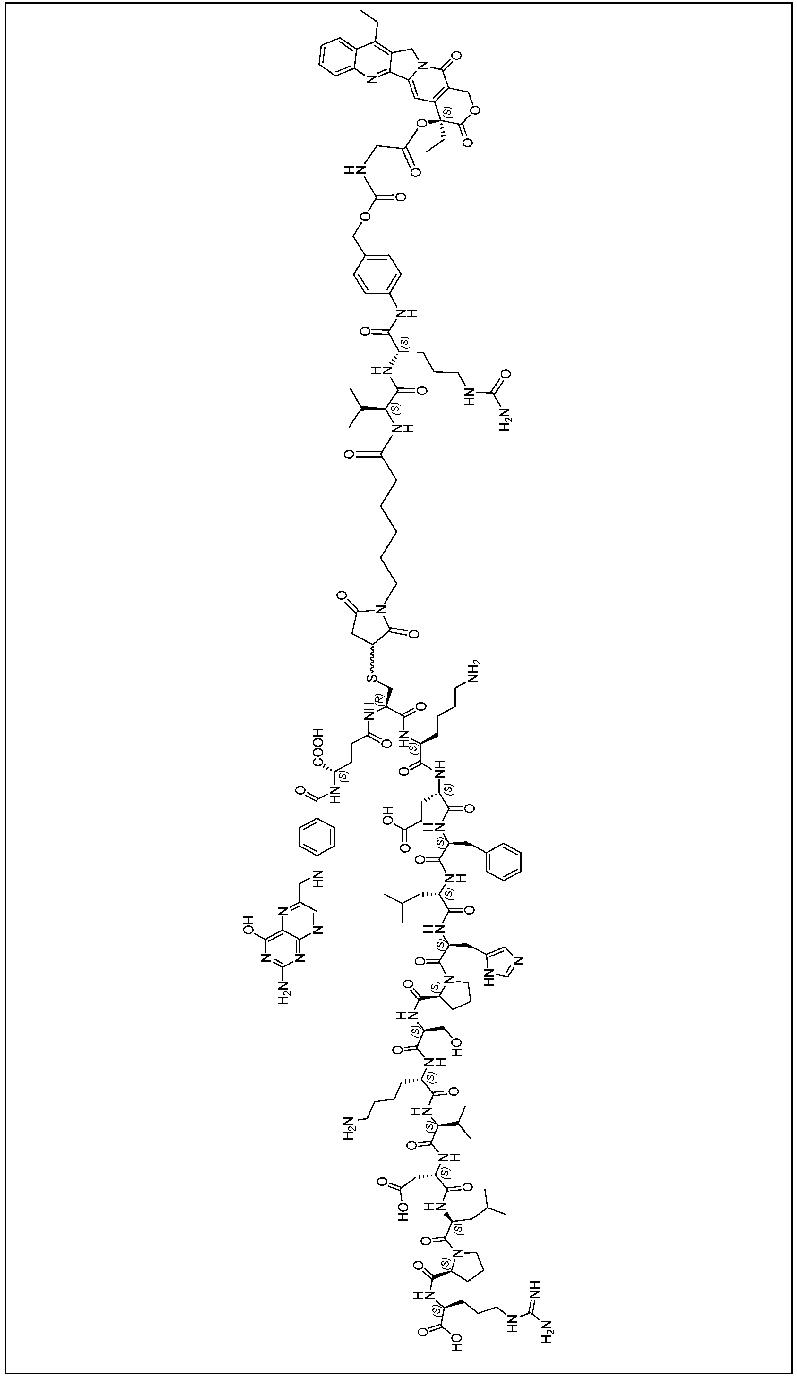

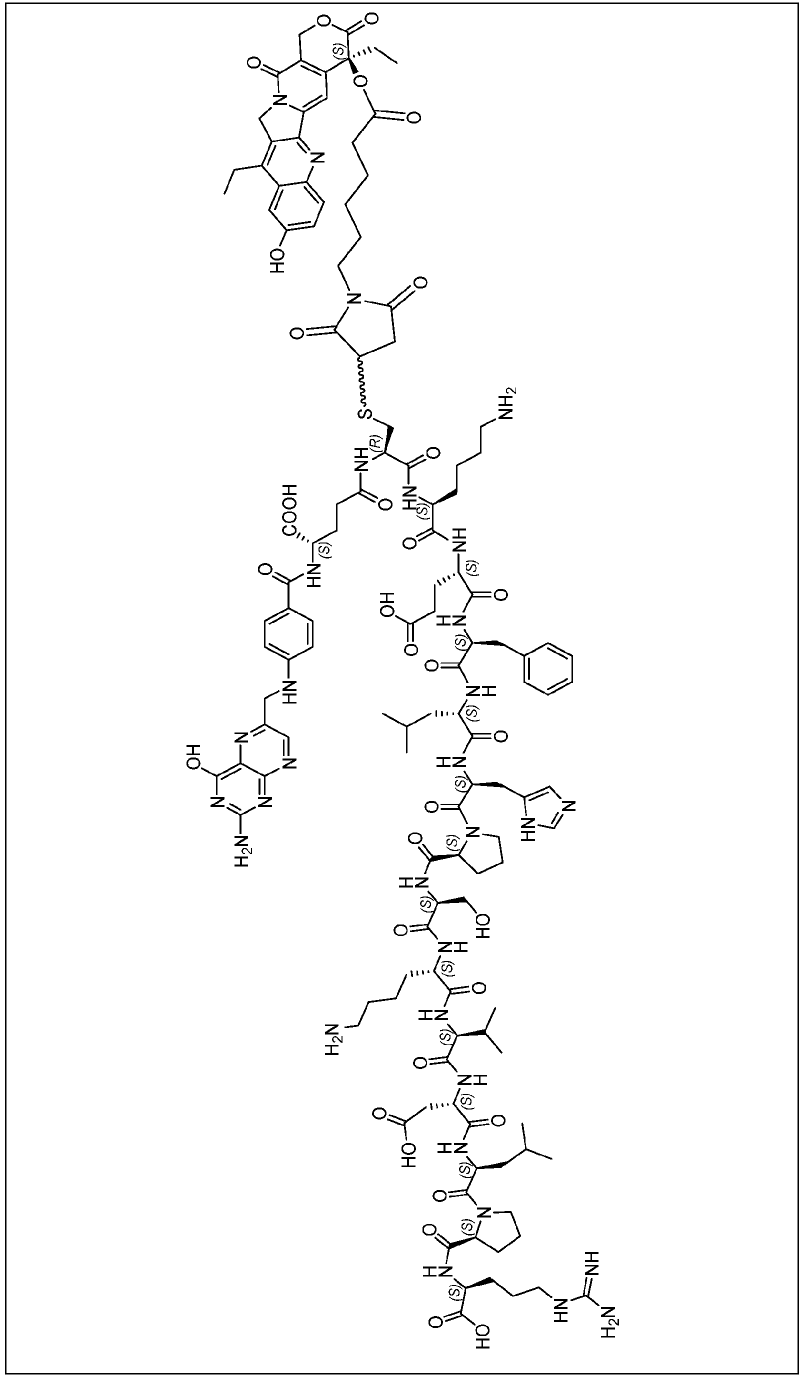

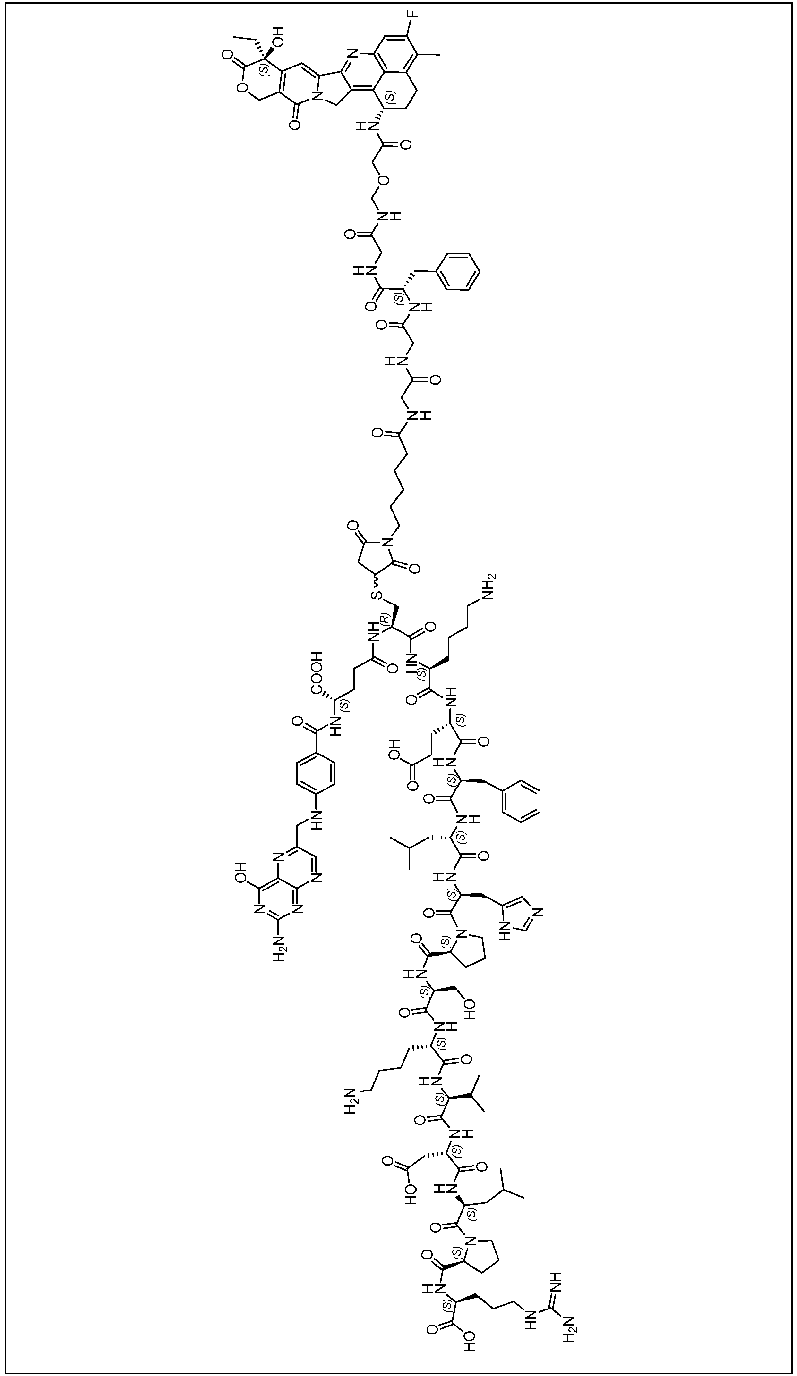

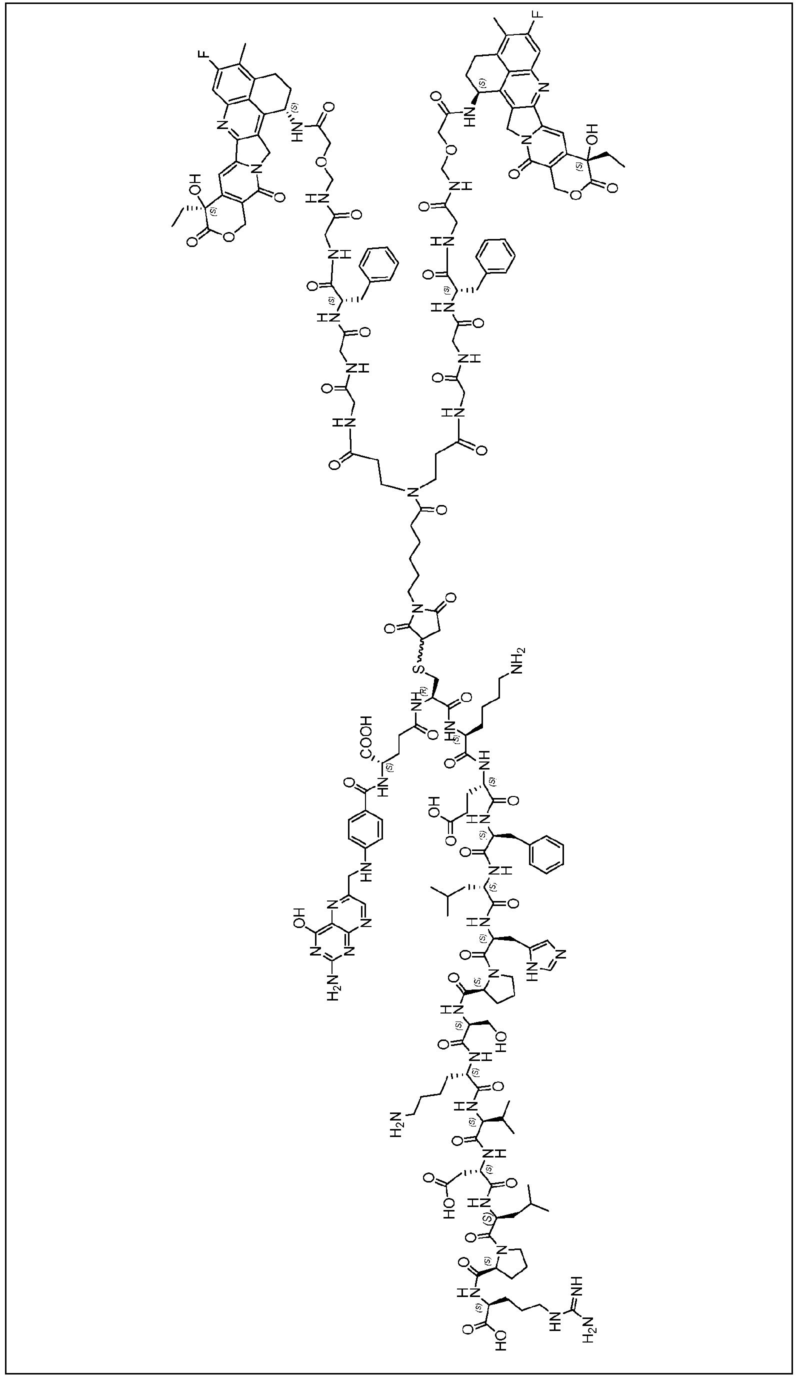

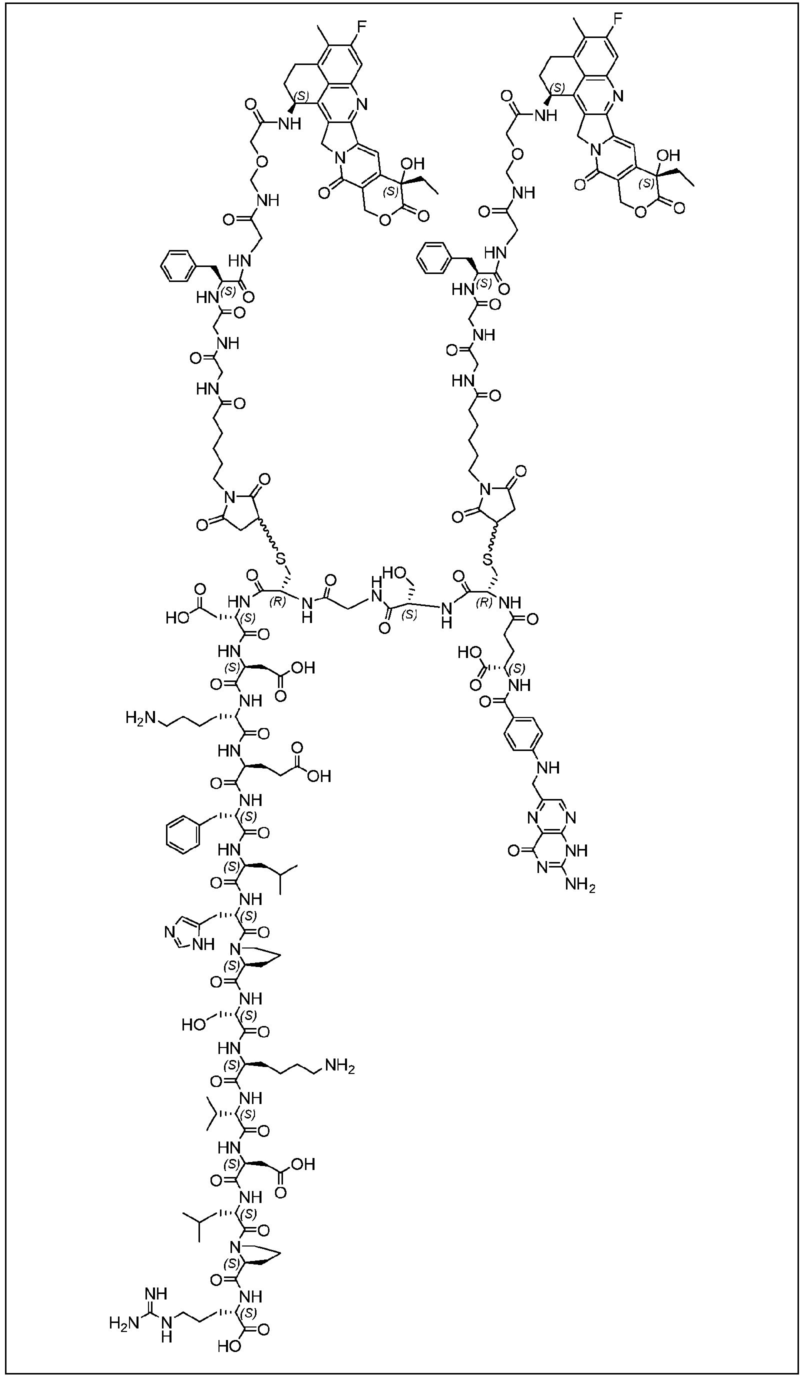

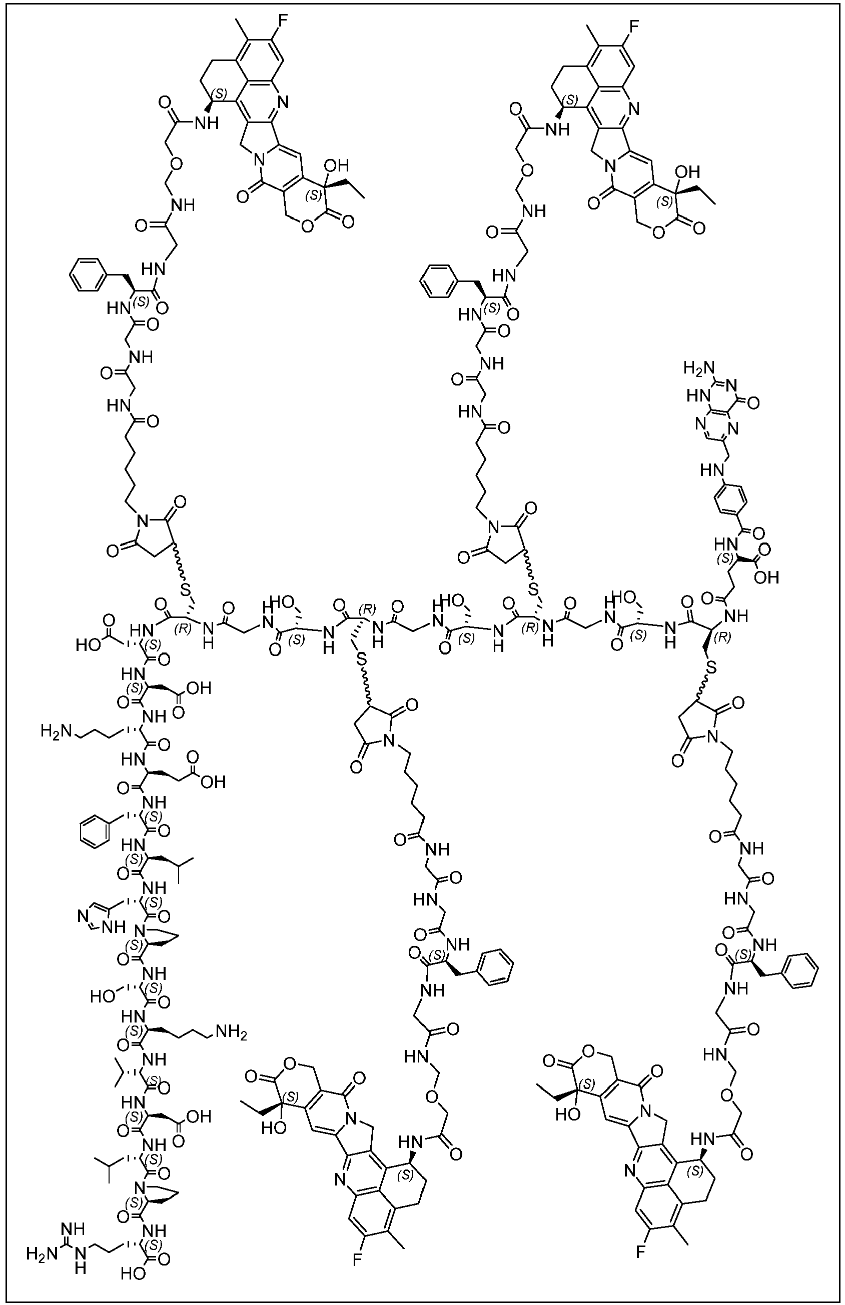

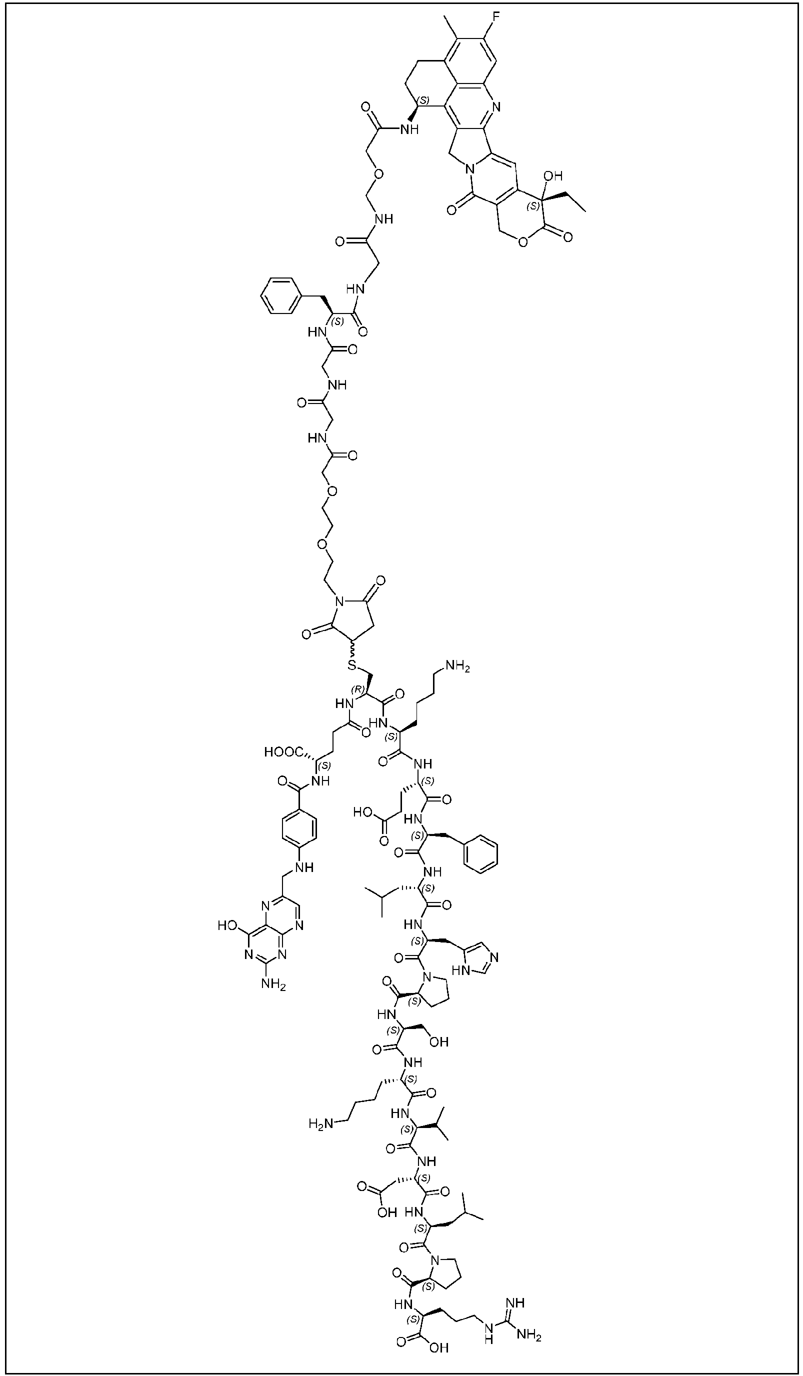

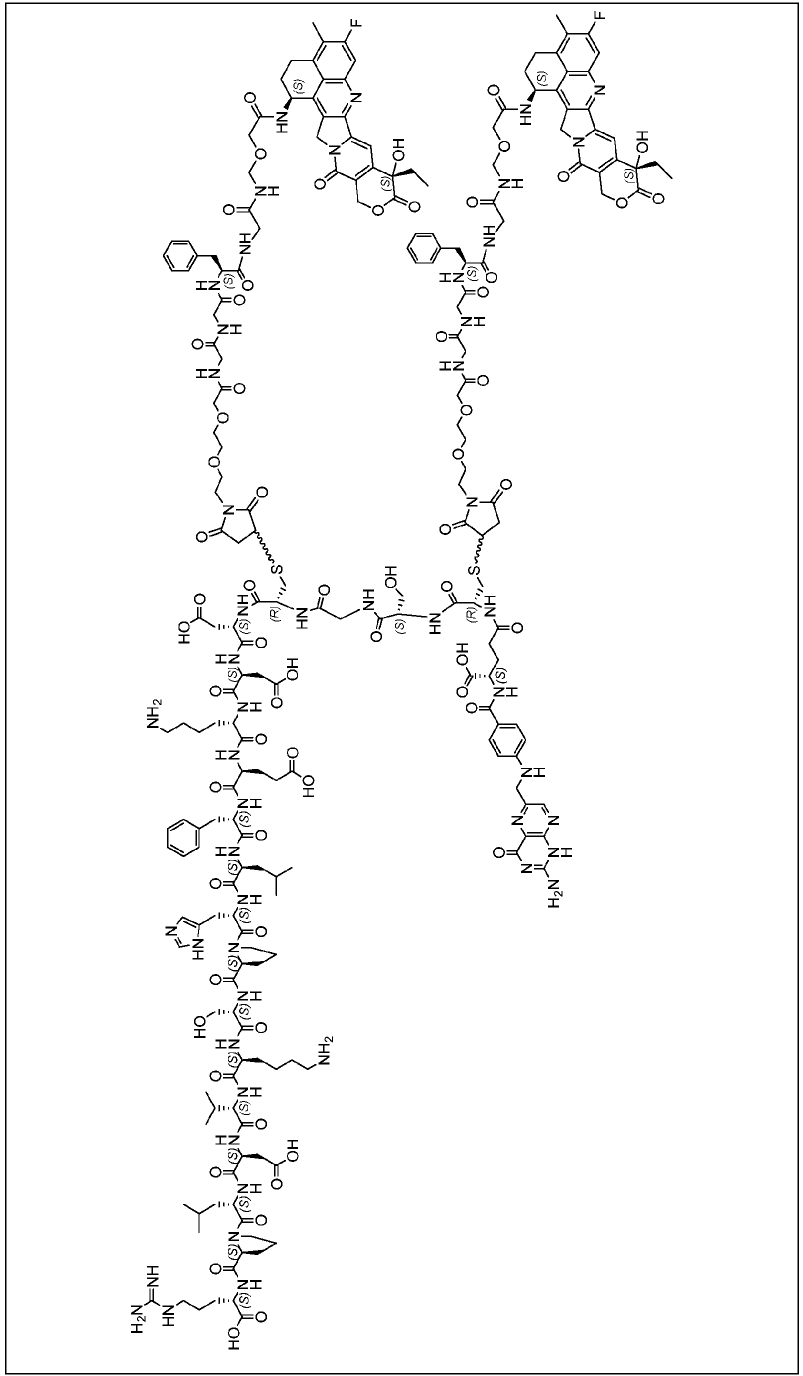

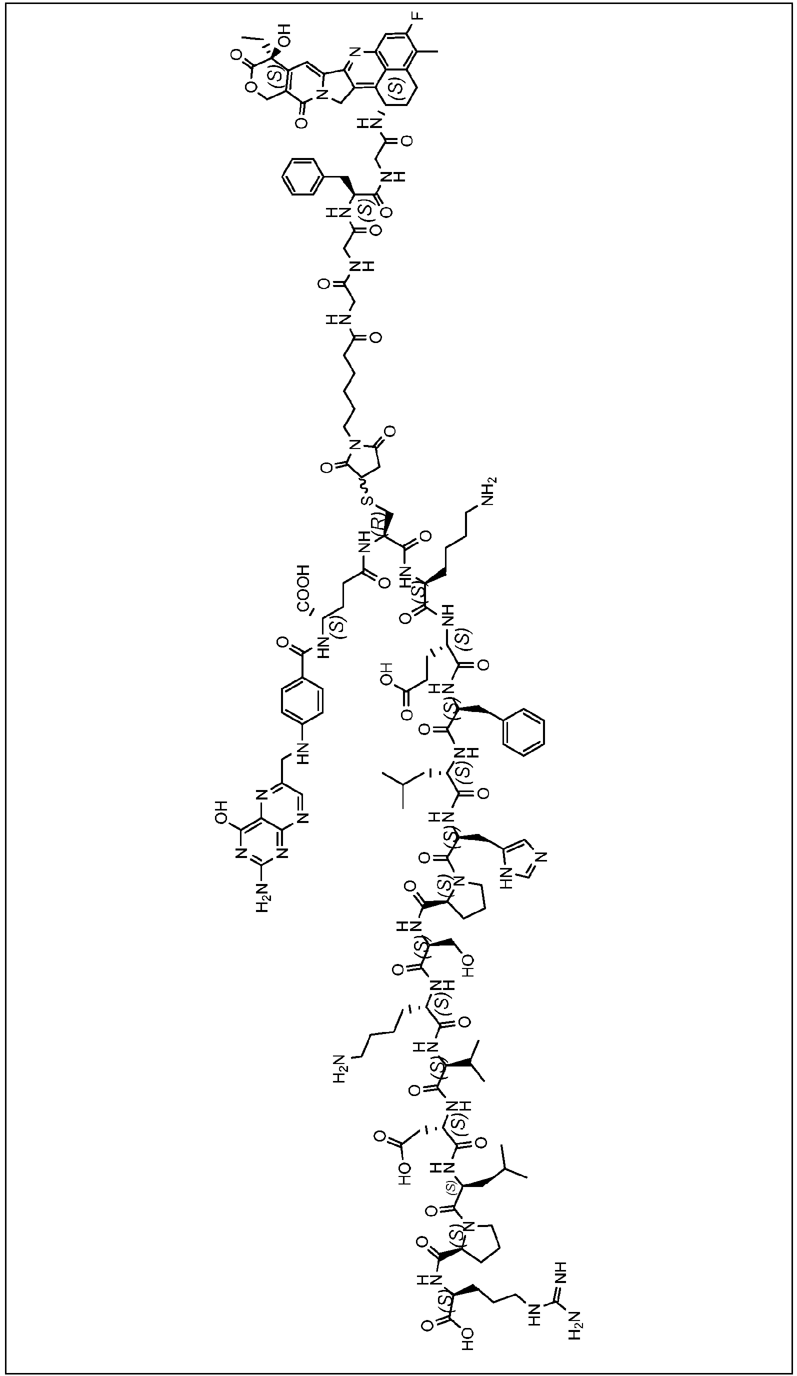

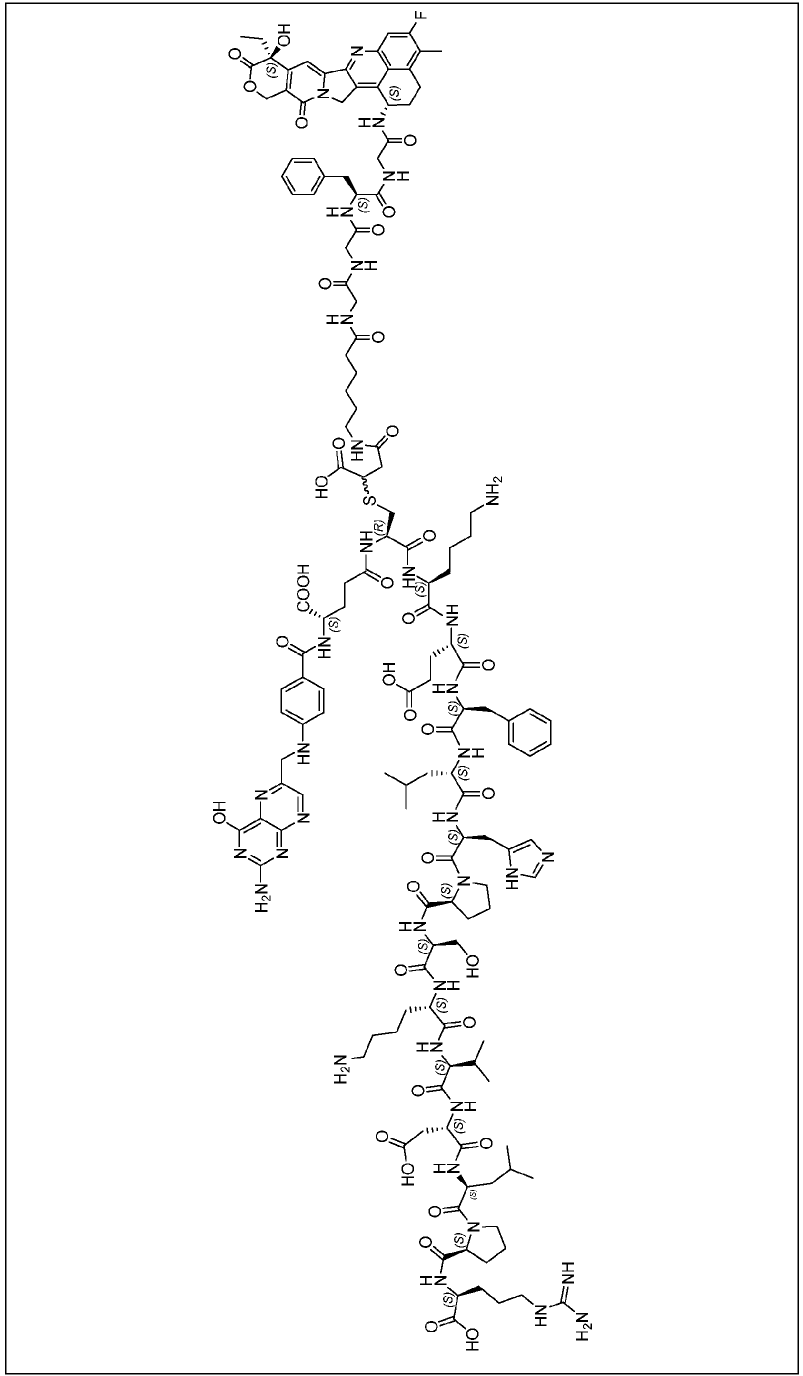

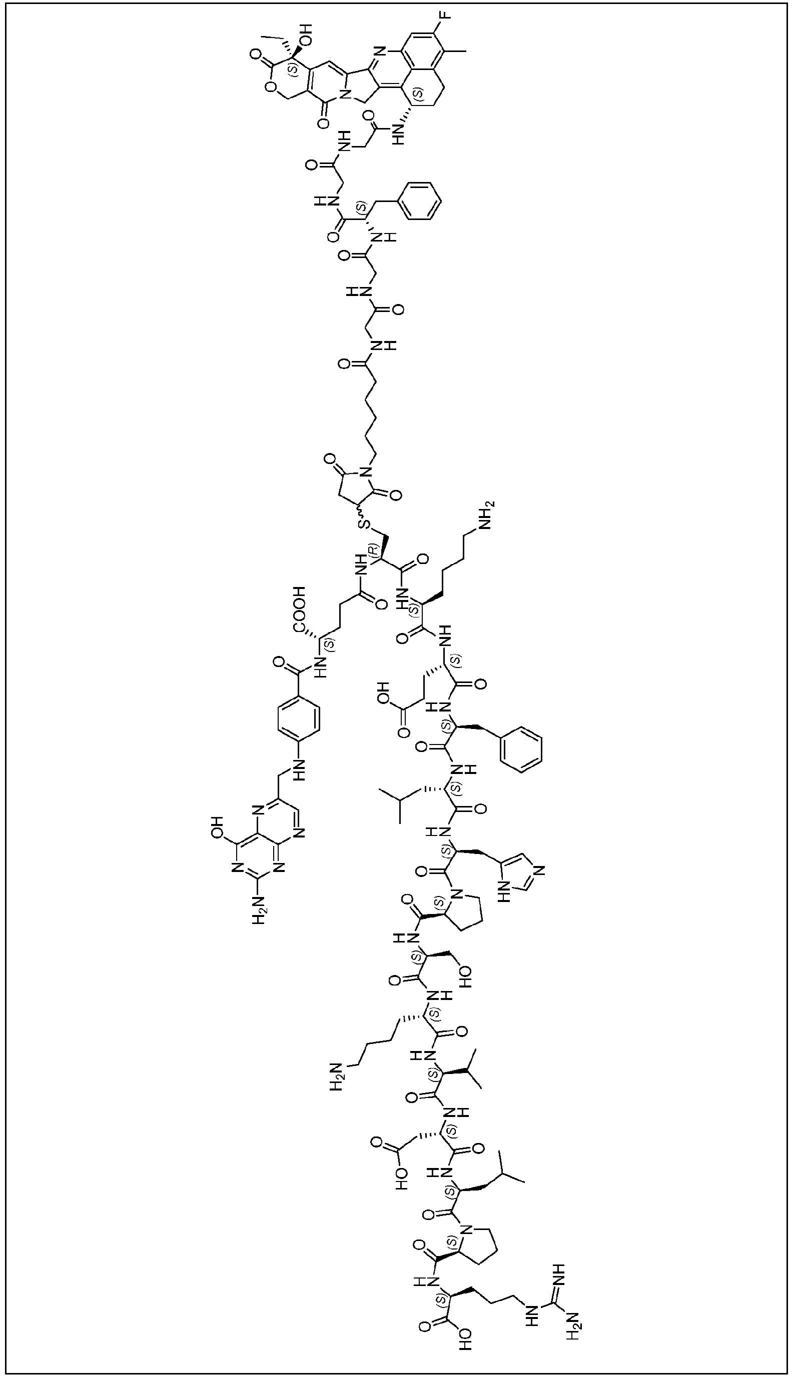

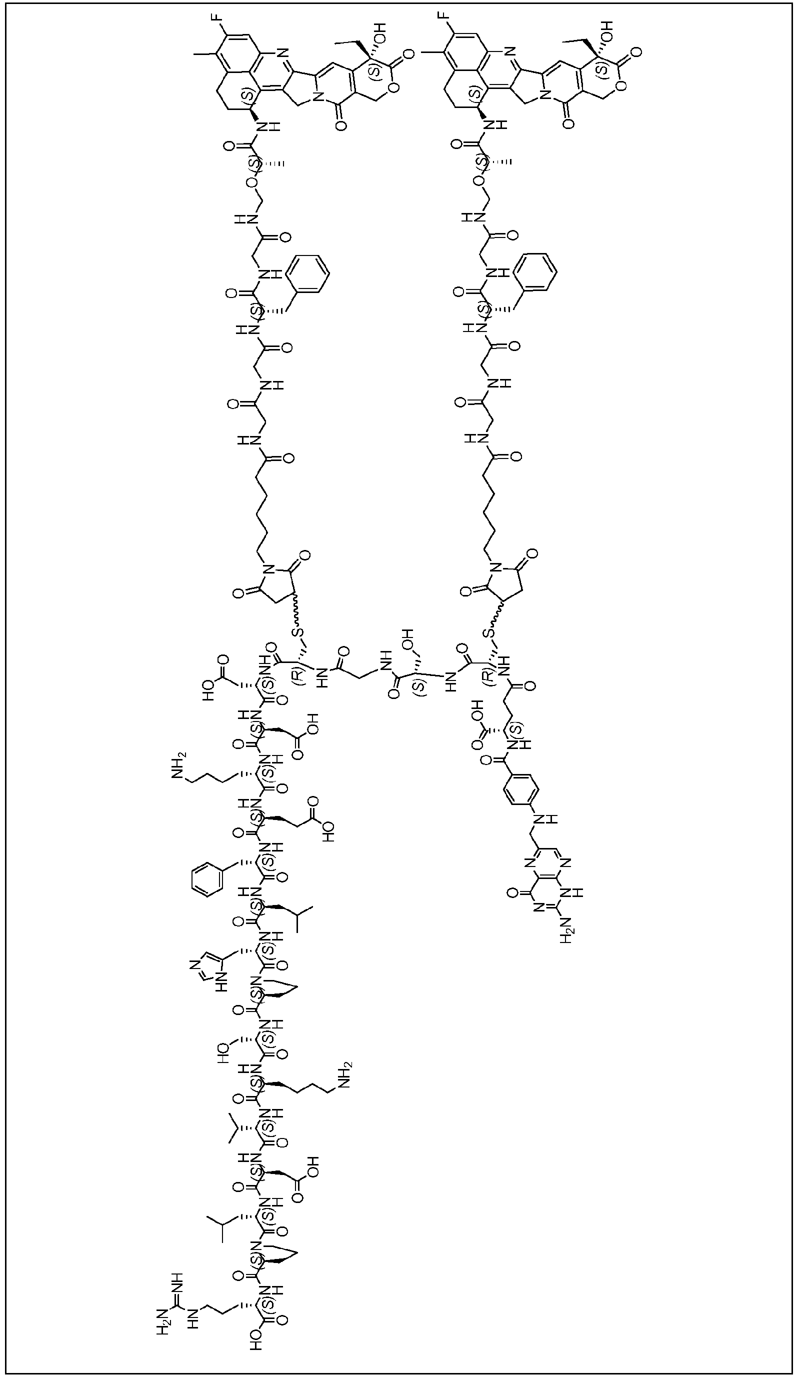

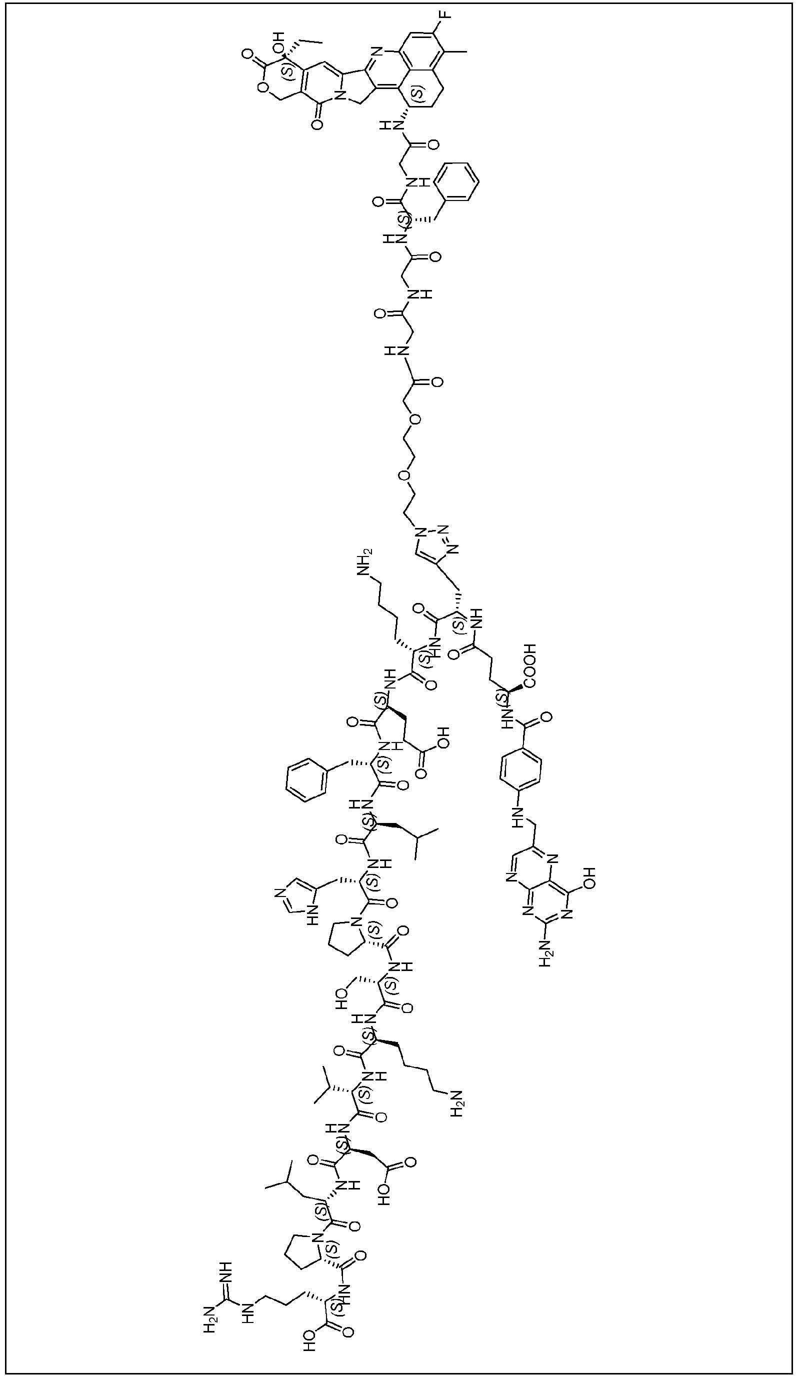

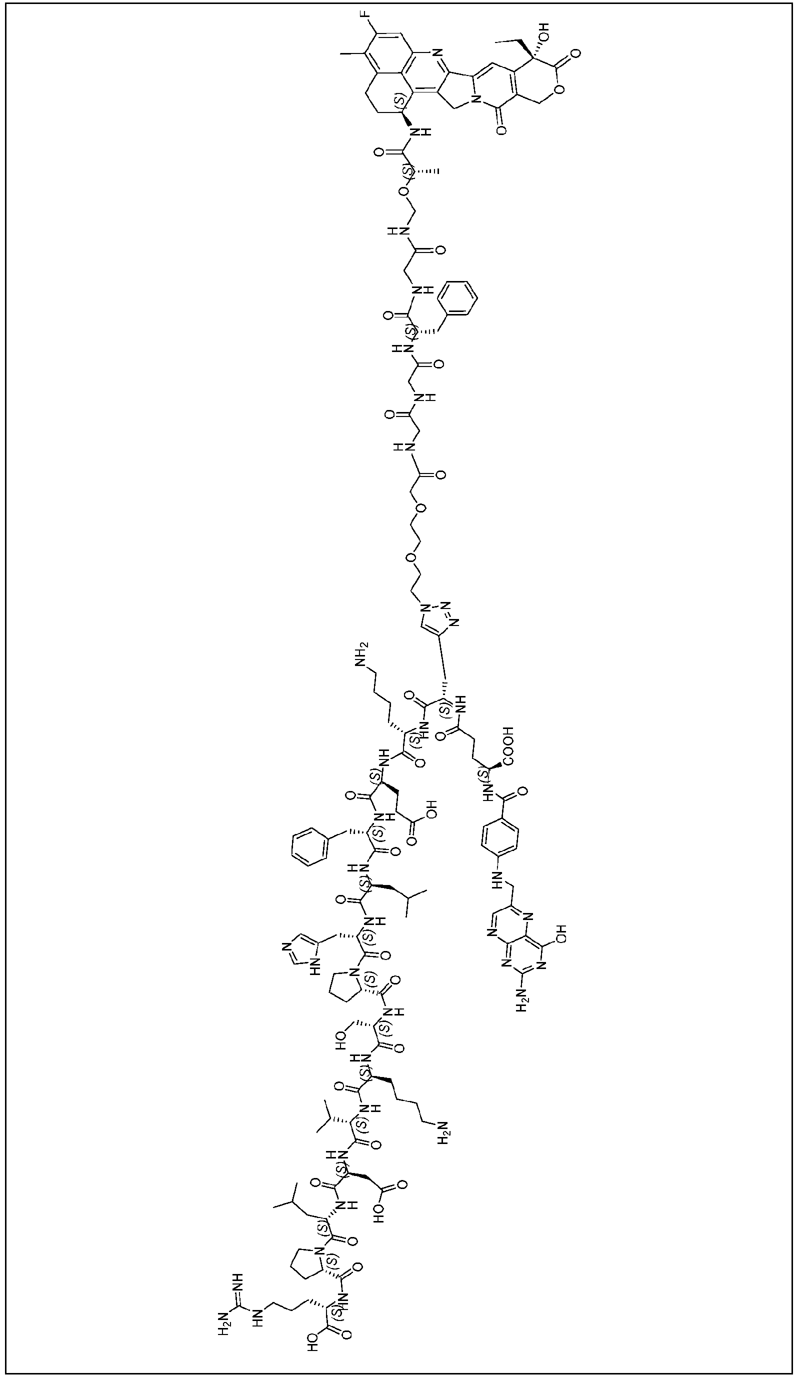

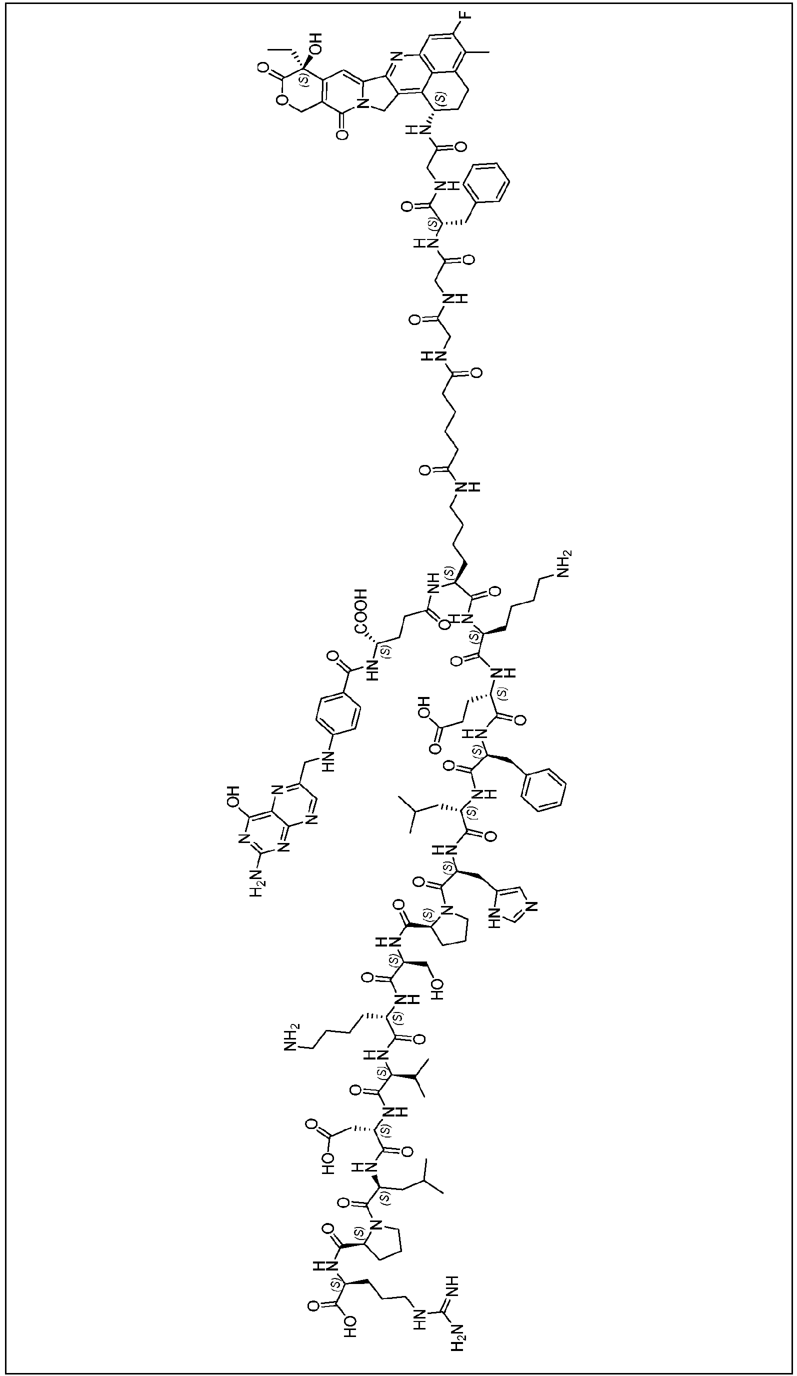

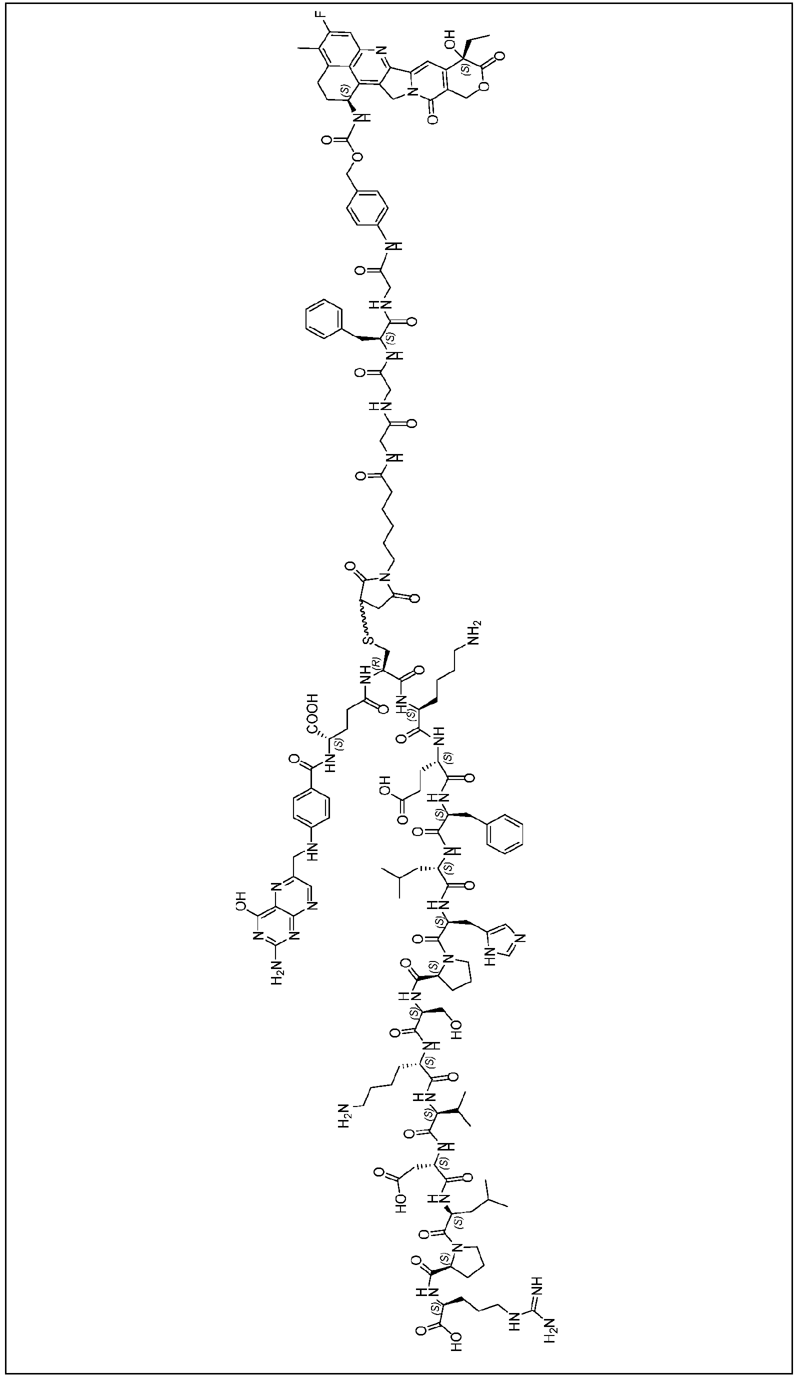

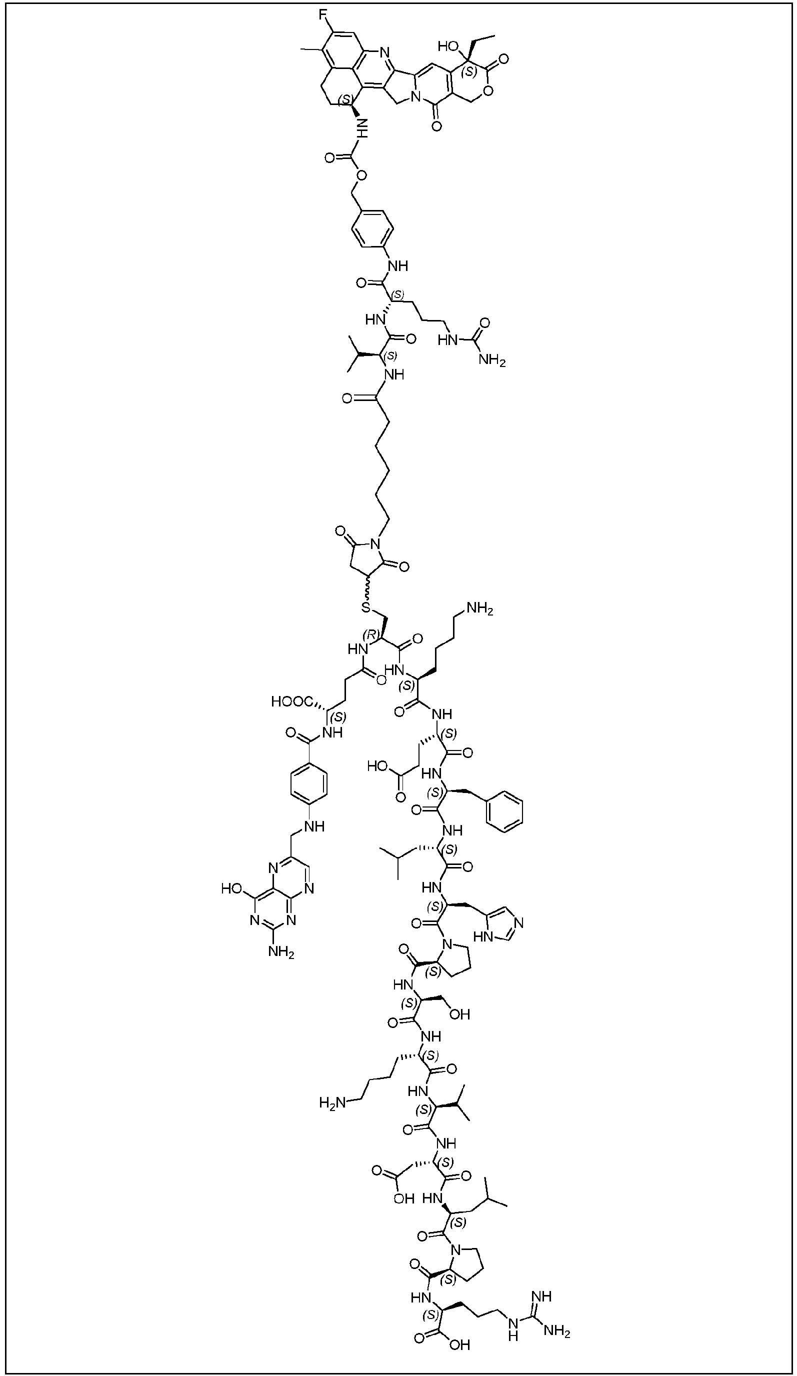

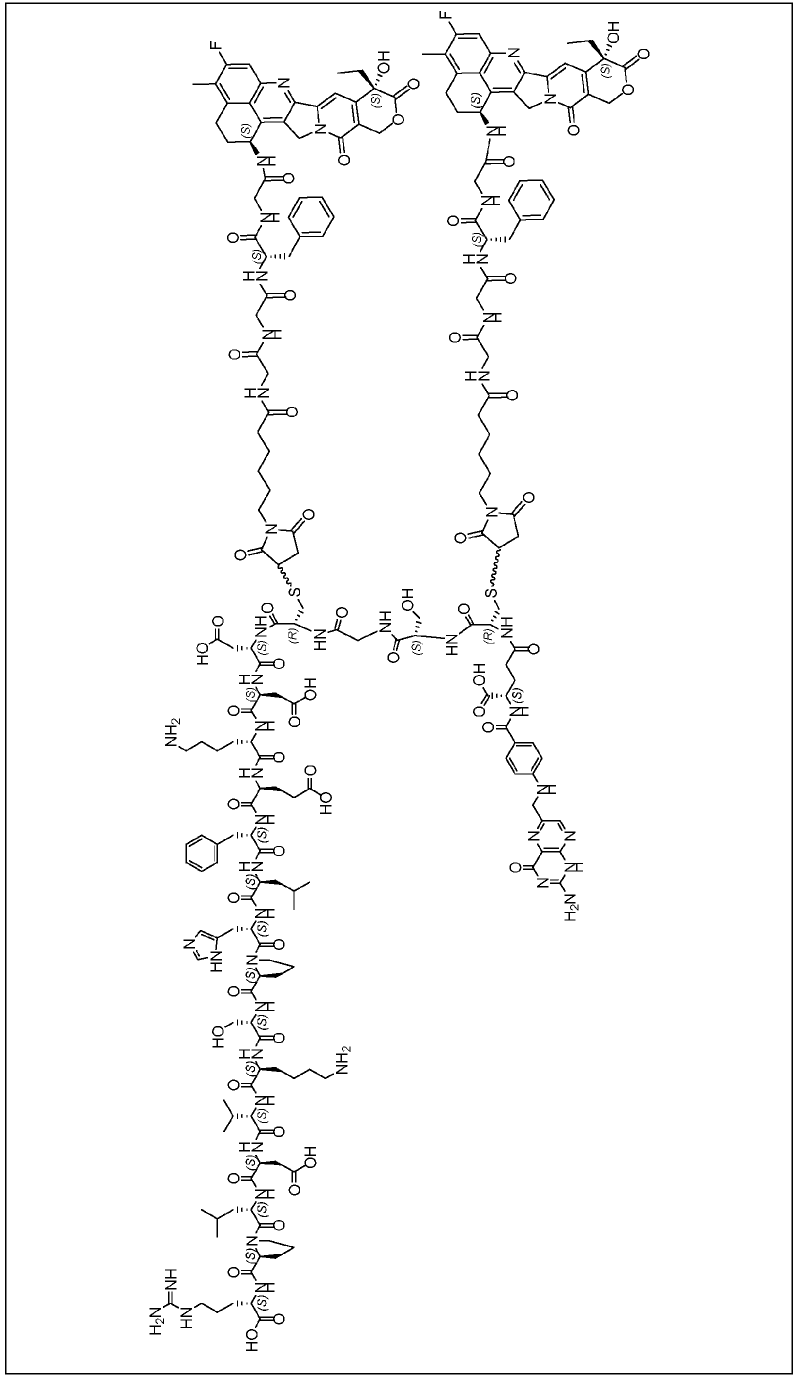

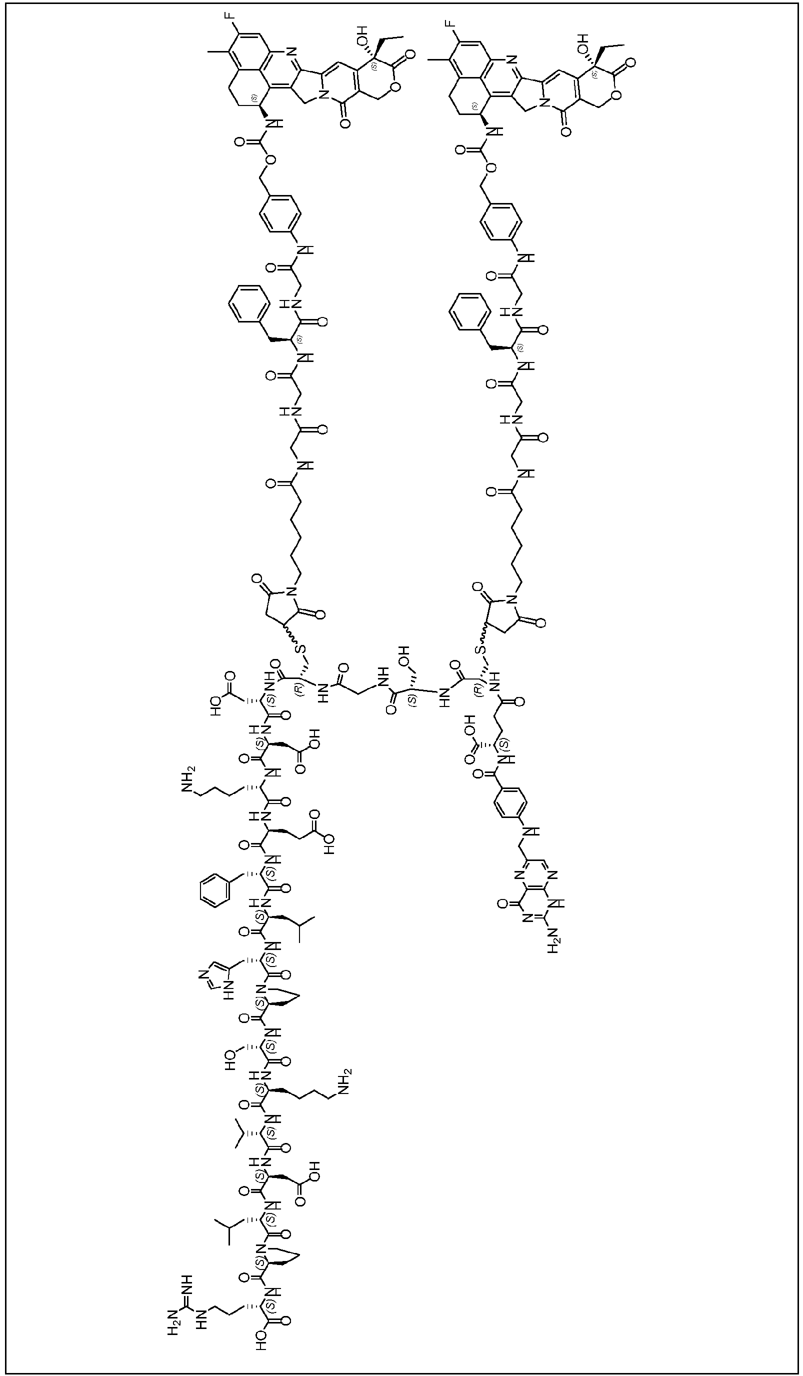

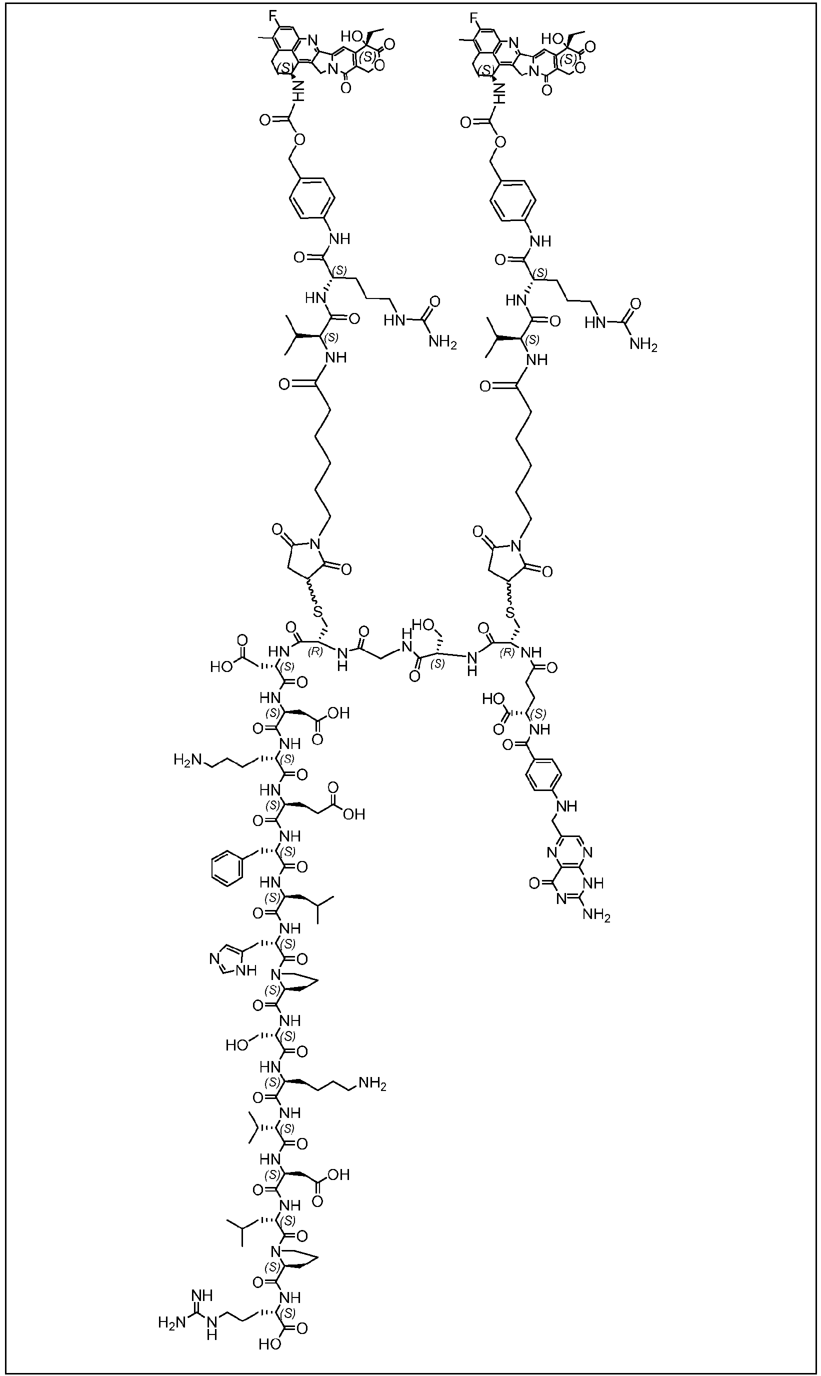

Another aspect of the present application provides a pharmaceutical composition, which comprises the conjugate compound or the pharmaceutically acceptable salt thereof as described in the present application, and a pharmaceutically acceptable carrier. In some embodiments, the pharmaceutical composition as described in the present application is administered intravenously, subcutaneously, orally, intramuscularly or intraventricularly.

Another aspect of the present application provides a method for delivering a payload to a subject in need thereof, the method comprises administering to the subject a therapeutically effective amount of the conjugate compound or the pharmaceutically acceptable salt thereof as described in the present application, or the pharmaceutical composition of the present application.

Another aspect of the present application provides a method for treating a disease in a subject, the method comprises administering to the subject a therapeutically effective amount of the conjugate compound or the pharmaceutically acceptable salt thereof as described in the present application, or the pharmaceutical composition of the present application. In some embodiments, the method further comprises administering one or more therapeutic agents in combination with the conjugate compound or the pharmaceutically acceptable salt thereof, or the pharmaceutical composition.

Another aspect of the present application provides the use of the conjugate compound or the pharmaceutically acceptable salt thereof of the present application, or the pharmaceutical composition as described in the present application in the preparation of a drug for treating a disease in a subject.

In some embodiments, the disease of the present application is selected from the group of: a cancer, an immune disease, a metabolic disease, and a neurological disease. In some embodiments, the cancer is selected from pancreatic cancer, biliary tract cancer, liver cancer, breast cancer, thyroid cancer, colorectal cancer, esophagus cancer, lung cancer, kidney cancer, leukemia, ovarian cancer, stomach cancer, uterine cancer, endometrial cancer, colon cancer, testicular cancer, skin cancer, prostate cancer, lymphoma, and multiple myeloma. In some embodiments, the immune disease is an autoimmune disease, preferably, the autoimmune disease is selected from a connective tissue disease, systemic sclerosis, rheumatoid arthritis, and systemic lupus erythematosus. The metabolic disease is selected from diabetes, gout, obesity, hypoglycemia, hyperglycemia, and dyslipidemia. In some embodiments, the neurological disease is selected from Alzheimer's disease, Parkinson's disease, Huntington's disease, head injury, multiple sclerosis, vertigo, coma, and epilepsy.

In some embodiments, the method of the present application further comprises administering one or more therapeutic agents in combination with the conjugate compound or the pharmaceutically acceptable salt thereof, or the pharmaceutical composition.

In some embodiments, the administration frequency of the conjugate compound or the pharmaceutically acceptable salt thereof or the pharmaceutical composition thereof of the present application is once a week, twice a week, three times a week, once every three days, once every two days, once a day, twice a day, or three times a day. In some embodiments, the administration frequency of the conjugate compound or the pharmaceutically acceptable salt thereof or the pharmaceutical composition thereof of the present application is once a week. In some embodiments, the administration frequency of the conjugate compound or the pharmaceutically acceptable salt thereof or the pharmaceutical composition thereof of the present application is three times a day.

In some embodiments, the conjugate compound or the pharmaceutically acceptable salt thereof or the pharmaceutical composition thereof as described in the present application is administered at a dose range of 1-150 mg/kg of the conjugate compound. In some embodiments, wherein the conjugate compound or the pharmaceutically acceptable salt thereof or the pharmaceutical composition thereof is administered at a dose of 1 mg/kg, 3 mg/kg, 10 mg/kg, 25 mg/kg, 50 mg/kg, 75 mg/kg, 100 mg/kg or 150 mg/kg of the conjugate compound. In some embodiments, the conjugate compound or the pharmaceutically acceptable salt thereof or the pharmaceutical composition thereof of the present application is administered at a dose of 50 mg/kg of the conjugate compound. In some embodiments, the conjugate compound or the pharmaceutically acceptable salt thereof or the pharmaceutical composition thereof of the present application is administered at a dose of 25 mg/kg of the conjugate compound.

### Brief Description of the Drawings

Fig. 1A shows the change in the body weight of the mice in the test over the administration time, and Fig. 1B shows the curve of the tumor volume of each group of the MIAPaCa-2 transplanted tumor model over time, and Figs. 1A-1B show that the compound of the present application has good tumor inhibition effect in MIAPaCa-2 transplanted tumor model.
Fig. 2A shows the change in the body weight of the mice in the test over the administration time, and Fig. 2B is the change in the body weight of the mice in the test from the initial body weight over the administration time.
Fig. 3A shows the change in the body weight of the rat in the test over the administration time, and Fig. 3B shows the change in the food intake of the test animals in each group during the administration period.

### Detailed Description of the Invention

The following description is only intended to illustrate various embodiments of the present application. Therefore, the specific embodiments herein should not be construed as a limitation to the scope of the present application. Those skilled in the art can easily obtain various equivalents and modifications based on the principles of the present invention and the description herein, and it should be understood that such equivalent embodiments are included in the scope of the present invention. All references cited in the present application, including public publication, patent, and patent application, are incorporated into the present application by reference in their entirety.

Unless the context clearly indicates otherwise, the singular form "a", "an" and "the" as used herein includes plural reference.

As used herein, terms such as "include", "comprise", "contain", "containing" and "have" as used in the present application are inclusive or open-ended and do not exclude additional, unrecited element or method step. The term "consist of" is a closed expression.

One aspect of the present application discloses a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 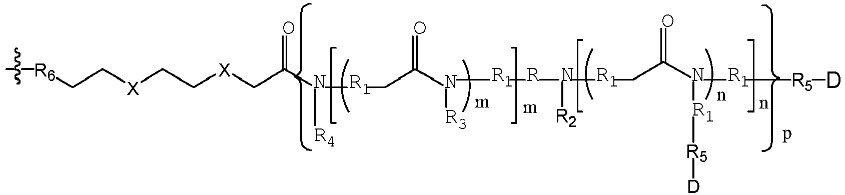 wherein:
   R is selected from 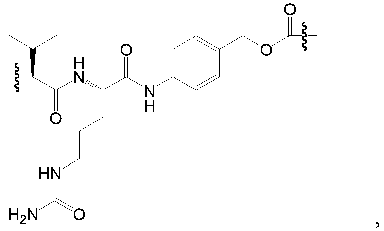 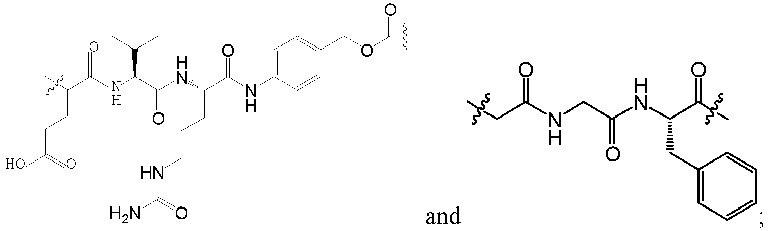
   each R₁ is independently selected from a bond and -CH₂CH₂C(O)-NH-;
   each R₂ is independently selected from hydrogen and 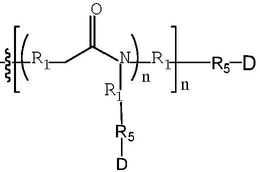 ;
   each R₃ is independently selected from hydrogen and 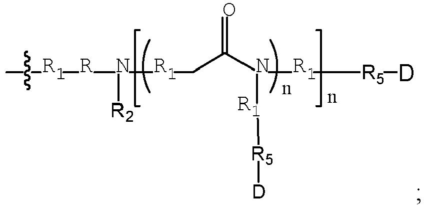
   each R₄ is independently selected from hydrogen and 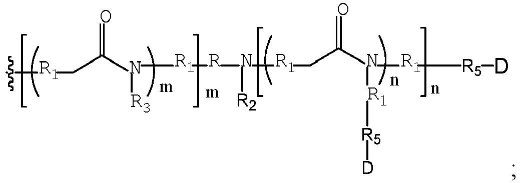
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 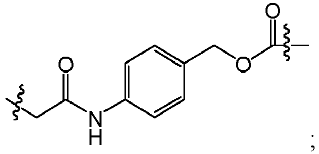
   each R₆ is independently selected from a bond, 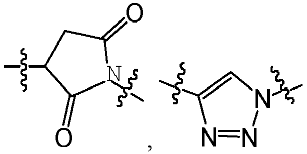 and 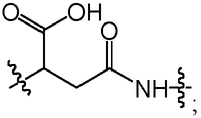
   each X is independently selected from a bond, C and O;
   each m is independently 0 or 1;
   each n is independently 0 or 1;
   p is 0 or 1;
   q is an integer from 1 to 4;
   and D is the payload.

Another aspect of the present application discloses a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 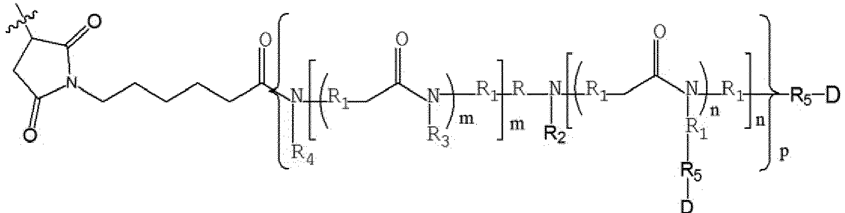 wherein:
   R is selected from 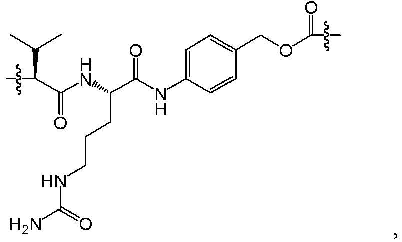 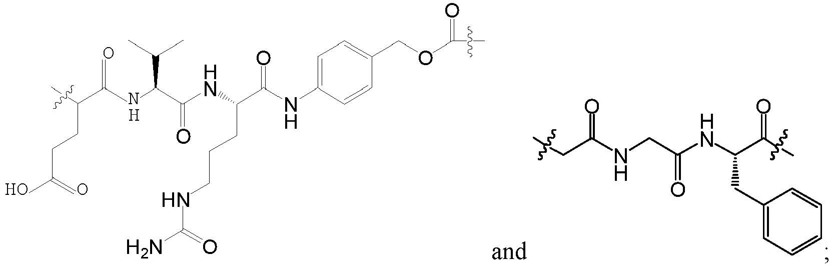
   each R₁ is independently selected from a bond and -CH₂CH₂C(O)-NH-;
   each R₂ is independently selected from hydrogen and 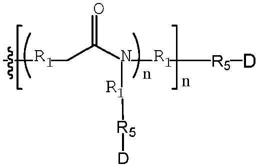 ;
   each R₃ is independently selected from hydrogen and 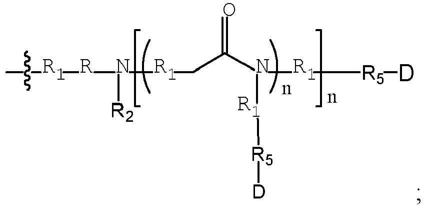
   each R₄ is independently selected from hydrogen and 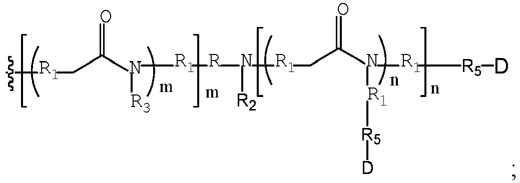
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 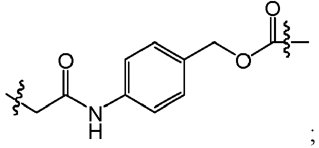
   each m is independently 0 or 1;
   each n is independently 0 or 1;
   p is 0 or 1;
   q is an integer from 1 to 4;
   and D is the payload.

Another aspect of the present application discloses a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 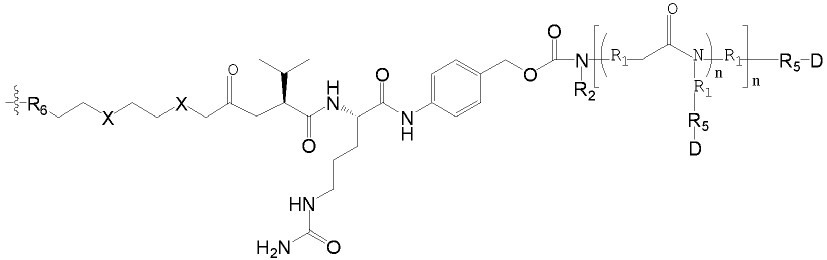 wherein:
   each R₁ is independently selected from a bond and -CH₂CH₂C(O)-NH-;
   each R₂ is independently selected from hydrogen and 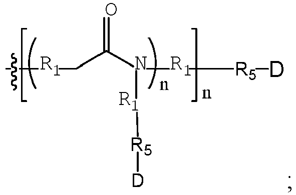
   each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 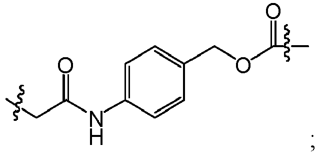
   each R₆ is independently selected from a bond, 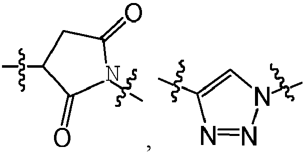 and 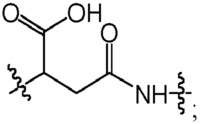
   each X is independently selected from a bond, C and O;
   each n is independently 0 or 1;
   q is an integer from 1 to 4;
   and D is the payload.

Another aspect of the present application discloses a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 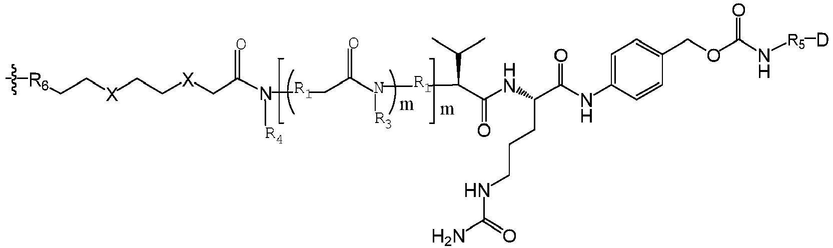 wherein:
   each R₁ is independently selected from a bond and -CH₂CH₂C(O)-NH-;
   each R₃ is independently selected from hydrogen and -R₁-R-NH-R₅-D;
   R₄ is hydrogen or 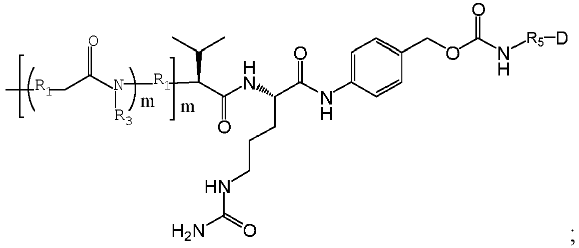
   R is selected from 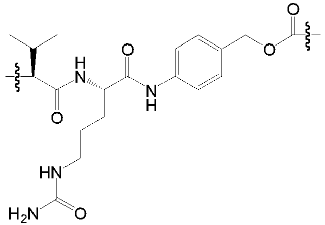 and 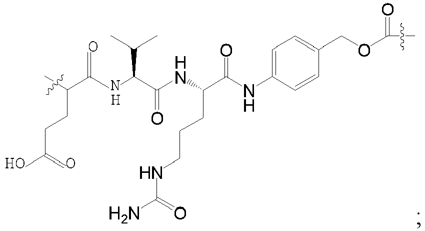
   each R₅ is independently selected from a bond, -(CH₂CH₂O)₃-CH₂CH₂-, - CH₂C(O)-NH-CH₂-, -CH₂C(O)-NH-CH₂C(O)- and -CHzC(O)-;
   each R₆ is independently selected from a bond, 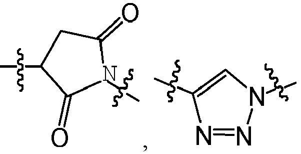 and 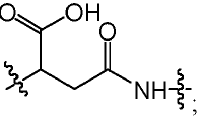
   each X is independently selected from a bond, C and O;
   each m is independently 0 or 1;
   q is an integer from 1 to 4;
   and D is the payload.

Another aspect of the present application discloses a conjugate compound or a pharmaceutically acceptable salt thereof, the conjugate compound having the following structure:

multi-ligand moiety-(linker-payload)_{q},

wherein:
the multi-ligand moiety comprises at least two ligands targeting different cell surface molecules,
and the linker-payload moiety has the following structure: 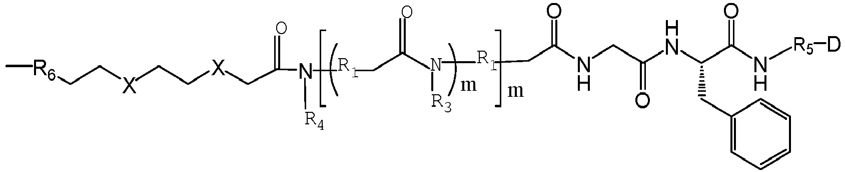 wherein:
   each R₁ is independently selected from a bond and -CH₂CH₂C(O)-NH-;
   each R₃ is independently selected from hydrogen and -R₁-R-NH-R₅-D;
   R₄ is hydrogen or 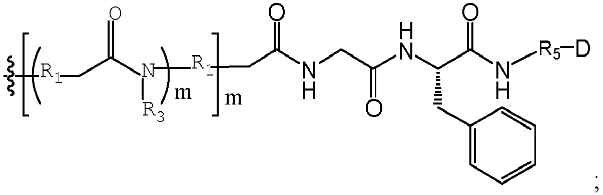
   R is 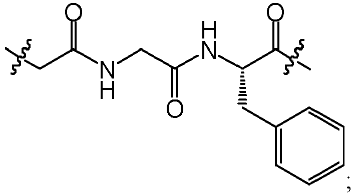
   each R₅ is independently selected from a bond, -(CH₂CH₂O)₃-CH₂CH₂-, - CH₂C(O)-NH-CH₂-, -CH₂C(O)-NH-CH₂C(O)-, -CH₂C(O)- and 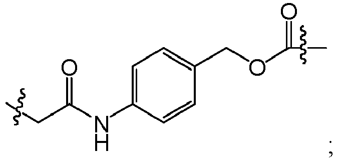
   each R₆ is independently selected from a bond, 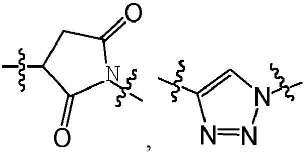 and 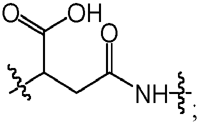
   each X is independently selected from a bond, C and O;
   each m is independently 0 or 1;
   q is an integer from 1 to 4;
   and D is the payload.

The term "ligand" as used herein can include a wide variety of chemical molecules or peptides, which can have a specific binding affinity to a selected target, wherein the selected target can be, for example, a cell surface receptor, a cell surface antigen, a cell, a tissue, an organ, etc. In some embodiments, the ligand can specifically bind to a protein or a marker expressed on the surface of a target cell. In some embodiments, the ligand of the present application binds to a cell surface protein or marker with an affinity of 10⁻⁶-10⁻¹¹ M (K_{d} value). In some embodiments, the ligand of the present application binds to a cell surface protein or marker with an affinity of at least 10⁻⁷, at least 10⁻⁸ and at least 10⁻⁹ M (K_{d} value). In some embodiments, the ligand of the present application binds to a cell surface protein or marker with an affinity of less than 10⁻⁶, less than 10⁻⁷ and less than 10⁻⁸ M (K_{d} value). In some embodiments, the ligand of the present application binds to a cell surface protein or marker with a certain affinity, wherein the certain affinity refers to the affinity of the ligand to a target cell surface protein or marker which is at least two, three, four, five, six, eight, ten, twenty, fifty, one hundred or more times higher than that to a non-target cell surface protein or marker. In some embodiments, the expression of the cell surface protein or marker of the present application in target cells (e.g., cancer cells) is significantly higher than that in normal cells. The term "significantly" as used herein refers to statistically significant differences, or significant differences that can be recognized by a person skilled in the art.

A "multi-ligand moiety" as described in this application comprises at least two ligands targeting different cell surface molecules. In some embodiments, the multi-ligand moiety comprises three ligands, wherein the first ligand and the second ligand target different cell surface molecules, and the cell surface molecule targeted by the third ligand may be the same as or different from those targeted by the first ligand and the second ligand. In some embodiments, the multi-ligand moiety can comprise four or more ligands.

The term "targeting" as used herein refers to the specific binding of a ligand or a ligand-containing conjugate compound to a cell surface molecule to trigger or facilitate the endocytosis of the ligand or the ligand-containing conjugate compound by the target cell, and trigger or facilitate the ligand or the ligand-containing conjugate compound to be enriched around the target cell and/or enter the target cell.

In some embodiments, R is

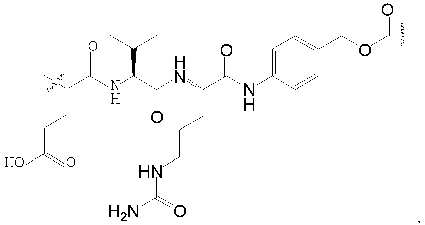

In some embodiments, R₁ is -CH₂CH₂C(O)-NH-.

In some embodiments, R₅ is -(CH₂CH₂O)₃-CH₂CH₂-. In other embodiments, R₅ is -CH₂C(O)-NH-CH₂C(O)-. In still other embodiments, R₅ is -CH₂C(O)-. In some embodiments, R₅ is 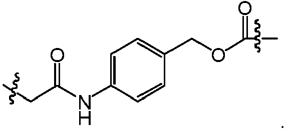 In other embodiments, R₅ is - CH₂C(O)-NH-CH₂-. In still other embodiments, R₅ is not -CH₂C(O)-NH-CH₂.

In some embodiments, each m is independently 0 or 1, and n is 0. In some embodiments, m is 0, and each n is independently 0 or 1. In some embodiments, m is 0 and n is 0. In still other embodiments, m is 0, n is 0, and R₅ is not -CH₂C(O)-NH-CH₂-.

In some embodiments, R₆ is 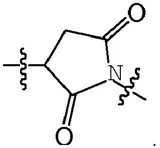 In some embodiments, R₆ is 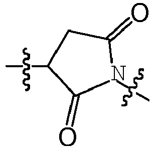 and X is a bond. In some embodiments, R₆ is 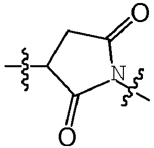 and X is O. In some embodiments, R₆ is 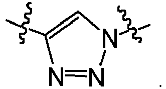 In some embodiments, R₆ is 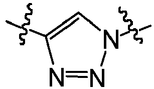 and X is a bond. In still other embodiments, R₆ is 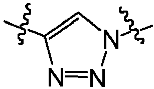 and X is O. In some embodiments, R₆ is 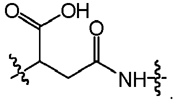 In some embodiments, R₆ is 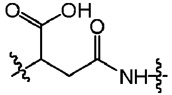 and X is a bond. In still other embodiments, R₆ is 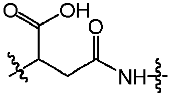 and X is O.

In some embodiments, p is 0.

The term "linker" as used herein refers to a molecule or moiety that covalently links a payload to a multi-ligand moiety. The linkers include a functional group for linking a payload to at least one ligand. In some embodiments, the functional group may comprise two reactive moieties, one for linking to a payload and the other for linking to a ligand. In some embodiments, the functional groups are different from each other. In some embodiments, the functional groups include a group containing a thiol-reacting moiety and an amine-reacting moiety. In some embodiments, the functional groups are identical to each other. In some embodiments, the carboxylic acid in the amino acid contained in the linker is amidated. In some embodiments, the linker contains a short chain polyethylene glycol (for example, comprising 2-10, 2-8, 3-8, 4-8, 4-7, 4-6, or 5 repeating units). In some embodiments, the linker contains an azide group. In some embodiments, the linker contains an alkynyl group.

In some embodiments, the molecules used to link the payload and the ligand are linked by a Click reaction prior to forming the linkers as described in the present application. The "click reaction" as described in the present application is a kind of chemical synthesis method with high efficiency and high selectivity. The click reaction splices several molecular fragments together by forming efficient linking units, and the whole reaction process is simple, fast and has few by-products. Some examples of the click reaction include, but are not limited to, the addition reaction of thiols to alkenes, and the cyclization reaction of azides to alkynes.

In some embodiments, the linker is sufficiently stable to avoid from unintended release of payloads during a blood circulation to increase an effective amount of payloads delivered to target cells or tissues and avoid toxicity. In some embodiments, the linker is capable of releasing the payloads around or within target cells to efficiently kill target cells or block functions of target cells.

In some embodiments, the linker is non-cleavable. The term "non-cleavable linker" as used herein refers to a linker which remains basically intact during intracellular metabolism.

In some embodiments, the linker is cleavable. In some embodiments, the cleavable functional group is sufficiently stable outside a target cell, but upon entry into the target cell, is cleaved to release a payload. In some embodiments, the cleavable functional group is cleaved at least 10, 20, 30, 50, 100 times or more efficiently in target cells than in the blood or serum. In some embodiments, the cleavable linker of the present application can be cleaved by hydrolysis, enzymatic reactions, or reduction reactions, or by pH changes. In some embodiments, the linker is cleavable under a certain physiological environment (for example, under an appropriate pH environment). In some embodiments, the linker is cleavable in an acidic environment with a pH of about 6.5 or lower, or by reagents such as enzymes. In some embodiments, the linker is sensitive to a cleavage agent. For example, pH, redox potential or the presence of a degrading molecule.

In some embodiments, the linker is linked internally by peptide bonds. In some embodiments, these peptide bonds are cleavable by a protease that is highly or specifically expressed around or in target cells, for example, cathepsin B in the lysosome or endosome.

In some embodiments, the linker has a structure selected from the group of:

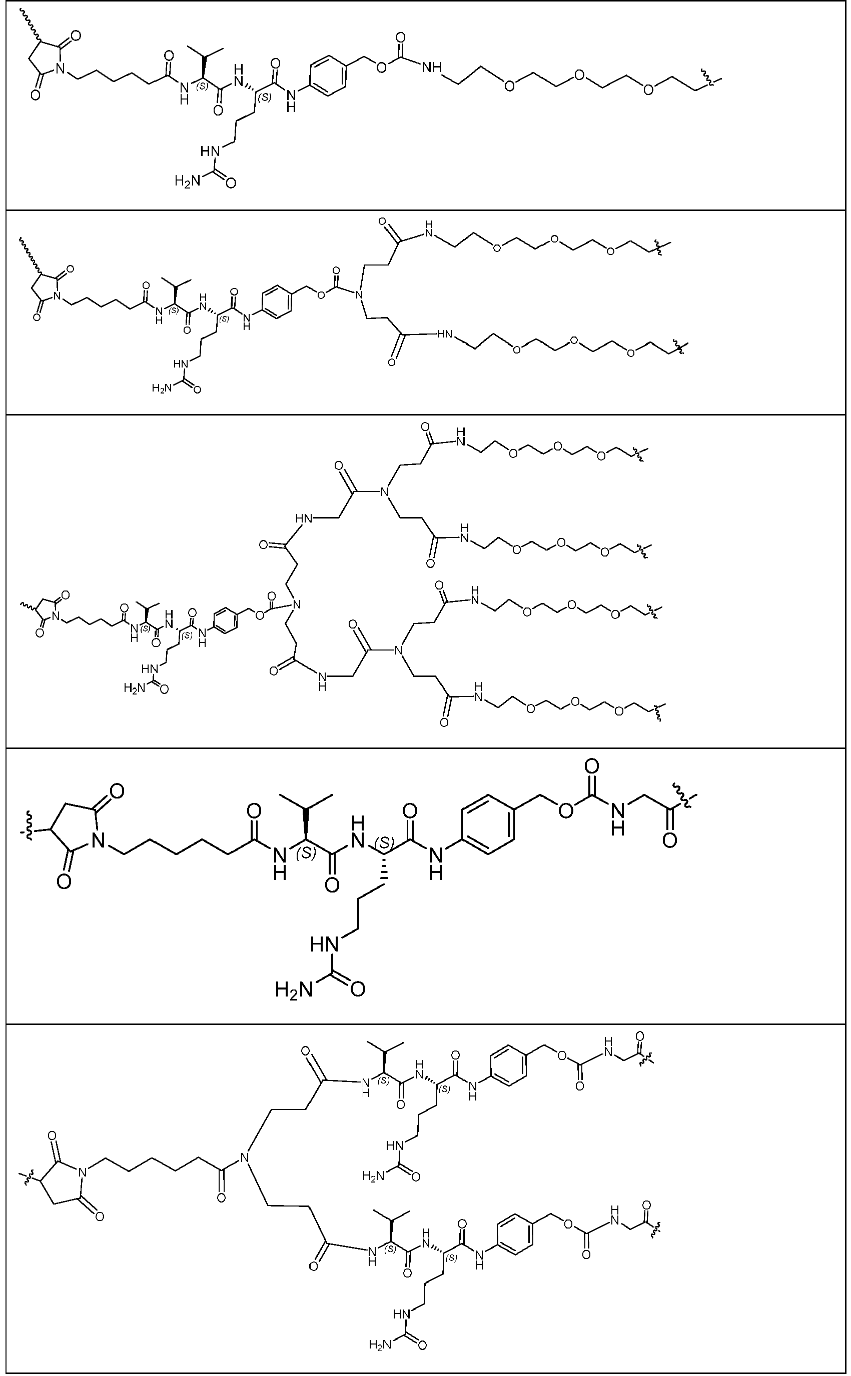

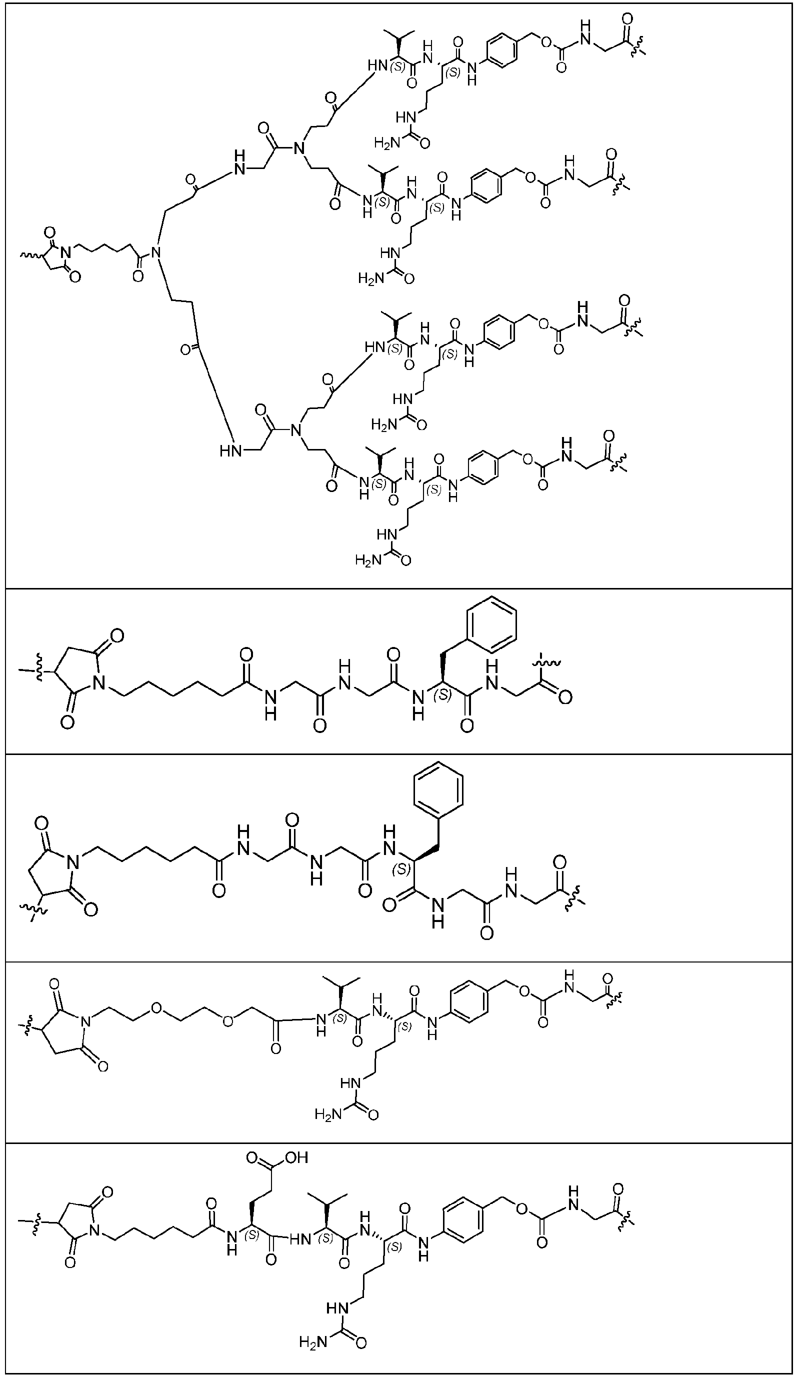

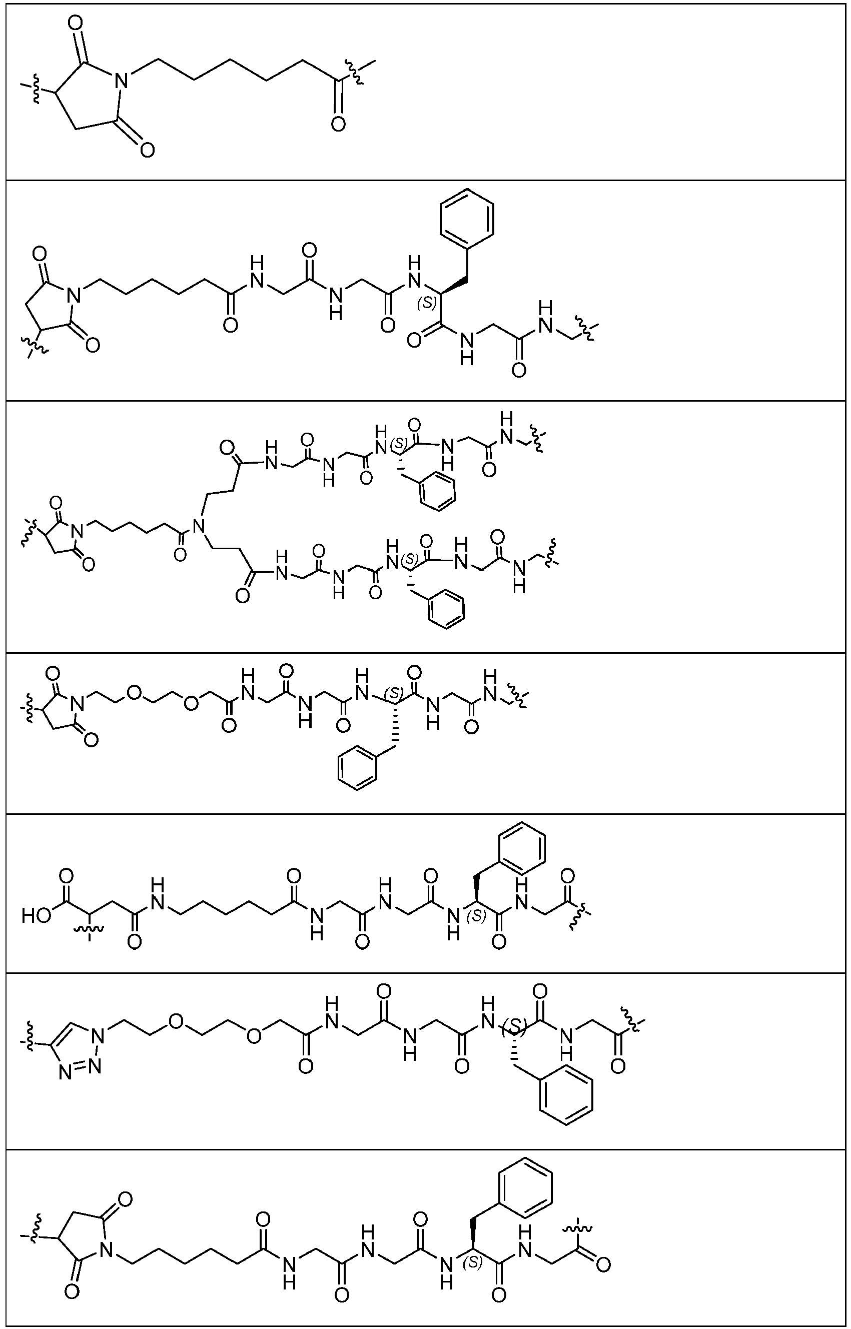

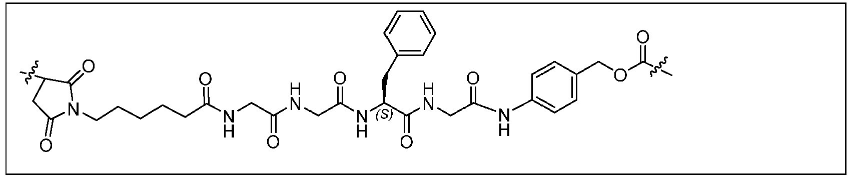

The term "payload" as used herein refers to a molecule or material to be delivered to a target cell or tissue. The molecule may be a biologically active molecule, for example, a toxic molecule. In some cases, the payload and the bioactive molecule are used interchangeably. Without limitation, the payload may be any molecule or material that is intended for use in the diagnosis, treatment, or prevention of a disease in a subject. In some embodiments, the payload as described in the present application may be any pharmaceutical agent that elicits biological or medicinal responses in a tissue, system, individual animal or human that is being sought by a researcher, veterinarian, medical doctor or other clinicians in preventing, inhibiting, ameliorating or treating a disease. In some embodiments, the payload has a molecular weight of less than or equal to about 5 kDa. In some embodiments, the payload has a molecular weight of less than or equal to about 1.5 kDa. In some embodiments, the payload is a drug or a diagnostic reagent that has been deemed safe and effective for use by appropriate drug approval and registration agencies (such as FDA, EMEA, or NMPA).

In some embodiments, the payloads of the present application include, but are not limited to: an anticancer drug, a radioactive substance, a vitamin, an anti-AIDS drug, an antibiotic, an immunosuppressant, an antiviral drug, an enzyme inhibitor, a neurotoxin, an opioid, a regulator of cell-extracellular matrix interaction, a vasodilator, an antihypertensive drug, a hypnotic, an antihistamine, an anticonvulsant, a muscle relaxant, an anti-Parkinson substance, an anticonvulsant and a drug for inhibiting muscle contraction, an antiparasitic drug and/or an anti-antiprotozoal drug, an analgesic drug, an antipyretic, a steroidal or non-steroidal anti-inflammatory drug, an anti-angiogenic factor, an anti secretory factor, an anticoagulant and/or an antithrombotic agent, a local anesthetic, a prostaglandin, an antidepressant, an antipsychotic, an antiemetic, or an imaging agent.

In some embodiments, the payload of the present application has a free amino or carboxyl group before being conjugated with the conjugate compound of the present application, and the payload is conjugated with the conjugate compound through an acylation reaction or a click reaction between the above-mentioned amino or carboxyl group and the corresponding part (for example, a linker) of the conjugate compound.

In some embodiments, the conjugate compound or the pharmaceutically acceptable salt thereof comprises one, two, three, four or more payloads. In some embodiments, the payload of the present application is a small molecule compound, a nucleotide (such as a DNA, a plasmid DNA, an RNA, an siRNA, an antisense oligonucleotide and an aptamer), a peptide, or a protein (for example, an enzyme). In some embodiments, the payload is a small molecule compound or a peptide.

In some embodiments, the peptide as the payload comprises at least 2, 3, 4, 5, 6, 7, 8, 9, 10, 12, 15, 18 or 20 amino acid residues. In some embodiments, the peptide as the payload comprises at most 4, 5, 6, 7, 8, 9, 10, 12, 15, 18 or 20 amino acid residues.

The "small molecule compound" as used herein refers to a compound having a molecular weight of less than or equal to about 2 kDa. In some embodiments, the small molecule compound has a molecular weight of less than or equal to about 1.5 kDa. In some preferred embodiments, the small molecule compound has a molecular weight of less than or equal to about 1 kDa, 800 Da, 700 Da, 600 Da, or 500 Da. In some embodiments, the small molecule compound of the present application is selected from the group consisting of: camptothecin and any derivative thereof (for example, SN38, Dx or Dxd), maytansine and any derivative thereof, a radioactive metal complex, a cyclooxygenase-2 inhibitor and any derivative thereof (for example, celecoxib), paclitaxel and any derivative thereof, epothilone and any derivative thereof, bleomycin and any derivative thereof, dactinomycin and any derivative thereof, plicamycin and any derivative thereof, and mitomycin C and any derivative thereof. In some preferred embodiments, the small molecule compound is camptothecin and any derivative thereof. In some preferred embodiments, the camptothecin is exatecan. In some embodiments, the small molecule compound as described in the present application is a drug for relieving or treating a cancer. In some embodiments, the small molecule compound as described in the present application is a drug for relieving or treating an autoimmune disease.

The term "camptothecin" as used herein refers to a cytotoxic alkaloid, mainly derived from Camptotheca acuminata (Nyssaceae) and showing a strong anti-tumor activity. The camptothecin and derivative thereof of the present application include a camptothecin and a derivative thereof that are already in existence or will be produced later. The camptothecin and derivative thereof of the present application include, but are not limited to: camptothecin, irinotecan, SN38, SN22, Dxd, exatecan (DX-8951), topotecan, GI-147211C, hycamtin, 9-aminocamptothecin, 7-hydroxymethyl camptothecin, 7-aminomethyl camptothecin, 10-hydroxy camptothecin, (20S)-camptothecin, 9-nitrocamptothecin, gimatecan, karenitecin, silatecan, lurtotecan, exatecan, diflomotecan, belotecan, lurtotecan and S39625. In some embodiments, the small molecule compound is SN38, SN22, Dxd, and exatecan (DX-8951). In some embodiments, the small molecule compound is lactic acid-exatecan. In some embodiments, the small molecule compound is exatecan (DX-8951).

In some embodiments, each D is independently selected from

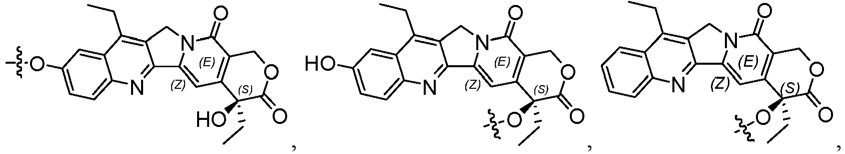

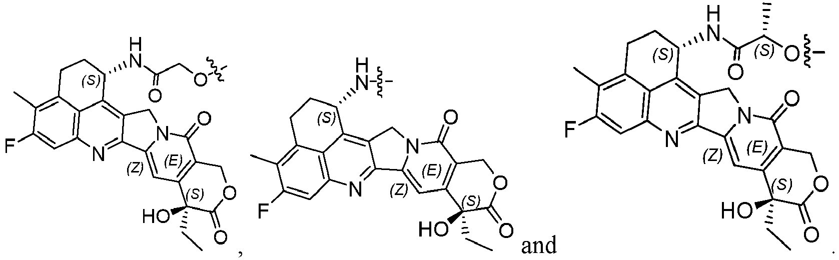

In some embodiments, the cell surface molecule is selected from TRPV6, FOLR1, PSMA, SSTR2, PD-L1, PD1, Her2, Trop2, Her3, 4-BBL, 4-1BB, CD70, CD27, CD155, CD122, CD113, CD28, CD86/80, CD160, ICOSL, ICOS, CD40, CD40L, OX40, OX40L, GITRL, GITR, NECTIN4, LRP1, GLUT1, EGFR1, AXL, CD44, Claudin18.2, APN, DLL3, CEACAM5, FZD10, TFRC, MET, IGFR1, CCKBR, LFA1, GPR87, GM-CSF, GM-CSFR, TIM3, TLR family, HHLA2, MHCII antigen, TCR, CTLA-4, IGIT, Galectin-3, Galectin-9, TIM-3, HVEM, BTLA, VISTA, phosphatidylserine, LAG3, LILRB4, SIGLEC15, NKG2A, NKG2D, SLAMF7, KIR2DL1, KIR2DL2, KIR2DL3, FGFR1, FGFR2, FGFR4, and NeuGcGM3.

In some embodiments, the cell surface molecule is selected from TRPV6, FOLR1, PSMA and SSTR2.

In some embodiments, the cell surface molecule is TRPV6 and FOLR1, PSMA and FOLR1, or SSTR2 and FOLR1, respectively. In some preferred embodiments, the cell surface molecule is TRPV6 and FOLR1, respectively.

In some embodiments, the ligand targeting FOLR1 is selected from folic acid or an analogue thereof. In some preferred embodiments, the folic acid analogue is selected from 5-methyltetrahydrofolate, 5-formyltetrahydrofolate, methotrexate , and 5,10-methylenetetrahydrofolate.

In some embodiments, the ligand targeting SSTR2 is selected from octreotide and any analogue thereof.

In some embodiments, the ligand moiety comprises more than three ligands that target cell surface molecules. In some preferred embodiments, the cell surface molecule is selected from TRPV6, FOLR1, PSMA and SSTR2.

TRPV6, *i.e.,* the transient receptor potential cation channel subfamily V member 6 (TRPV6), is a highly selective calcium ion transmembrane transport channel that mediates the active transport of calcium ions from extracellular to intracellular. TRPV6 is expressed in normal human kidney, gastrointestinal tract, pancreas, breast, salivary gland, *etc.,* and mainly expressed in intestinal epithelial cells. Since TRPV6 participates in the transport of calcium ions into a cell, when the number or function of TRPV6 channels changes, it can cause changes in the regulation of calcium ions, and further lead to structural or functional abnormalities of related tissues and organs. Compared with normal tissues, the expression of TRPV6 is significantly increased in malignant tumors such as breast cancer, bile duct cancer, ovarian cancer, lung squamous cell carcinoma, and prostate cancer, and the abnormal expression thereof may be related to the formation and progression of the tumors.

FOLR1 is a glycosylphosphatidylinositol (GPI)-anchored glycoprotein that binds folate with nanomolar affinity, thereby promoting receptor-mediated endocytosis. Rapidly growing solid malignancies, including ovarian and lung cancers, depend on folic acid for metabolism and nucleic acid synthesis. In some embodiments, the targeting molecule as described in the present application includes a ligand that target FOLR1.

PSMA refers to "prostate-specific membrane antigen", which is a type II transmembrane glycoprotein that exists in the membrane of prostate epithelial cells and consists of 750 amino acids, comprising 19 amino acids in the intracellular region, 24 amino acids in the transmembrane region and 707 amino acids in the extracellular region. The prostate-specific membrane antigen is expressed in normal prostate epithelial cells, but is expressed at a much higher level in prostate cancer cells. Compared with the traditional prostate-specific antigen used for clinical detection, the prostate-specific membrane antigen is a more sensitive and specific prostate cancer tumor marker, and especially, it is highly expressed in both hormone-refractory prostate cancer and prostate cancer metastatic lesions, and has a high sensitivity and specificity in distinguishing prostate cancer from other types of malignant tumors. Moreover, in a variety of nonprostate solid tumors (such as lung cancer, bladder cancer, gastric cancer, pancreatic cancer, kidney cancer and colorectal cancer), the prostate-specific membrane antigen is also highly and specifically expressed on tumor vascular endothelial cells.

SSTR2, i.e., somatostatin receptor-2, is a G protein-coupled receptor that can be activated by somatostatin or its synthetic analogues. SSTR2 is one of five subtypes of somatostatin receptors and thought to be involved in tumor development. Somatostatin or somatostatin analogues can inhibit the proliferation of tumor cells through SSTR2.

In some embodiments, the ligands targeting different cell surface molecules are linked to each other directly, or via a spacer. In some embodiments, the spacer consists of amino acids. In some preferred embodiments, the spacer consists of natural amino acids or unnatural amino acids. In still other preferred embodiments, the spacer is glycine. In some embodiments, the spacer is Arg-Arg, Ala-Ser-Asn, Ala-Ala-Ala, Ser-Ser-Arg, Pro-Arg, and Pro-Leu-Gly. In some embodiments, the spacer is cleavable under a certain physiological environment by protease cleavage or reduction. In some embodiments, the spacer is cleavable by a protease that is specifically expressed in target cells or to be expressed in target cells.

"Cleavable" or "cleavage" as used herein includes a metabolic process or reaction process on the conjugate compound provided in the present application, whereby a linker between a payload and a multi-ligand moiety, or a spacer between ligands is broken to release the free payload or the ligand.

In some embodiments, the ligand moiety has the following structure:

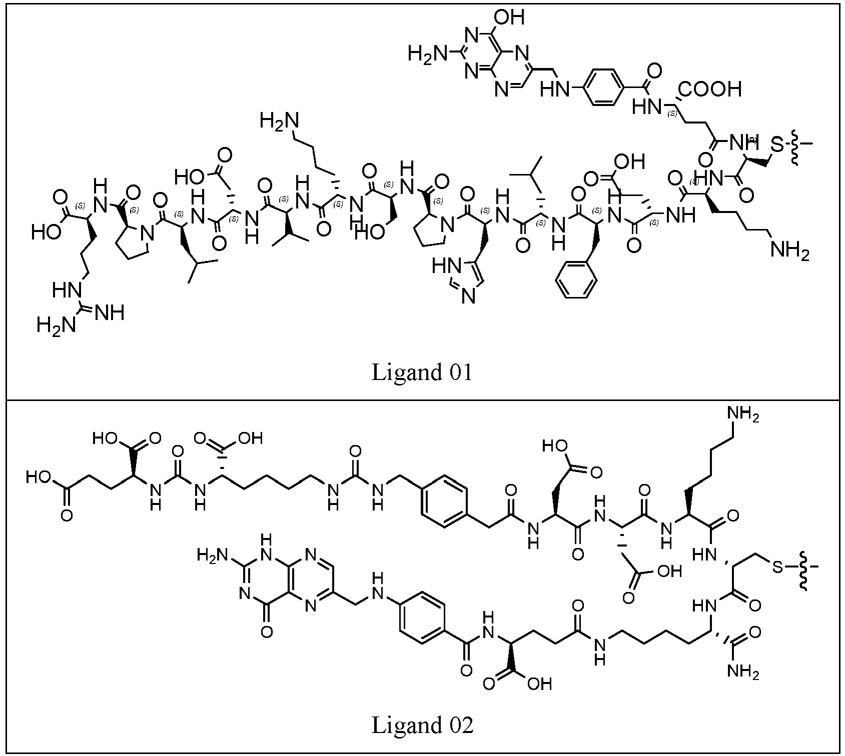

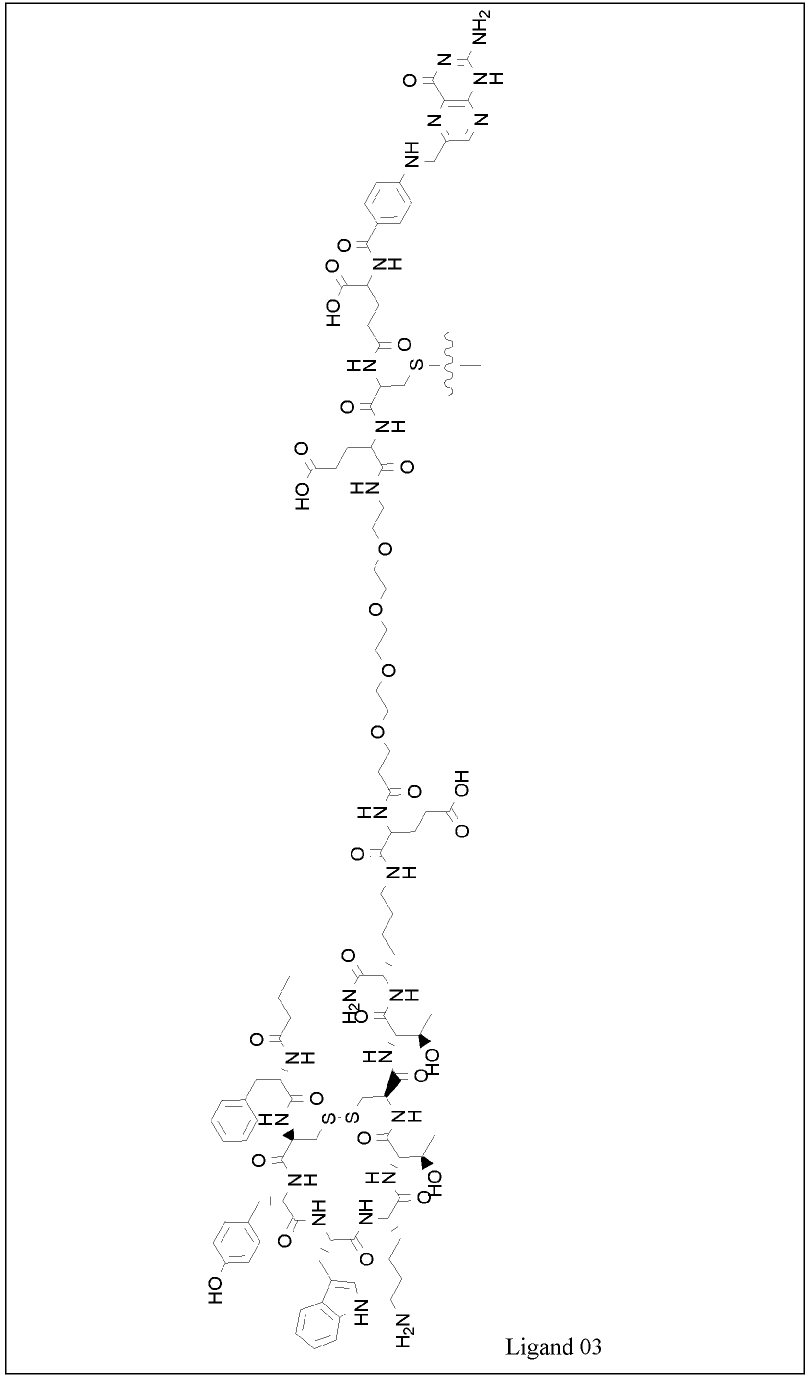

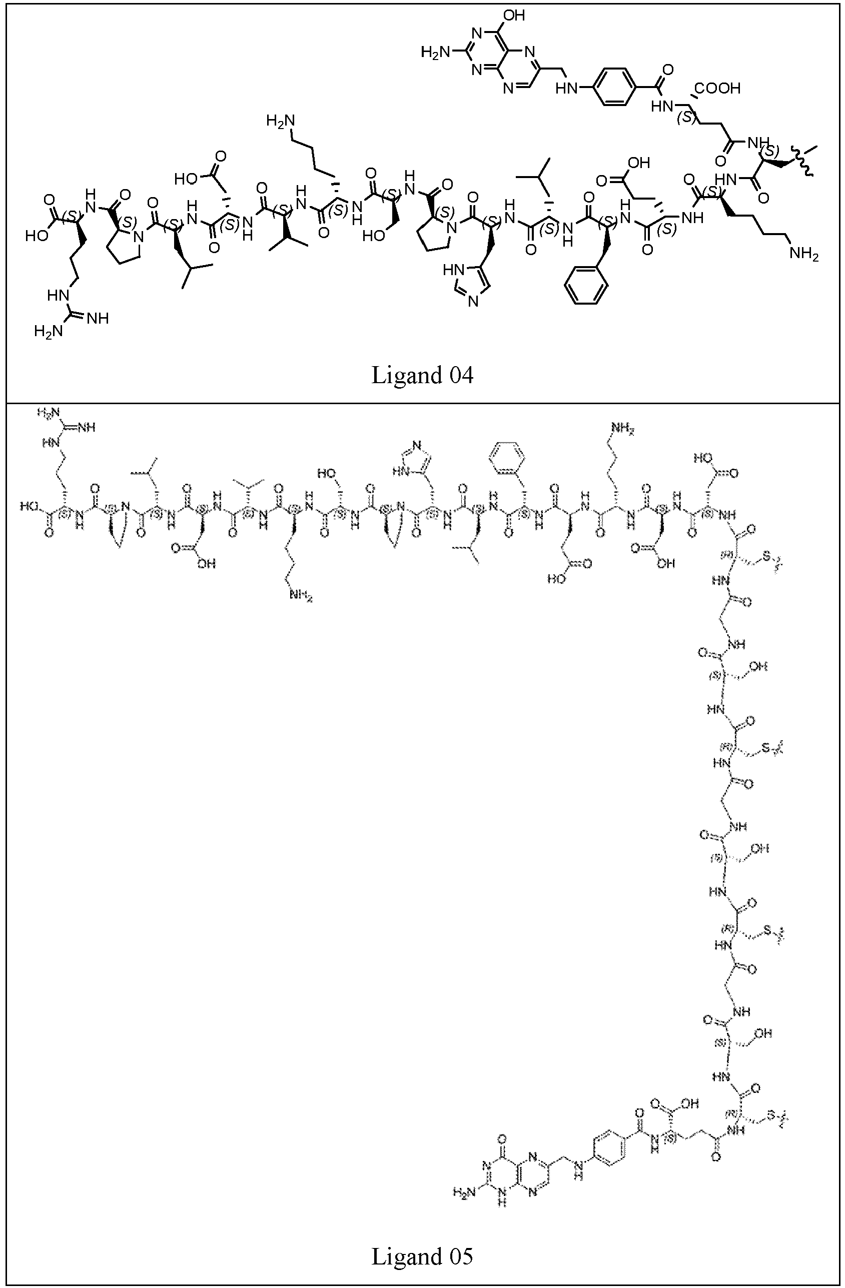

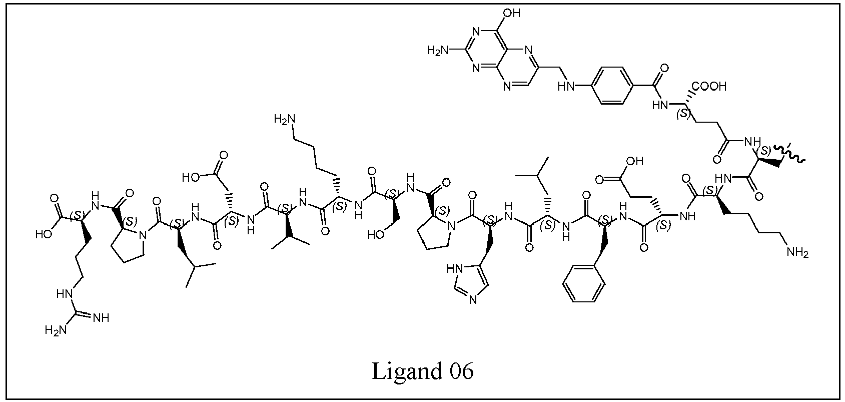

In some embodiments, q is 1. In some embodiments, q is 2. In some embodiments, q is 3. In some embodiments, q is 4.

One aspect of the present application discloses a conjugate compound or a pharmaceutically acceptable salt thereof, the conjugate compound is selected from:

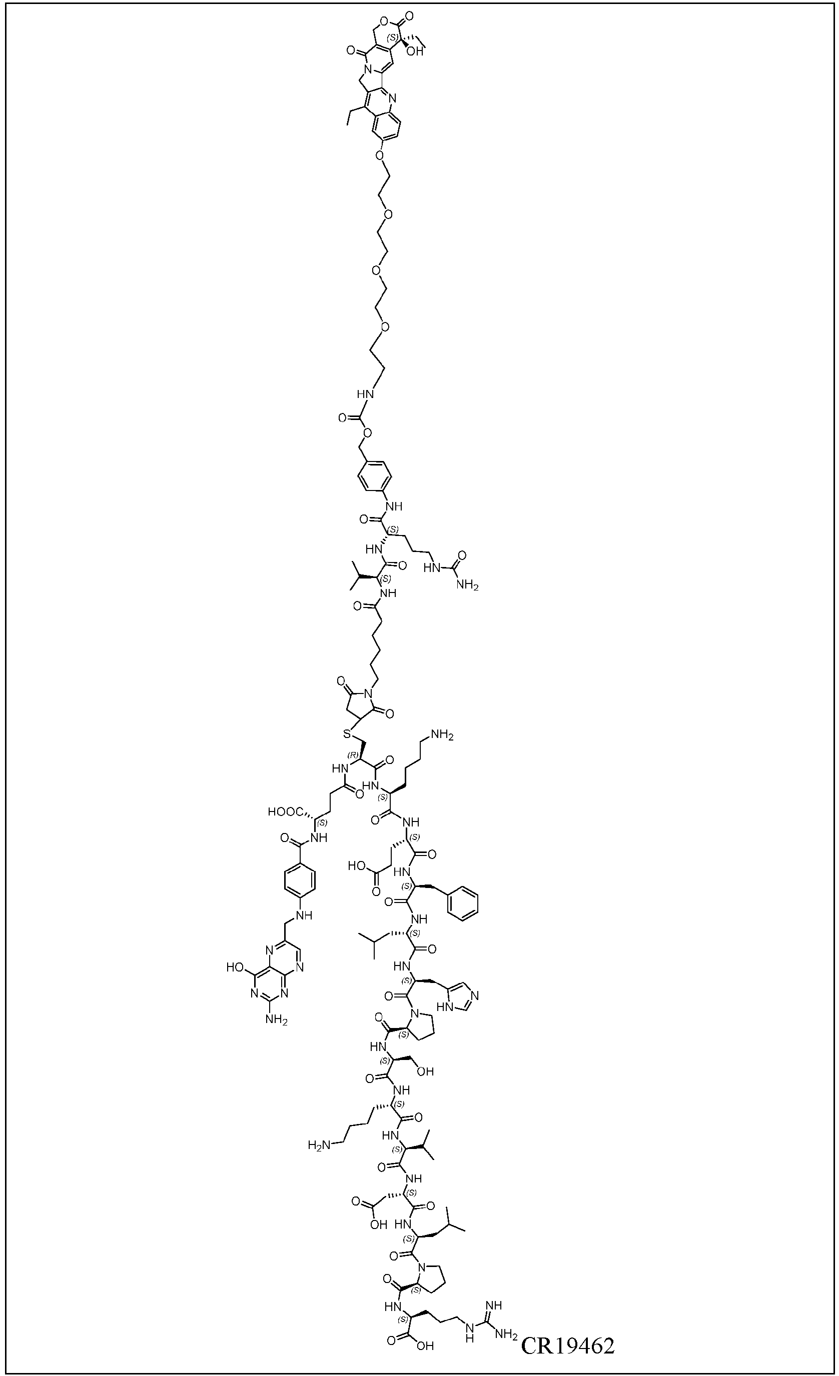

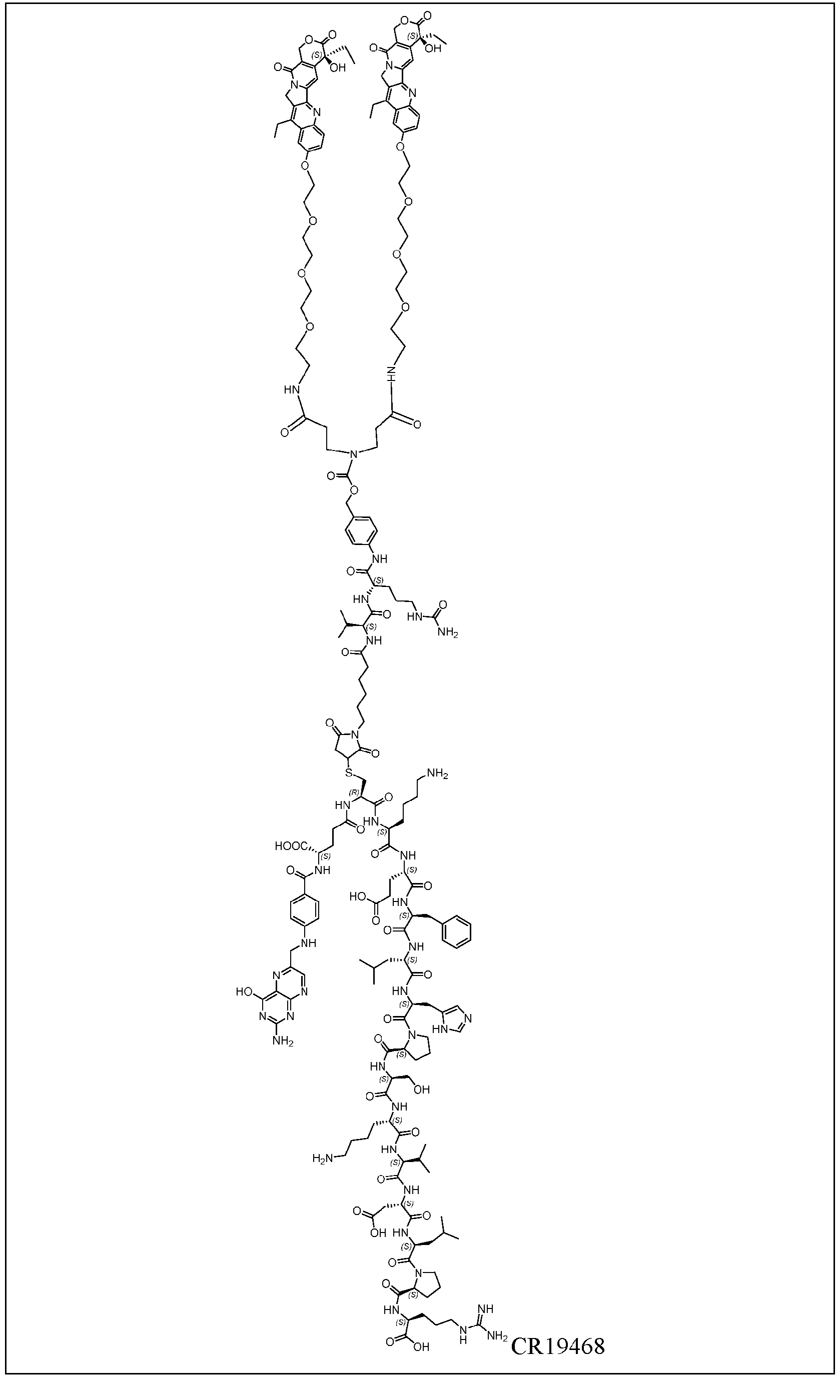

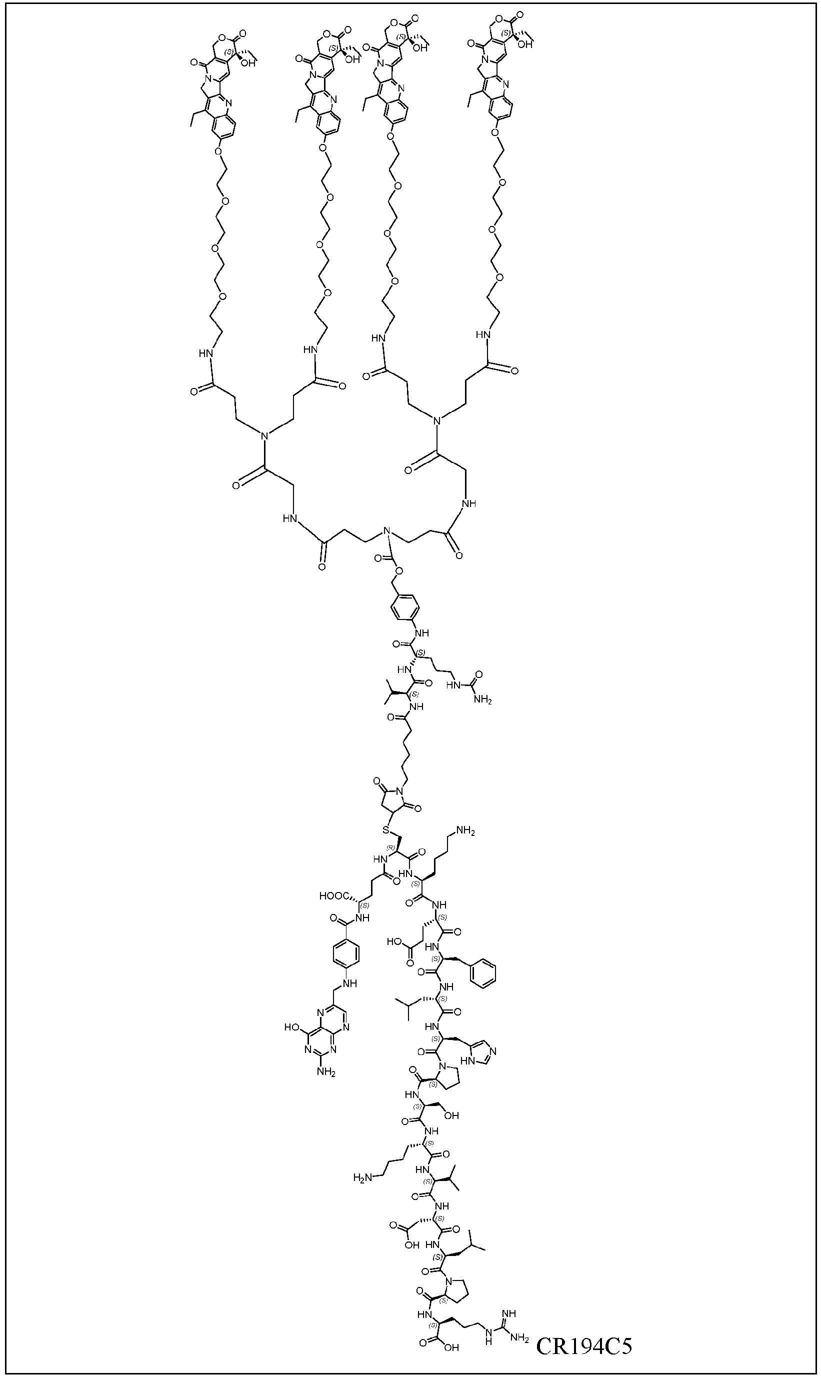

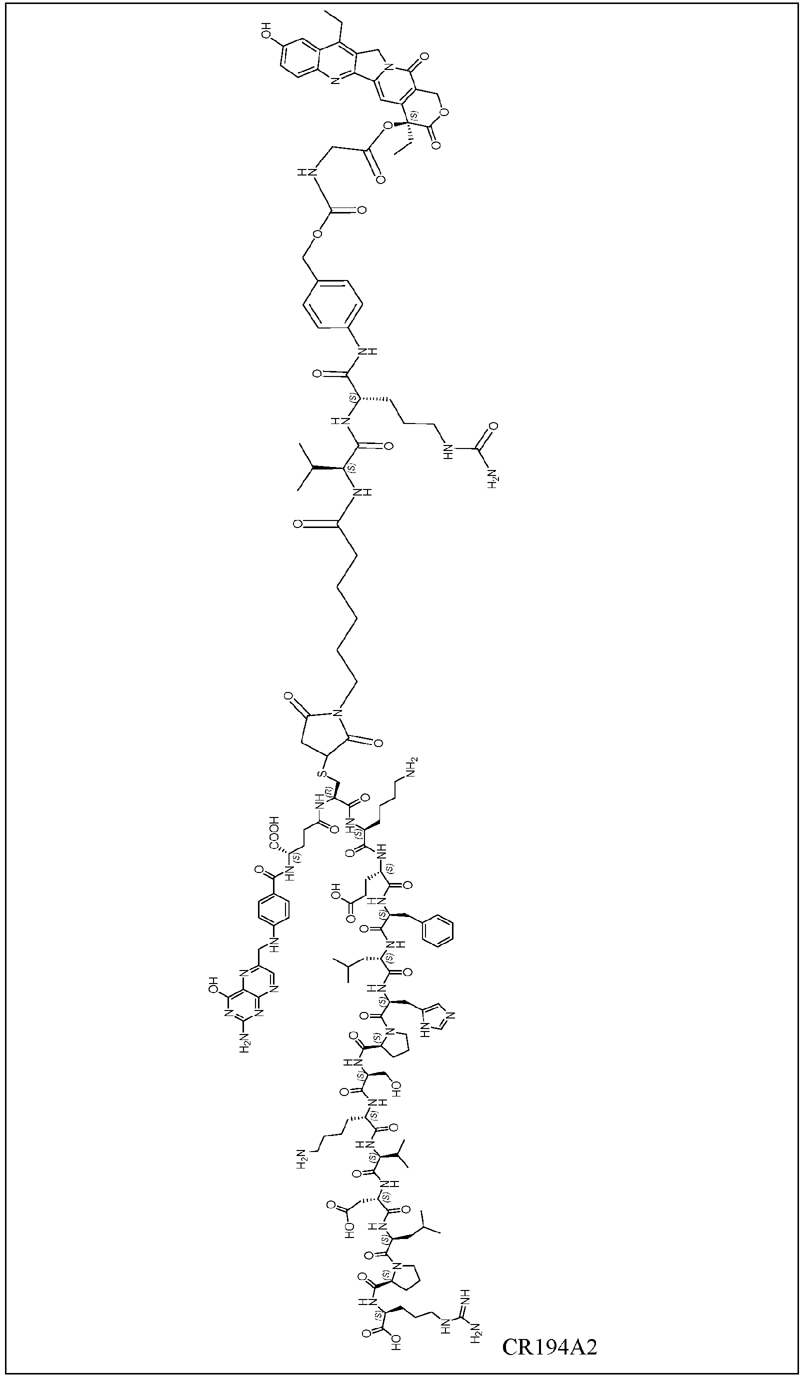

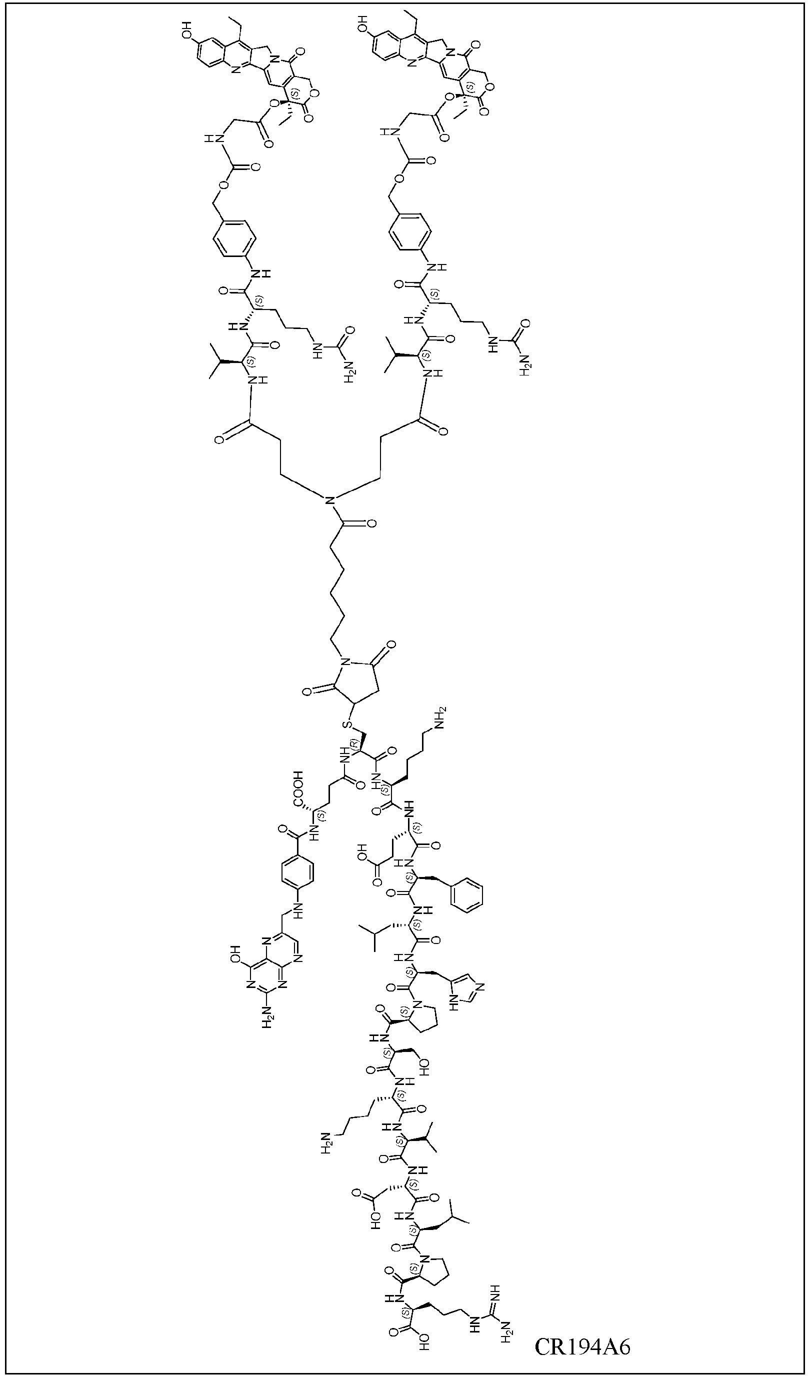

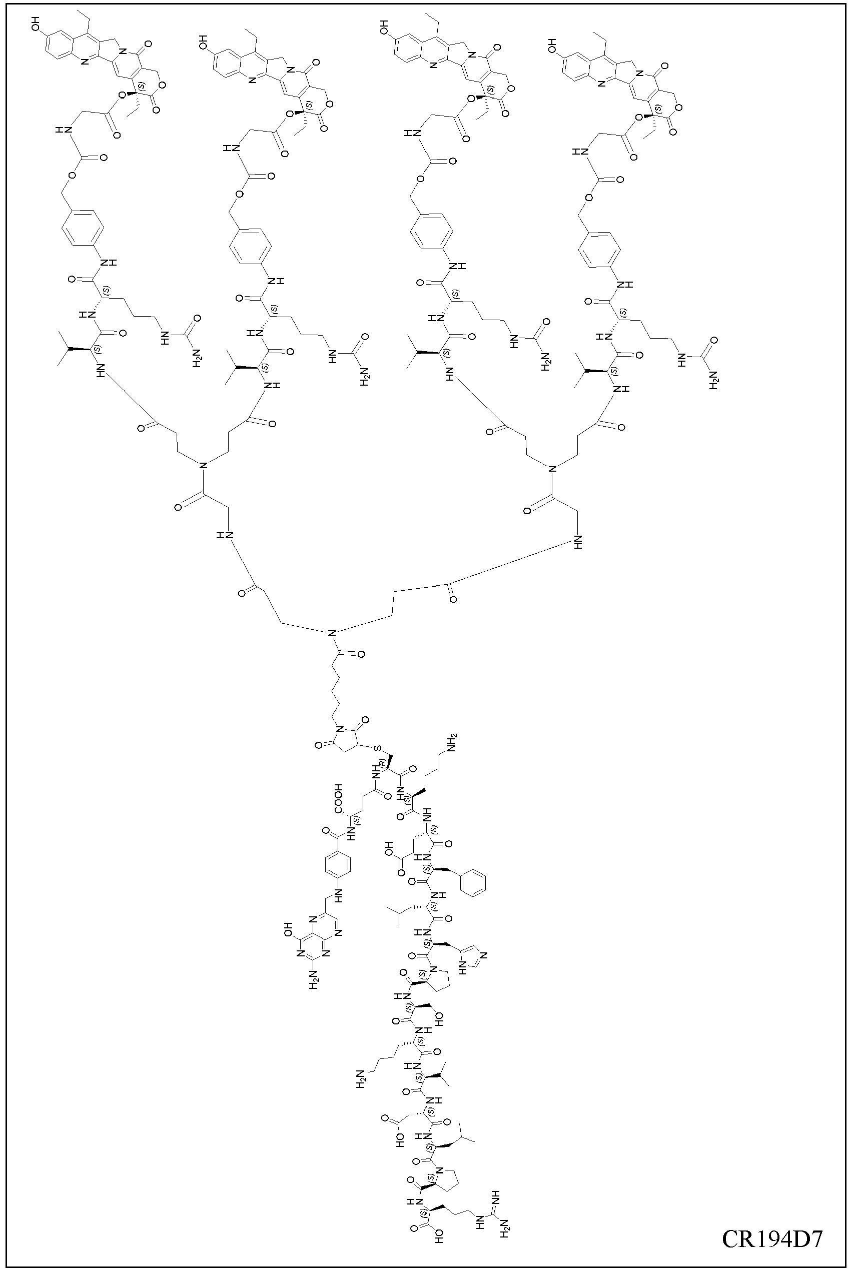

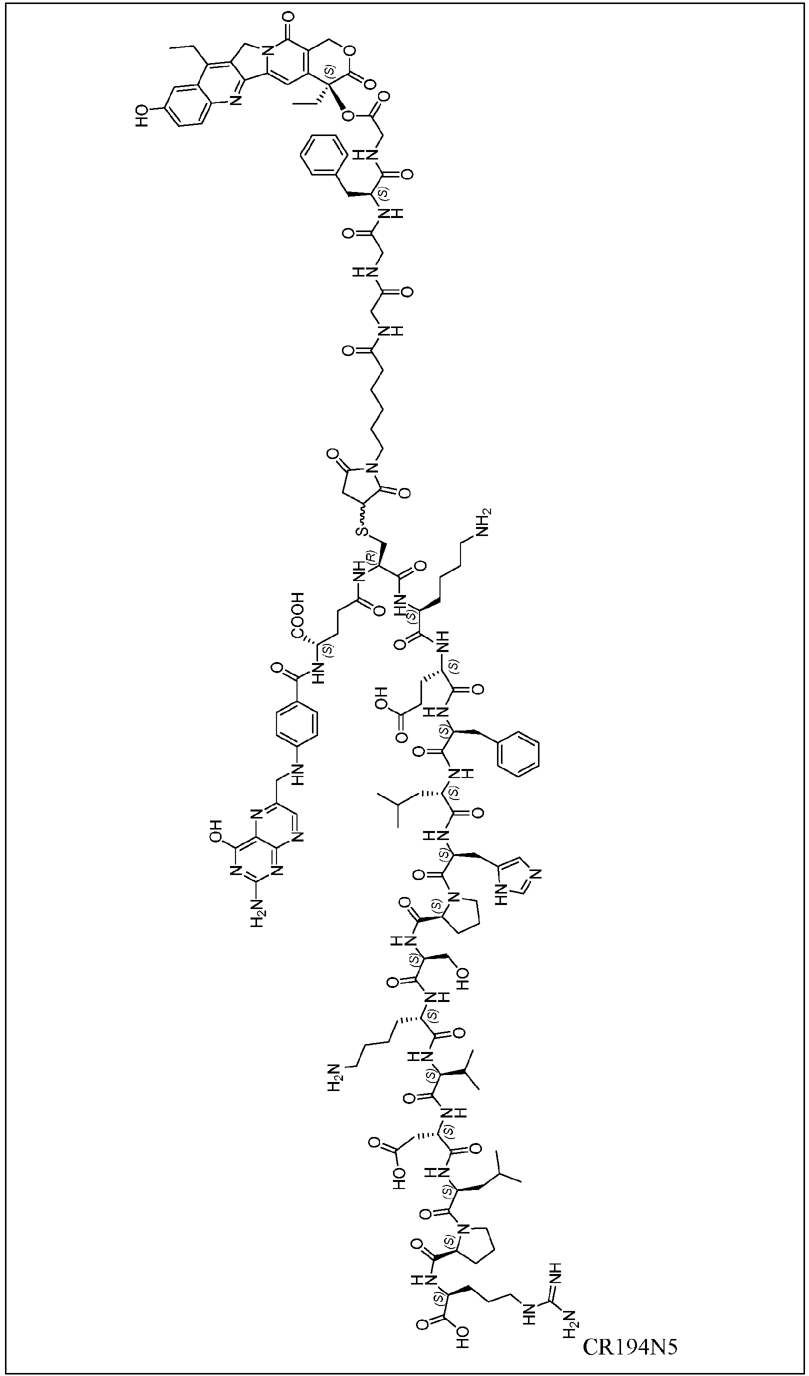

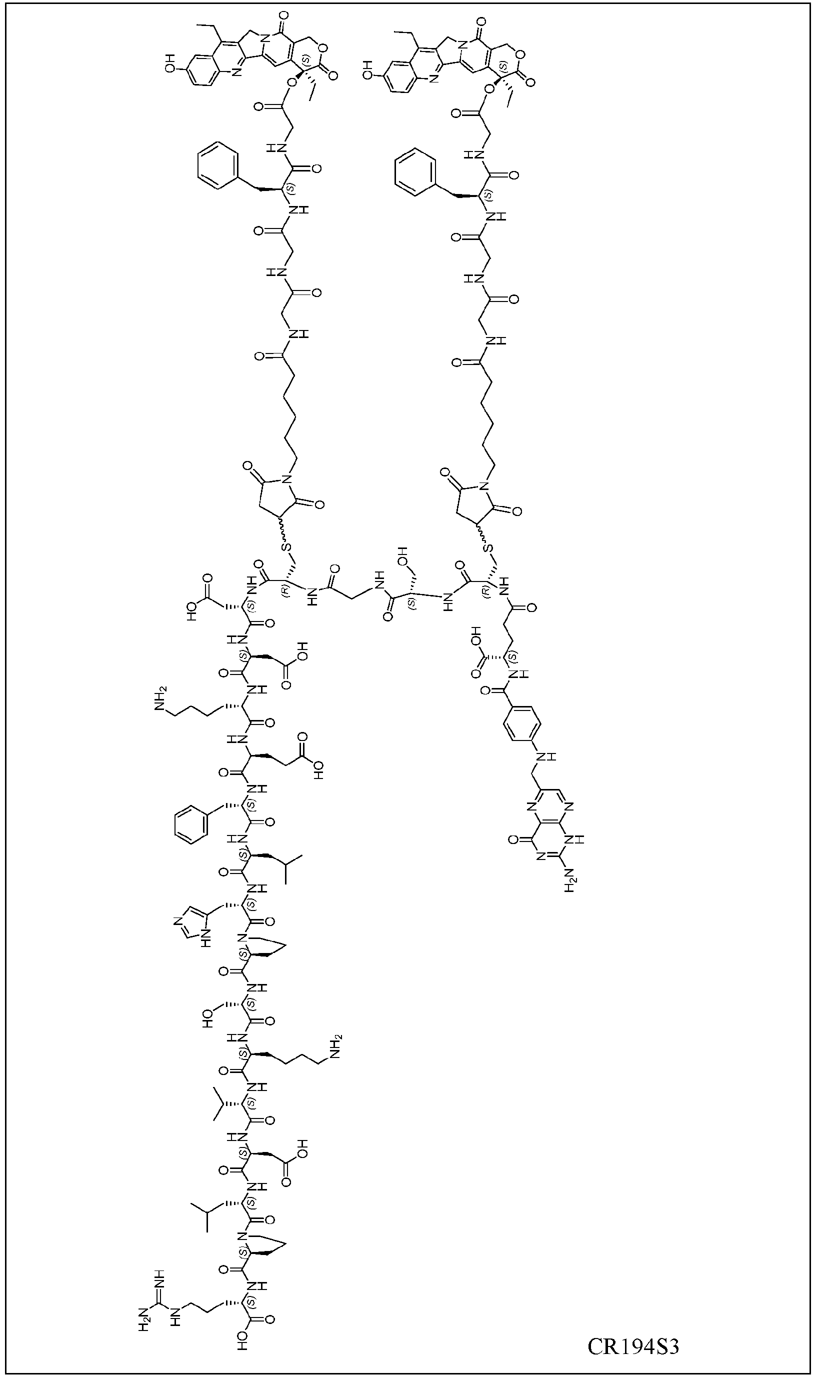

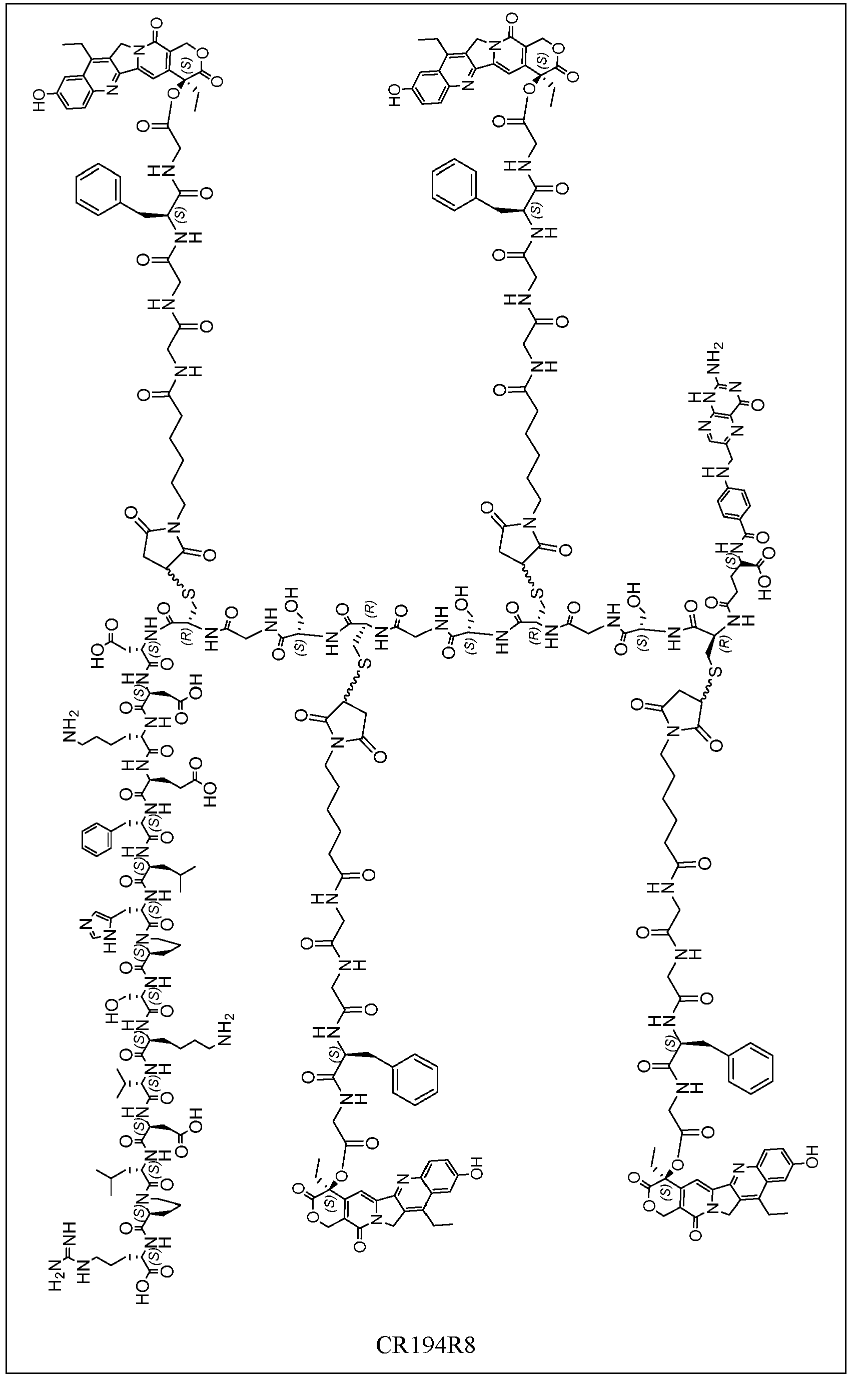

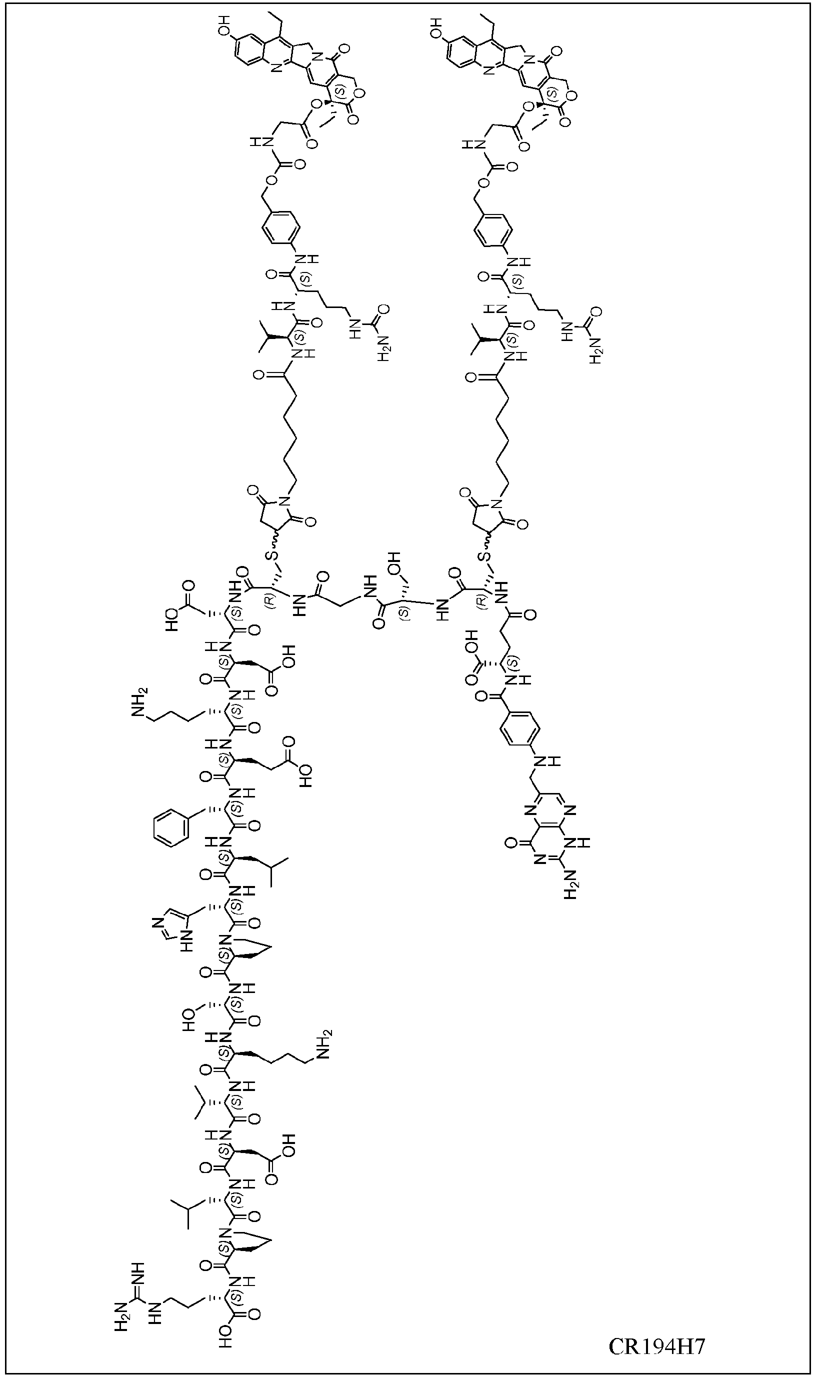

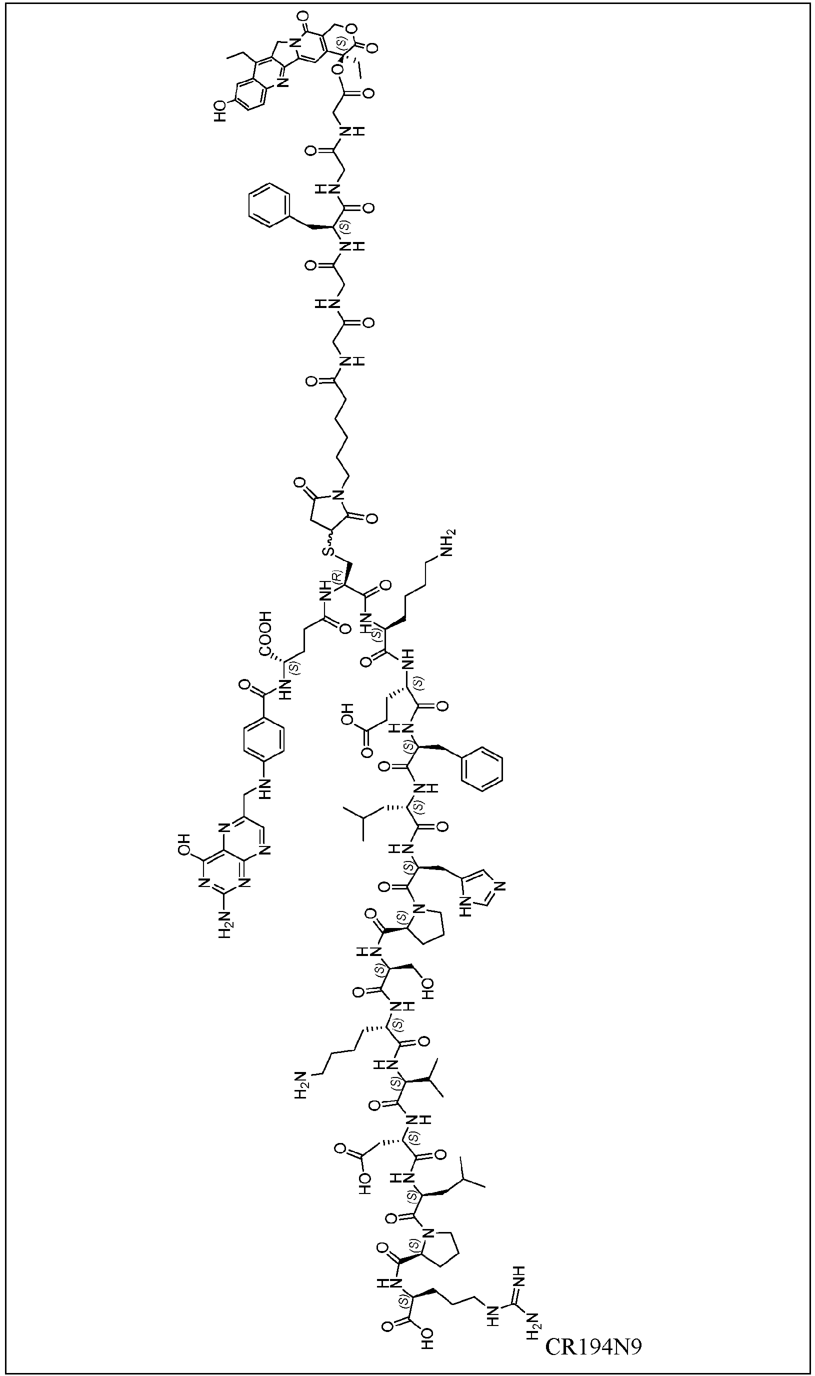

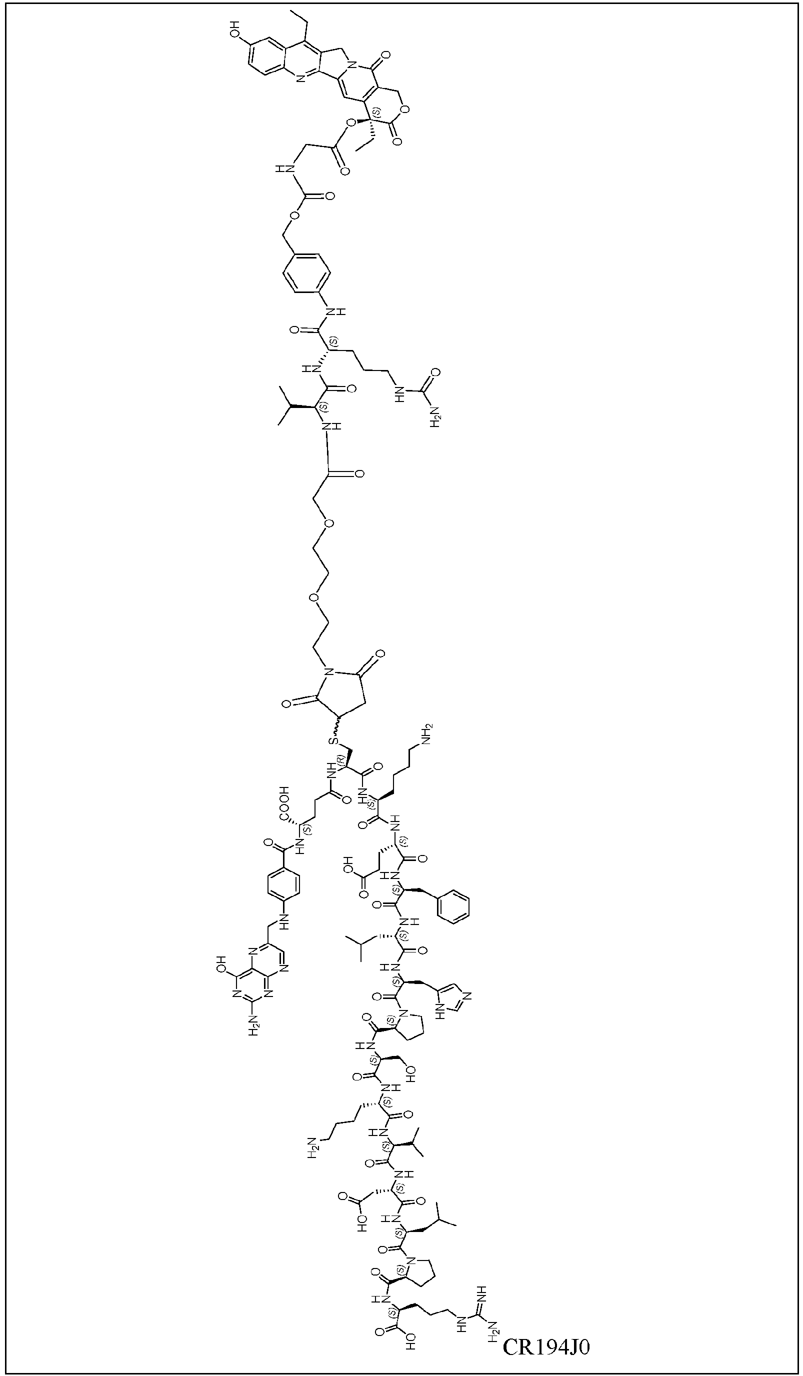

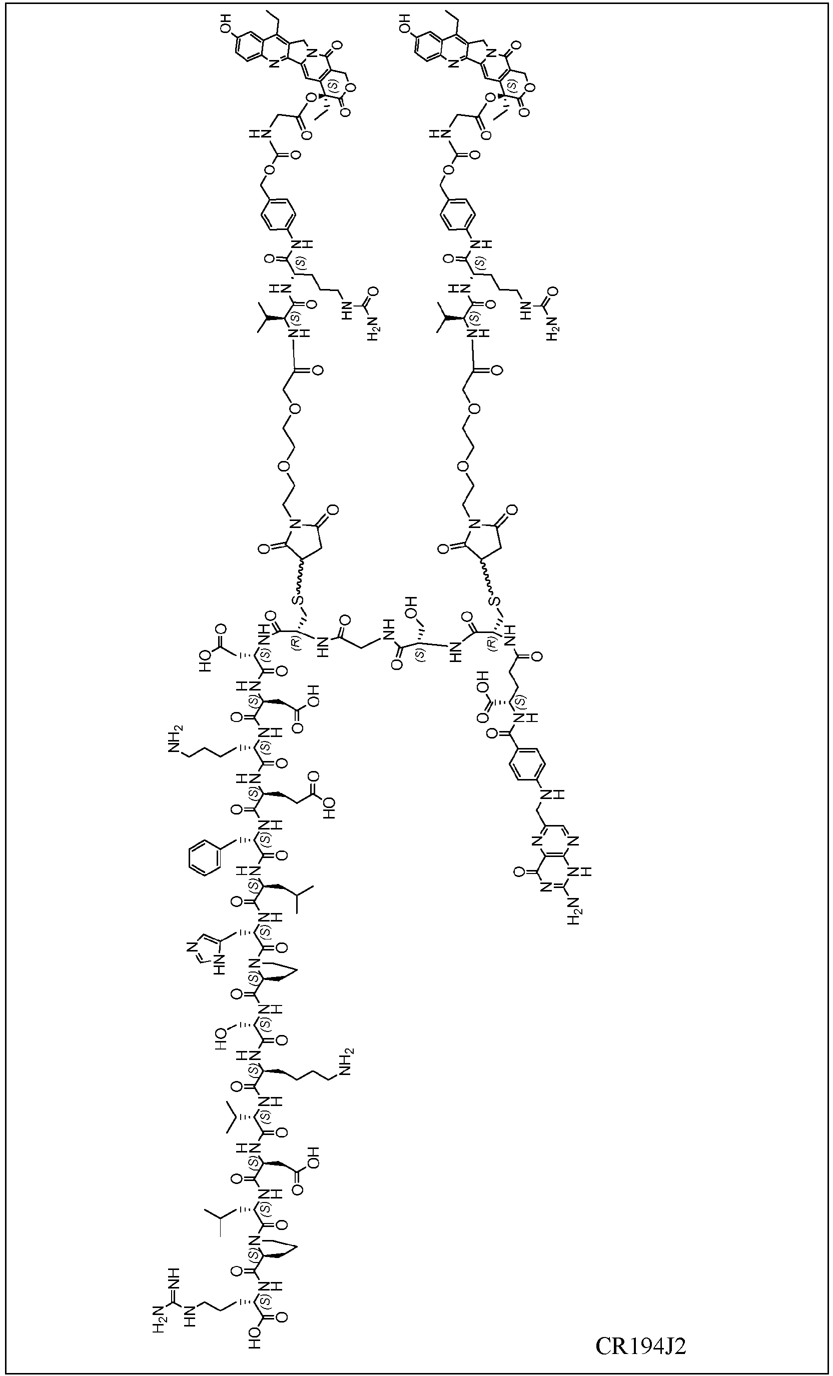

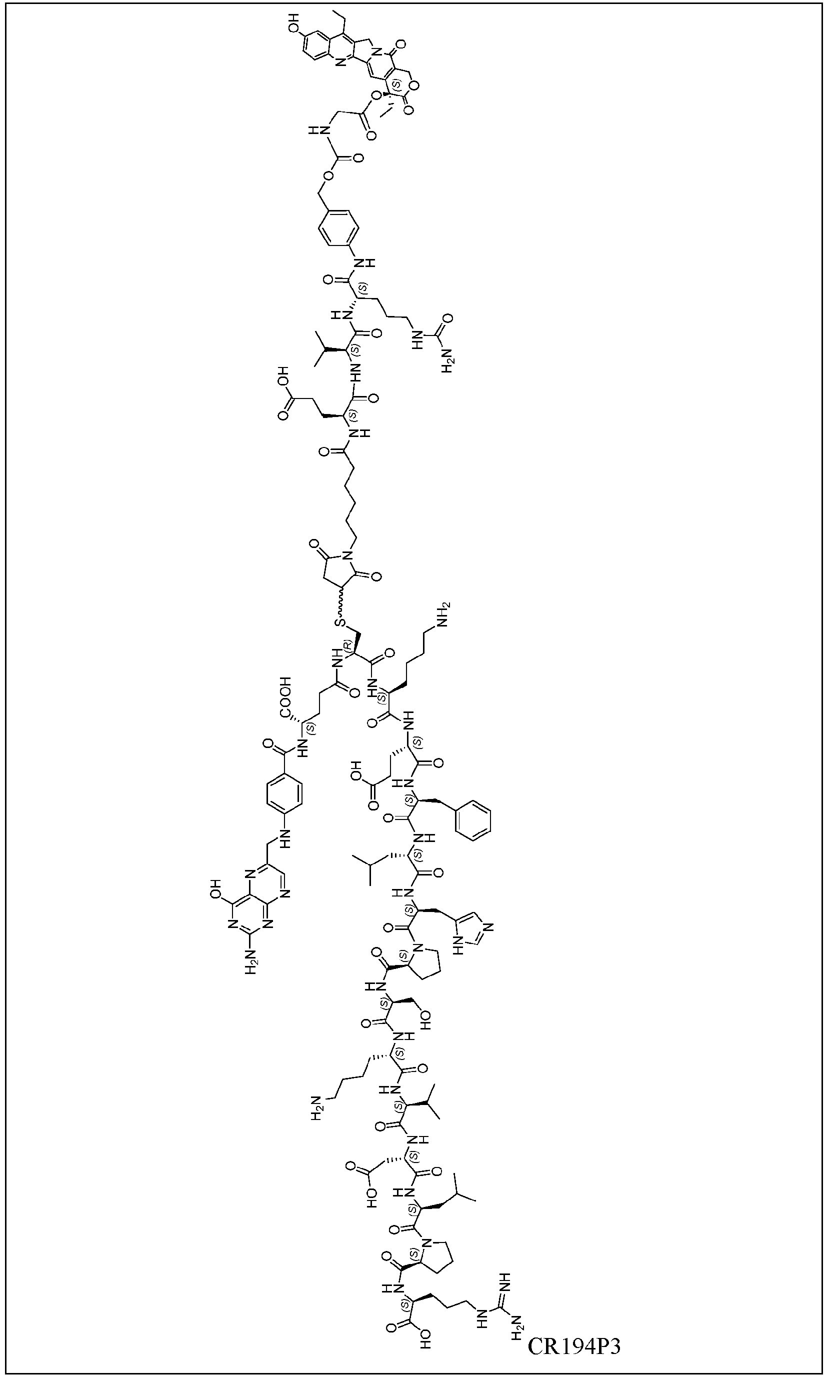

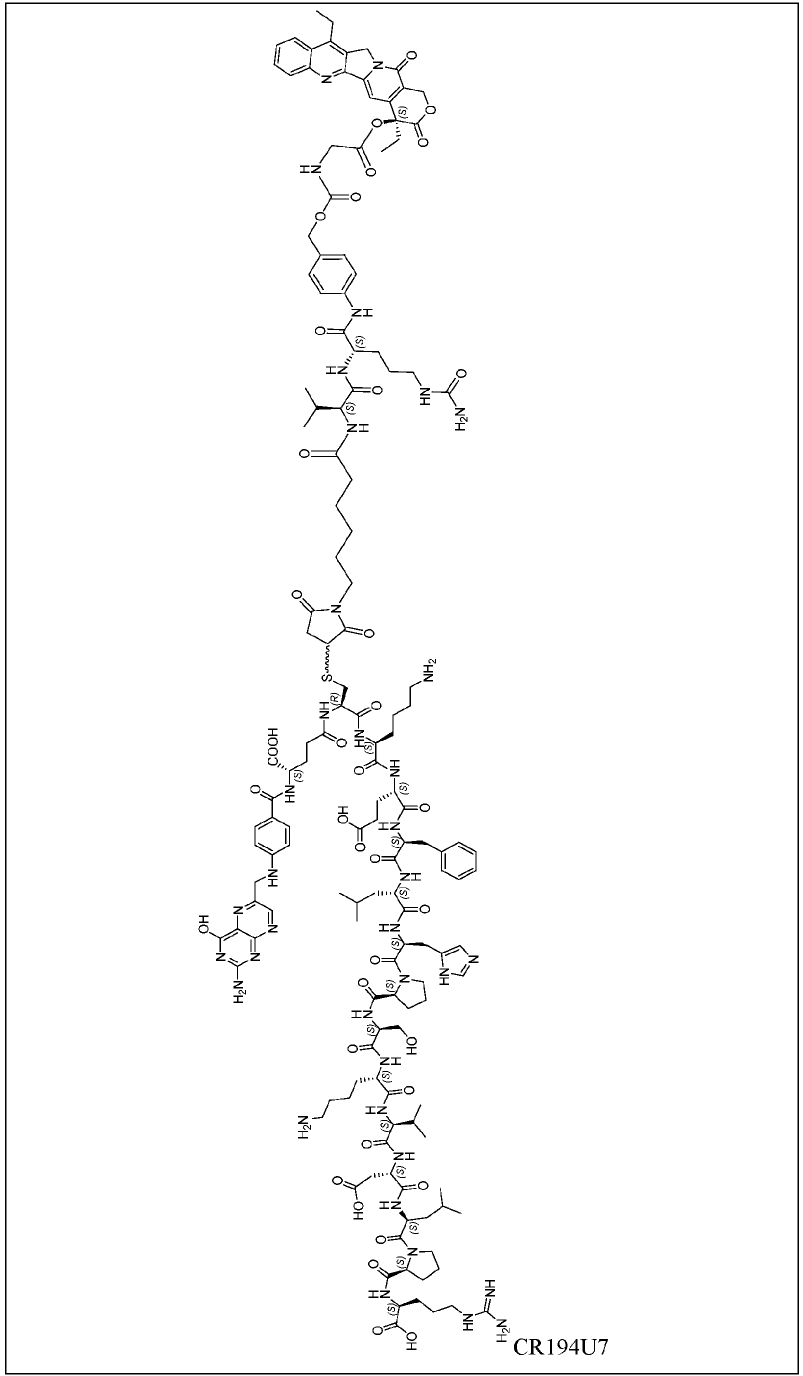

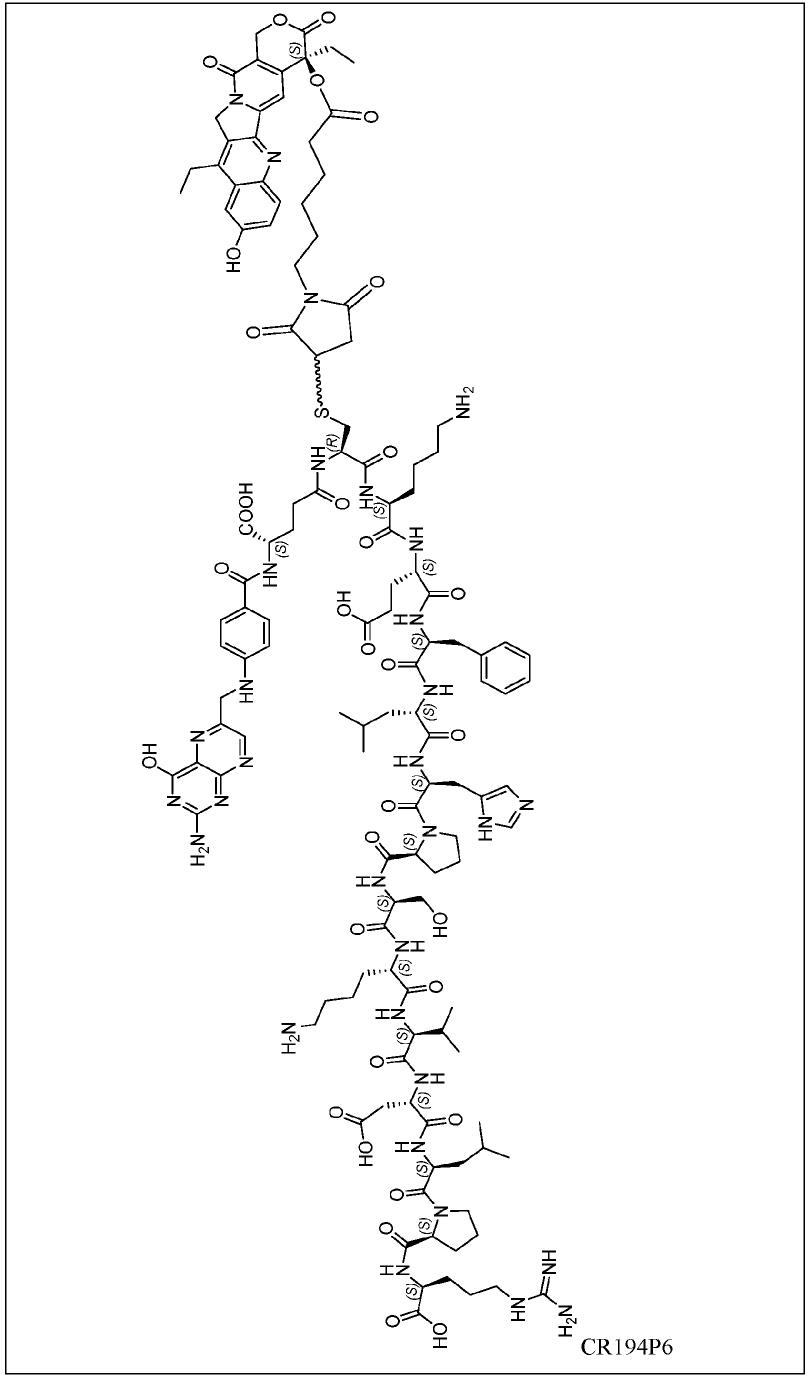

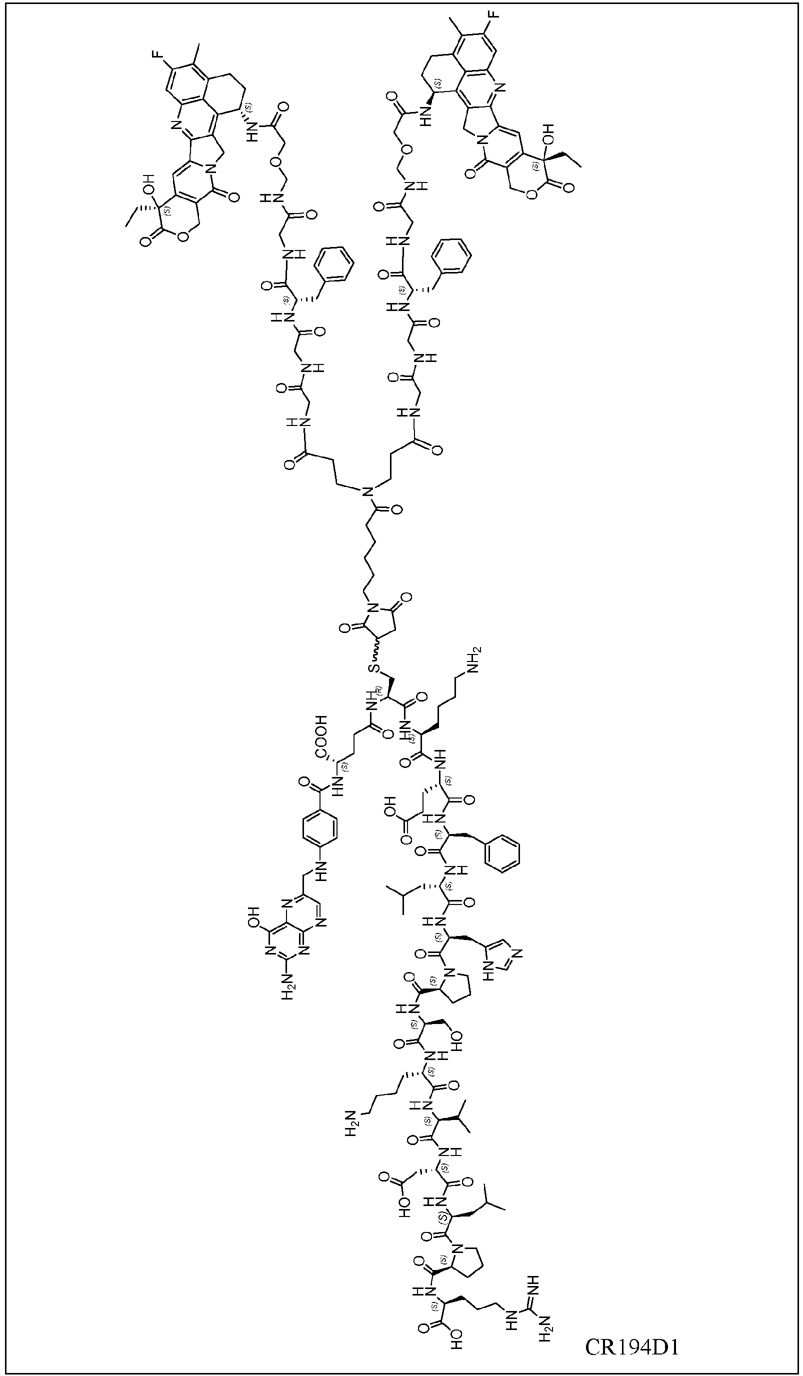

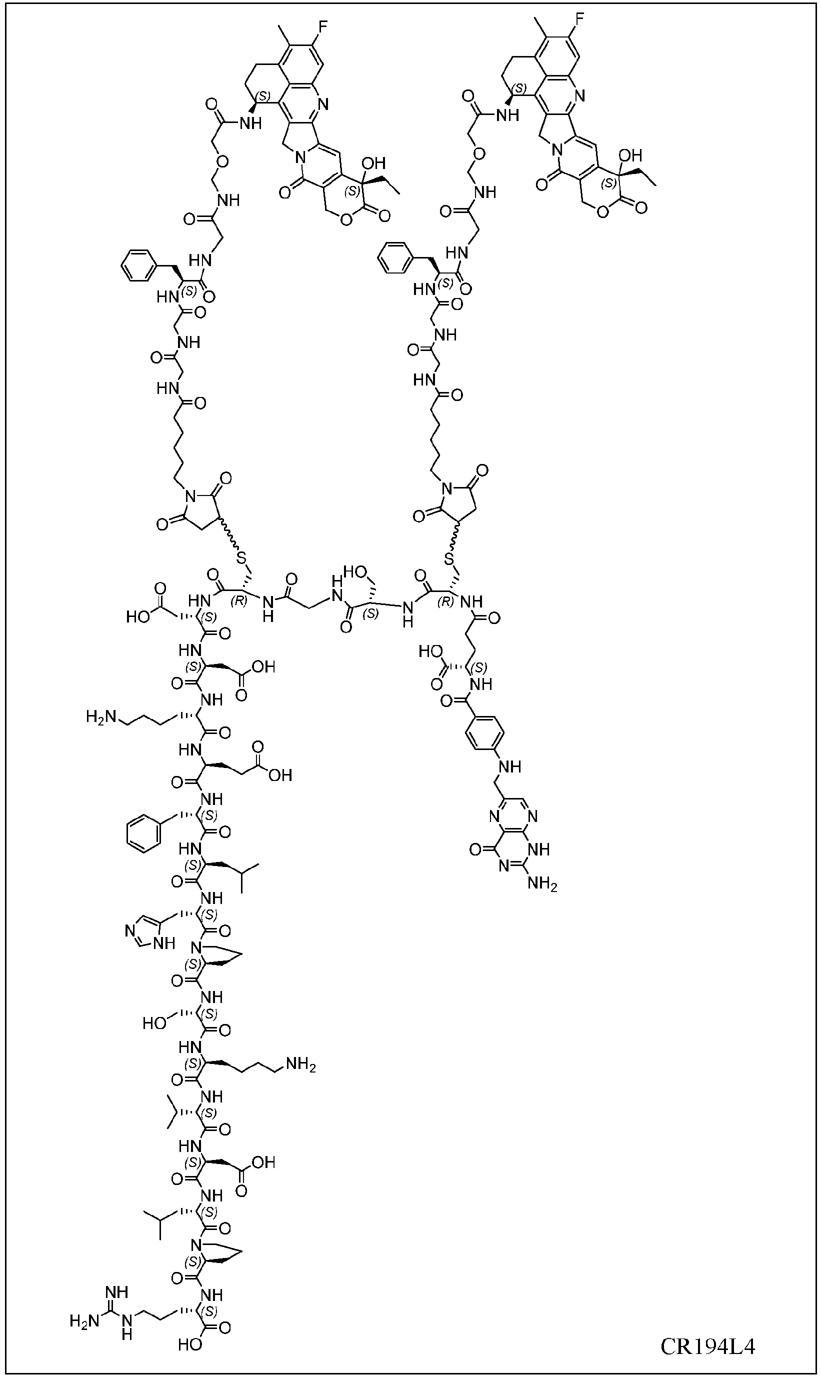

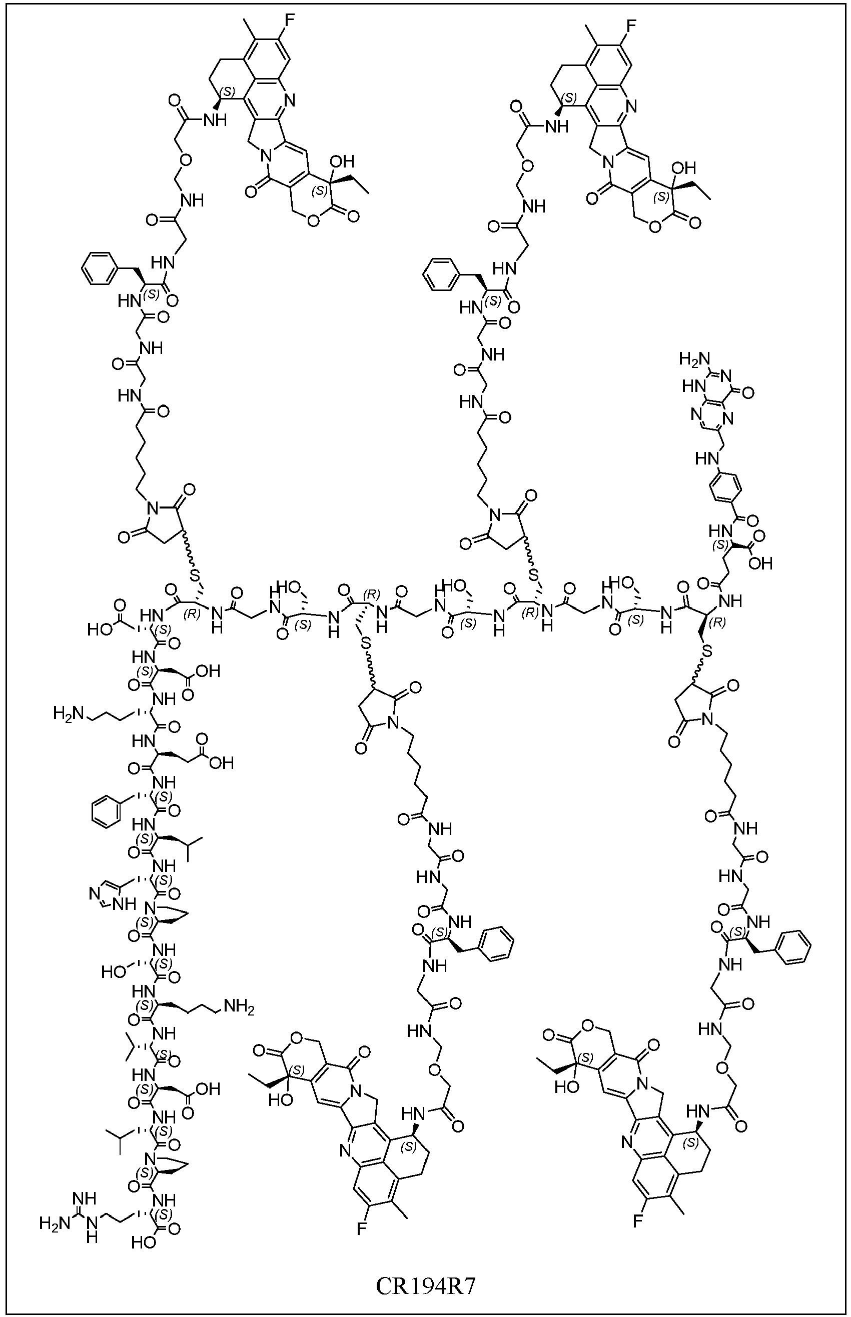

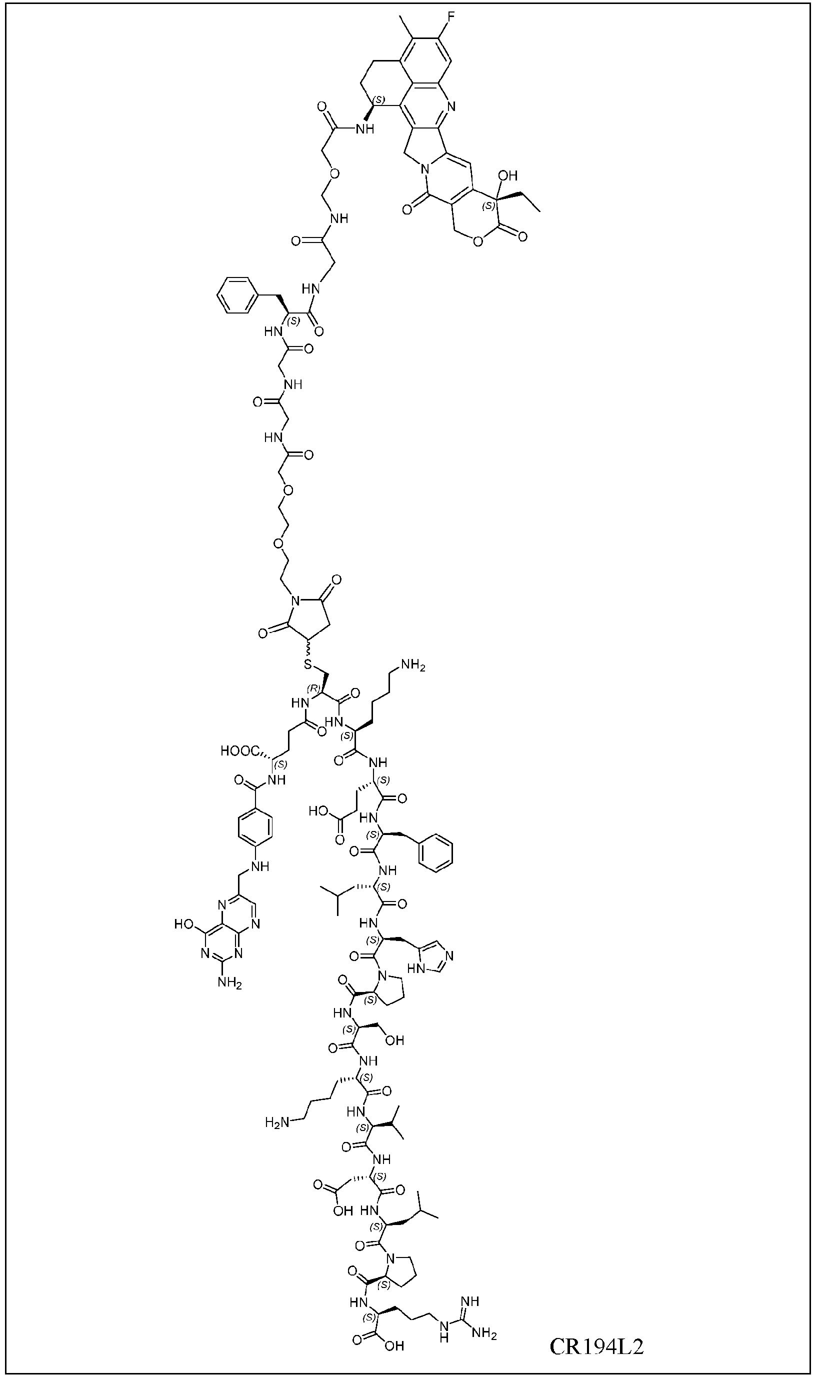

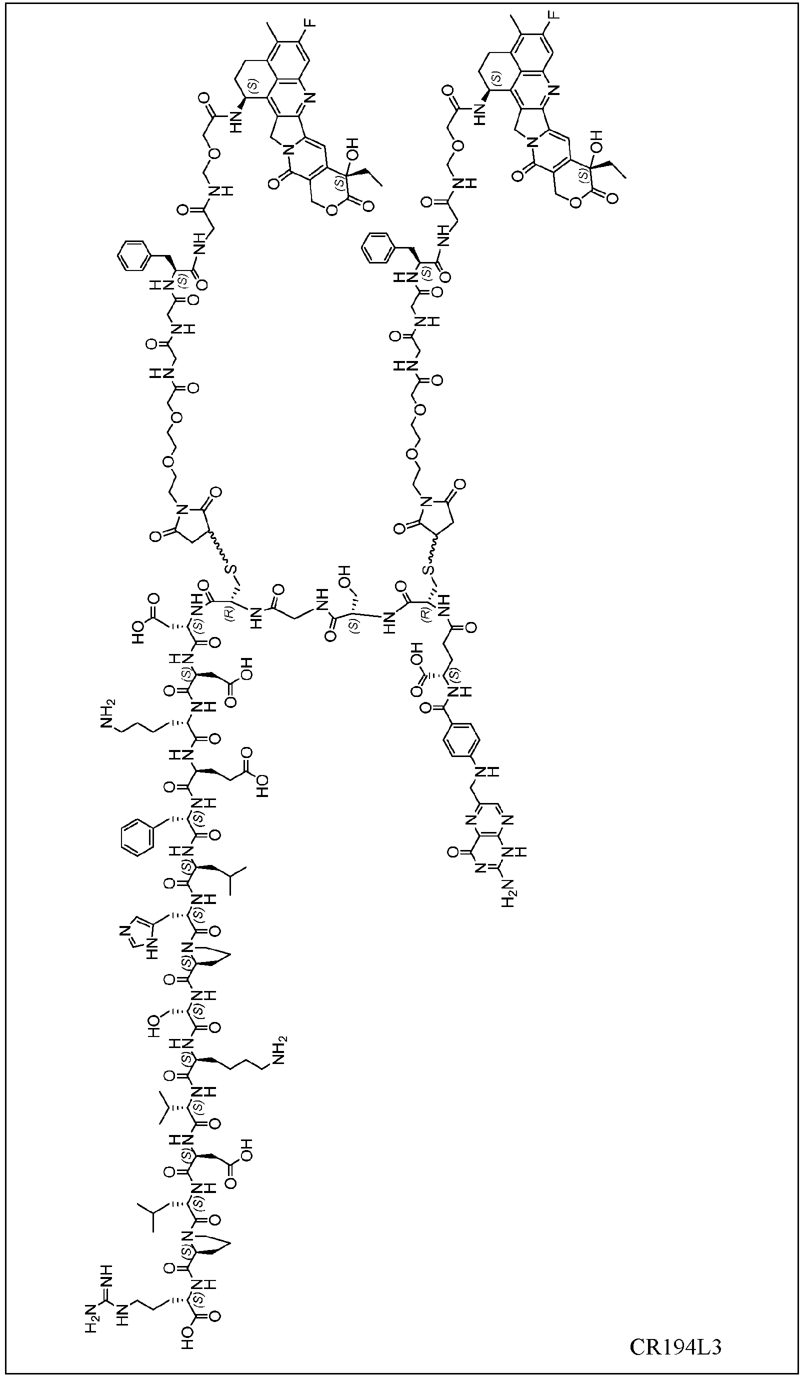

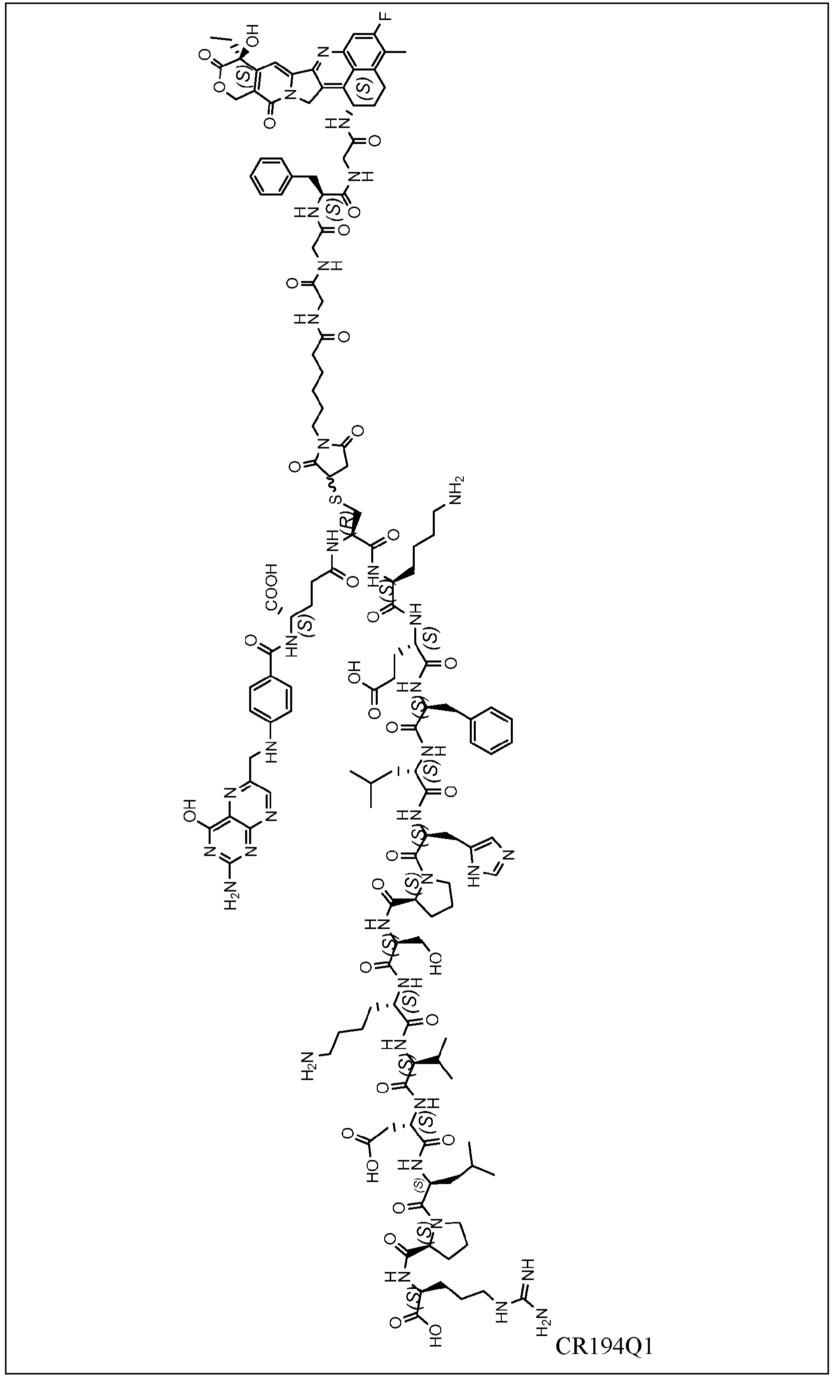

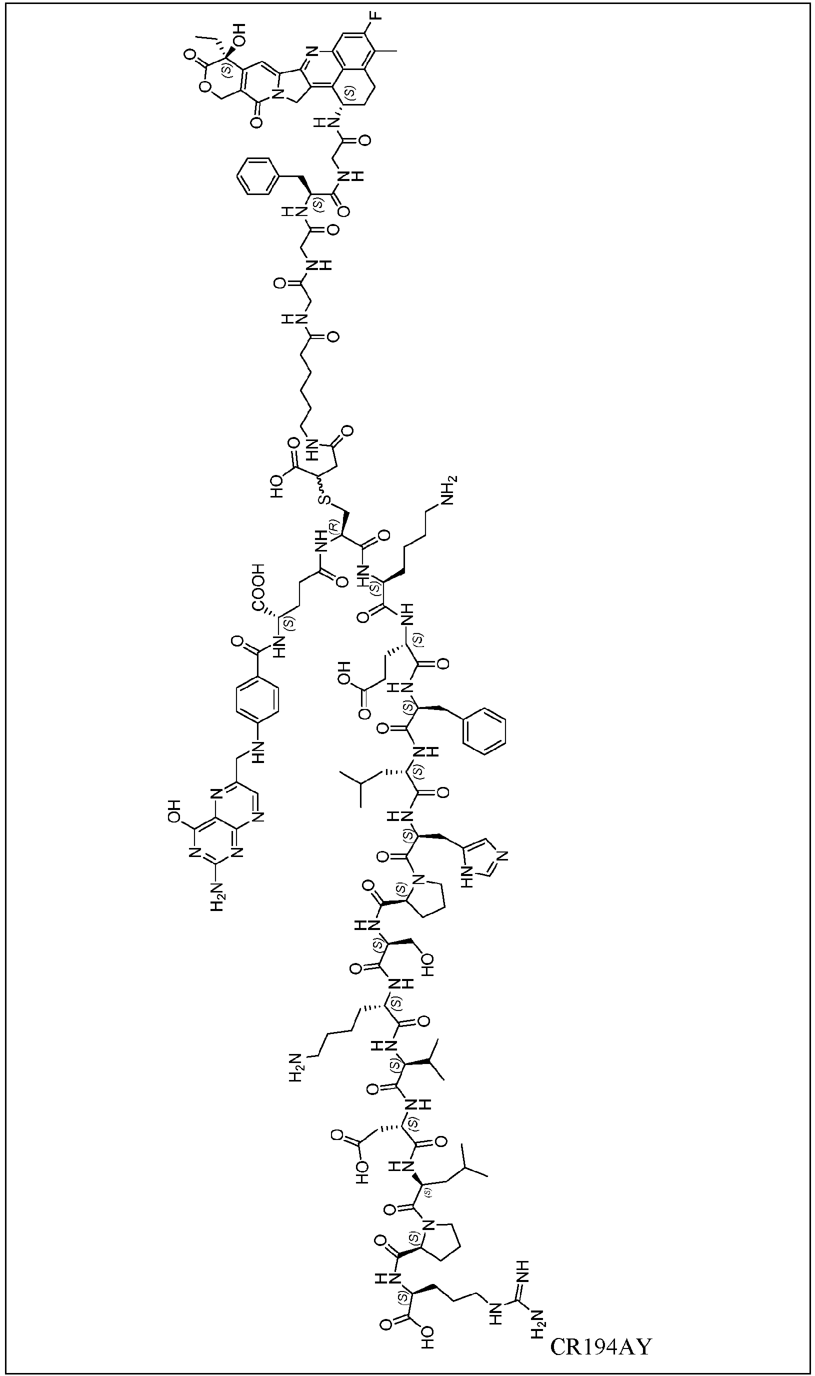

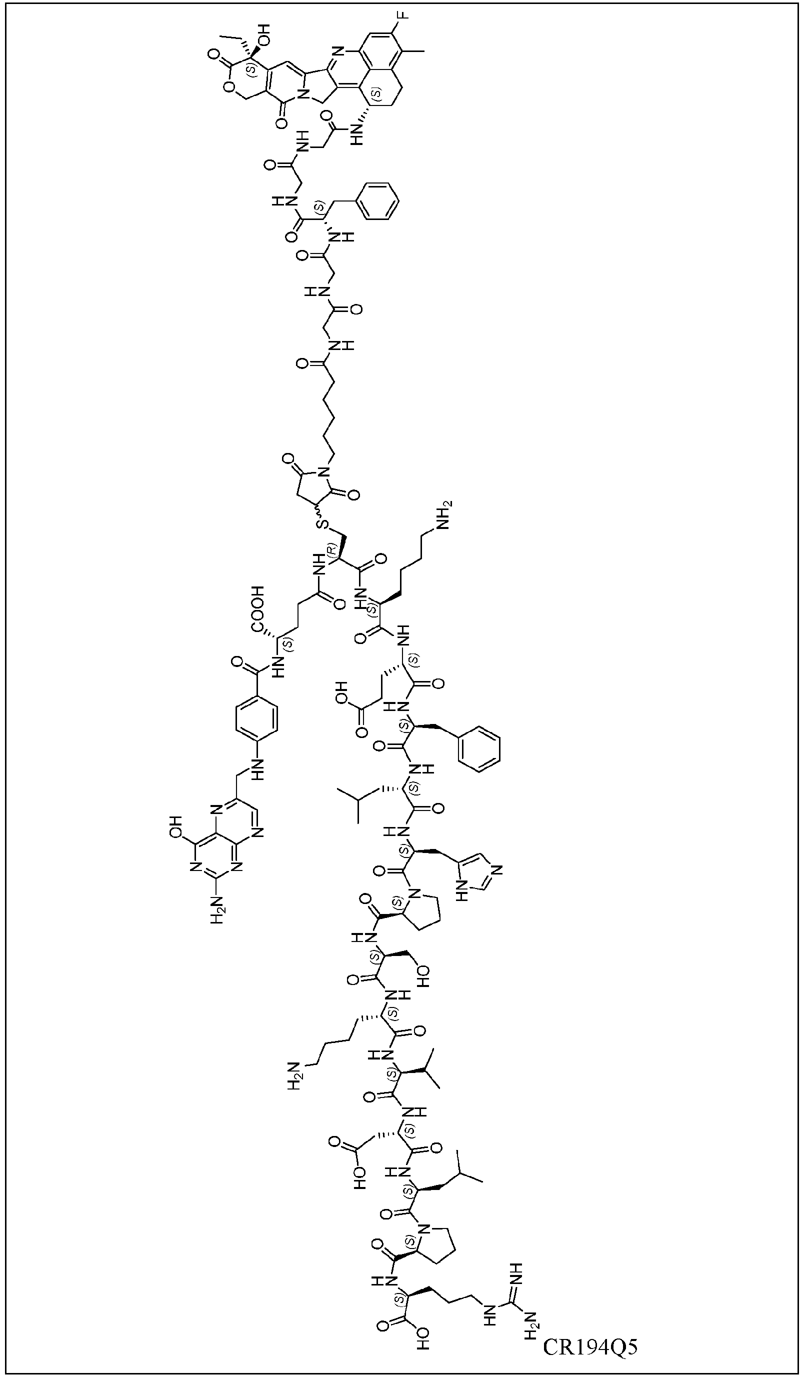

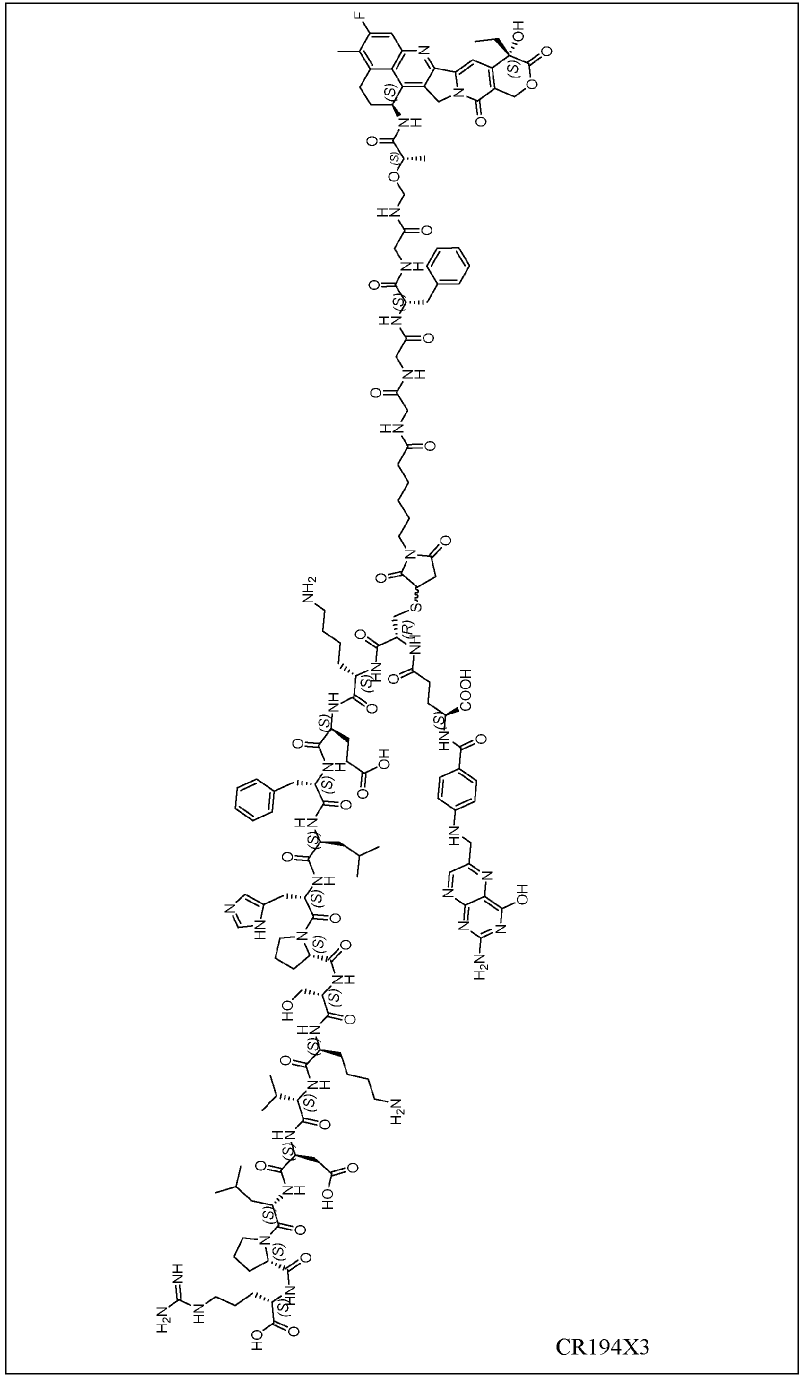

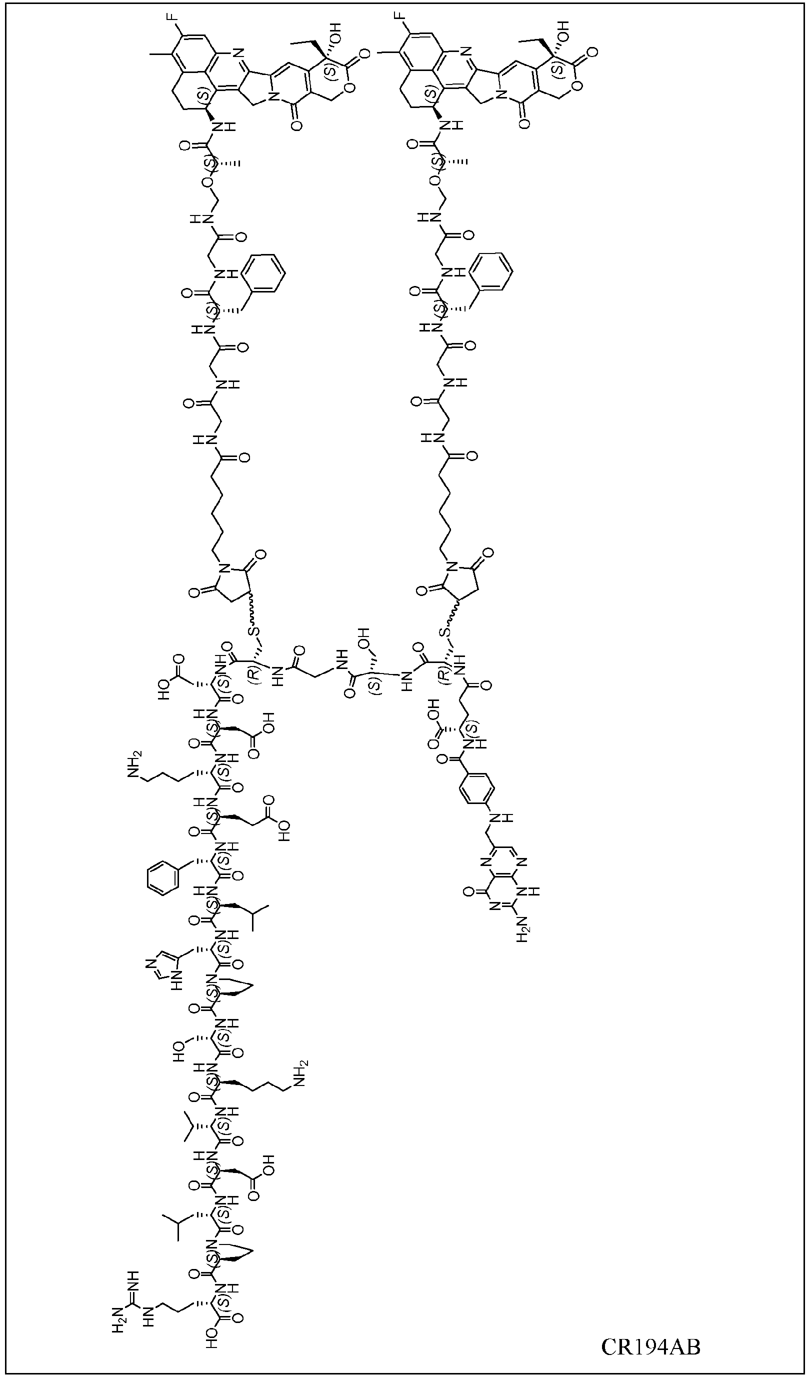

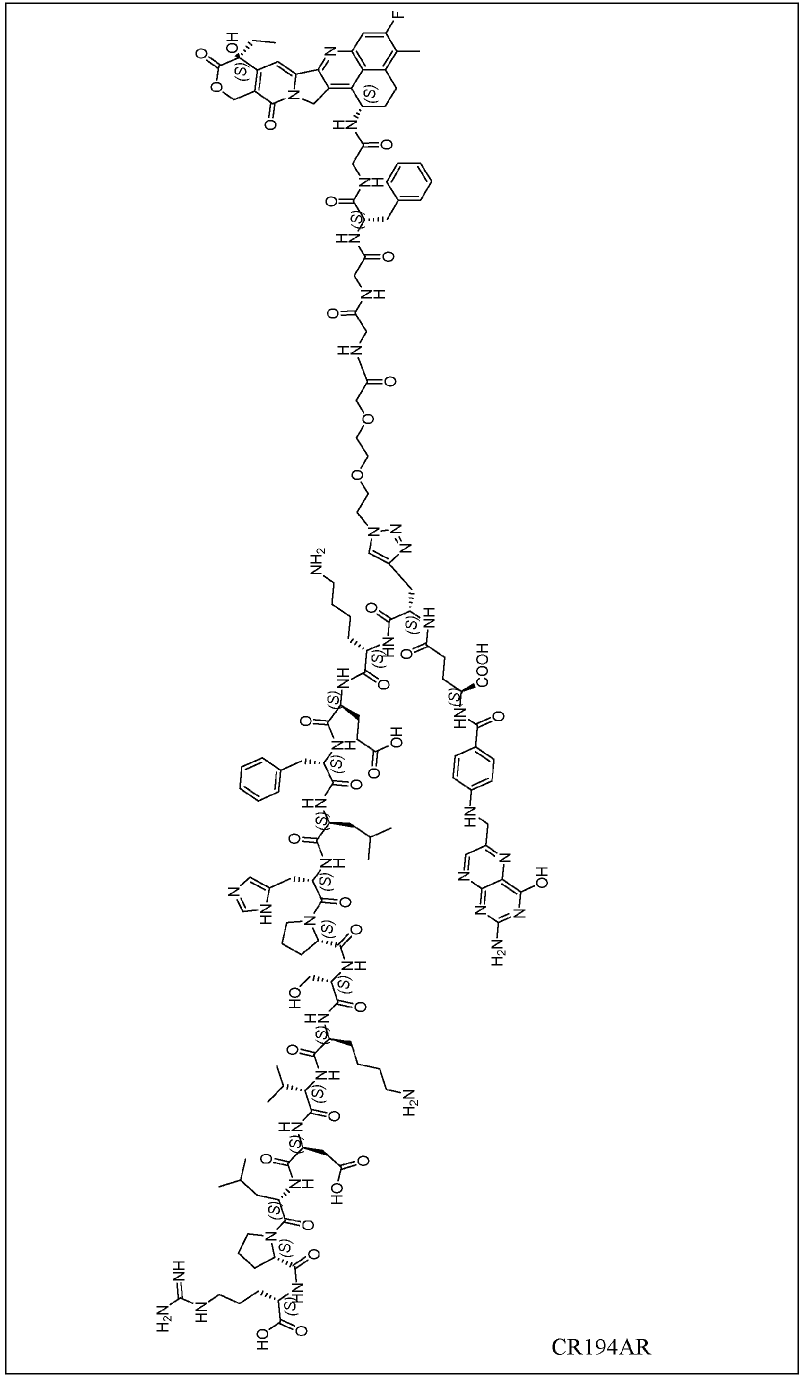

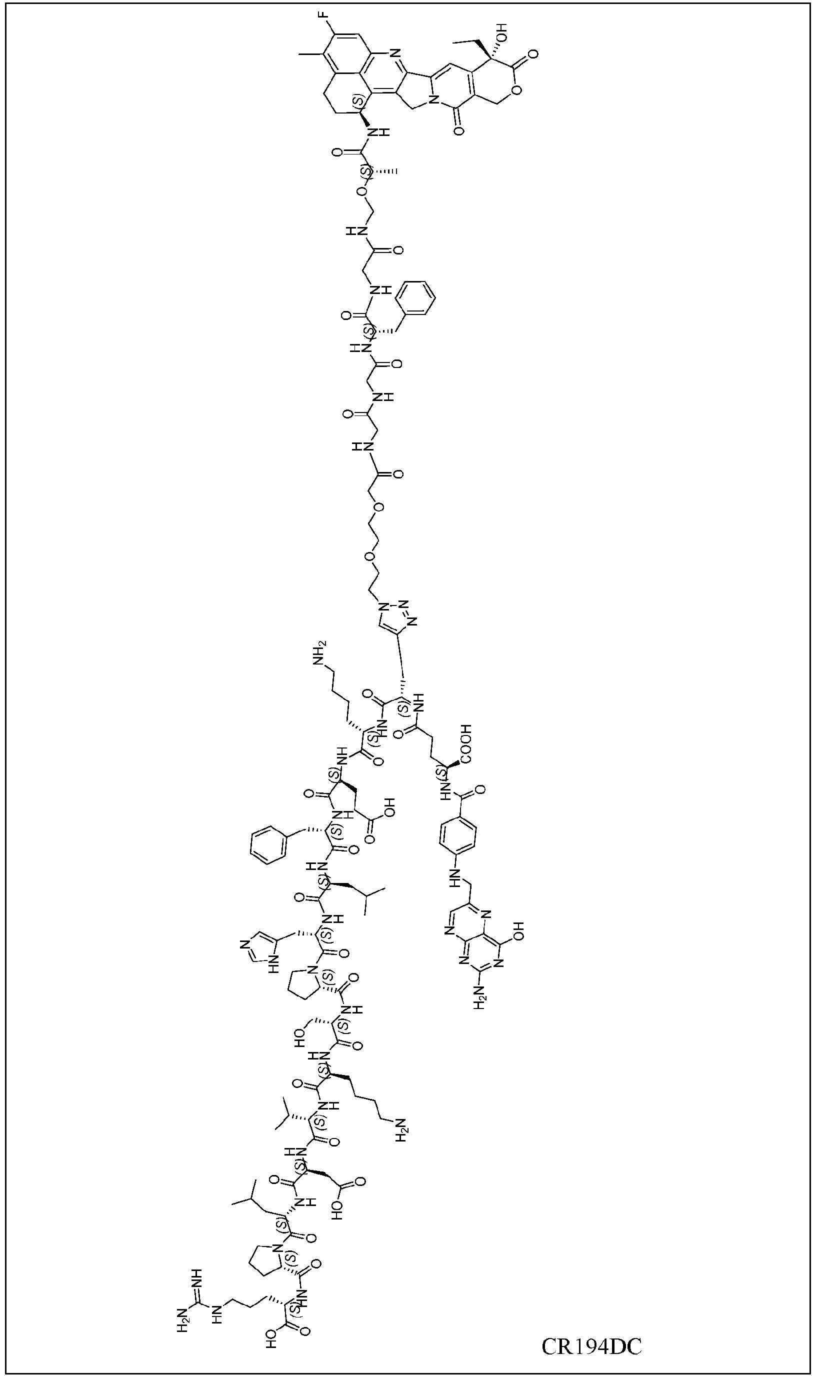

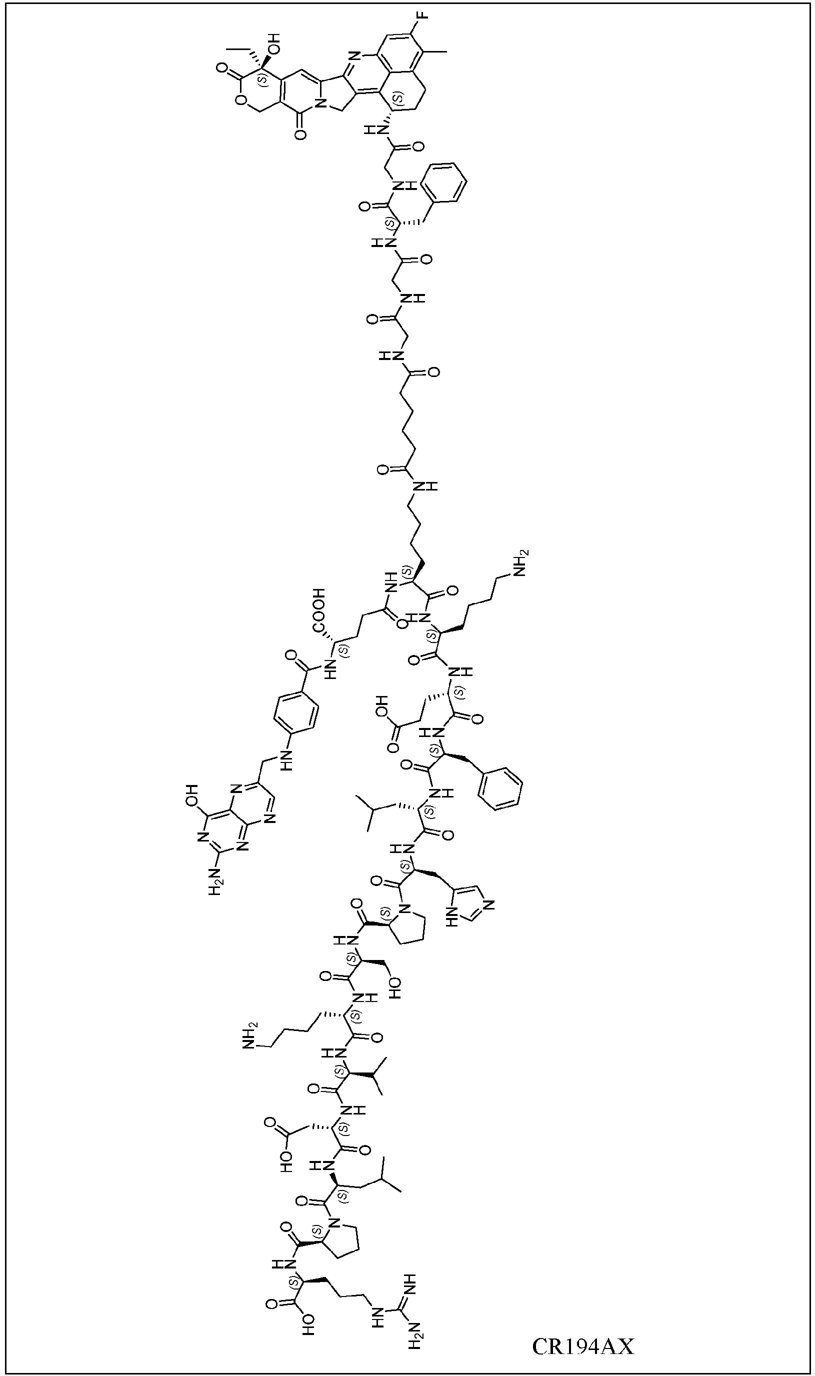

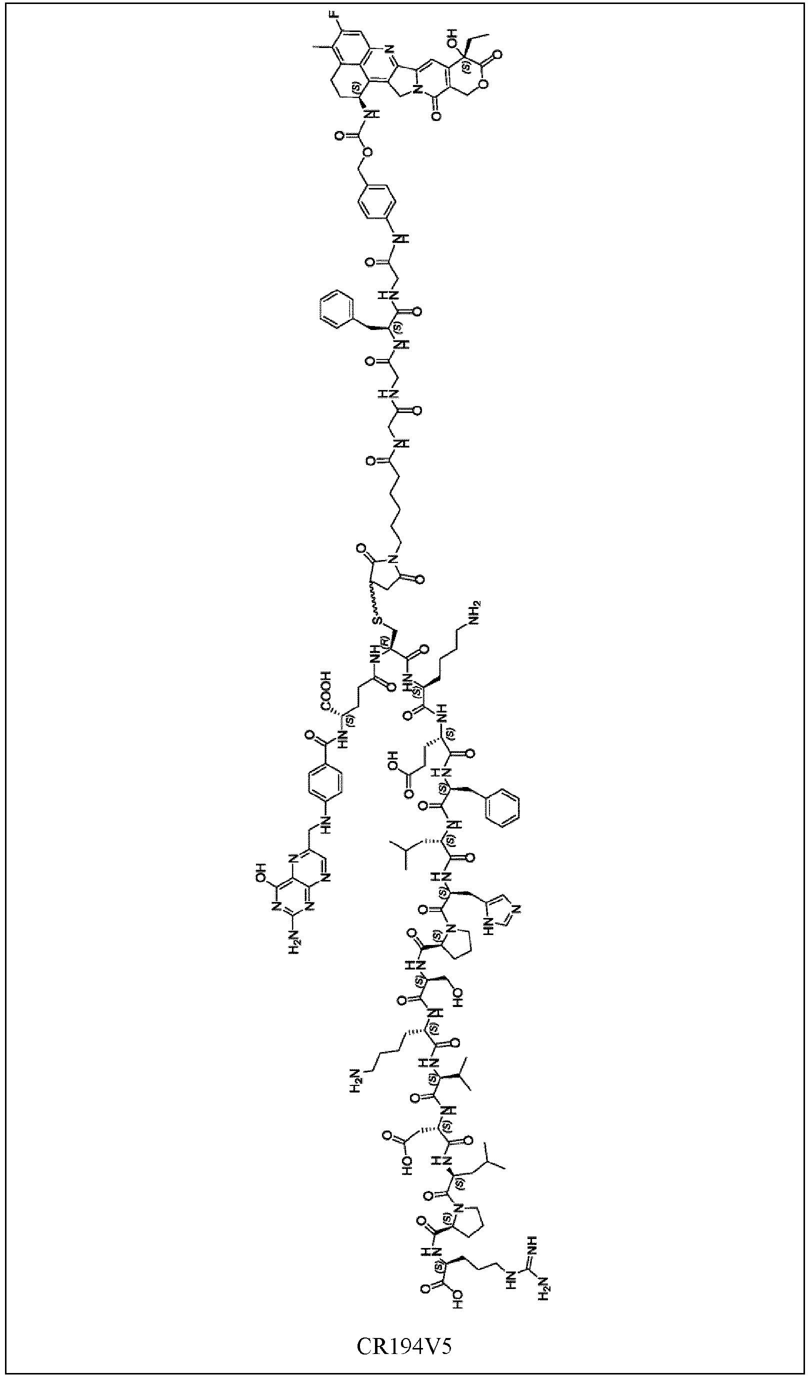

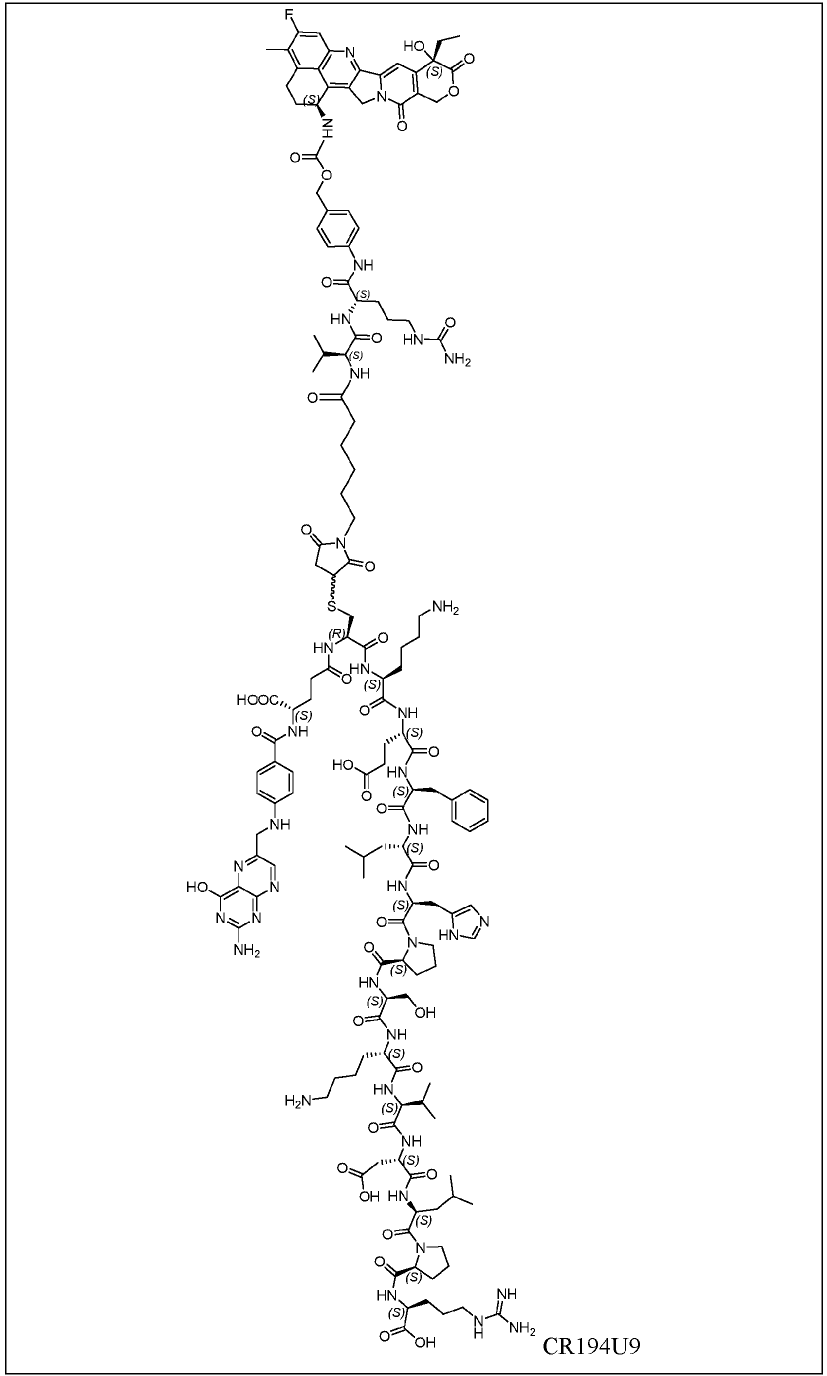

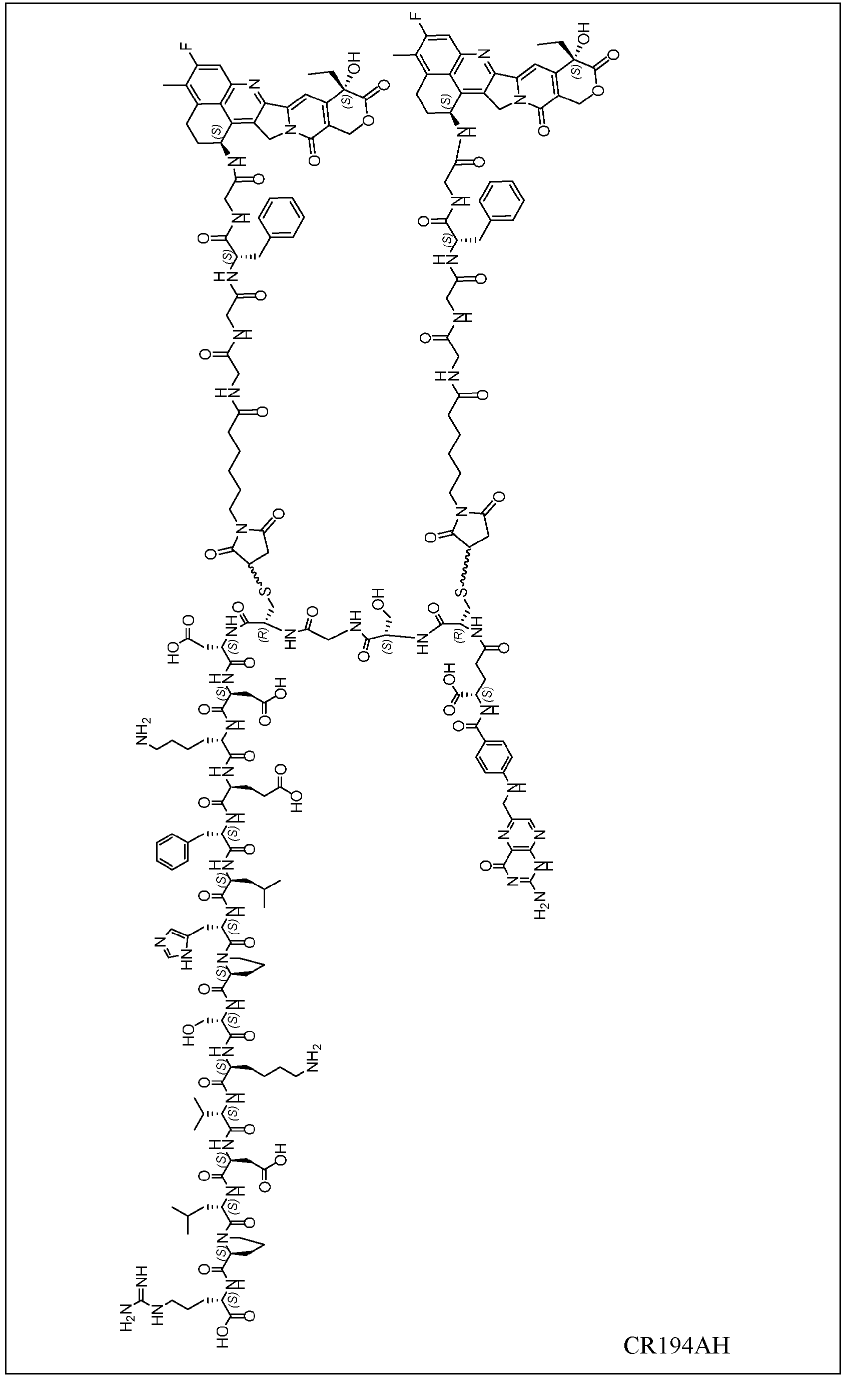

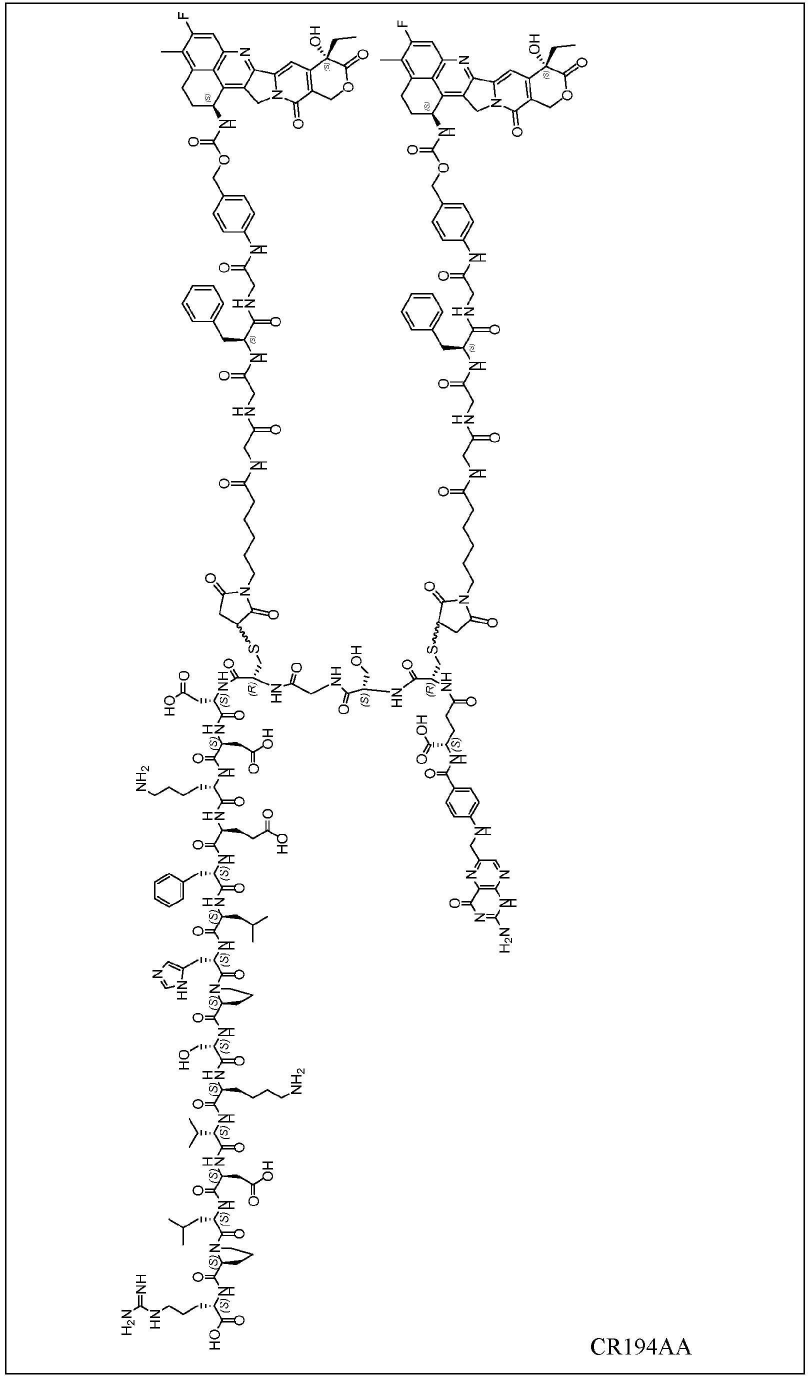

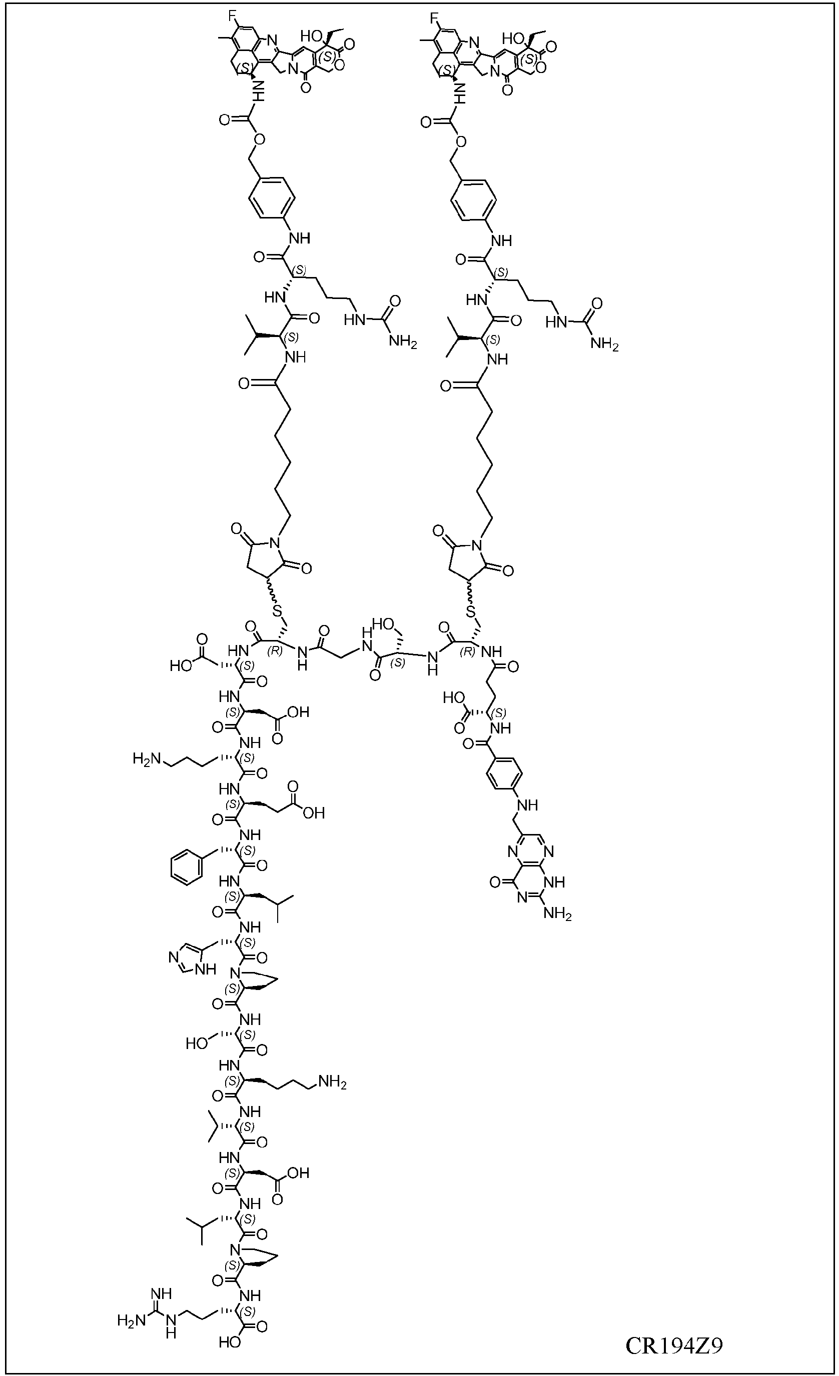

Another aspect of the present application discloses a pharmaceutical composition, which comprises the conjugate compound or the pharmaceutically acceptable salt thereof as described in the present application, and a pharmaceutically acceptable carrier. In some embodiments, the pharmaceutical composition as described in the present application is administered intravenously, subcutaneously, orally, intramuscularly or intraventricularly.

The term "pharmaceutically acceptable" as used herein means, within the scope of sound medical judgment, being suitable for contact with cells of human beings and other animals without undue toxicity, irritation, allergic response, etc., and being commensurate with a reasonable benefit/risk ratio.

The term "pharmaceutically acceptable salt" as used herein refers to a relatively non-toxic, inorganic and organic acid addition salt and base addition salt, of the conjugate compound of the present application. Representative acid addition salts include hydrobromides, hydrochlorides, sulfates, bisulfates, phosphates, nitrates, acetates, oxalates, valerates, oleates, palmitates, stearates, laurates, borates, benzoates, lactates, phosphates, tosylates, citrates, maleates, fumarates, succinates, tartrates, naphthylates, mesylates, glucoheptonates, lactiobionates, sulphamates, malonates, salicylates, propionates, methylene-bis-b-hydroxynaphthoates, gentisates, isethionates, di-p-toluoyltartrates, methanesulphonates, ethanesulphonates, benzenesulphonates, p-toluenesulphonates, cyclohexylsulphamates, quinateslaurylsulphonate salts, and the like. Base addition salts include pharmaceutically acceptable metal and amine salts. Suitable metal salts include sodium, potassium, calcium, barium, zinc, magnesium, and aluminum salts. In some embodiments, sodium and potassium salts are preferred. Suitable inorganic base addition salts are prepared from metal bases which include, for example, sodium hydride, sodium hydroxide, potassium hydroxide, calcium hydroxide, aluminum hydroxide, lithium hydroxide, magnesium hydroxide, and zinc hydroxide. Suitable amine base addition salts are prepared from amines which have sufficient basicity to form a stable salt, and preferably include the following amines which are frequently used in medicinal chemistry because of their low toxicity and acceptability for medical use: ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e.g., lysine and arginine, dicyclohexylamine, and the like.

The term "pharmaceutically acceptable carrier" as used herein refers to a pharmaceutically acceptable solvent, suspension or any other pharmacologically inert vehicle for delivering the conjugate compound provided in the present application to a subject, without interfering with the structures and properties of the conjugate compound. Such carriers enable the conjugate compound to be formulated as, for example, tablets, pills, capsules, liquids, gels, syrups, slurries, suspensions and pastilles, for oral ingestion by a subject. Such carriers enable the conjugate compound to be formulated as injections, infusions or preparations for local administration.

The pharmaceutically acceptable carriers for use in the pharmaceutical composition provided in the present application may include, but are not limited to, for example, pharmaceutically acceptable liquids, gels, or solid carriers, aqueous vehicles (such as sodium chloride injection, Ringer's injection, isotonic dextrose injection, sterile water injection, or dextrose and lactated Ringer's injection), nonaqueous vehicles (such as fixed oils derived from vegetables, cottonseed oil, corn oil, sesame oil, or peanut oil), antimicrobial agents, isotonic agents (such as sodium chloride or dextrose), buffers (such as phosphate or citrate buffers), antioxidants (such as sodium bisulfate), anesthetics (such as procaine hydrochloride), suspensions/dispersions (such as sodium carboxymethylcellulose, hydroxypropyl methylcellulose, or polyvinylpyrrolidone), chelating agents (such as EDTA (ethylenediamine tetraacetic acid) or EGTA (ethylene glycol tetraacetic acid)), emulsifying agents (such as polysorbate 80 (Tween-80)), diluents, adjuvants, excipients or non-toxic auxiliary substances, other components well known in the art, or various combinations thereof. Suitable components may include, for example, fillers, binders, buffers, preservatives, lubricants, flavoring agents, thickening agents, coloring agents, or emulsifying agents.

In some embodiments, the pharmaceutical composition is an injection preparation. The injection preparations include sterile water solutions or dispersions, suspensions or emulsions. In all cases, the injection preparations should be sterile and be a fluid for easy injection. It should be stable under the conditions of manufacture and storage and must be preserved against the contaminating action of microorganisms such as bacteria and fungi. The carriers can be solvents or dispersion mediums containing, for example, water, ethanol, polyol (for example, glycerol, propylene glycol, and liquid polyethylene glycol), and suitable mixtures thereof and/or vegetable oils. The injection preparations should maintain appropriate fluidity. The appropriate fluidity can be maintained, for example, by the use of coatings such as lecithin, by the use of surfactants, and the like. Prevention of the action of microorganisms can be achieved by various antibacterial and antifungal agents, for example, parabens, chlorobutanol, phenol, sorbic acid, thimerosal, and the like.

In some embodiments, the pharmaceutical composition is an oral preparation. The oral preparations include, but are not limited to, capsules, cachets, pills, tablets, lozenges (using a flavored basis, usually sucrose and acacia or tragacanth), powders, granules, or as solutions or suspensions in aqueous or non-aqueous liquids, or as oil-in-water or water-in-oil liquid emulsions, or as elixirs or syrups, or as pastilles (using an insert base, such as gelatin and glycerin, or sucrose and acacia) and/or as mouth washes.

In solid dosage forms for oral administration (e.g., capsules, tablets, pills, dragees, pulvis, granules, *etc*.), the conjugate compound is mixed with one or more pharmaceutically acceptable carriers, such as sodium citrate or dicalcium phosphate, and/or any of the followings: (1) fillers or extenders, such as starches, lactose, sucrose, glucose, mannitol, and/or silicic acid; (2) binders, such as carboxymethylcellulose, alginates, gelatins, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as acetyl alcohol and glycerol monostearate; (8) absorbents, such as kaolin and bentonite clay; (9) lubricants, such as talc, calcium stearate, magnesium stearate, solid polyethylene glycol, sodium lauryl sulfate, and mixtures thereof; and (10) coloring agents.

In liquid dosage forms for oral administration, the conjugate compound is mixed with any of the followings: pharmaceutically acceptable emulsions, microemulsions, solutions, suspensions, syrups and elixirs. In addition to the conjugate compound, the liquid dosage forms may contain inert diluents commonly used in the art, such as, water or other solvents, solubilizing agents and emulsifying agents, such as ethyl alcohol, isopropyl alcohol, ethyl carbonate, ethyl acetate, benzyl alcohol, benzyl benzoate, isopropanol, 1,3-butylene glycol, oils (in particular, cottonseed oil, peanut oil, corn oil, olive oil, castor oil and sesame oil), glycerol, tetrahydrofurfuryl alcohol, polyethylene glycol and fatty acid esters of sorbitan, and mixtures thereof. Besides inert diluents, an oral composition can also include adjuvants such as wetting agents, emulsifying agents and suspensions, sweetening agents, flavoring agents, coloring agents, perfuming agents and preservatives.

In some embodiments, the pharmaceutical composition is a mouth spray preparation or a nasal spray preparation. The spray preparations include, but are not limited to, aqueous aerosols, nonaqueous suspensions, lipidosome preparations or solid granular preparations. Aqueous aerosols are prepared by mixing aqueous solutions or suspensions of agents with conventional pharmaceutically acceptable carriers and stabilizers. The carriers and stabilizers vary according to the requirements of specific compounds, but in general, they include nonionic surfactants (Tweens or polyethylene glycol), oleic acid, lecithin, amino acids such as glycine, buffer solution, salts, sugar or sugar alcohol. Aerosols are generally prepared from isotonic solutions, and can be delivered by sprayers.

In some embodiments, the pharmaceutical composition can be used by mixing with one or more other drugs. In some embodiments, the pharmaceutical composition comprises at least one other drug. In some embodiments, the other drugs are antineoplastic drugs, cardiovascular drugs, anti-inflammatory drugs, antiviral drugs, digestive system drugs, nervous system drugs, respiratory system drugs, immune system drugs, dermatologic drugs, metabolic drugs, etc.

In some embodiments, the pharmaceutical compositions can be administered to a subject in need thereof by appropriate routes, including without limitation, oral, injection (such as intravenous, intramuscular, subcutaneous, intracutaneous, intracardiac, intrathecal, intrapleural and intraperitoneal injection), mucosal (such as nasal and intraoral administration), sublingual, rectal, percutaneous, intraocular, and pulmonary administration. In some embodiments, the pharmaceutical composition can be administered intravenously, subcutaneously, orally, intramuscularly or intraventricularly.

Due to the properties of some payloads, such as high toxicity and high hydrophilicity, it is desired to deliver the payloads more specifically and more efficiently to a subject in need thereof. For example, in cancer treatment, it is desired to specifically deliver chemotherapeutic agents to cancer cells without toxicity to normal cells. Therefore, in another aspect, the present application discloses a method for delivering a payload to a subject in need thereof, comprising administering to the subject a therapeutically effective amount of the conjugate compound or the pharmaceutically acceptable salt thereof provided in the present application, or the pharmaceutical composition provided in the present application.

Another aspect of the present application discloses a method for treating a disease in a subject, the method comprises administering to the subject a therapeutically effective amount of the conjugate compound or the pharmaceutically acceptable salt thereof as described in the present application, or the pharmaceutical composition of the present application. In some embodiments, the method further comprises administering one or more therapeutic agents in combination with the conjugate compound or the pharmaceutically acceptable salt thereof, or the pharmaceutical composition.

Another aspect of the present application discloses the use of the conjugate compound or the pharmaceutically acceptable salt thereof of the present application, or the pharmaceutical composition as described in the present application in the preparation of a drug for treating a disease in a subject.

The term "therapeutically effective amount" as used herein refers to an amount of the conjugate compound or the pharmaceutically acceptable salt thereof, or the pharmaceutical composition which relieves to some extent one or more symptoms of a disease or disorder in a subject, returns to normal either partially or completely one or more physiological or biochemical parameters associated with or causative of the disease or disorder, and/or reduces the likelihood of the onset of the disease or disorder. Such amounts generally vary according to a number of factors which can be determined and explained, according to the scope of the specification provided in the present application, by those of ordinary skill in the art. These factors include, without limitation: the particular subject and the age, weight, height, general physical condition, and medical history thereof, the particular compound used, the carrier of the preparation and the administration route selected; and the nature and severity of the condition being treated.

In some embodiments, the amount of the conjugate compound, or the pharmaceutically acceptable salt thereof, or the pharmaceutical composition is sufficient to inhibit a disease or disorder in a subject, or prophylactically inhibit or prevent the onset of a disease or disorder in a subject. Although the therapeutically effective amount may vary in different subjects, it is generally ranged from 0.01 to 150 mg/kg, for example 0.01 to 140 mg/kg, 0.01 to 130 mg/kg, 0.01 to 120 mg/kg, 0.01 to 110 mg/kg, 0.01 to 100 mg/kg, 0.01 to 90 mg/kg, 0.01 to 80 mg/kg, 0.01 to 70 mg/kg, 0.01 to 60 mg/kg, 0.01 to 50 mg/kg, 0.01 to 40 mg/kg, 0.01 to 30 mg/kg, 0.01 to 20 mg/kg, 0.01 to 10 mg/kg, 0.01 to 5 mg/kg, 0.01 to 4 mg/kg, 0.01 to 3 mg/kg, 0.01 to 2 mg/kg, 0.01 to 1 mg/kg, 0.01 to 0.1 mg/kg. In some embodiments, the conjugate compound or the pharmaceutically acceptable salt thereof or the pharmaceutical composition thereof of the present application is administered at a dose range of 1-150 mg/kg of the conjugate compound. In some embodiments, the conjugate compound or the pharmaceutically acceptable salt thereof or the pharmaceutical composition thereof of the present application is administered at a dose of 1-25 mg/kg, 1-50 mg/kg, 1-75 mg/kg, 1-100 mg/kg, 1-125 mg/kg, 1-150 mg/kg, 25-75 mg/kg, 25-50 mg/kg, 25-40 mg/kg, 25-30 mg/kg, 1-20 mg/kg, 1-15 mg/kg, 1-10 mg/kg, 1-5 mg/kg, or 1-3 mg/kg of the conjugate compound. In some embodiments, the conjugate compound or the pharmaceutically acceptable salt thereof or the pharmaceutical composition thereof of the present application is administered at a dose range of 1 mg/kg, 3 mg/kg, 10 mg/kg, 25 mg/kg, 50 mg/kg, 75 mg/kg, 100 mg/kg, or 150 mg/kg of the conjugate compound. In some embodiments, the conjugate compound or the pharmaceutically acceptable salt thereof or the pharmaceutical composition thereof of the present application can be administered every day (for example: once, twice, three times or four times a day), or at intervals of one or more days (for example, once a week, once every two weeks, once every three weeks, once a month or once a quarter). In some embodiments, the administration frequency of the conjugate compound or the pharmaceutically acceptable salt thereof or the pharmaceutical composition thereof of the present application is once a week, twice a week, three times a week, once every three days, once every two days, once a day, twice a day, or three times a day. In some embodiments, the administration frequency of the conjugate compound or the pharmaceutically acceptable salt thereof or the pharmaceutical composition thereof of the present application is once a week. In some embodiments, the administration frequency of the conjugate compound or the pharmaceutically acceptable salt thereof or the pharmaceutical composition thereof of the present application is three times a day. The therapeutically effective amount as described in the present application can be equal to any value within the above numerical range, including the end values of this range.

The term "object" as used herein refers to human and non-human animals. The non-human animal includes all vertebrates, such as a mammal and a non-mammal. The subject may also be a livestock animal such as cattle, swine, sheep, poultry and horse, or a domestic animal such as dog and cat. The subject may be male (e.g., man) or female (e.g., woman), may be elderly, and may be an adult, adolescent, child, or infant. A human subject may be Caucasian, African, Asian, Semitic, or human with other racial backgrounds or a mixture of such racial backgrounds.

In some embodiments, the disease of the present application is selected from the group of: a cancer, an immune disease, a metabolic disease, and a neurological disease. In some embodiments, the cancer is selected from pancreatic cancer, biliary tract cancer, liver cancer, breast cancer, thyroid cancer, colorectal cancer, esophagus cancer, lung cancer, kidney cancer, leukemia, ovarian cancer, stomach cancer, uterine cancer, endometrial cancer, colon cancer, testicular cancer, skin cancer, prostate cancer, lymphoma, and multiple myeloma. In some embodiments, the immune disease is an autoimmune disease, preferably, the autoimmune disease is selected from a connective tissue disease, systemic sclerosis, rheumatoid arthritis, and systemic lupus erythematosus. The metabolic disease is selected from diabetes, gout, obesity, hypoglycemia, hyperglycemia, and dyslipidemia. In some embodiments, the neurological disease is selected from Alzheimer's disease, Parkinson's disease, Huntington's disease, head injury, multiple sclerosis, vertigo, coma, and epilepsy.

In some embodiments, the method of the present application further comprises administering one or more therapeutic agents in combination with the conjugate compound or the pharmaceutically acceptable salt thereof, or the pharmaceutical composition.

The present application will be described in greater detail by way of specific examples. The following examples are offered for illustrative purposes only, and are not intended to limit the invention in any manner. A person skilled in the art will readily recognize a variety of noncritical parameters which can be changed or modified to yield essentially the same results.

### Example

The following examples are intended to better illustrate the present invention, and should not be construed as a limitation to the scope of the present invention. All the following specific compositions, materials and methods, in whole or in part, are within the scope of the present invention. The particular compositions, materials and methods are not intended to limit the present invention but merely to illustrate that the particular embodiment is within the scope of the present invention. Those skilled in the art can develop equivalent compositions, materials and methods without adding inventive step or departing from the scope of the present invention. It should be understood that various modifications to the methods of the present invention can still be included within the scope of the present invention. The inventor intends to include such modifications in the scope of the present invention.

### Example 1 Preparation of Multi-Ligand Moiety

### Preparation of double ligand Ligand-01

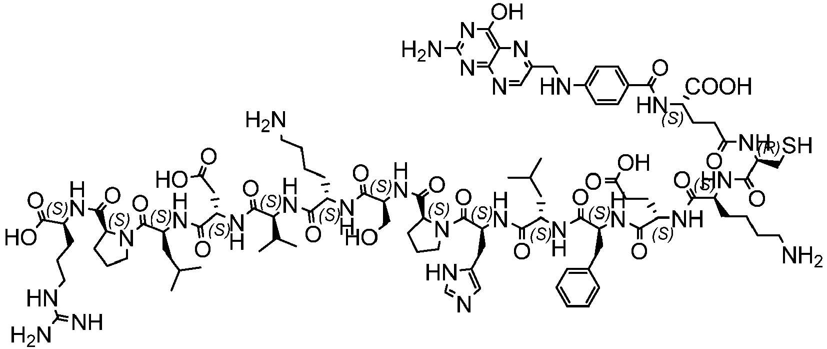

The peptide sequence of Pteroic acid (pteroic acid)-Glu-Cys-Lys-Glu-Phe-Leu-His-Pro-Ser-Lys-Val-Asp-Leu-Pro-Arg-OH was synthesized by solid-phase synthesis using Fmoc chemical amino resins. The resin was loaded in a solid-phase reaction column, DMF was added, nitrogen was bubbled into the solvent, and the resin was swelled for 30 min. Fmoc-Arg-OH, DCC and DMAP were added, reacted at 25°C for 3 h, then capped with acetic anhydride and pyridine for 1 h, and washed with DMF for 3 times. The Fmoc protecting group on the resin was removed with DBLK, and washing was performed with DMF for 5 times. Fmoc-Pro-OH and HOBt were weighed and dissolved in DMF. DIC was added to the above solution under an ice-water bath at 0°C and mixed for activation for 5 minutes. The solution was added to the above reaction column and reacted for 3 h, then, the solvent was drained, and the resin in the reaction column was washed 3 times. Then DBLK was used to remove the Fmoc protecting group. The above-mentioned operations were repeated, and Fmoc-Leu-OH, Fmoc-Asp(OtBu)-OH, Fmoc-Val-OH, Fmoc-Lys(Boc)OH, Fmoc-Ser(tBu)-OH, Fmoc-Pro-OH, Fmoc-His(Trt)-OH, Fmoc-Leu-OH, Fmoc-Phe-OH, Fmoc-Glu(OtBu)-OH, Fmoc-Lys(Boc)-OH, Fmoc-Cys(Trt)-OH, Fmoc-Glu-OtBu and pteroic acid were conjugated successively according to the structures. The resin was lysed with a lysis buffer and then filtered, and the lysate was poured into MTBE to precipitate a solid, which was washed with MTBE to obtain a crude product, which was purified by Pre-HPLC to prepare Ligand01.

Similarly, Ligand02, Ligand03, Ligand04, Ligand05 and Ligand06 can be obtained through steps similar to the above method.

### Example 2 Preparation of Conjugate Compounds

### 1. Synthesis of conjugate compound CR194AR

### Step 1: Preparation of CR194AQ

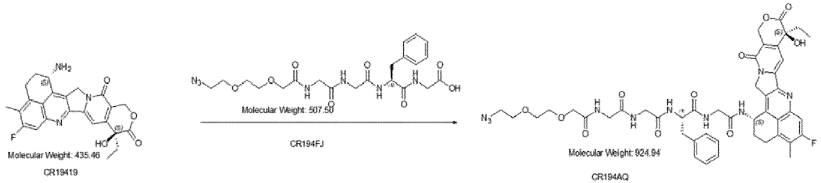

CR19419 (1.0 eq, see the diagram for the structure) was weighed into a 50 mL single-necked bottle and dissolved by adding DMF, then CR194FJ (1.0 eq, see the diagram for the structure), HATU (1.1 eq) and DIPEA (2.5 eq) were added and stirred for 4 h at 25°C. After the reaction was completed, the reaction solution was poured into an aqueous acetic acid solution, extracted three times with ethyl acetate, and the organic phase was washed with water and saturated brine, and dried to obtain CR194AQ (see the diagram for the structure).

### Step 2: Preparation of CR194AR

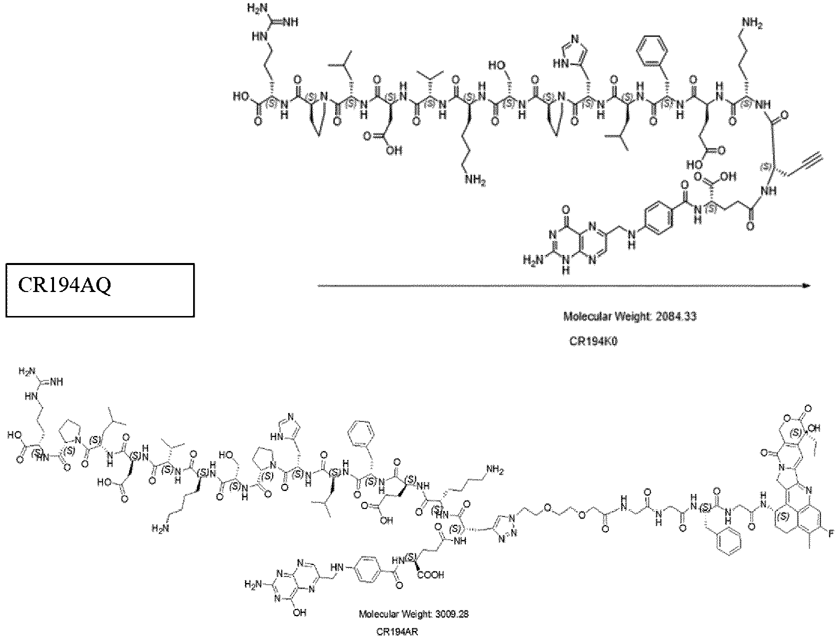

CR194AQ (1.0 eq) was weighed into a 50 mL single-necked bottle and dissolved by adding a mixed solution of DMF : water = 1 : 1, then CR194K0 (1.2 eq, see the diagram for the structure) and CuI (1.0 eq) were added and stirred for 4 h at 25°C. After the reaction was completed, the reaction solution was subjected to preparative purification to prepare a pure product which was lyophilized to obtain CR194AR.

### 2. Synthesis of conjugate compound CR194X3

### Step 1: Preparation of CR194X0

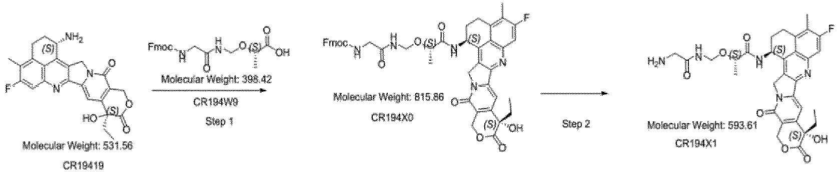

CR19419 (1.0 eq, see the diagram for the structure) was weighed into a 50 mL single-necked bottle and dissolved by adding DMF, then CR194W9 (1.0 eq, see the diagram for the structure), HATU (1.1 eq) and DIPEA (2.5 eq) were added and stirred for 4 h at 25°C. After the reaction was completed, the reaction solution was poured into an aqueous acetic acid solution; a solid was precipitated and filtered, and the filter cake was washed with an aqueous acetic acid solution and dried to obtain CR194X0 (see the diagram for the structure).

### Step 2: Preparation of CR194X1

CR194X0 (1.0 eq, see the diagram for the structure) was weighed into a 50 mL single-necked bottle, dissolved by adding DMF, and cooled down under an ice bath, and then DBU (1.1 eq) was added and reacted under an ice bath for 3 h. After the reaction was completed, the reaction solution was poured into MTBE; a solid was precipitated and filtered, and the filter cake was washed with MTBE for three times, and dried under vacuum to obtain CR194X1 (see the diagram for the structure).

### Step 3: Preparation of CR194X2

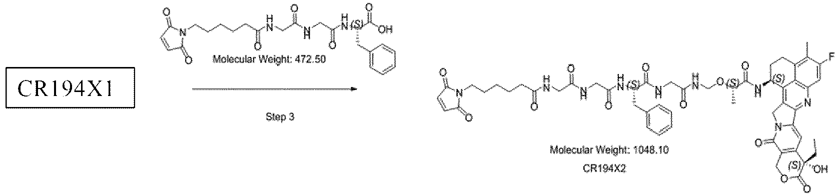

CR194X1 (1.0 eq) was weighed into a 50 mL single-necked bottle and dissolved by adding DMF, then CR19424 (1.0 eq, see the diagram for the structure), HATU (1.1 eq) and DIPEA (2.5 eq) were added and stirred for 4 h at 25°C. After the reaction was completed, the reaction solution was poured into an aqueous acetic acid solution, extracted three times with ethyl acetate, and the organic phase was washed with water and saturated brine, and dried to obtain CR194X2 (see the diagram for the structure).

### Step 4: Preparation of CR194X3

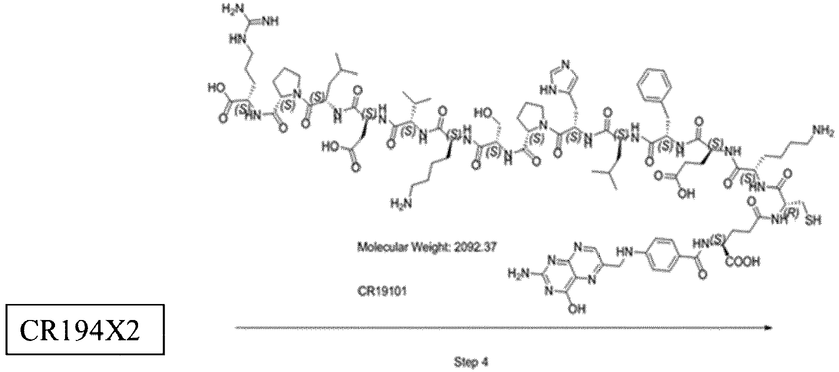

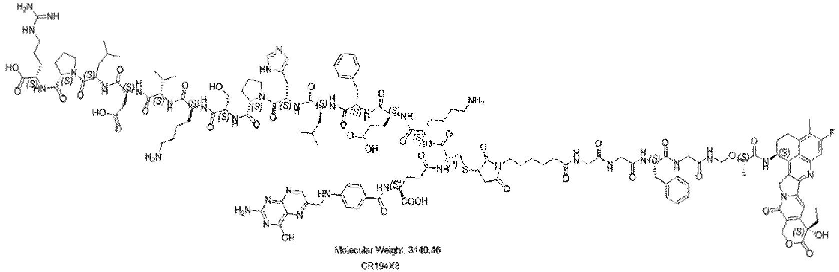

CR194X2 (1.0 eq) was weighed into a 50 mL single-necked bottle and dissolved by adding methanol, then 10 mM PBS buffer was added and stirred under an ice bath cooling for 10 min to obtain a white suspension, pH = 8-9. 0.1 M NaH₂PO₄ was added to adjust the pH to pH = 7. CR19101 (see the diagram for the structure) was weighed and added in batches, and the pH was controlled to equal to 7. The reaction was stirred under an ice bath for 15 min. HPLC controlled the complete reaction of raw materials, and the reaction solution was subjected to preparative purification and lyophilization to obtain CR194X3 (see the diagram for the structure).

### 3. Synthesis of conjugate compound CR194AX

### Step 1: Preparation of CR194CZ

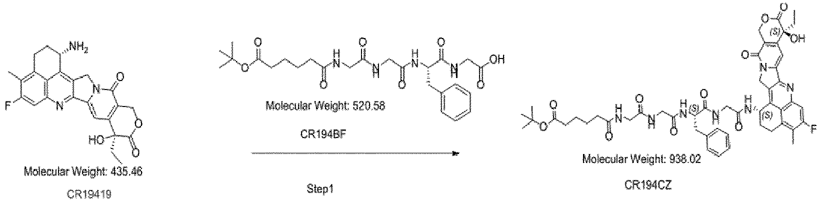

CR19419 (1.0 eq) was weighed into a 50 mL single-necked bottle and dissolved by adding DMF, then CR194BF (1.0 eq), HATU (1.1 eq) and DIPEA (2.5 eq) were added and stirred for 4 h at 25°C. After the reaction was completed, the reaction solution was poured into an aqueous acetic acid solution, extracted three times with ethyl acetate, and the organic phase was washed with water and saturated brine, and dried to obtain CR194CZ.

### Step 2: Preparation of CR194AW

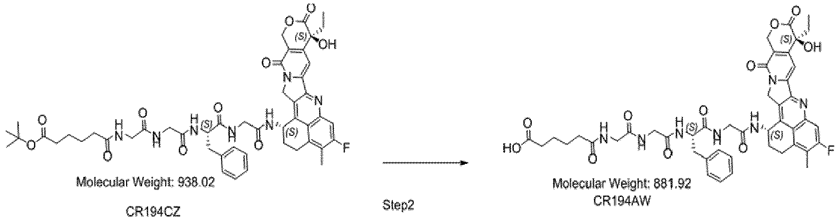

CR194CZ (1.0 eq) was weighed into a 50 mL single-necked bottle, and TFA : DCM = 1 : 1 solution was added, and reacted for 5 h. After the reaction was completed, the reaction solution was poured into MTBE; a solid was precipitated and filtered, and the filter cake was washed with MTBE for three times, and dried under vacuum to obtain CR194AW.

### Step 3: Preparation of CR194AX

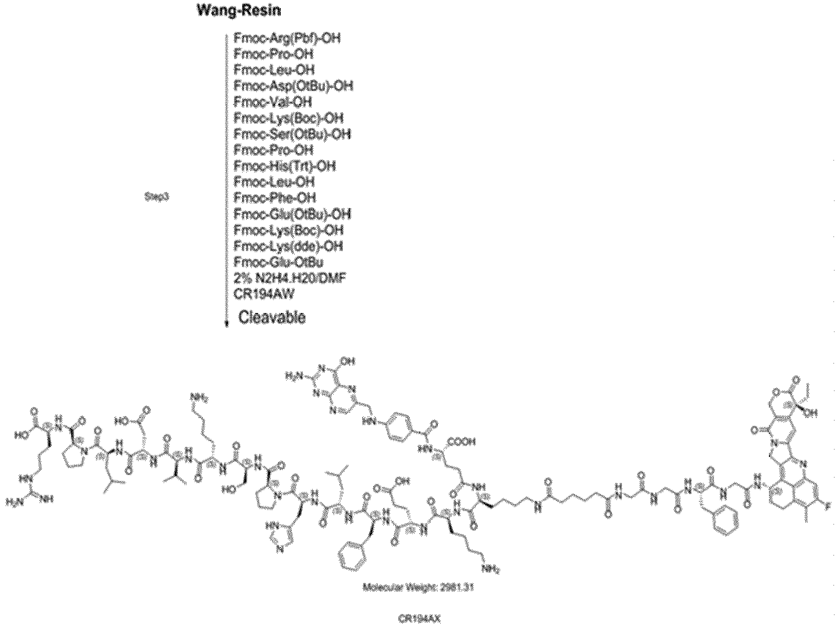

The synthesis of CR194AX was realized by solid-phase synthesis using Fmoc chemical amino resin. The resin was loaded in a solid-phase reaction column, DMF was added, nitrogen was bubbled into the solvent, and the resin was swelled for 30 min. Fmoc-Arg-OH, DCC and DMAP were added, reacted at 25°C for 3 h, then capped with acetic anhydride and pyridine for 1 h, and washed with DMF for 3 times. The Fmoc protecting group on the resin was removed with DBLK, and washing was performed with DMF for 5 times. Fmoc-Pro-OH and HOBt were weighed and dissolved in DMF. DIC was added to the above solution under an ice-water bath at 0°C and mixed for activation for 5 minutes. The solution was added to the above reaction column and reacted for 3 h, then, the solvent was drained, and the resin in the reaction column was washed 3 times. Then DBLK was used to remove the Fmoc protecting group. The above-mentioned operations were repeated, and Fmoc-Leu-OH, Fmoc-Asp(OtBu)-OH, Fmoc-Val-OH, Fmoc-Lys(Boc)OH, Fmoc-Ser(tBu)-OH, Fmoc-Pro-OH, Fmoc-His(Trt)-OH, Fmoc-Leu-OH, Fmoc-Phe-OH, Fmoc-Glu(OtBu)-OH, Fmoc-Lys(dde)-OH, Fmoc-Cys(Trt)-OH, Fmoc-Glu-OtBu and pteroic acid were conjugated successively according to the structures. 4% hydrazine hydrate/DMF solution was used to remove the dde protecting group, and washing was performed with DMF for 5 times. CR194AW, HBTU, and DIEA were added and reacted for 3 h. After the reaction was completed, the reaction solution was washed with DMF, DCM, and MeOH. The resin was blown dry with nitrogen. The resin was lysed with a lysis buffer and then filtered, and the lysate was poured into MTBE to precipitate a solid, which was washed with MTBE to obtain a crude product, which was subjected to Pre-HPLC to prepare CR194AX.

### 4. Synthesis of conjugate compound CR194AB

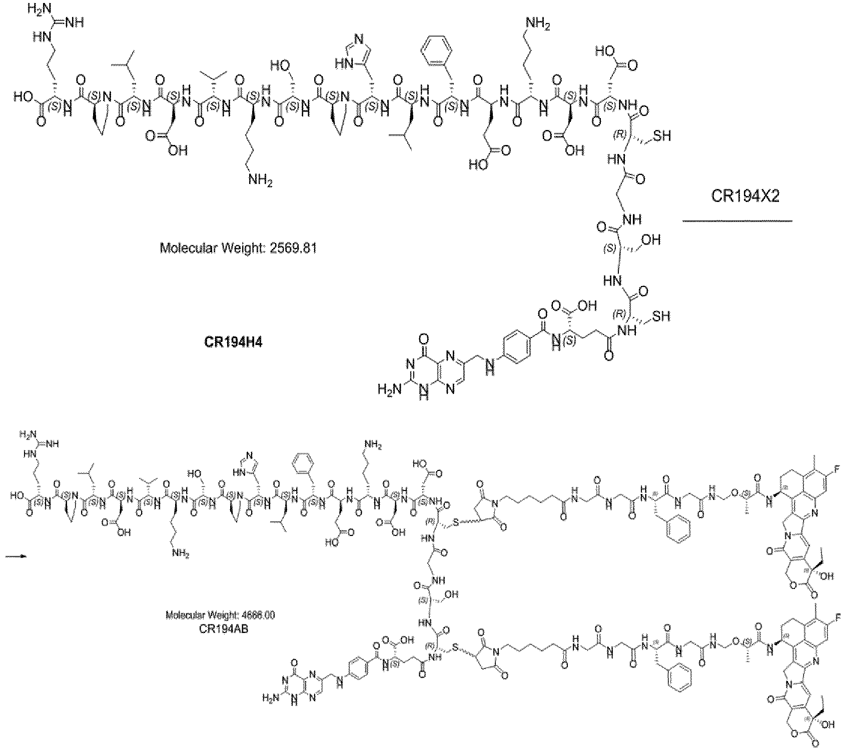

CR194X2 (1.0 eq) was weighed into a 50 mL single-necked bottle and dissolved by adding methanol, then 10 mM PBS buffer was added and stirred under an ice bath cooling for 10 min to obtain a white suspension, pH = 8-9. 0.1 M NaH₂PO₄ was added to adjust the pH to pH = 7. CR194H4 (see the diagram for the structure) was weighed and added, and the pH was controlled to equal to 7. The reaction was stirred under an ice bath for 15 min. HPLC controlled the complete reaction of raw materials, and the reaction solution was subjected to preparative purification and lyophilization to obtain CR194AB (see the diagram for the structure).

### 5. Synthesis of conjugate compound CR194A2

### Step 1: Preparation of CR19498

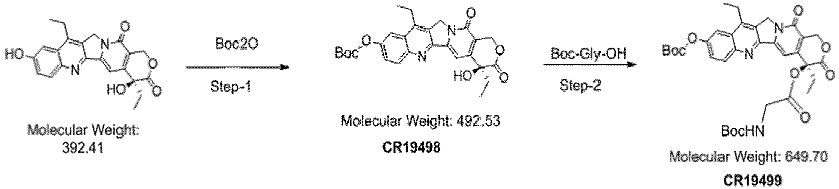

SN38 (1.0 eq) was weighed into a 50 mL single-necked flask and dissolved by adding DMF, then (Boc)₂O (1.2 eq) and DIPEA (1.5 eq) were added and stirred overnight at room temperature. TLC controlled the complete reaction of raw materials, the reaction solution was poured into water, extracted with EtOAc, and the organic phase was dried over anhydrous sodium sulfate, and rotary evaporated under reduced pressure to obtain CR19498 (see the diagram for the structure).

### Step 2: Preparation of CR19499

CR19498 (1.0 eq) and Boc-Gly-OH (1.1 eq) were weighed into a 50 mL single-necked flask, dissolved by adding DMF, and cooled under an ice bath, then HATU (1.1 eq), DMAP (0.1 eq) and DIPEA (2 eq) were added. Then the ice bath was removed and the stirring was performed at room temperature for 1 h. The reaction solution was quenched by adding water, extracted with EtOAc, and the organic phase was dried over anhydrous sodium sulfate, and rotary evaporated under reduced pressure to obtain CR19499 (see the diagram for the structure).

### Step 3: Preparation of CR194A0

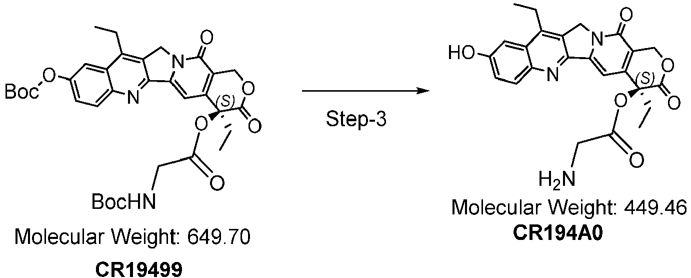

CR19499 (1.0 eq) was weighed and dissolved in 30% TFA in dichloromethane, stirred at room temperature for 1 h. The reaction solution was rotary evaporated under reduced pressure to remove DCM and TFA, then MTBE was added to precipitate a solid, and CR194A0 was obtained by suction filtration (see the diagram for the structure).

### Step 4: Preparation of CR194A1

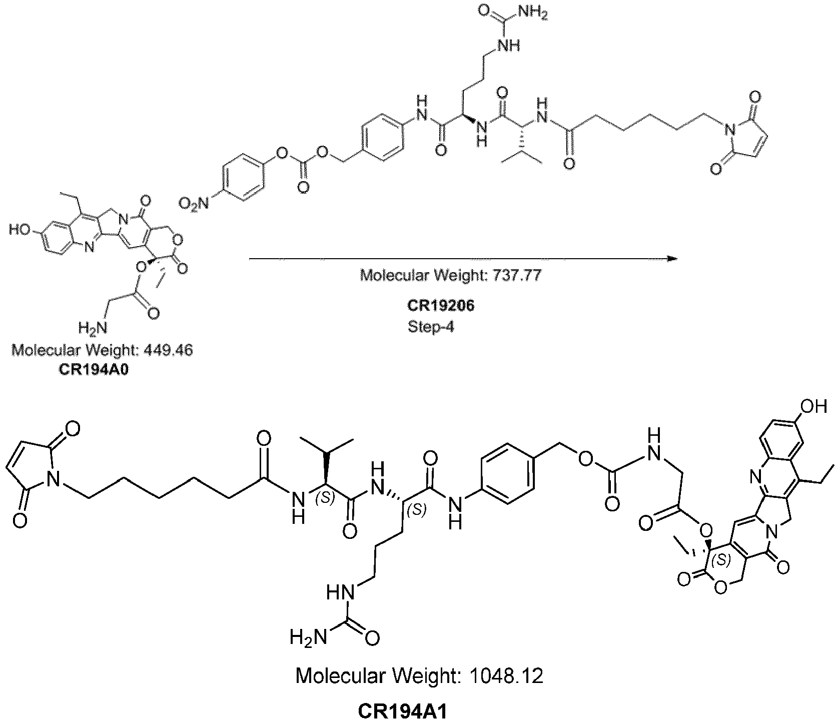

CR194A0 (1.0 eq), CR19206 (1.05 eq), and HOBT (0.1 eq) were weighed into a 50 mL single-necked flask and dissolved by adding DMF, then DIPEA (2 eq) was added and stirred at room temperature overnight. The reaction solution was subjected to preparative purification and lyophilization to obtain CR194A1 (see the diagram for the structure).

### Step 5: Preparation of CR194A2

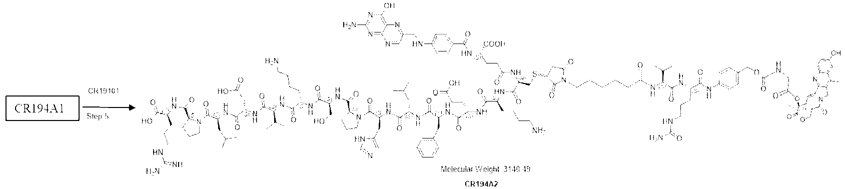

CR194A1 (1.0 eq) was weighed into a 50 mL single-necked bottle and dissolved by adding methanol, then 10 mM PBS buffer was added and stirred under an ice bath cooling for 10 min, pH = 8-9. 0.1 M NaH₂PO₄ was added to adjust the pH to pH = 7. CR19101 (1.0 eq) was weighed and added, and the pH was controlled to equal to 7. The reaction was stirred under an ice bath for 15 min. HPLC controlled the complete reaction of raw materials, and the reaction solution was subjected to preparative purification and lyophilization to obtain CR194A2 (see the diagram for the structure).

### 6. Synthesis of conjugate compound CR194A6

### Step 1: Preparation of CR194A3

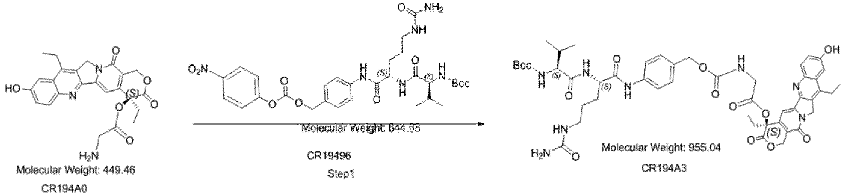

CR194A0 (1.0 eq), CR19496 (1.05 eq), and HOBT (0.1 eq) were weighed into a 50 mL single-necked flask and dissolved by adding DMF, then DIPEA (2 eq) was added and stirred at room temperature overnight. The reaction solution was subjected to preparative purification and lyophilization to obtain CR194A3 (see the diagram for the structure).

### Step 2: Preparation of CR194A4

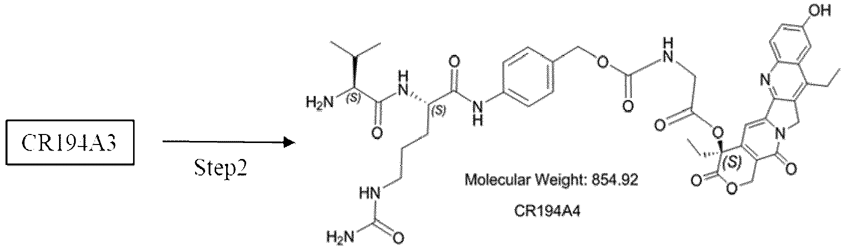

CR194A3 (1.0 eq) was weighed and dissolved in 30% TFA in dichloromethane, stirred at room temperature for 1 h. The reaction solution was rotary evaporated under reduced pressure to remove DCM and TFA, then MTBE was added to precipitate a solid, and CR194A4 was obtained by suction filtration (see the diagram for the structure).

### Step 3: Preparation of CR194A5

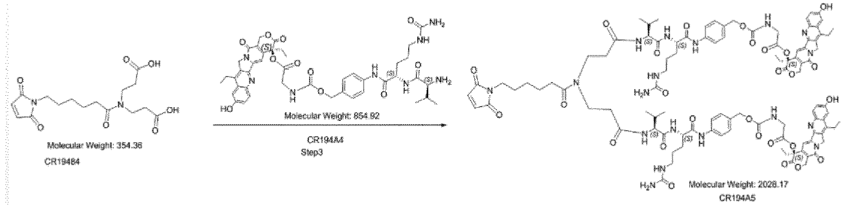

CR19484 (1.0 eq) and CR194A4 (2.0 eq) were weighed into a 50 mL single-necked flask, dissolved by adding DMF, and cooled under an ice bath, then HATU (2.1 eq) and DIPEA (3 eq) were added, stirred at room temperature for 2 h, and quenched by adding 5% aqueous acetic acid solution. A solid was precipitated out. The crude product of CR194A5 was obtained by suction filtration and subjected to preparative purification and lyophilization to obtain CR194A5 (see the diagram for the structure).

### Step 4: Preparation of CR194A6

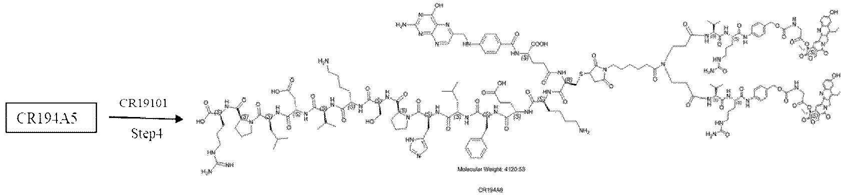

CR194A5 (1.0 eq) was weighed into a 50 mL single-necked bottle and dissolved by adding methanol, then 10 mM PBS buffer was added and stirred under an ice bath cooling for 10 min, pH = 8-9. 0.1 M NaH₂PO₄ was added to adjust the pH to pH = 7. CR19101 (1.0 eq) was weighed and added, and the pH was controlled to equal to 7. The reaction was stirred under an ice bath for 15 min. HPLC controlled the complete reaction of raw materials, and the reaction solution was subjected to preparative purification and lyophilization to obtain CR194A6 (see the diagram for the structure).

### 7. Synthesis of conjugate compound CR19462

### Step 1: Preparation of CR19459

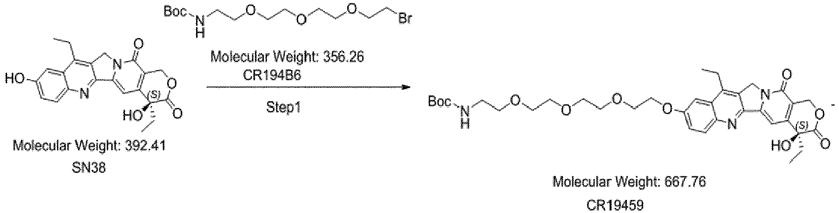

SN38 (1.0 eq) was weighed into a 50 mL single-necked flask and dissolved by adding DMF, then potassium carbonate (1.5 eq) and CR194B6 (1.2 eq) were added, heated to 50°C and stirred for 5 h. The reaction solution was cooled under an ice bath, quenched by adding water and extracted with ethyl acetate. The organic phase was washed with saturated sodium chloride, dried over anhydrous sodium sulfate, concentrated to remove the solvent, and purified on a silica gel column to obtain CR19459 (see the diagram for the structure).

### Step 2: Preparation of CR19460

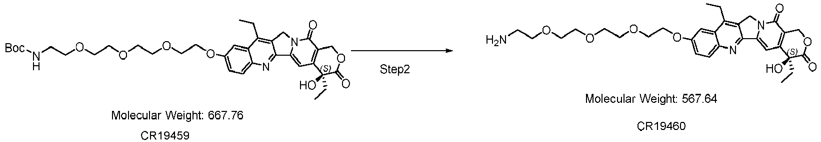

CR19459 (1.0 eq) was weighed into a 50 mL single-necked flask, 30% TFA in dichloromethane was added, stirred at room temperature for 1 h, and subjected to rotary evaporation under reduced pressure to remove the solvent. MTBE was added to precipitate a solid, and CR19460 was obtained by suction filtration (see the diagram for the structure).

### Step 3: Preparation of CR19461

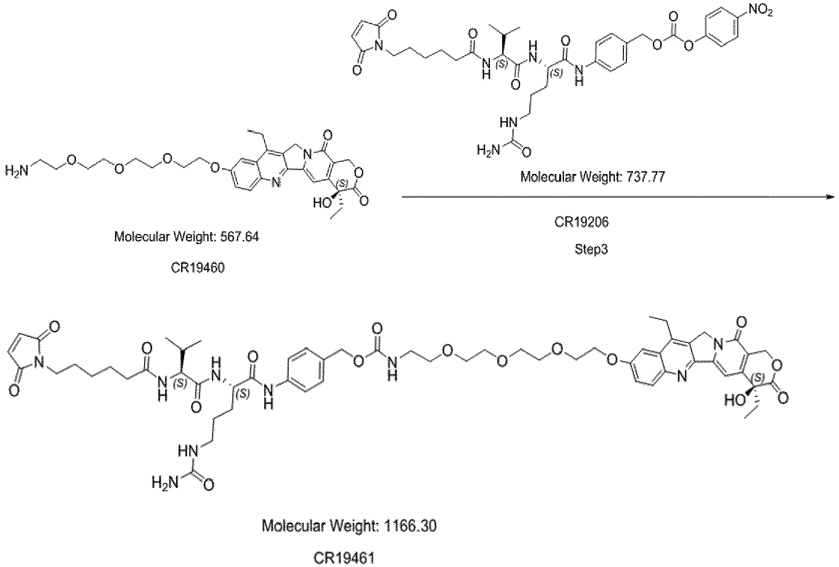

CR19460 (1.0 eq), CR19206 (1.05 eq), and HOBT (1.0 eq) were weighed into a 50 mL single-necked flask and dissolved by adding DMF, then DIPEA (2.0 eq) was added and stirred at room temperature overnight. The reaction solution was subjected to preparative purification and lyophilization to obtain CR19461 (see the diagram for the structure).

### Step 4: Preparation of CR19462

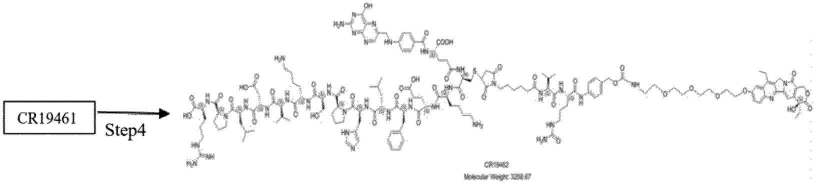

CR19461 (1.0 eq) was weighed into a 50 mL single-necked bottle and dissolved by adding methanol, then 10 mM PBS buffer was added and stirred under an ice bath cooling for 10 min, pH = 8-9. 0.1 M NaH₂PO₄ was added to adjust the pH to pH = 7. CR19101 (1.0 eq) was weighed and added, and the pH was controlled to equal to 7. The reaction was stirred under an ice bath for 15 min. HPLC controlled the complete reaction of raw materials, and the reaction solution was subjected to preparative purification and lyophilization to obtain CR19462. (see the diagram for the structure).

Other conjugate compounds as described in the present application can be prepared by using a preparation method similar to that described above.

### Example 3 Affinity Experiment

Experiments were carried out according to the Reichert SPR4 manual, and the affinities of the analyte CR194AR to FOLR1 and TRPV6 were respectively determined. Among them, the CM5 chip was coupled with ligands FOLR1 (Sinobiological Co., Ltd., Cat. No. 11241-H08H) and TRPV6 (Aero, Cat. No. MAES), respectively. The experimental results are shown in Table 1 below:

**Table 1: Affinity test result**

| Name | (FOLR1 end) KD (M) | (TRPV6 end) KD (M) |
|---|---|---|
| CR194AR | 4.54E-10 | 7.88E-5 |

The results showed that CR194AR was specifically bound to FOLR1 and TRPV6 with high affinity.

Similar to the above determination method, other conjugate compounds of the present application had good affinity with corresponding cell surface receptors.

### Example 4 Cell Expansion Inhibition Assay

Sample information: CR194AR, CR194X3, CR194V5, CR194AA, CR194AB, CR194U9, CR194Z9, CR194AH, CR194AY, CR194AX, and Dx.

Cell lines: HEK293T-FOLR1 (a stably transformed cell line overexpressing FOLR1), SK-BR-3 (human breast cancer cell) and HEK293T (human embryonic kidney cell). The protein expression levels of FOLR1 in different cell lines are shown in Table 2 below.

**Table 2: Protein expression levels of FOLR1 in different cell lines**

| **Cell lines** | **FOLR1** |
|---|---|
| HEK293T-FOLR1 | +++ (high expression) |
| SK-BR-3 | + (low expression) |
| HEK293T | - (no visible expression) |

Main reagents: 1640 folic acid-free medium, fetal bovine serum, penicillinstreptomycin solution, L-glutamine and CCK8.

Experimental operations:
(1) Cell plating: Cells were prepared in advance, digested with Trypsin, collected and counted. A complete cell culture medium was used to dilute HEK293T-FOLR1 and HEK293T cells to 6 × 10³ cells/ml, and dilute SK-BR-3 cells to 1.5 × 10⁴ cells/ml. 100 µl of diluted cell solution was added to each well of 96-well plates, and negative and blank control wells were set on each plate. The 96-well plates added with the cells were placed in an incubator at 37°C, 5% COz for culturing overnight.
(2) Diluting and adding samples: The samples were serially diluted with medium (the starting concentration was 30 µM, 5-fold serial dilution), and a total of 10 concentration gradients were set. To each well of the 96-well plates that were cultured overnight, 50 µl of the diluted samples were added, with 3 replicate wells; negative controls and blank controls were set; and the plates were placed in an incubator at 37°C, 5% CO2 for culturing 72 h.
(3) Color development and reading plate: To each well, 15 µl (10% of the liquid volume in a well) of a color developing solution from CCK-8 was added; the plates were incubated at 37°C for an appropriate time (try to keep the OD value in the range of 1.0-2.5); the covers of the 96-well plates were removed, and the plates were read at 450 nm on a microplate reader (Molecular Devices SpectraMax iD5).
(4) Data processing: The data was edited using SoftMax Pro 7.1 and a four-parameter fitting curve was plotted.

The experimental results are shown in Table 3 below:

**Table 3: Inhibitory activity against HEK293T-FOLR1, SK-BR-3 and HEK293T cells**

| **Sample name** | **HEK293T-FOLR1** | **HEK293T** | **SK-BR-3** |
|---|---|---|---|
| | IC₅₀ | IC₅₀ | IC₅₀ |
| CR194AR | 0.4 | 2371 | 52 |
| CR194X3 | 1.594 | 5.035 | 4.024 |
| CR194V5 | 2.17 | 8.284 | 4.961 |
| CR194AA | 0.633 | 18.13 | 5.085 |
| CR194AB | 0.58 | 27.38 | 6.149 |
| CR194U9 | 1.541 | 180.6 | 35.37 |
| CR194Z9 | 1.19 | 145.7 | 20.06 |
| CR194AH | 0.308 | 346 | 38.78 |
| CR194AY | 0.755 | 5143 | 50.42 |
| CR194AX | 0.195 | 1211 | 132.6 |
| Dx | 1.017 | 1.12 | 0.599 |

It can be seen from Table 3 that the IC₅₀ of CR194AR, CR194AB, CR194U9, CR194Z9, CR194AH, CR194AY, CR194AX, CR194X3, CR194V5 and CR194AA against the cell lines HEK293T-FOLR1 and SK-BR-3 with relatively high expression of the receptors was significantly lower than the cell line HEK293T with relatively low expression of the receptors. It can be seen that the above-mentioned compounds showed a tumor-inhibiting effect related to the expression level of related receptors in cells and played an important role in inhibiting the proliferation of tumor cells.

Sample information: CR194A2, CR194A6, CR194N5, CR194N9, CR194P6, CR194P3, CR194S3, CR194R8, CR194L4, CR194U7, CR194Q1, CR194Q5, CR194U9, CR194R7, Dxd, Dx, and SN38.

Cell lines: HEK293T-FOLR1 (a stably transformed cell line overexpressing FOLR1), SK-BR-3 (human breast cancer cell with low expression of FOLR1) and MIA PaCa2 (human pancreatic cancer cell without expression of FOLR1).

Main reagents: 1640 folic acid-free medium, fetal bovine serum, penicillin-streptomycin solution, L-glutamine and CCK8.

Experimental operations:
(1) Cell plating: Cells were prepared in advance, digested with Trypsin, collected and counted. A complete cell culture medium was used to dilute SK-BR-3 cells to 3.0 × 10⁴ cells/ml, HEK293T-FOLR1 cells to 1.5 × 10⁴ cells/ml, and MIA PaCa-2 cells to 2.0 × 10⁴ cells/ml. 100 µl of diluted cell solution was added to each well of 96-well plates, and negative and blank control wells were set on each plate. The 96-well plates added with the cells were placed in an incubator at 37°C, 5% COz for culturing overnight.
(2) Diluting and adding samples: The samples were serially diluted with medium (the starting concentration was 30 µM, 5-fold serial dilution), and a total of 10 concentration gradients were set. To each well of the 96-well plates that were cultured overnight, 50 µl of the diluted samples were added, with 3 replicate wells; negative controls and blank controls were set; and the plates were placed in an incubator at 37°C, 5% CO2 for culturing 72 h.
(3) Color development and reading plate: To each well, 15 µl (10% of the liquid volume in a well) of a color developing solution from CCK-8 was added; the plates were incubated at 37°C for an appropriate time (try to keep the OD value in the range of 1.0-2.5); the covers of the 96-well plates were removed, and the plates were read at 450 nm on a microplate reader (Molecular Devices SpectraMax iD5).
(4) Data processing: The data was edited using SoftMax Pro 7.1 and a four-parameter fitting curve was plotted.

The experimental results are shown in Table 4 below:

**Table 4: Inhibitory activity against HEK293T-FOLR1, SK-BR-3 and MIA PaCa2 cells**

| **Sample name** | **HEK293T-FOLR1** | **MIAPACA-2** | **SK-BR-3** |
|---|---|---|---|
| | IC₅₀ | IC₅₀ | IC₅₀ |
| CR194A2 | 1.069 | 20.15 | 12.6 |
| CR194A6 | 2.513 | 15.54 | 1.719 |
| CR194N5 | 2.754 | 19.79 | 3.204 |
| CR194N9 | 4.957 | 16.9 | 3.239 |
| CR194P6 | 200.9 | 434.8 | 283.1 |
| CR194P3 | 1.473 | 75.28 | 20.27 |
| CR194S3 | 0.76 | 10.04 | 1.051 |
| CR194R8 | 0.269 | 0.412 | 0.537 |
| CR194L4 | 0.436 | 23.76 | / |
| CR194U7 | 18.66 | 148.8 | 29 |
| CR194Q1 | 0.935 | / | 2508 |
| CR194Q5 | 0.908 | 1.35 | 126 |
| CR194U9 | 26.04 | 1064 | 237.5 |
| CR194R7 | 0.132 | 262.5 | 62.42 |
| Dxd | 1.677 | 110.9 | / |
| Dx | 0.995 | 5.529 | 0.569 |
| SN38 | 0.943 | 7.027 | 0.947 |

| | | | |
|---|---|---|---|
| Notes: "/" indicates that no valid IC₅₀ value was fitted. | | | |

### Example 5 Pharmacodynamic Study of CR194AX, CR194X3 and CR194AR in CDX Model

Experiment purpose: to study the efficacy of conjugate compounds CR194AX, CR194X3 and CR194AR in the subcutaneous xenograft model (CDX) of 293T-FOLR1 cell line overexpressing FOLR1.

### CDX model: 293T-FOLR1

Experimental scheme: The cells were prepared and subcutaneously inoculated into the right flank of BALB/c-nude mice. When the tumor volume expanded to 100-150 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumor growth and treatment on normal behaviors of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumor were measured with a vernier caliper, and the tumor volume, relative tumor proliferation rate, tumor volume inhibition rate and other indicators were calculated.
The formula of tumor volume is TV = 0.5 a × b², wherein a is the long diameter of a tumor and b is the short diameter of a tumor.

The experimental results are shown in Table 5 below. It can be seen from the experimental results that the test samples CR194AX, CR194X3 and CR194AR show different degrees of tumor growth inhibition effects with a regimen of administration via tail vein. CR194AX, CR194X3 and CR194AR all had certain anti-tumor growth effects at a medium dose (3 mg/kg) compared with the negative control group; all had excellent anti-tumor growth effects at a high dose (10 mg/kg) compared with the negative control group, and the anti-tumor effects were dose-dependent, and the data are shown in Table 5.

**Table 5: Efficacy in subcutaneous xenograft model (CDX) of 293T-FOLR1 cell line**

| Test samples | Model | FOLR1 Expression level | TRPV6 Expression level | Tumor growth inhibition rate (TGI) (%) | Administration dosage (time) |
|---|---|---|---|---|---|
| CR194AX | 293T-FOLR1 | +++ | - | -5.94 | 1 mg/kg (days 1, 8, 15) |
| | | | | 73.35 | 3 mg/kg (days 1, 8, 15) |
| | | | | 97.95 | 10 mg/kg (days 1, 8, 15) |
| CR194X3 | | | | 53.15 | 1 mg/kg (days 1, 8, 15) |
| | | | | 64.80 | 3 mg/kg (days 1, 8, 15) |
| | | | | 89.98 | 10 mg/kg (days 1, 8, 15) |
| CR194AR | | | | -9.95 | 1 mg/kg (days 1, 8, 15) |
| | | | | 58.21 | 3 mg/kg (days 1, 8, 15) |
| | | | | 96.37 | 10 mg/kg (days 1, 8, 15) |

### Example 6 Pharmacodynamic Study of Conjugate Compounds in CDX Model

Experiment purpose: to study the efficacy of conjugate compounds CR194Q1, CR194V5, CR194AH, CR194X3, CR194U9, CR194Z9, CR194AB, CR194AX, CR194AY and CR194AR in the CDX model of CFPAC-1.

### CDX model: CFPAC-1 (human pancreatic cancer cell)

Experimental scheme: The cells were prepared and subcutaneously inoculated into the right flank of BALB/c-nude mice. When the tumor volume expanded to 100-150 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumor growth and treatment on normal behaviors of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumor were measured with a vernier caliper, and the tumor volume, relative tumor proliferation rate, tumor volume inhibition rate and other indicators were calculated. The formula of tumor volume is TV = 0.5 a × b², wherein a is the long diameter of a tumor and b is the short diameter of a tumor.

The experimental results are shown in Table 6 below. It can be seen from the experimental results that the test samples CR194Q1, CR194V5, CR194AH, CR194X3, CR194U9, CR194Z9, CR194AB, CR194AX, CR194AY and CR194AR all have a good inhibitory effect on the CDX tumor model of mouse CFPAC-1.

| Test samples | Model | FOLR1 Expression level | TRPV6 Expression level | Tumor growth inhibition rate (TGI) (%) | Administration dosage/time |
|---|---|---|---|---|---|
| CR194V5 | CFPAC -1 | ++ | - | 85.11 | 25 mg/kg (days 1, 4, 7, 10, 13) |
| CR194AH | | | | 81.64 | 25 mg/kg (days 1, 4, 7, 10, 13) |
| CR194U9 | | | | 88.85 | 25 mg/kg (days 1, 4, 7, 10, 13) |
| CR194Z9 | | | | 90.26 | 25 mg/kg (days 1, 4, 7, 10, 13) |
| CR194AB | | | | 71.60 | 25 mg/kg (days 1, 4, 7, 10, 13) |
| CR194Q1 | | | | 93.84 | 25 mg/kg (days 1, 4, 7, 10, 13) |
| | | | | 99.84 | 50 mg/kg (days 1, 4, 7, 10, 13) |
| | | | | 99.75 | 75 mg/kg (days 1, 4, 7, 10, 13) |
| CR194X3 | | | | 87.71 | 25 mg/kg (days 1, 4, 7, 10, 13) |
| | | | | 98.53 | 50 mg/kg (days 1, 4, 7, 10, 13) |
| | | | | 99.70 | 75 mg/kg (days 1, 4, 7, 10, 13) |
| CR194AX | | | | 99.54 | 50 mg/kg (days 1, 4, 7, 10, 13) |
| CR194AY | | | | 33.46 | 50 mg/kg (days 1, 4, 7, 10, 13) |
| CR194AR | | | | 99.55 | 50 mg/kg (days 1, 4, 7, 10, 13) |

### Example 7 Tumor Growth Inhibition Experiment of Conjugate Compounds in HuPrime^{®} xenograft PDX Model

Experiment purpose: pharmacodynamic study of conjugate compounds CR194Q1, CR194X3, CR194AX, CR194AR and CR194AY in the PDX model of OV2423

Model: OV2423, an animal model of ovarian cancer, from Crown Biotechnology (Taicang) Co., Ltd.

Experimental scheme: The tumor tissue mass was prepared and subcutaneously inoculated into the anterior right flank of BALB/c-nude mice. When the tumor volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumor growth and treatment on normal behaviors of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumor were measured with a vernier caliper, and the tumor volume, relative tumor proliferation rate, tumor volume inhibition rate and other indicators were calculated.
The formula of tumor volume is TV = 0.5 a × b², wherein a is the long diameter of a tumor and b is the short diameter of a tumor.

The experimental results are shown in Table 7 below. It can be seen from the experimental results that the test samples CR194Q1, CR194X3, CR194AX, CR194AR and CR194AY show excellent anti-tumor growth effects in the FOLR1 single expression model OV2423.

| Test samples | Model | FOLR1 | TRPV6 | TGI (%) | Dosage/time |
|---|---|---|---|---|---|
| CR194Q1 | OV2423 | 9+ | - | 100 | 25 mg/kg, (days 1, 4, 7, 10, 13, 16) |
| CR194Q1 | | | | 100 | 50 mg/kg, (days 1, 4, 7, 10, 13, 16) |
| CR194X3 | | | | 84.02 | 10 mg/kg, (days 1, 4, 7, 10, 13, 16) |
| CR194X3 | | | | 94.92 | 25 mg/kg, (days 1, 4, 7, 10, 13, 16) |
| CR194AX | | | | 99.41 | 25 mg/kg, (days 1, 4, 7, 10, 13, 16) |
| CR194AR | | | | 99.37 | 25 mg/kg, (days 1, 4, 7, 10, 13, 16) |
| CR194AY | | | | 92.16 | 25 mg/kg, (days 1, 4, 7, 10, 13, 16) |

### Example 8 Safety Evaluation of Conjugate Compounds in MIAPaca-2 Human Pancreatic Cancer Model BALB/c Nude Mice

Experiment purpose: to test the effect of the conjugate compounds on the toxicity of tumor-bearing nude mice model transplanted with MIAPaCa-2 human pancreatic cancer cells subcutaneously under the condition of equal dose.

Sample preparation: An appropriate amount of the compound was weighed and dissolved in PBS, and an appropriate amount of alkaline solution was added to formulate a sample mother solution with a neutral pH, which was filtered and diluted with filtered physiological saline to a sample solution with a working concentration for later use.

Experimental scheme: The tumor tissue mass was prepared and subcutaneously inoculated into the anterior right flank of BALB/c-nude mice. When the tumor volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumor growth and treatment on normal behaviors of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumor were measured twice a week with a vernier caliper, and the tumor volume, relative tumor proliferation rate, tumor volume inhibition rate and other indicators were calculated. The formula of tumor volume is TV = 0.5 a × b², wherein a is the long diameter of a tumor and b is the short diameter of a tumor. The experimental design was as shown in Table 8 below.

| **Group** | **Number of animals** | **Test object** | **Dosage (mg/kg)** | **Mode of administration** | **Planned administration cycle** |
|---|---|---|---|---|---|
| 1 | 6 | control | - | - | - |
| 3 | 6 | CR194L4 | 100 | iv | Once every 3 days, a total of 6 administrations (q3d*6) |
| 4 | 6 | CR194A6 | 100 | iv | Once every 3 days, a total of 3 administrations (q3d*3) |
| 5 | 6 | CR194N5 | 100 | iv | q3d*3 |
| 6 | 6 | CR194P6 | 100 | iv | q3d*3 |

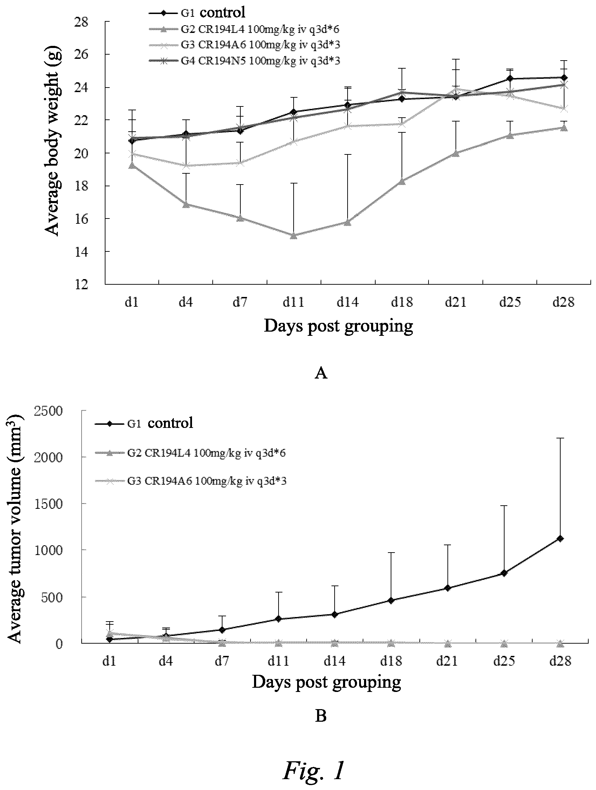

As shown in Figs. 1A-1B, CR194L4 (100 mg/kg iv q3d*6) and CR194A6 (100 mg/kg iv q3d*3) had good tumor growth inhibition effects on MIAPaCa-2. After the first administration of CR194L4 (100 mg/kg iv q3d*6), the body weight of the whole group has been slowly decreasing, but after the fourth administration, the administration was stopped and the body weight of the animals increased slowly; for CR194A6 (100 mg/kg iv q3d*3), one experimental animal died on the experimental day d2, and the body weight of the experimental animals in the same group increased slowly; the body weight values of the experimental animals in the remaining groups fluctuated slowly, but the overall trend was increasing.

### Example 9 Safety Evaluation of Conjugate Compound CR194AX in ICR Mice

### Experiment purpose: Safety evaluation of CR194AX in ICR mice

Sample preparation: An appropriate amount of CR194AX was weighed and dissolved in PBS, and an appropriate amount of alkaline solution was added to formulate a sample mother solution with a neutral pH, which was filtered and diluted with filtered PBS to a sample solution with a working concentration for later use.

Experimental design: ICR mice (female, 6-8 weeks old, weighing about 25 g) were randomly grouped according to body weight, and the administration was started on the first day of grouping. The dosage was adjusted according to the latest body weight of the mice, and the administration was performed via tail vein injection at an administration volume of 10 µl/g. After grouping and administration, the effects of the drugs on the normal behaviors of the animals, including the activity, food intake and drinking status, body weight, eyes, hair, feces, secretions and other abnormalities of the experimental animals, were tested. The body weight of the mice was measured twice a week and observed for 8 days after the last administration. At the end of the experiment, the liver and spleen were weighed, and the liver, spleen, lung, kidney and stomach were taken out and placed in 10% formalin neutral fixative for HE analysis. Sample preparation: An appropriate amount of the compound was weighed and dissolved in PBS, and an appropriate amount of alkaline solution was added to formulate a sample mother solution with a neutral pH, which was filtered and diluted with filtered physiological saline to a sample solution with a working concentration for later use.

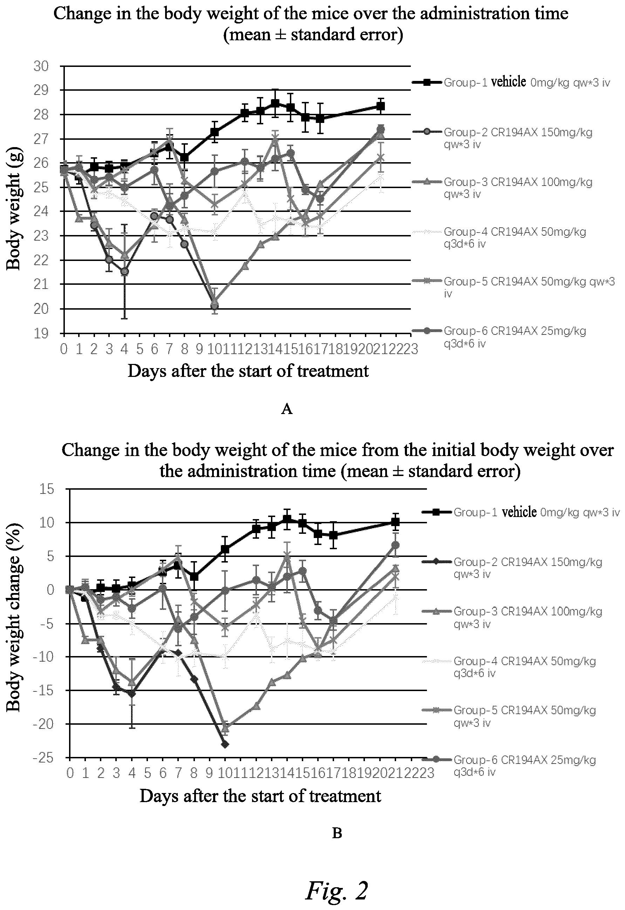

Experimental results and analysis: CR194AX at 50 mg/kg was administered once a week for 3 administrations (qw*3) and administered once every three days for 6 administrations (q3d*6), no mice died during the administration period, and the average weight loss of the mice did not exceed 10%, showing good tolerance; during the administration of CR194AX 25 mg/kg q3d*6, no mice died, and the average weight loss of the mice did not exceed 5%, showing good tolerance (see Figs. 2A-2B). At the end of the experiment, the liver and spleen were taken, and the results showed that: compared with the negative control group, there was no significant difference in the weights of the liver and spleen of the mice in each administration group.

### Example 10 Safety Evaluation of Conjugate Compound CR194X3 in BALB/c Nude Mice

### Experiment purpose: Safety evaluation of CR194X3 in BALB/c nude mice

Sample preparation: An appropriate amount of CR194X3 was weighed and dissolved in PBS, and an appropriate amount of alkaline solution was added to formulate a sample mother solution with a neutral pH, which was filtered and diluted with filtered PBS to a sample solution with a working concentration for later use.

BALB/c nude mice (female, 6-8 weeks old, weighing about 25 g) were randomly grouped according to body weight (6 mice in each group), and the administration was started on the first day of grouping. The dosage was adjusted according to the latest body weight of the mice, and the administration was performed via tail vein injection at an administration volume of 10 µl/g. After grouping and administration, the effects of the drugs on the normal behaviors of the animals, including the activity, food intake and drinking status, body weight, eyes, hair, feces, secretions and other abnormalities of the experimental animals, were tested. The body weight of the mice was measured twice a week and observed for 7 days after the last administration. At the end of the experiment, the liver and spleen were weighed, and the lung, liver, spleen and small intestine were taken out and placed in 10% formalin neutral fixative for HE analysis.

Experimental results and analysis: the mice had a good tolerance to the test substance CR194X3 (150 mg/kg). At the end of the experiment, the liver and spleen were taken, and the results showed that: compared with the negative control group, there was no significant difference in the weights of the liver and spleen of the mice in each administration group.

### Example 11 Safety Evaluation of CR194AX and CR194X3 in SD Rats

### Experiment purpose: Safety evaluation of CR194 series compounds in SD rats

Sample preparation: An appropriate amount of the compound was weighed and dissolved in physiological saline, and an appropriate amount of alkaline solution was added to formulate a sample mother solution with a neutral pH, which was filtered and diluted with filtered physiological saline to a sample solution with a working concentration for later use.

Experimental scheme: SD rats (female, weighing 160-180 g) were randomly grouped according to body weight (5 mice in each group), and the administration was started on the first day of grouping. The dosage was adjusted according to the latest body weight of the rats, and the administration was performed via tail vein injection at an administration volume of 10 ml/kg. After grouping and administration, the effects of the drugs on the normal behaviors of the animals, including the activity, food intake and drinking status, body weight, eyes, hair, feces, secretions and other abnormalities of the experimental animals, were tested. The body weight of the rats was measured twice a week and observed for 14 days after administration.

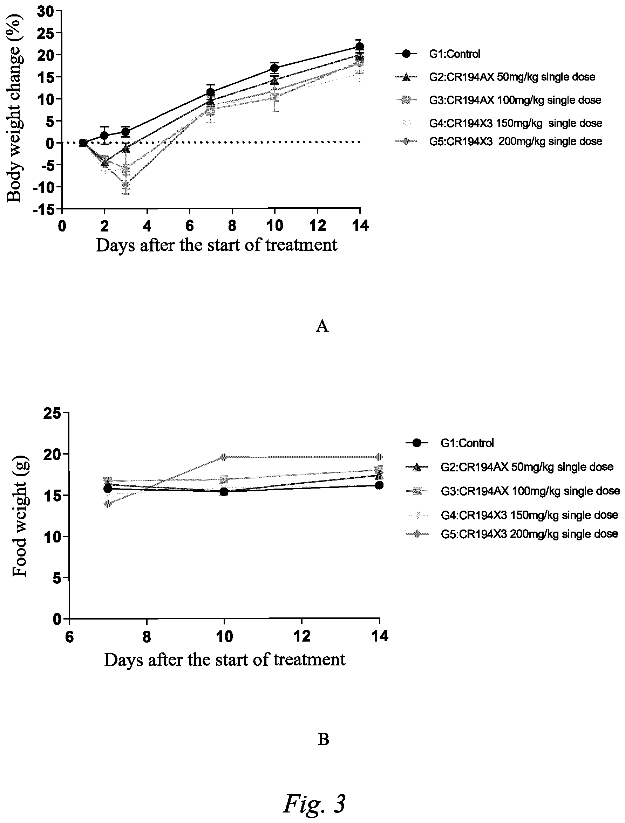

Experimental results and analysis: the body weight of the animals in each administration group continued to decrease after the administration to day 3, but did not decrease by more than 10%. Clinical observations showed no abnormalities (Figs. 3A-3B).

## Claims

1. A conjugate compound or a pharmaceutically acceptable salt thereof, the conjugate compound having the following structure:
multi-ligand moiety-(linker-payload)_{q},
wherein:
the multi-ligand moiety comprises at least two ligands targeting different cell surface molecules,
and the linker-payload moiety has the following structure: 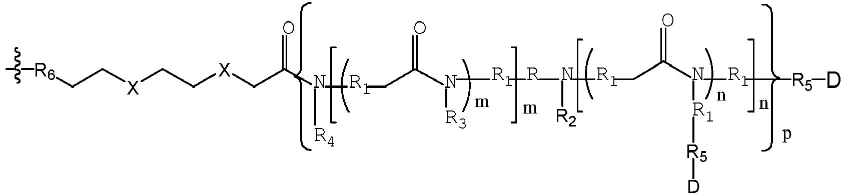 wherein:
R is selected from 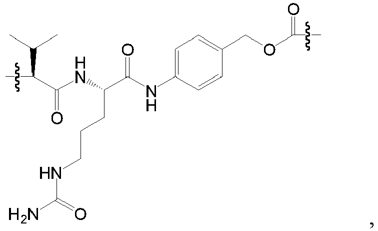 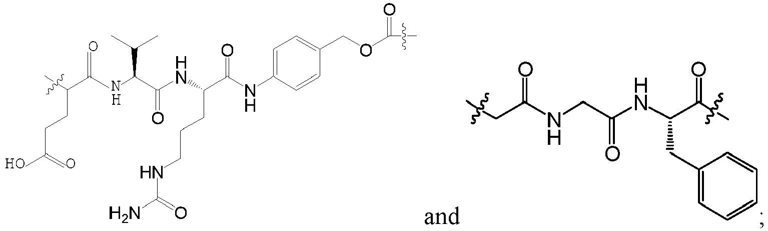
each R₁ is independently selected from a bond and -CH₂CH₂C(O)-NH-;
each R₂ is independently selected from hydrogen and 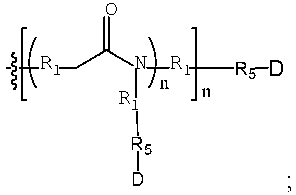
each R₃ is independently selected from hydrogen and 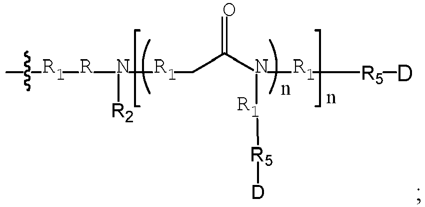
each R₄ is independently selected from hydrogen and 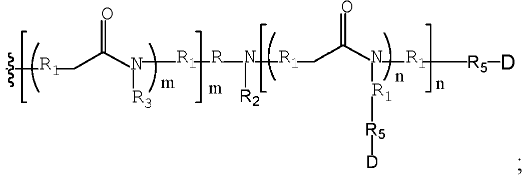
each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 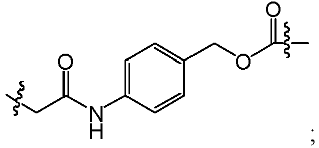
each R₆ is independently selected from a bond, 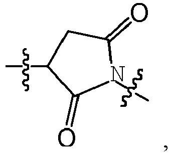 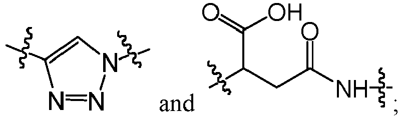
each X is independently selected from a bond, C and O;
each m is independently 0 or 1;
each n is independently 0 or 1;
p is 0 or 1;
q is an integer from 1 to 4;
and D is the payload.

2. A conjugate compound or a pharmaceutically acceptable salt thereof, the conjugate compound having the following structure:
multi-ligand moiety-(linker-payload)_{q},
wherein:
the multi-ligand moiety comprises at least two ligands targeting different cell surface molecules,
and the linker-payload moiety has the following structure: 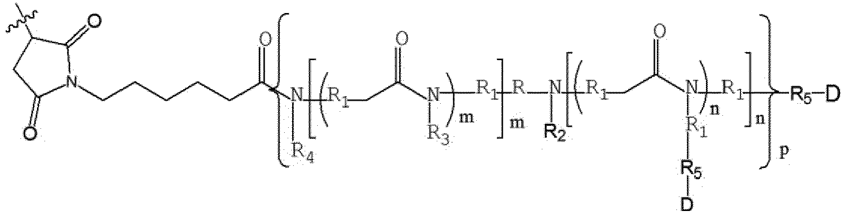 wherein:
R is selected from 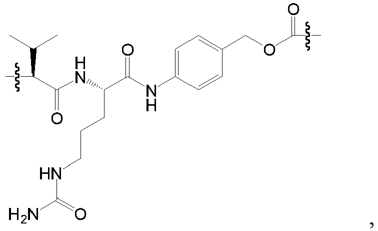 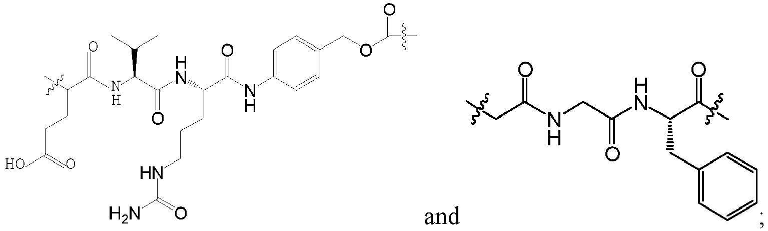
each R₁ is independently selected from a bond and -CH₂CH₂C(O)-NH-;
each R₂ is independently selected from hydrogen and 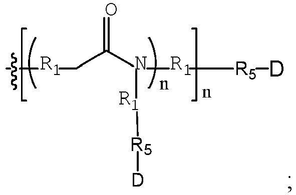
each R₃ is independently selected from hydrogen and 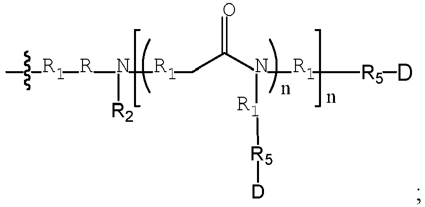
each R₄ is independently selected from hydrogen and 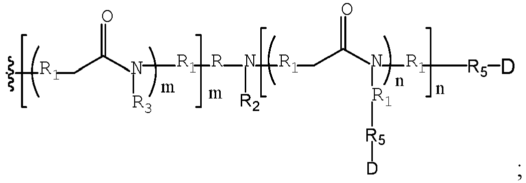
each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 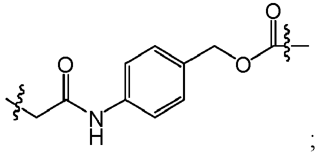
each m is independently 0 or 1;
each n is independently 0 or 1;
p is 0 or 1;
q is an integer from 1 to 4;
and D is the payload.

3. A conjugate compound or a pharmaceutically acceptable salt thereof, the conjugate compound having the following structure:
multi-ligand moiety-(linker-payload)_{q},
wherein:
the multi-ligand moiety comprises at least two ligands targeting different cell surface molecules,
and the linker-payload moiety has the following structure: 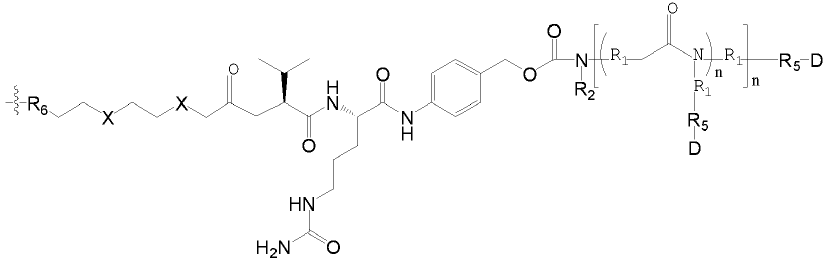 wherein:
each R₁ is independently selected from a bond and -CH₂CH₂C(O)-NH-;
each R₂ is independently selected from hydrogen and 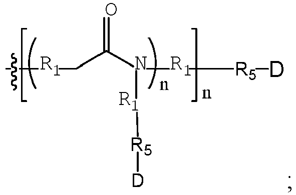
each R₅ is independently selected from a bond, -(CH₂CH₂O)₃-CH₂CH₂-, - CH₂C(O)-NH-CH₂-, -CH₂C(O)-, -CH₂C(O)-NH-CH₂C(O)- and 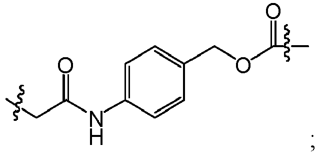
each R₆ is independently selected from a bond, 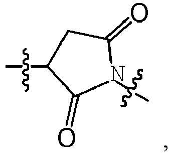 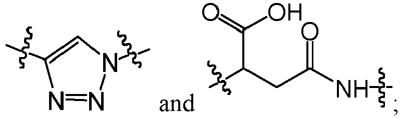
each X is independently selected from a bond, C and O;
each n is independently 0 or 1;
q is an integer from 1 to 4;
and D is the payload.

4. A conjugate compound or a pharmaceutically acceptable salt thereof, the conjugate compound having the following structure:
multi-ligand moiety-(linker-payload)_{q},
wherein:
the multi-ligand moiety comprises at least two ligands targeting different cell surface molecules,
and the linker-payload moiety has the following structure: 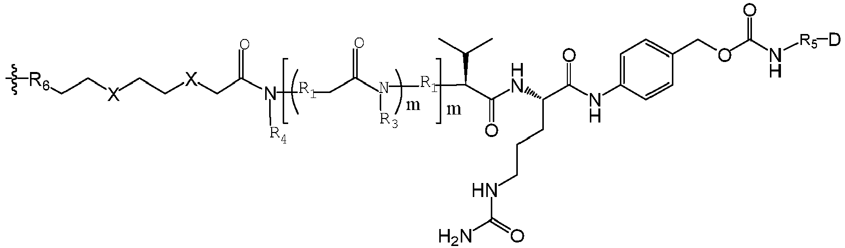 wherein:
each R₁ is independently selected from a bond and -CH₂CH₂C(O)-NH-;
each R₃ is independently selected from hydrogen and -R₁-R-NH-R₅-D;
R₄ is hydrogen or 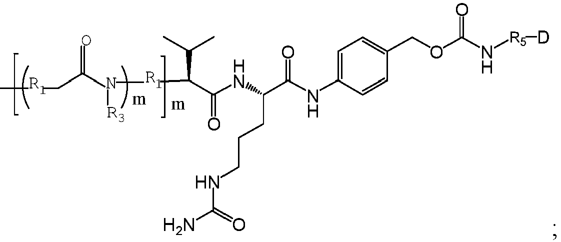
R is selected from 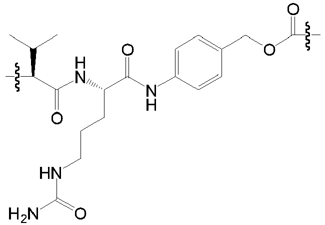 and 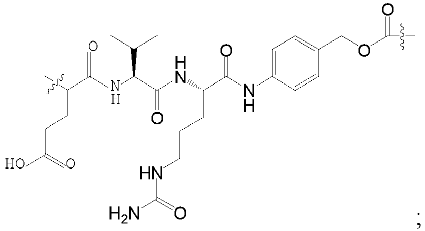
each R₅ is independently selected from a bond, -(CH₂CH₂O)₃-CH₂CH₂-, - CH₂C(O)-NH-CH₂-, -CH₂C(O)-NH-CH₂C(O)- and -CHzC(O)-;
each R₆ is independently selected from a bond, 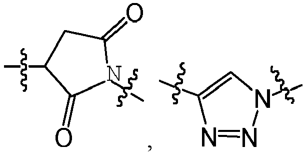 and 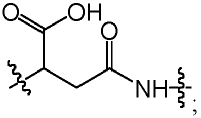
each X is independently selected from a bond, C and O;
each m is independently 0 or 1;
q is an integer from 1 to 4;
and D is the payload.

5. A conjugate compound or a pharmaceutically acceptable salt thereof, the conjugate compound having the following structure:
multi-ligand moiety-(linker-payload)_{q},
wherein:
the multi-ligand moiety comprises at least two ligands targeting different cell surface molecules,
and the linker-payload moiety has the following structure: 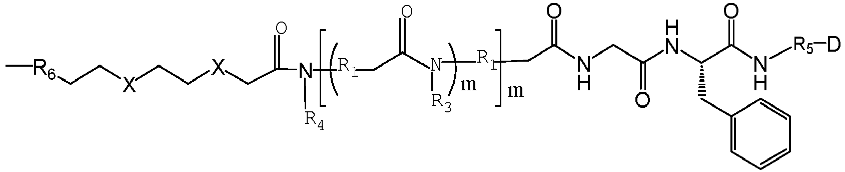 wherein:
each R₁ is independently selected from a bond and -CH₂CH₂C(O)-NH-;
each R₃ is independently selected from hydrogen and -R₁-R-NH-R₅-D;
R₄ is hydrogen or 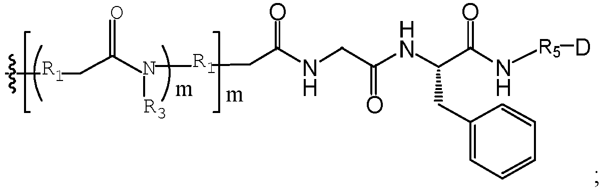
R is 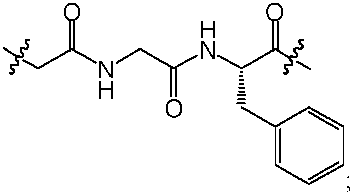
each R₅ is independently selected from a bond, -(CH₂CH₂O)₃-CH₂CH₂-, - CH₂C(O)-NH-CH₂-, -CH₂C(O)-NH-CH₂C(O)-, -CH₂C(O)- and 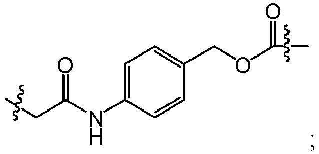
each R₆ is independently selected from a bond, 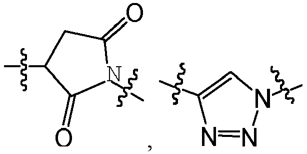 and 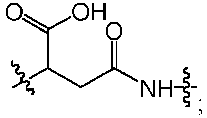
each X is independently selected from a bond, C and O;
each m is independently 0 or 1;
q is an integer from 1 to 4;
and D is the payload.

6. The conjugate compound or the pharmaceutically acceptable salt thereof according to claim 1, wherein R is

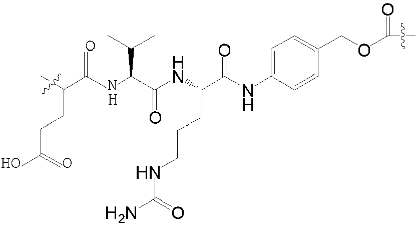

7. The conjugate compound or the pharmaceutically acceptable salt thereof according to any one of claims 1-6, wherein R₁ is -CH₂CH₂C(O)-NH-.

8. The conjugate compound or the pharmaceutically acceptable salt thereof according to any one of claims 1-7, wherein R₅ is -(CH₂CH₂O)₃-CH₂CH₂-.

9. The conjugate compound or the pharmaceutically acceptable salt thereof according to any one of claims 1-7, wherein R₅ is -CH₂C(O)-NH-CH₂C(O)-

10. The conjugate compound or the pharmaceutically acceptable salt thereof according to any one of claims 1-7, wherein R₅ is -CH₂C(O)-.

11. The conjugate compound or the pharmaceutically acceptable salt thereof according to any one of claims 1-7, wherein R₅ is

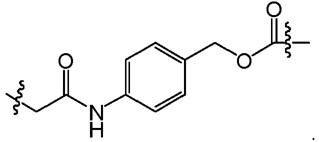

12. The conjugate compound or the pharmaceutically acceptable salt thereof according to any one of claims 1-7, wherein R₅ is -CH₂C(O)-NH-CH₂-.

13. The conjugate compound or the pharmaceutically acceptable salt thereof according to any one of claims 1-7, wherein R₅ is not -CH₂C(O)-NH-CH₂-.

14. The conjugate compound or the pharmaceutically acceptable salt thereof according to any one of claims 1-13, wherein each m is independently 0 or 1, and n is 0.

15. The conjugate compound or the pharmaceutically acceptable salt thereof according to any one of claims 1-13, wherein m is 0, and each n is independently 0 or 1.

16. The conjugate compound or the pharmaceutically acceptable salt thereof according to any one of claims 1-13, wherein m is 0, and n is 0.

17. The conjugate compound or the pharmaceutically acceptable salt thereof according to any one of claims 1-13, wherein m is 0, n is 0, and R₅ is not - CH₂C(O)-NH-CH₂-.

18. The conjugate compound or the pharmaceutically acceptable salt thereof according to any one of claims 1 or 3-17, wherein R₆ is

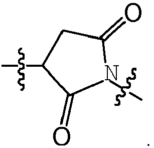

19. The conjugate compound or the pharmaceutically acceptable salt thereof according to any one of claims 1 or 3-18, wherein R₆ is 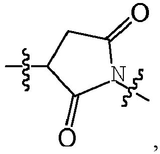 and X is a bond.

20. The conjugate compound or the pharmaceutically acceptable salt thereof according to any one of claims 1 or 3-18, wherein R₆ is 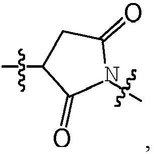 and X is O.

21. The conjugate compound or the pharmaceutically acceptable salt thereof according to any one of claims 1 or 3-17, wherein R₆ is

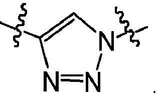

22. The conjugate compound or the pharmaceutically acceptable salt thereof according to claim 21, wherein X is a bond.

23. The conjugate compound or the pharmaceutically acceptable salt thereof according to claim 21, wherein X is O.

24. The conjugate compound or the pharmaceutically acceptable salt thereof according to any one of claims 1 or 3-17, wherein R₆ is

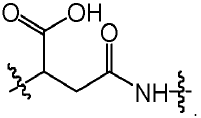

25. The conjugate compound or the pharmaceutically acceptable salt thereof according to claim 24, wherein X is a bond.

26. The conjugate compound or the pharmaceutically acceptable salt thereof according to claim 24, wherein X is O.

27. The conjugate compound or the pharmaceutically acceptable salt thereof according to any one of claims 1-2 or 7-26, wherein p is 0.

28. The conjugate compound or the pharmaceutically acceptable salt thereof according to any one of claims 1-5, wherein the linker has a structure selected from the group of:

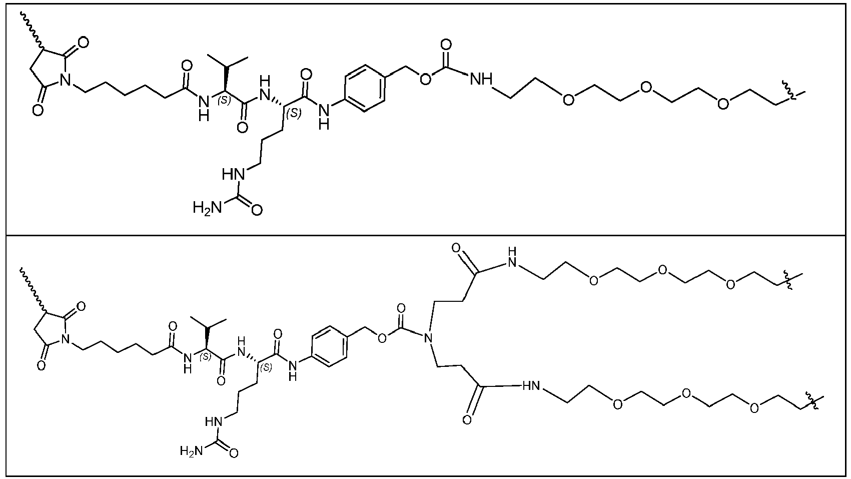

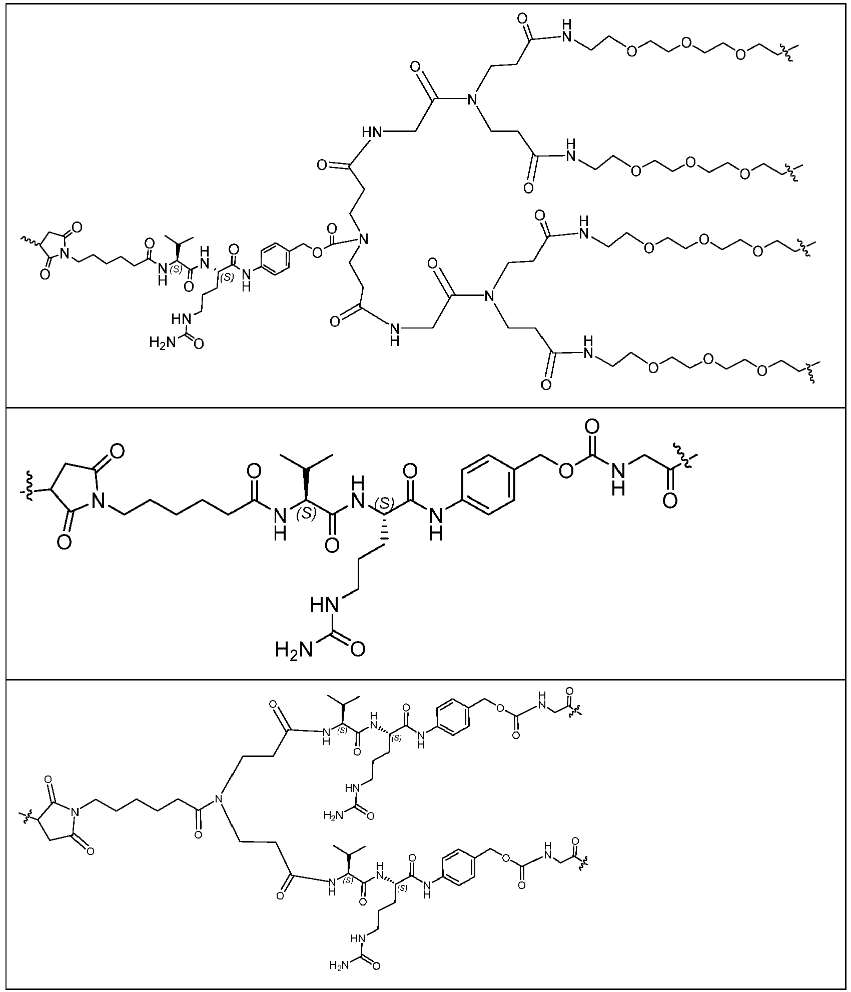

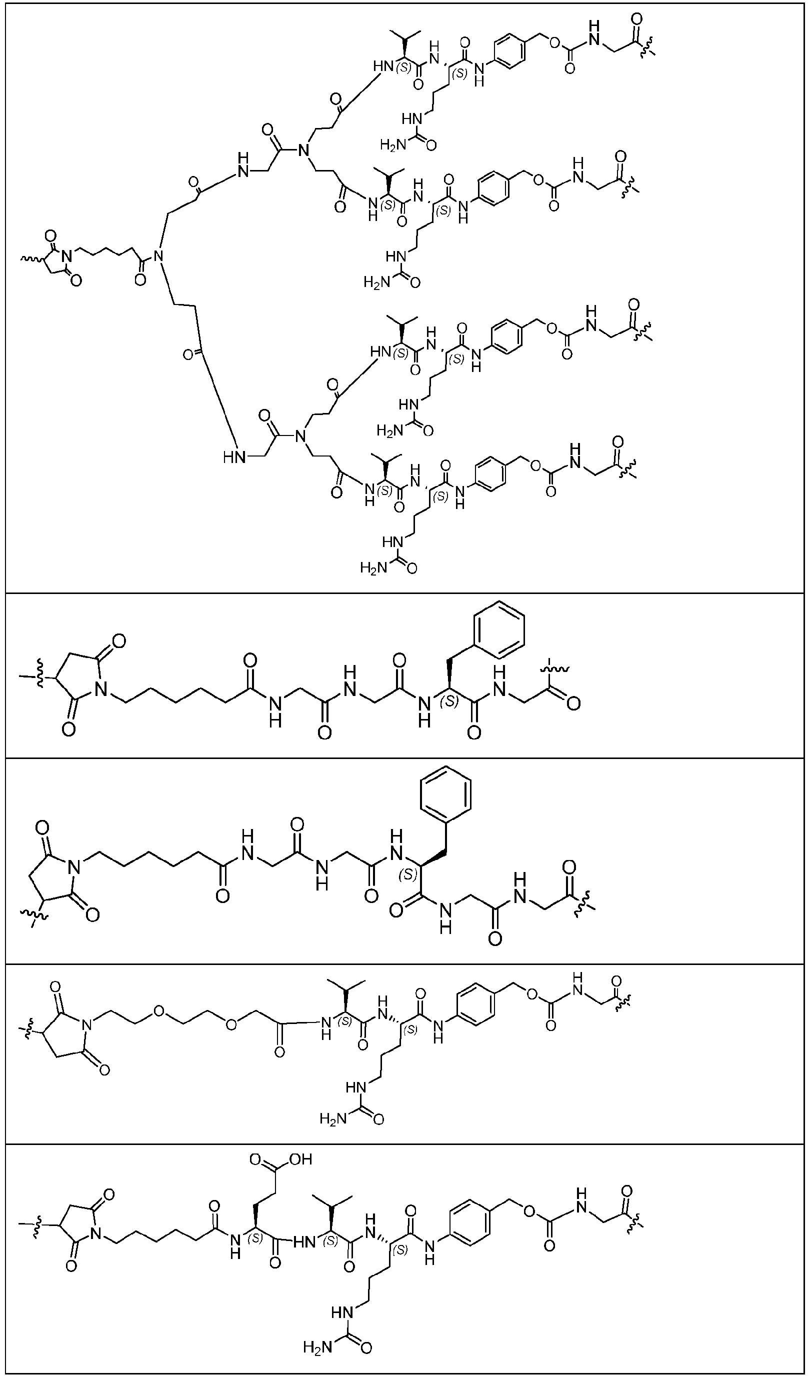

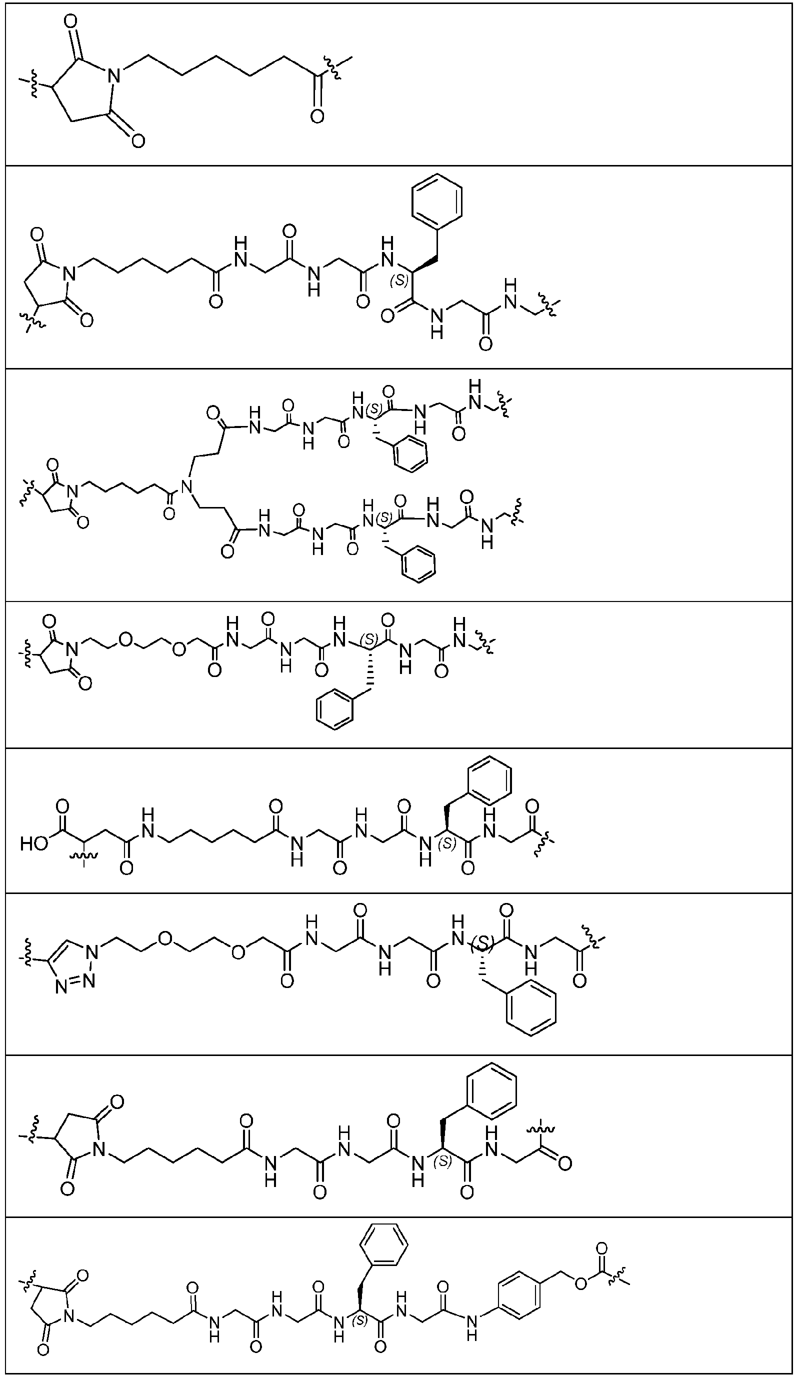

29. The conjugate compound or the pharmaceutically acceptable salt thereof according to any one of claims 1-28, wherein the payload is selected from the group of: a small molecule compound, a nucleotide and a peptide, and preferably, the payload is a small molecule compound.

30. The conjugate compound or the pharmaceutically acceptable salt thereof according to claim 29, wherein the small molecular compound is selected from the group of: camptothecin and any derivative thereof, maytansine and any derivative thereof, a radioactive metal complex, a cyclooxygenase-2 inhibitor and any derivative thereof, paclitaxel and any derivative thereof, epothilone and any derivative thereof, bleomycin and any derivative thereof, dactinomycin and any derivative thereof, plicamycin and any derivative thereof, and mitomycin C and any derivative thereof, preferably, the small molecular compound is camptothecin and any derivative thereof, and preferably, the camptothecin is exatecan.

31. The conjugate compound or the pharmaceutically acceptable salt thereof according to any one of claims 1-30, wherein each D is independently selected from 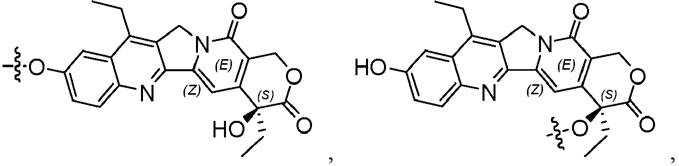 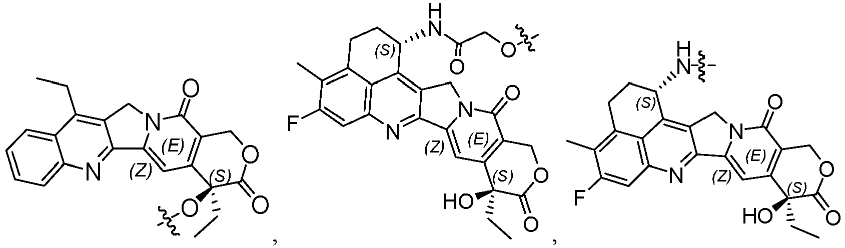 and

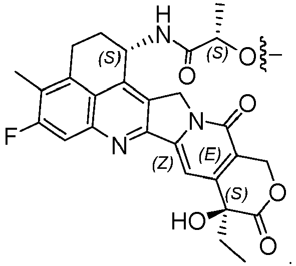

32. The conjugate compound or the pharmaceutically acceptable salt thereof according to any one of claims 1-31, wherein the cell surface molecule is selected from TRPV6, FOLR1, PSMA and SSTR2.

33. The conjugate compound or the pharmaceutically acceptable salt thereof according to any one of claims 1-32, wherein the cell surface molecule is TRPV6 and FOLR1, PSMA and FOLR1, or SSTR2 and FOLR1, respectively.

34. The conjugate compound or the pharmaceutically acceptable salt thereof according to any one of claims 1-33, wherein the cell surface molecule is TRPV6 and FOLR1, respectively.

35. The conjugate compound or the pharmaceutically acceptable salt thereof according to any one of claims 32-34, wherein the ligand targeting FOLR1 is selected from folic acid or an analogue thereof, preferably, the folic acid analogue is selected from 5-methyltetrahydrofolate, 5-formyltetrahydrofolate, methotrexate, and 5,10-methylenetetrahydrofolate, wherein the ligand targeting SSTR2 is selected from octreotide and any analogue thereof.

36. The conjugate compound or the pharmaceutically acceptable salt thereof according to any one of claims 1-32, wherein the ligand moiety comprises more than three ligands targeting cell surface molecules, and preferably, the cell surface molecules are selected from TRPV6, FOLR1, PSMA and SSTR2.

37. The conjugate compound or the pharmaceutically acceptable salt thereof according to any one of claims 32-36, wherein the ligands targeting different cell surface molecules are linked to each other directly, or via a spacer.

38. The conjugate compound or the pharmaceutically acceptable salt thereof according to claim 37, wherein the spacer consists of amino acids; preferably, the spacer consists of natural amino acids or unnatural amino acids; and preferably, the spacer is glycine.

39. The conjugate compound or the pharmaceutically acceptable salt thereof according to any one of claims 37-38, wherein the spacer is cleavable under a certain physiological environment by a protease or by reduction.

40. The conjugate compound or the pharmaceutically acceptable salt thereof according to any one of claims 1-39, wherein the ligand moiety has the following structure:

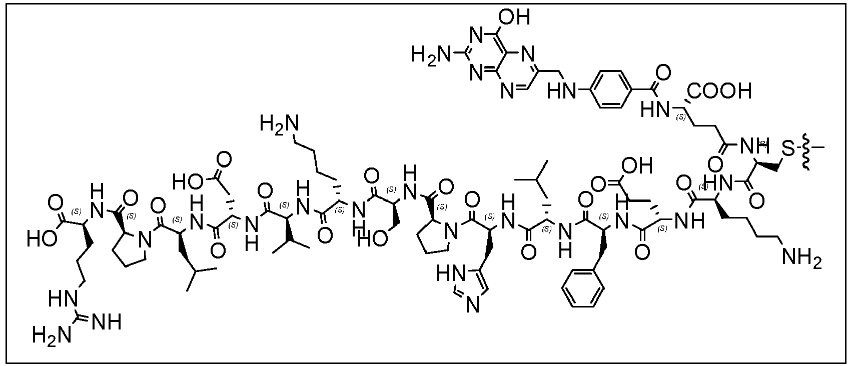

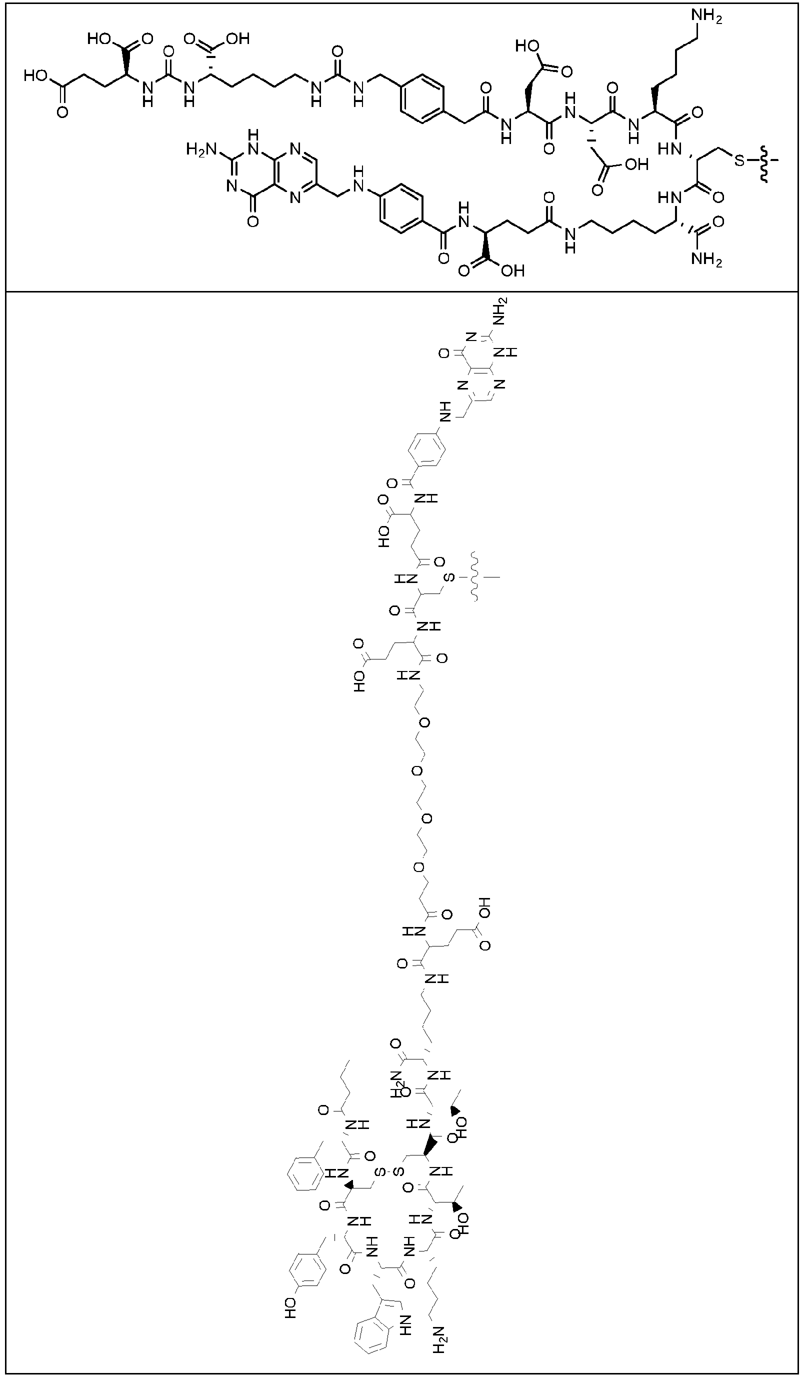

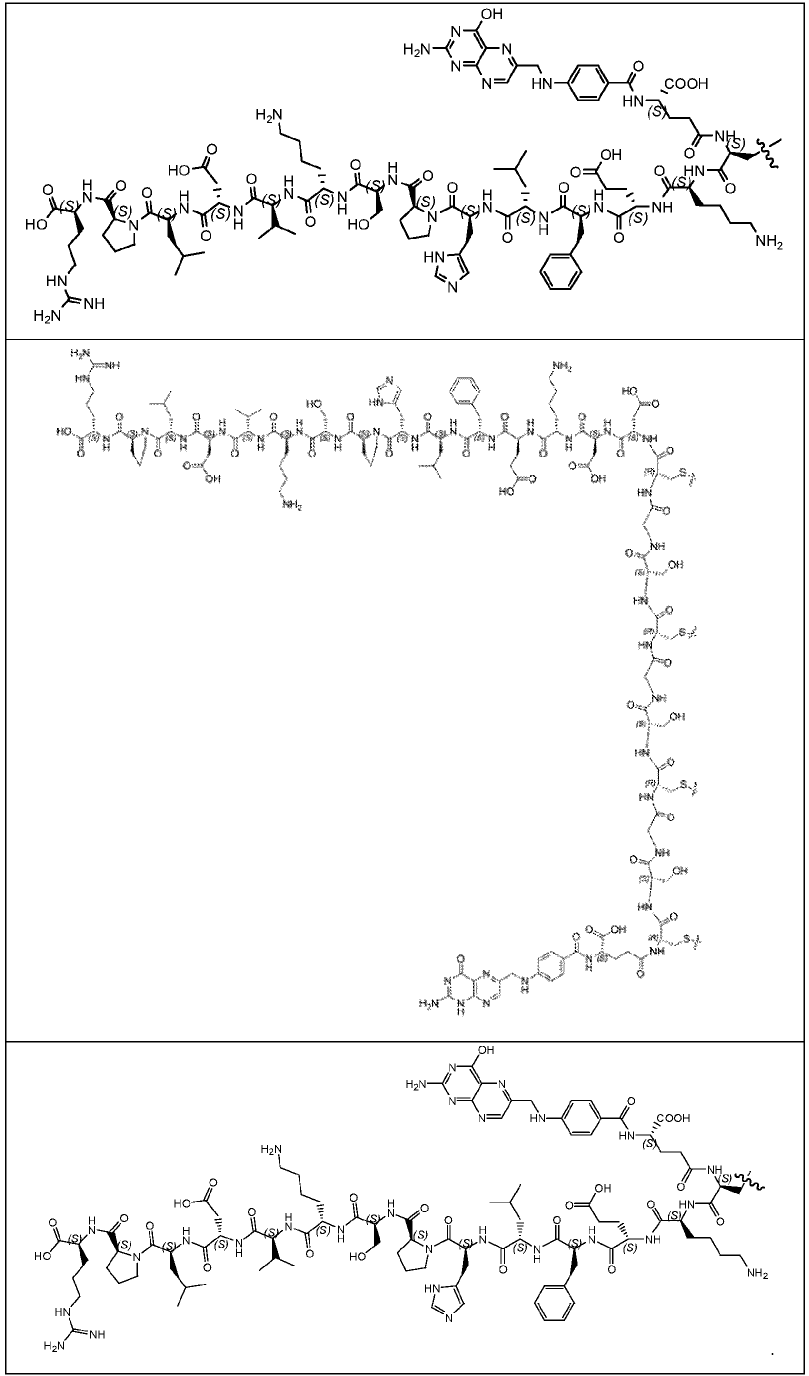

41. The conjugate compound or the pharmaceutically acceptable salt thereof according to any one of claims 1-40, wherein q is 1.

42. The conjugate compound or the pharmaceutically acceptable salt thereof according to any one of claims 1-41, wherein q is 2.

43. The conjugate compound or the pharmaceutically acceptable salt thereof according to any one of claims 1-42, wherein q is 3.

44. The conjugate compound or the pharmaceutically acceptable salt thereof according to any one of claims 1-43, wherein q is 4.

45. A conjugate compound or a pharmaceutically acceptable salt thereof, wherein the conjugate compound is selected from:

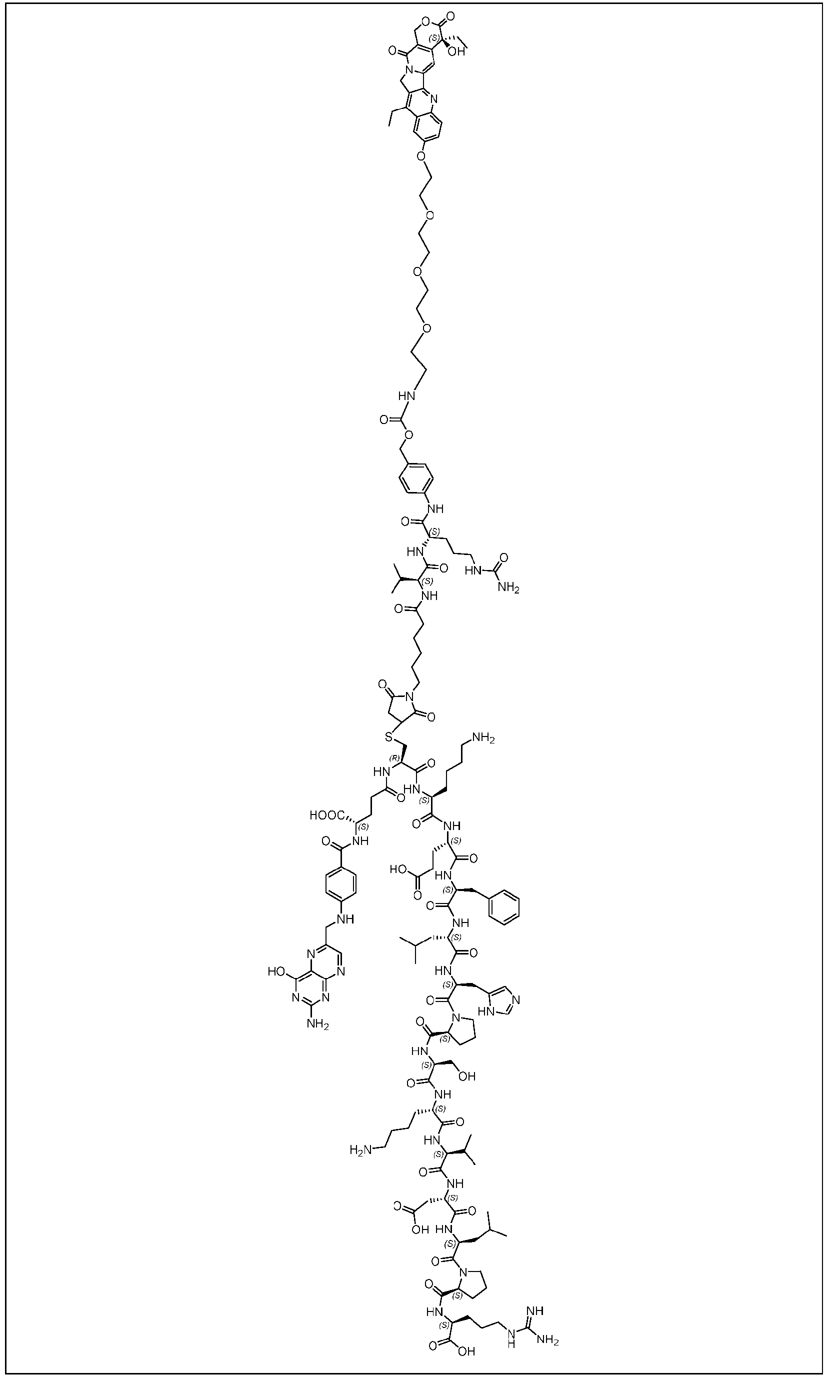

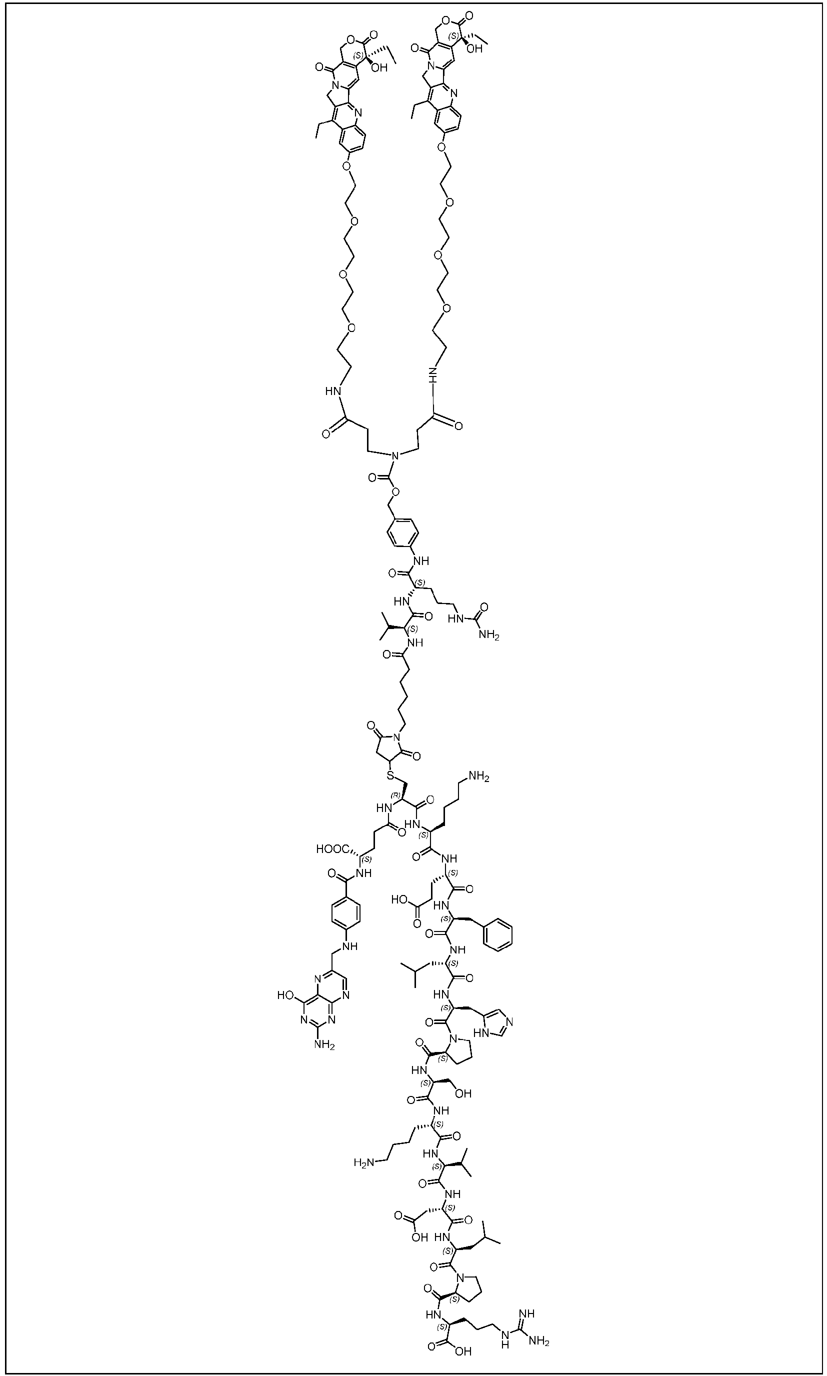

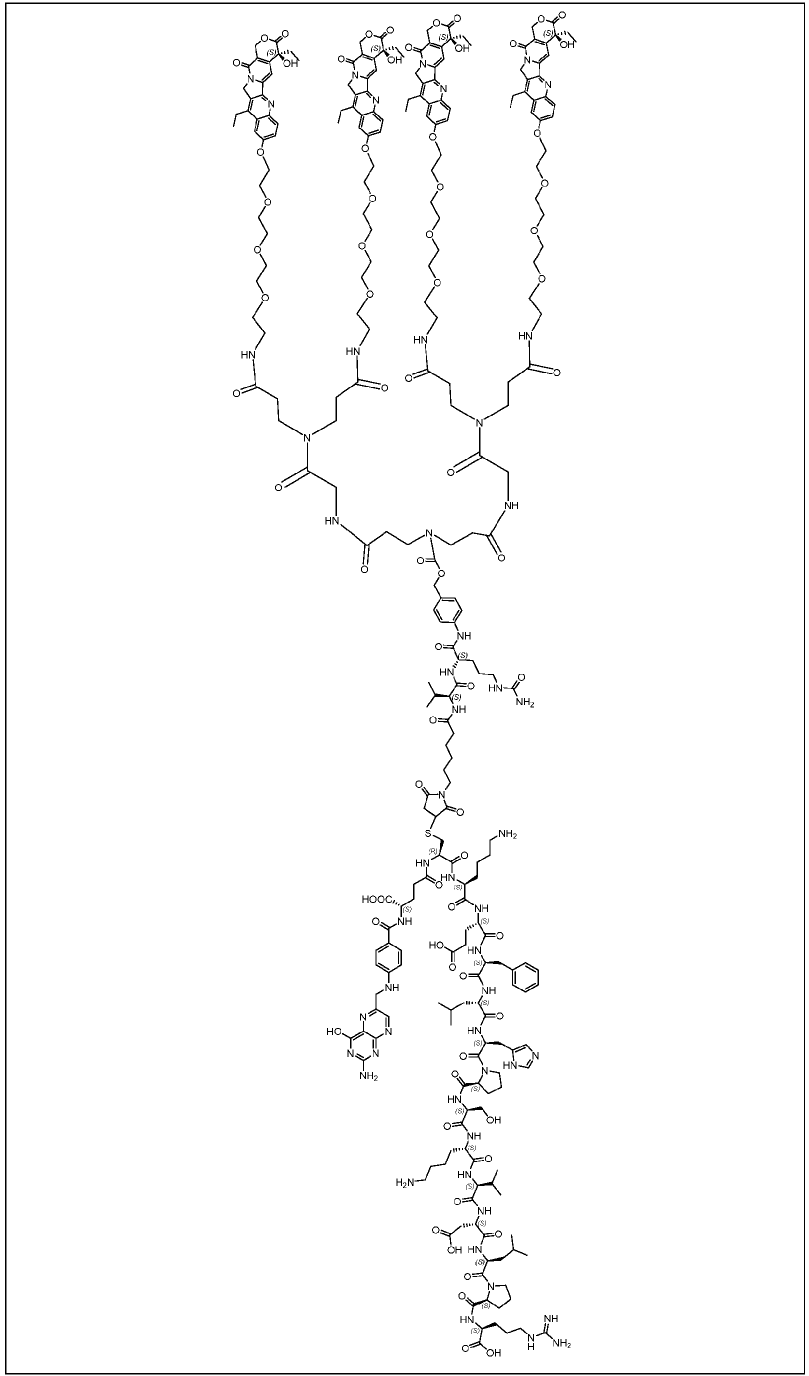

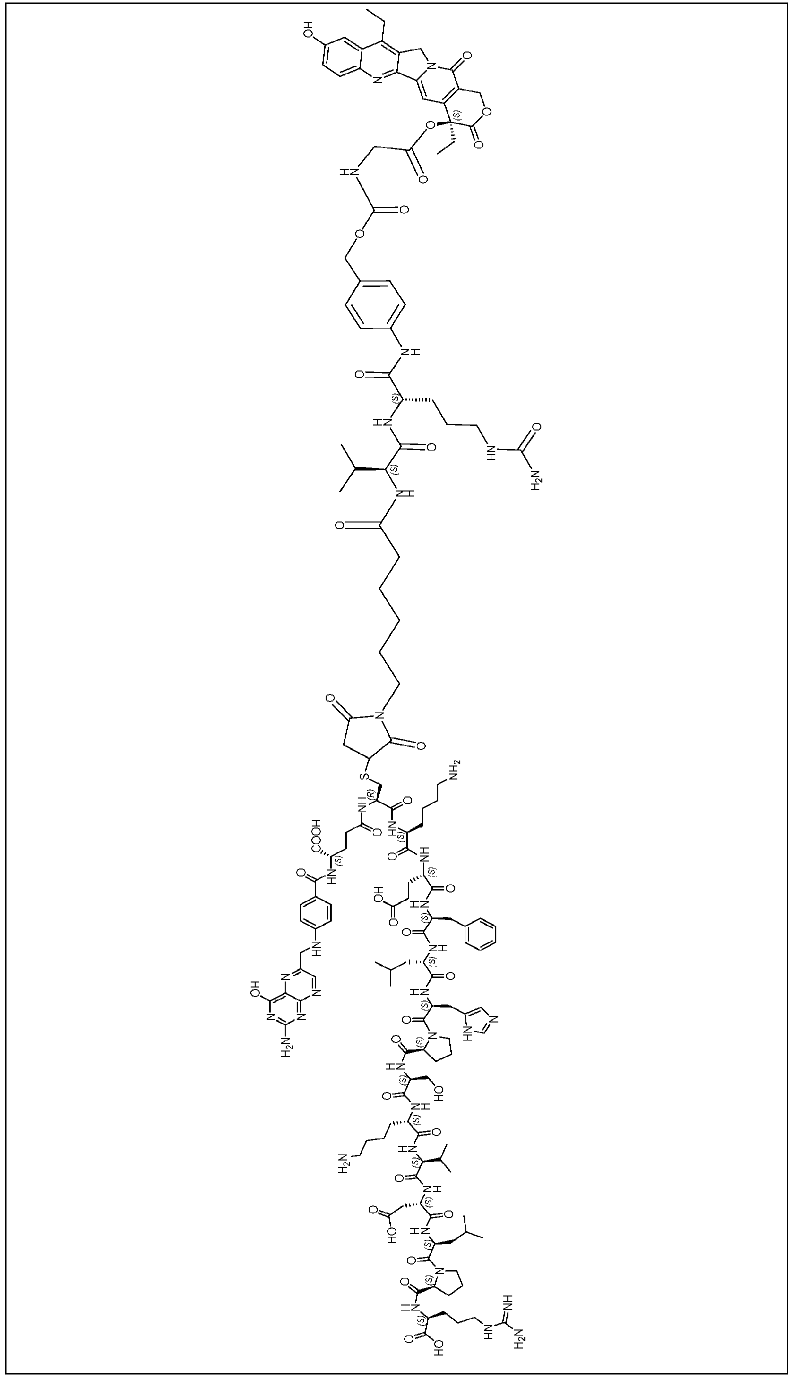

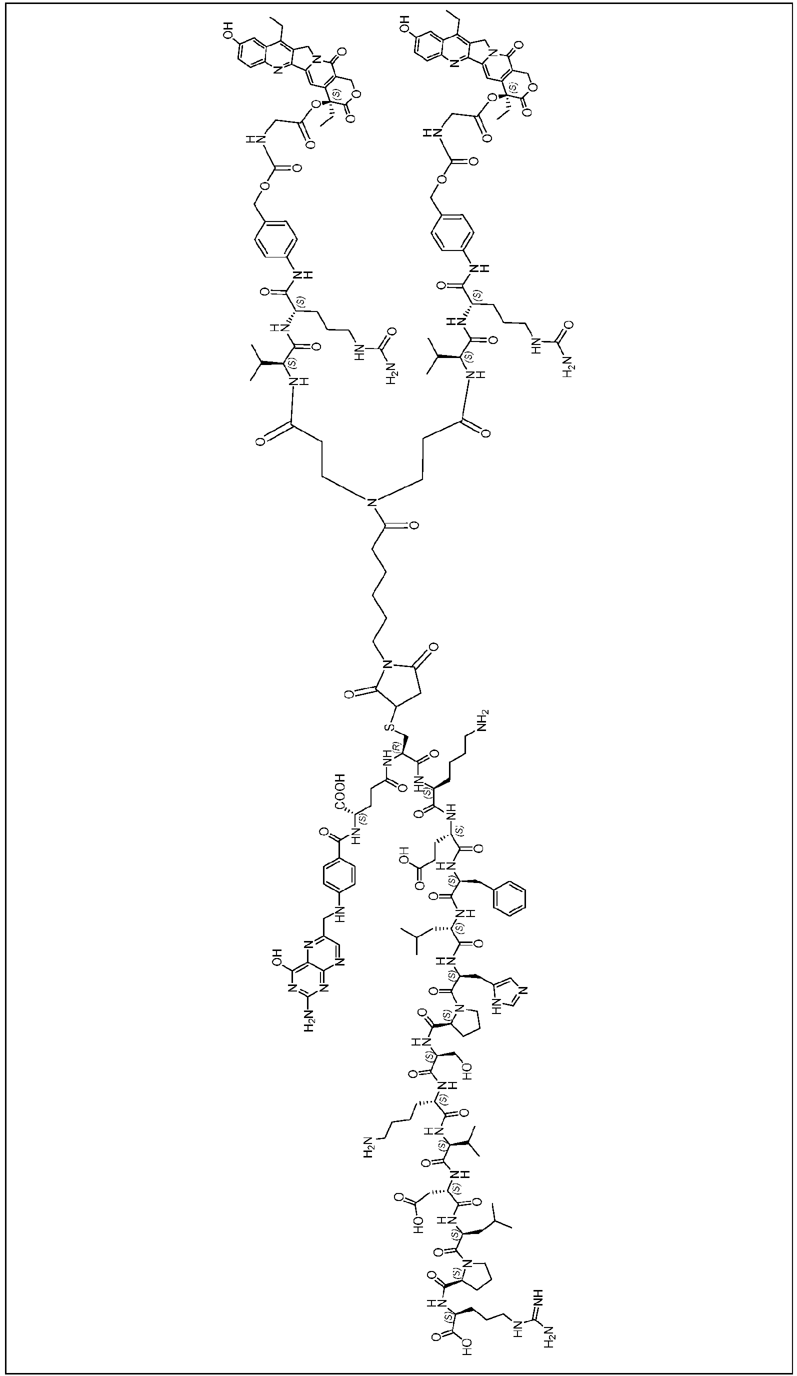

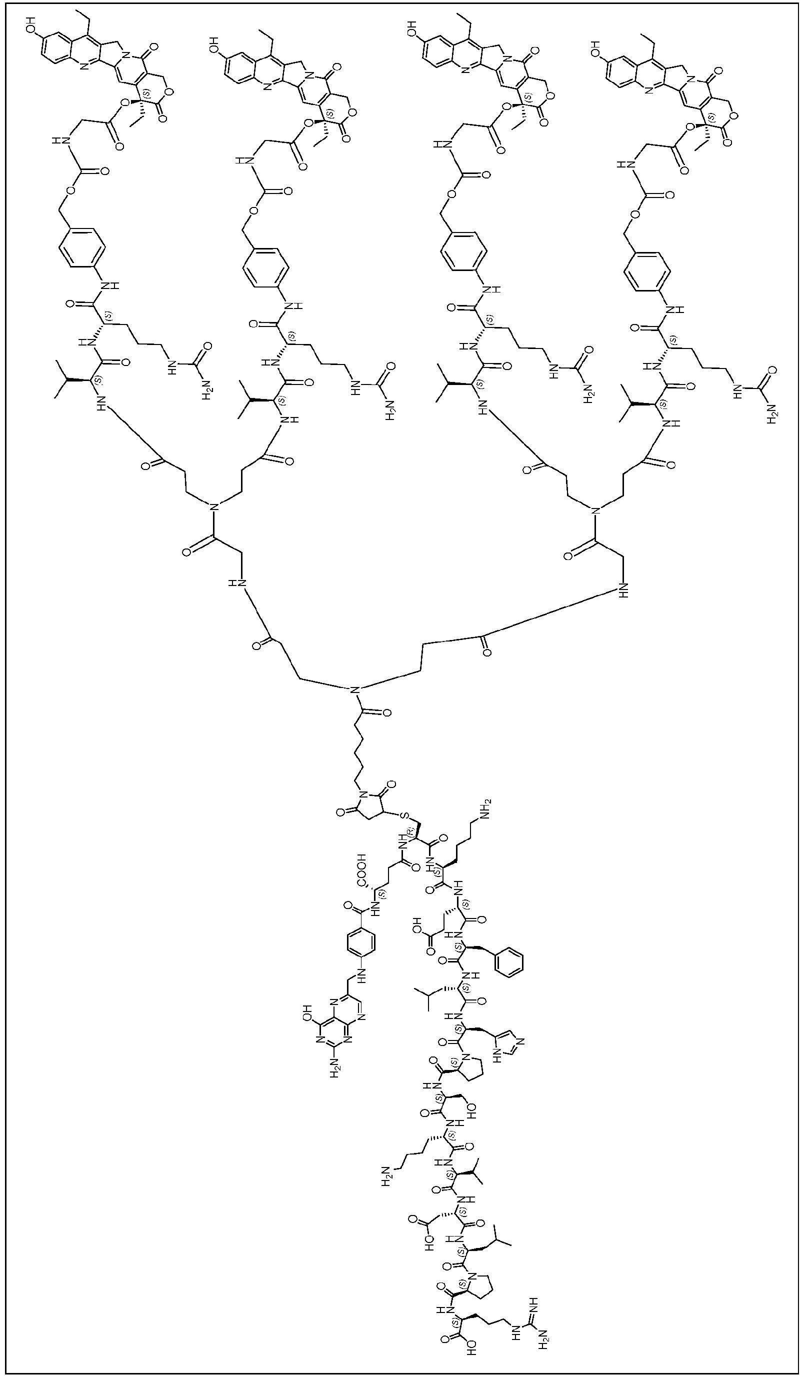

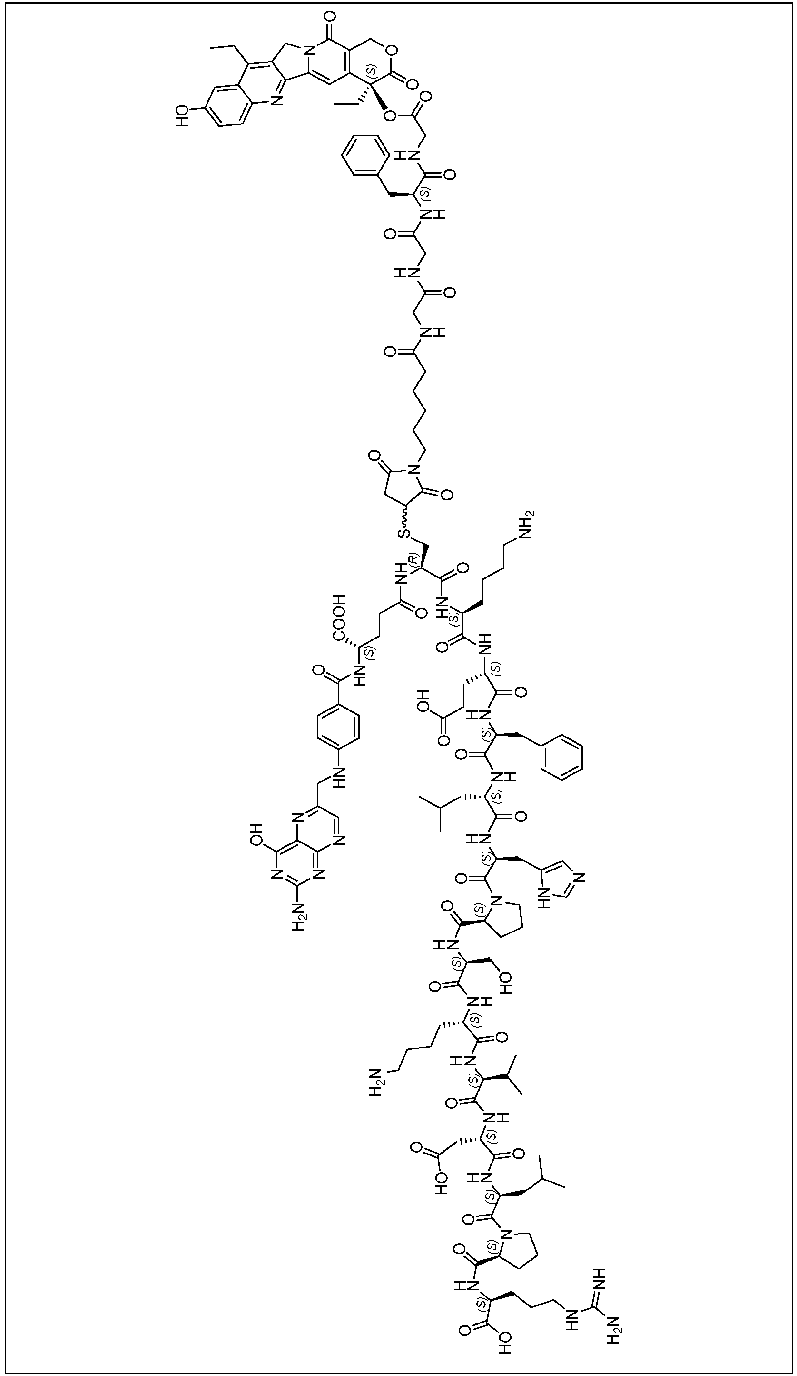

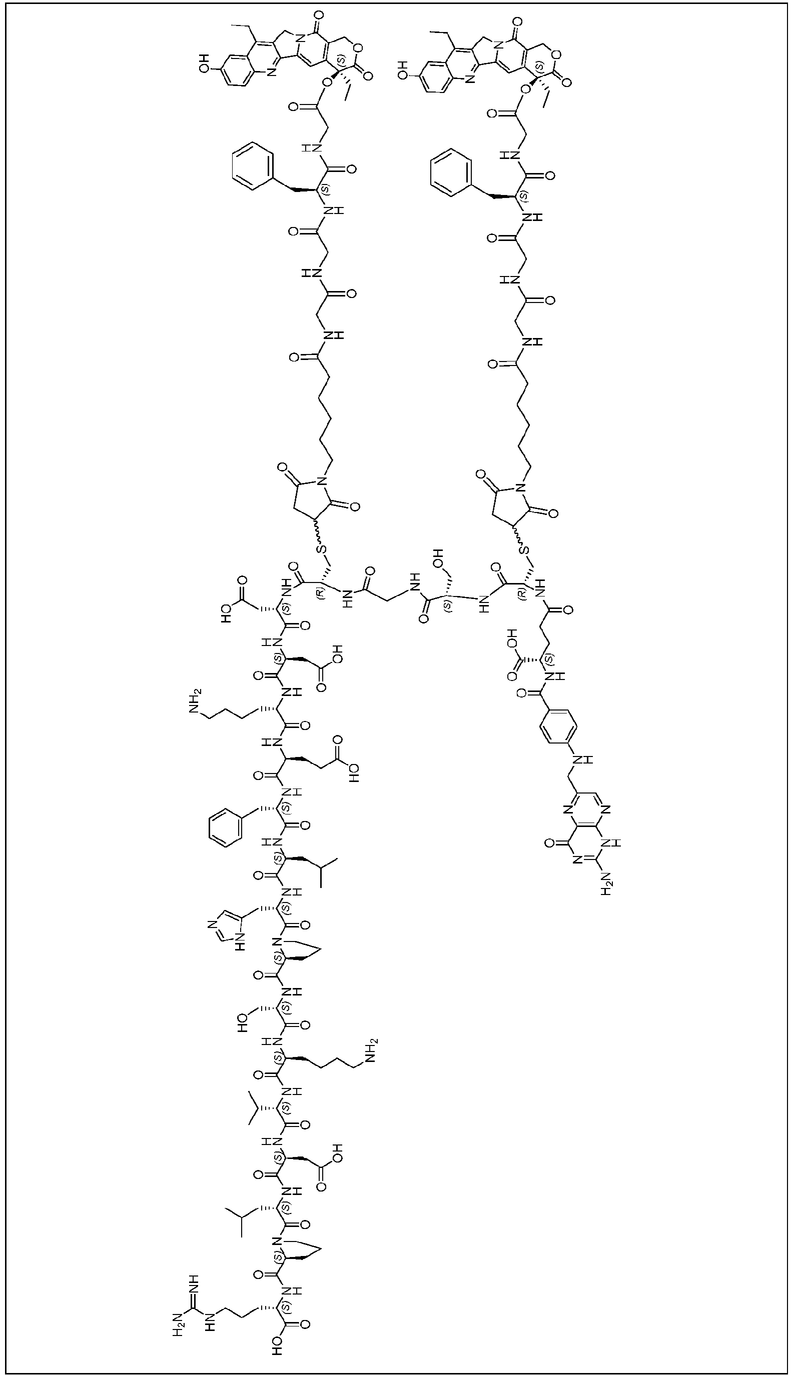

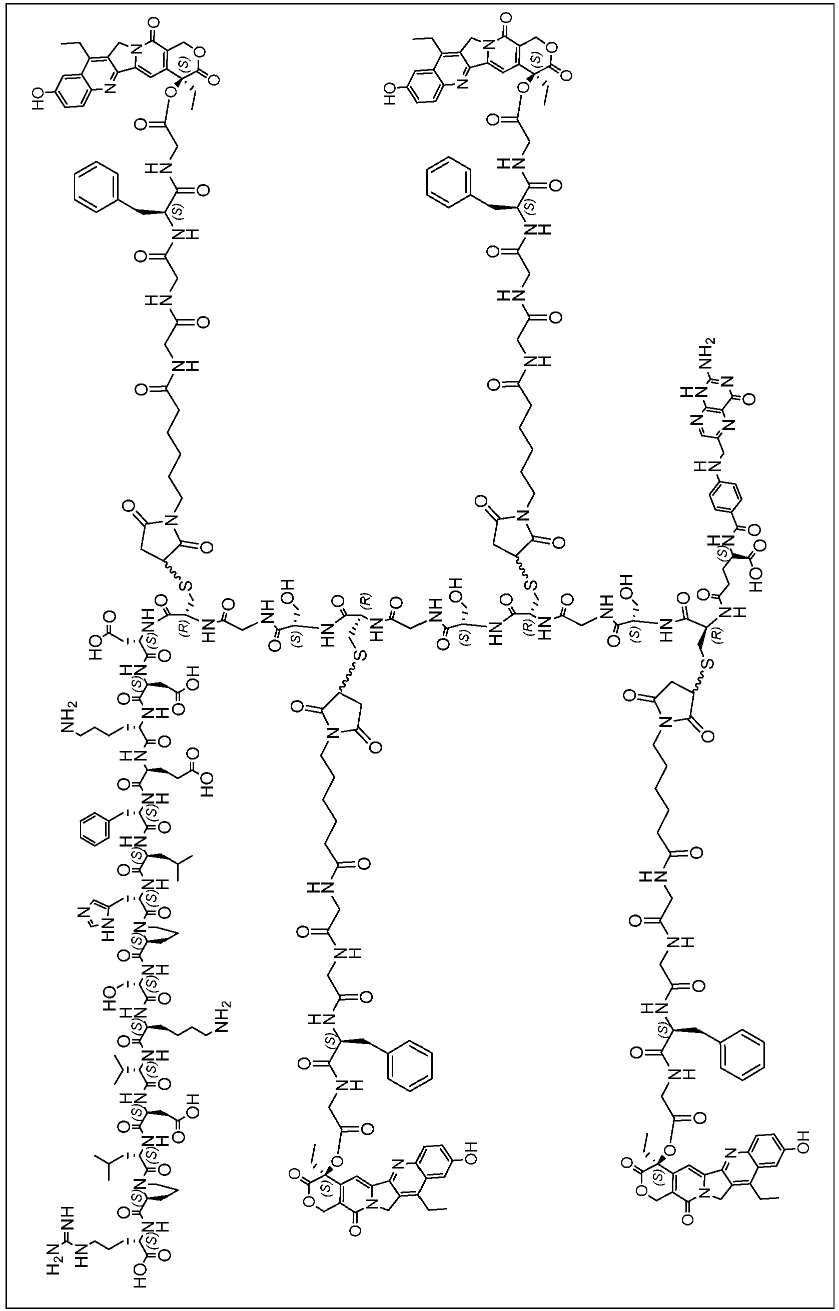

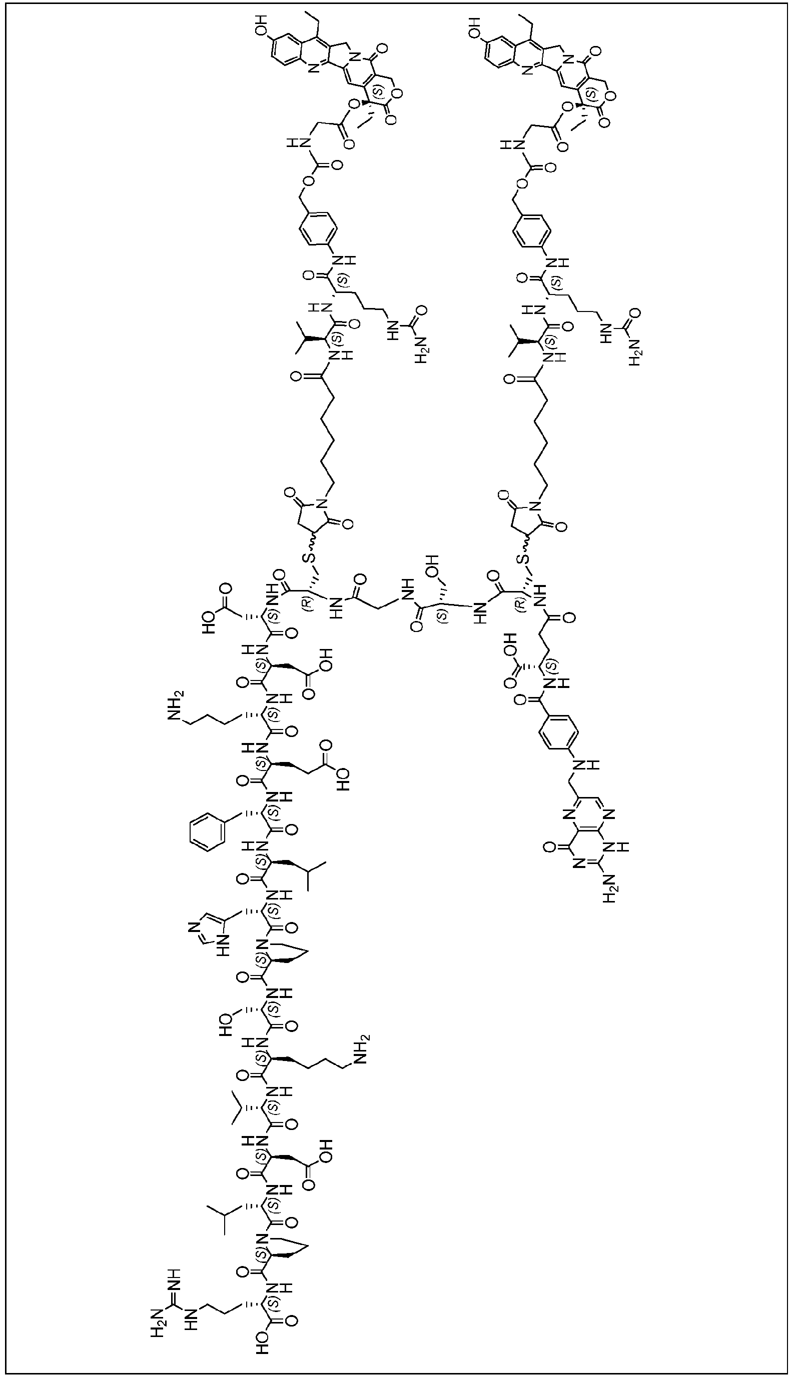

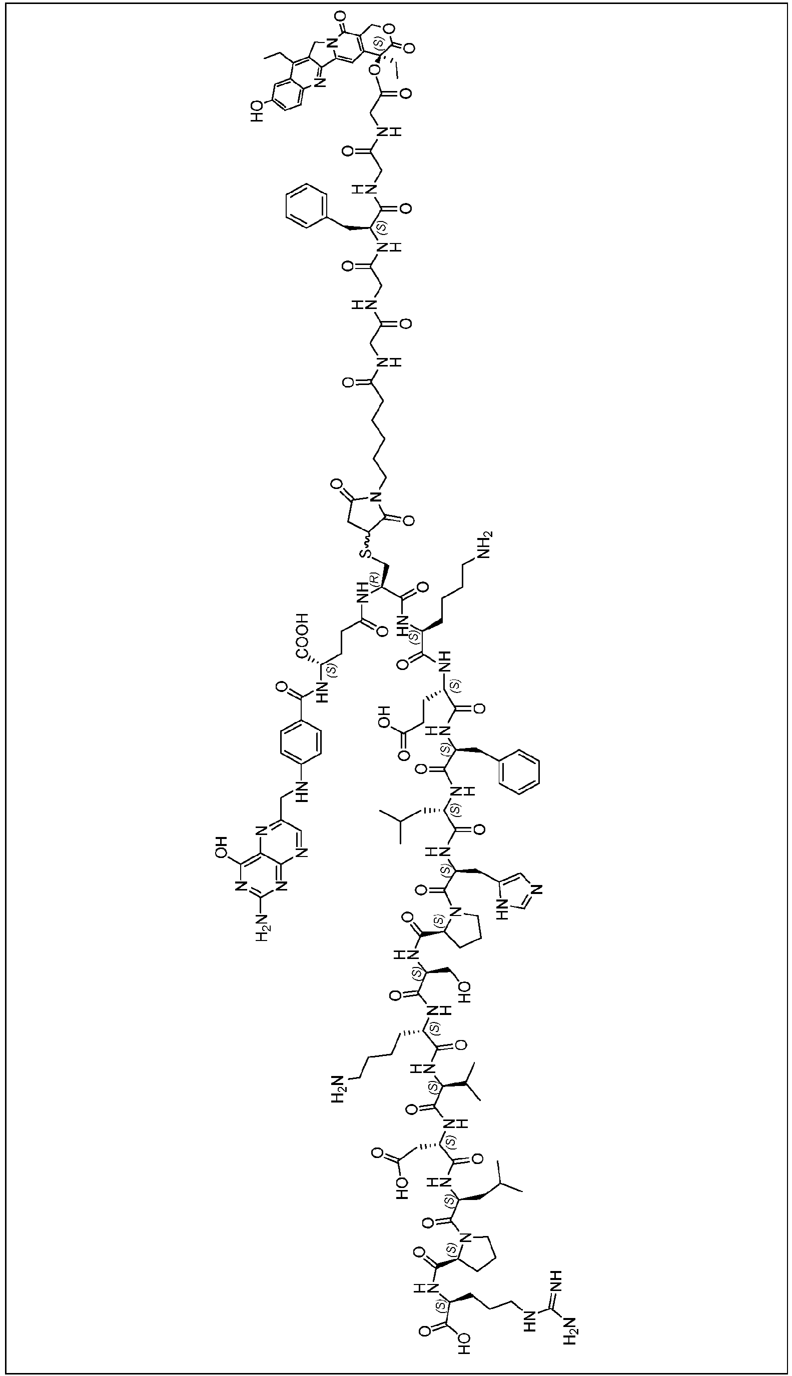

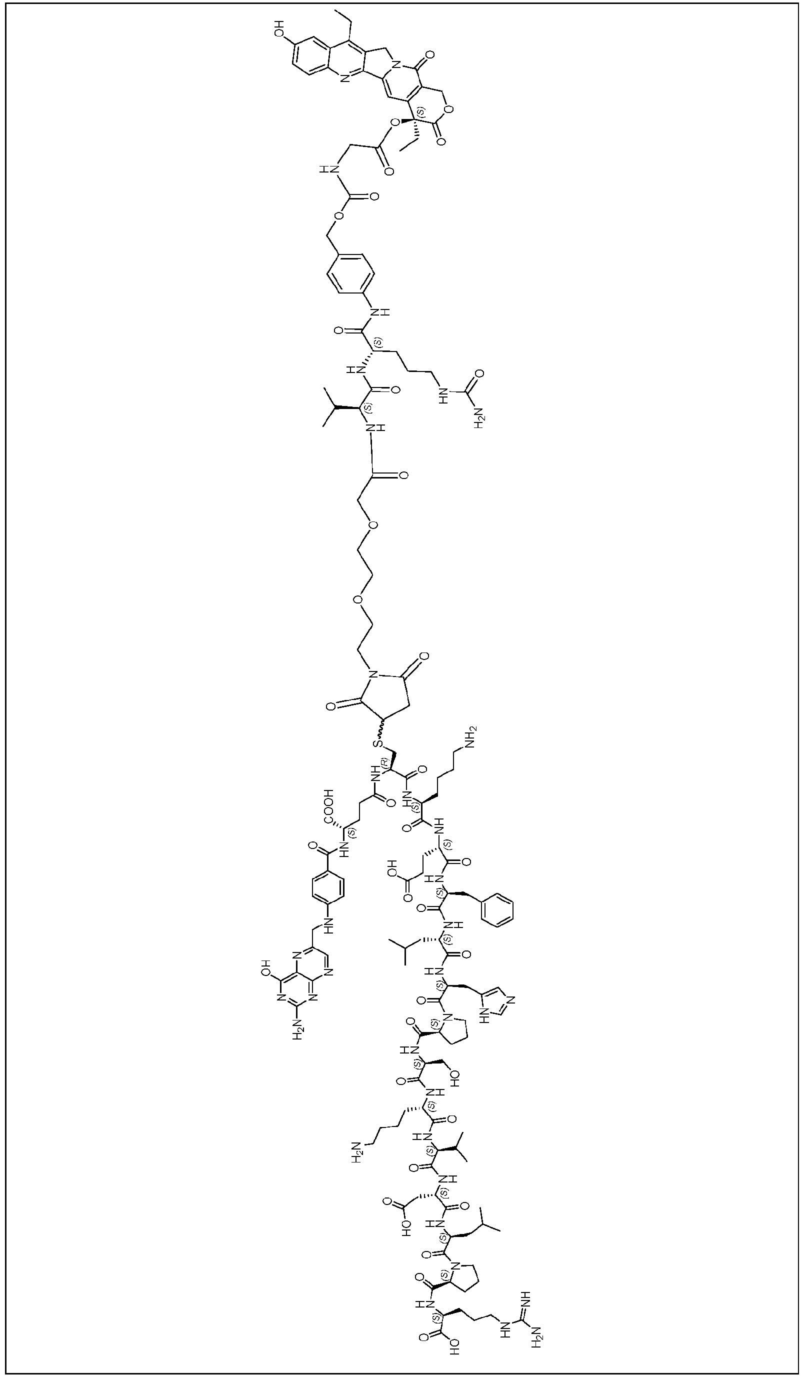

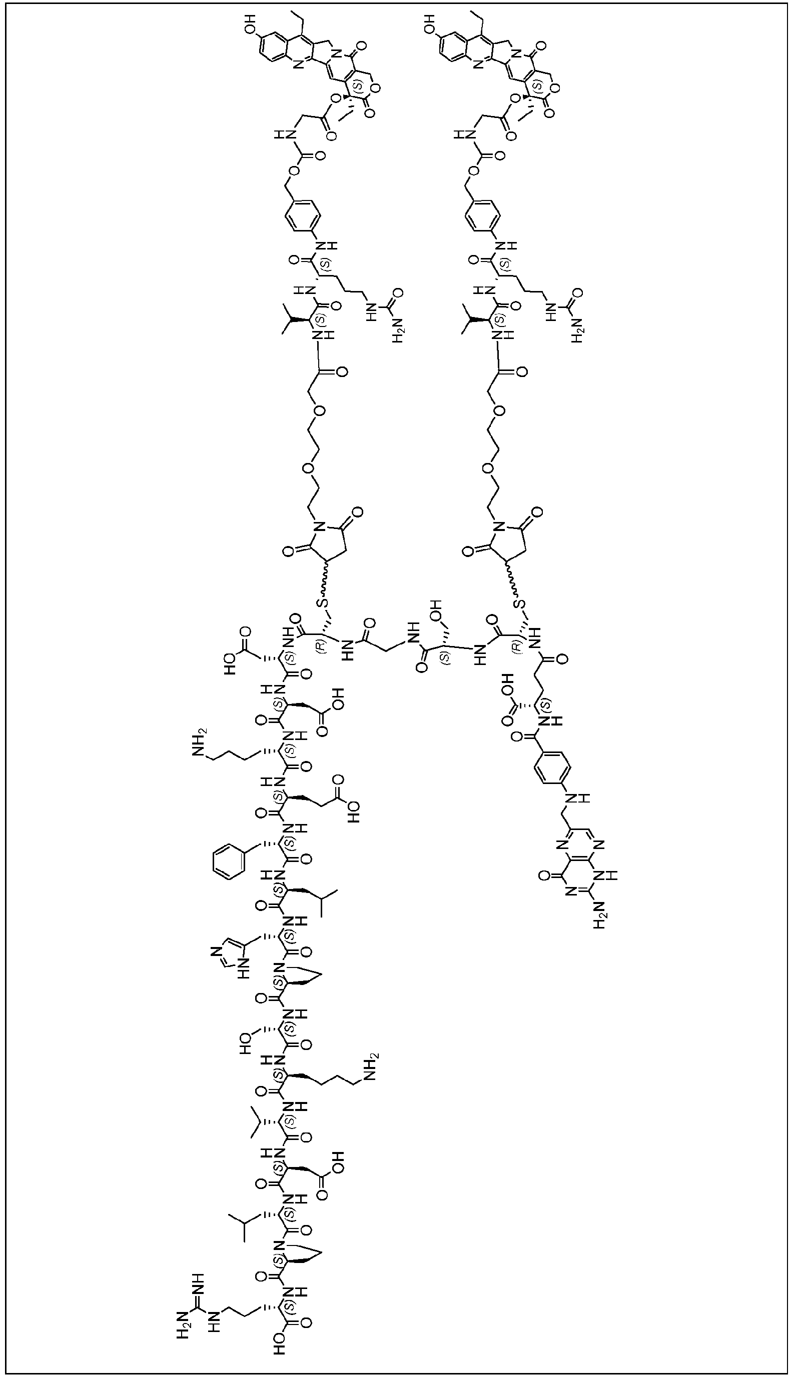

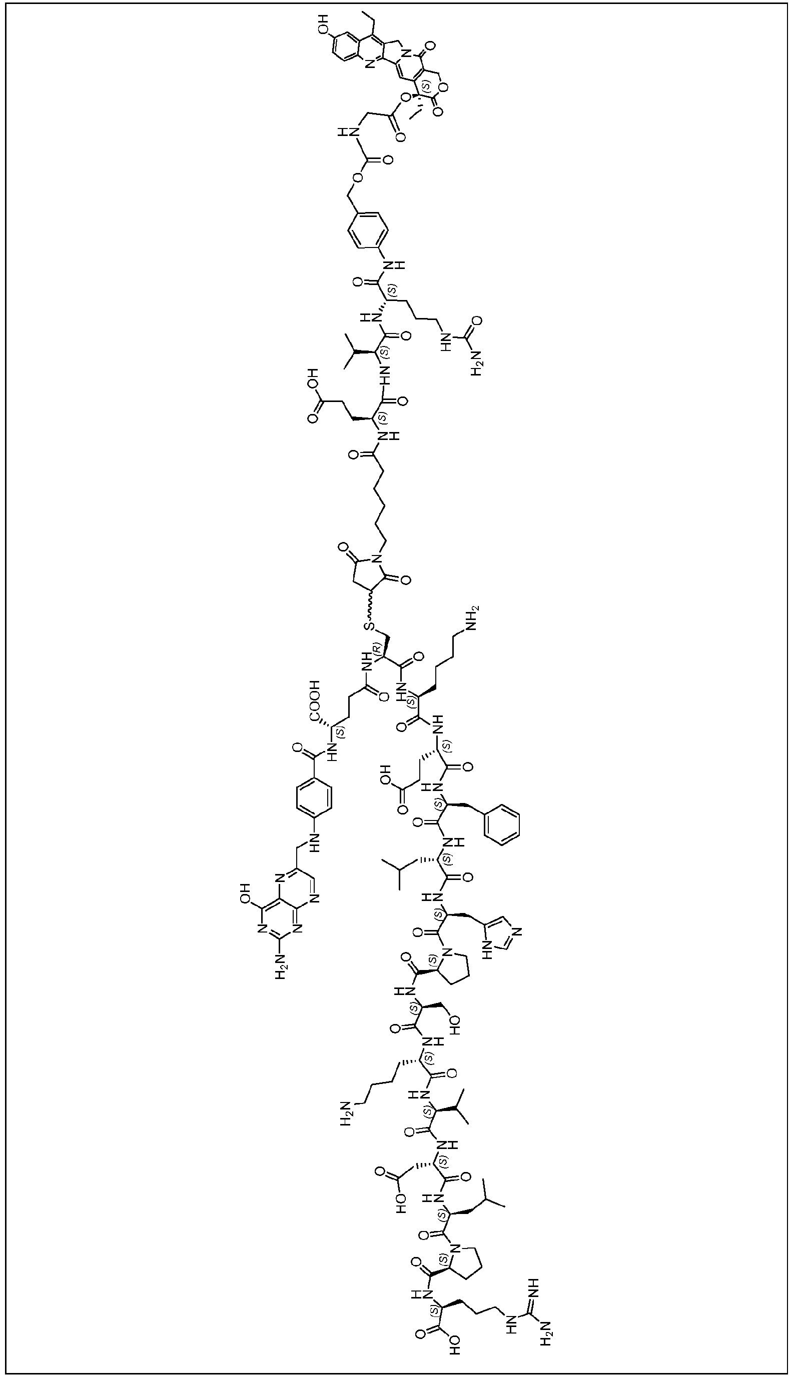

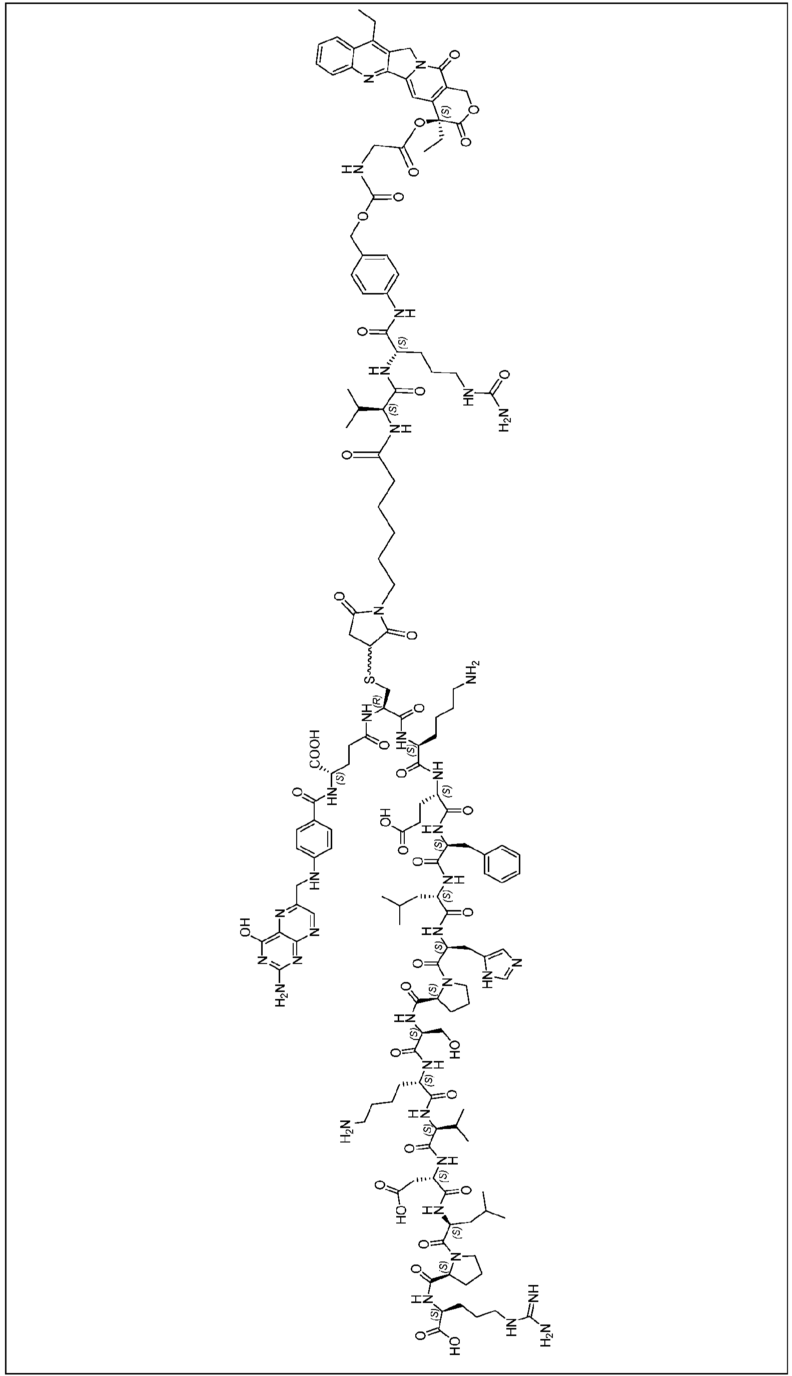

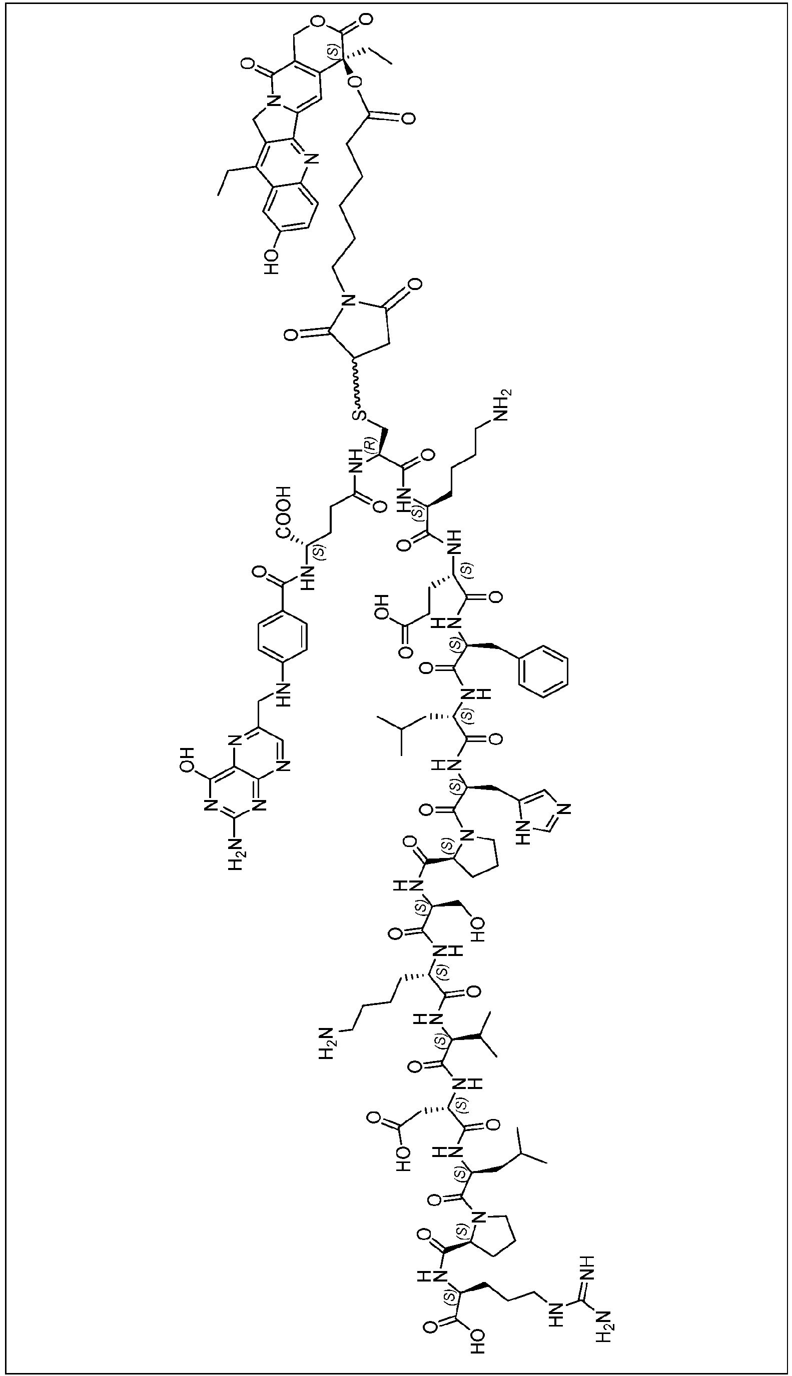

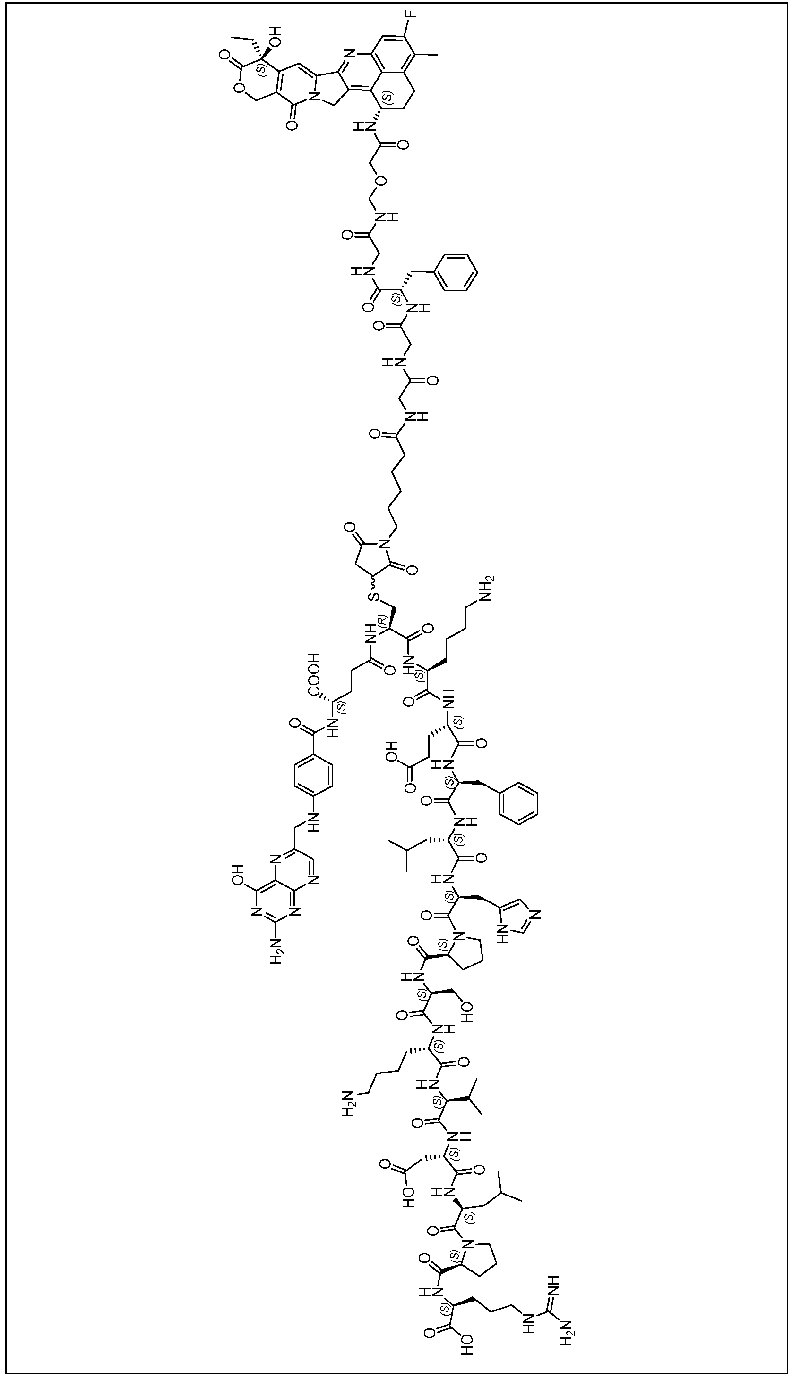

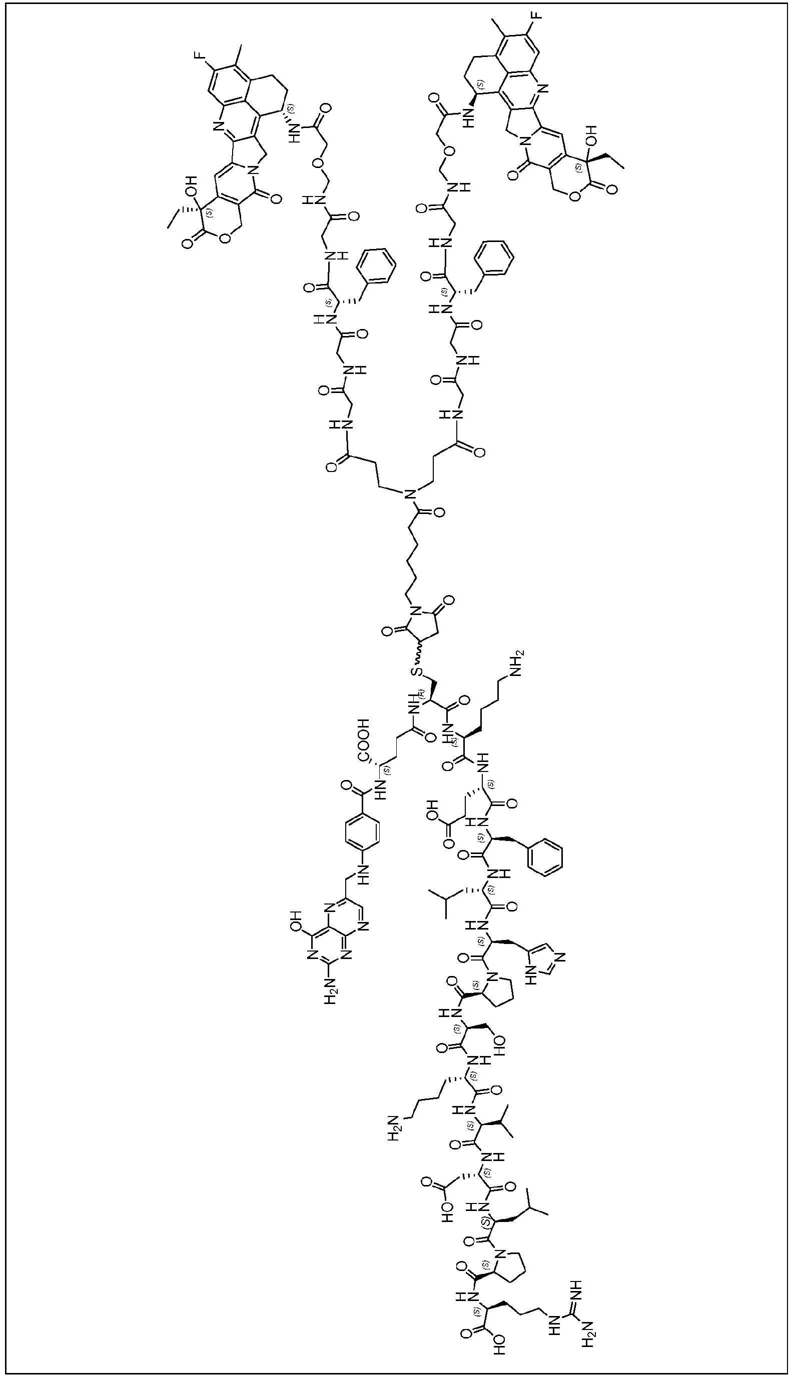

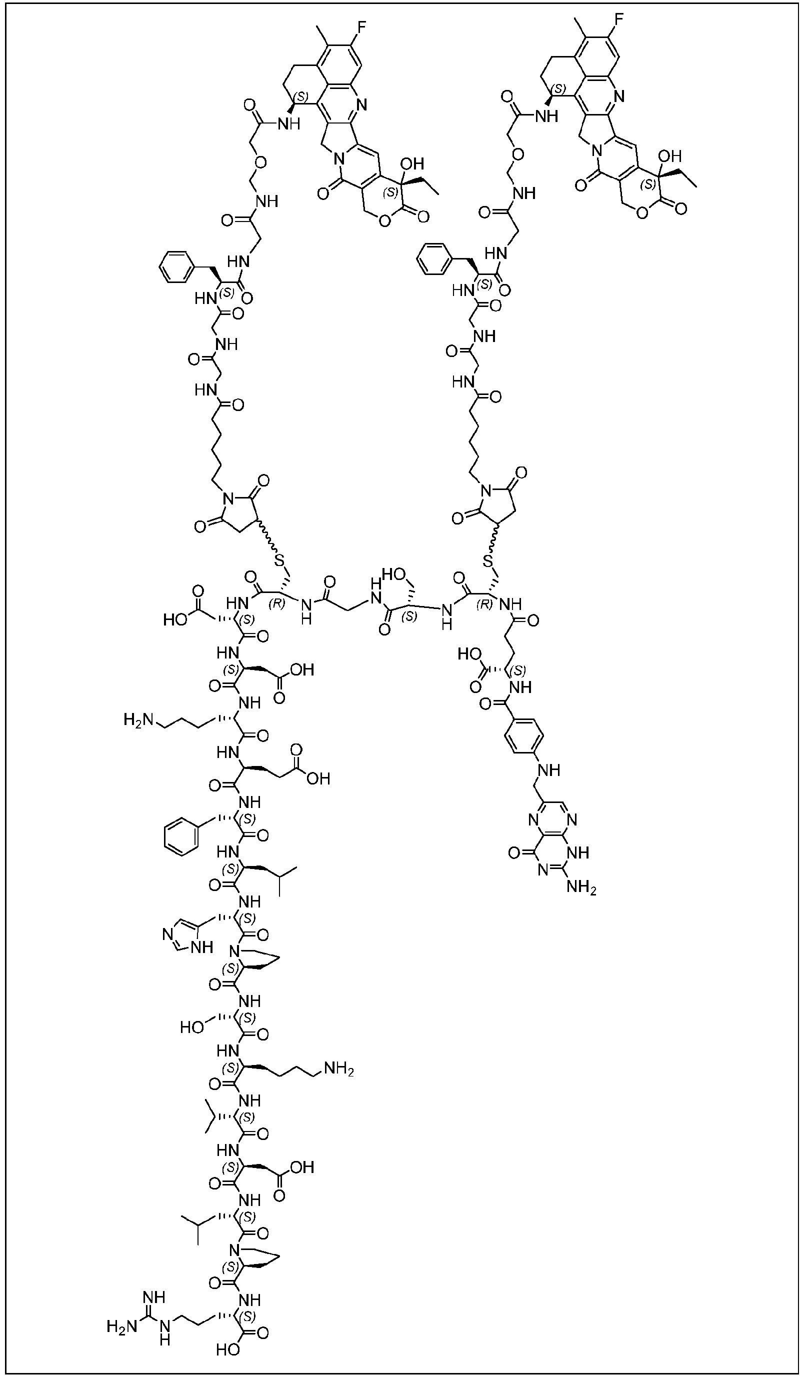

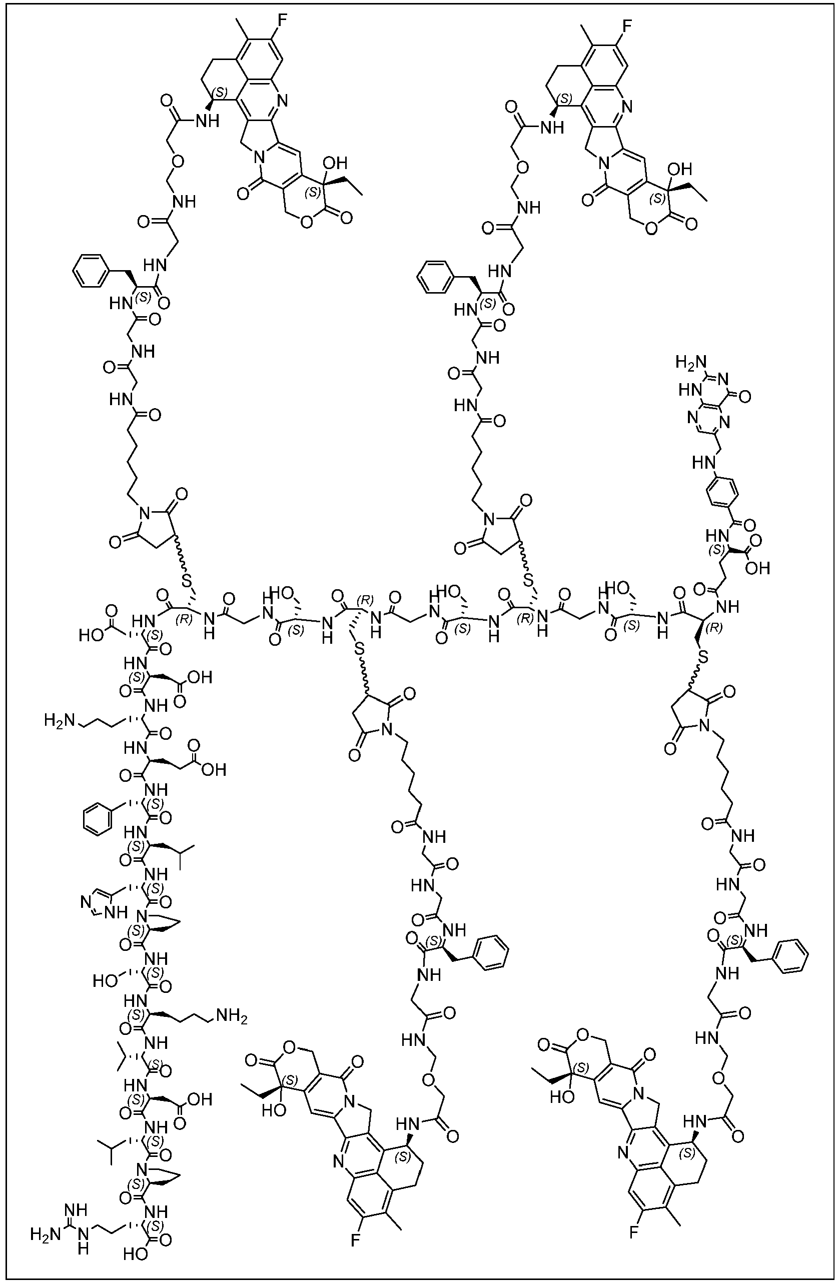

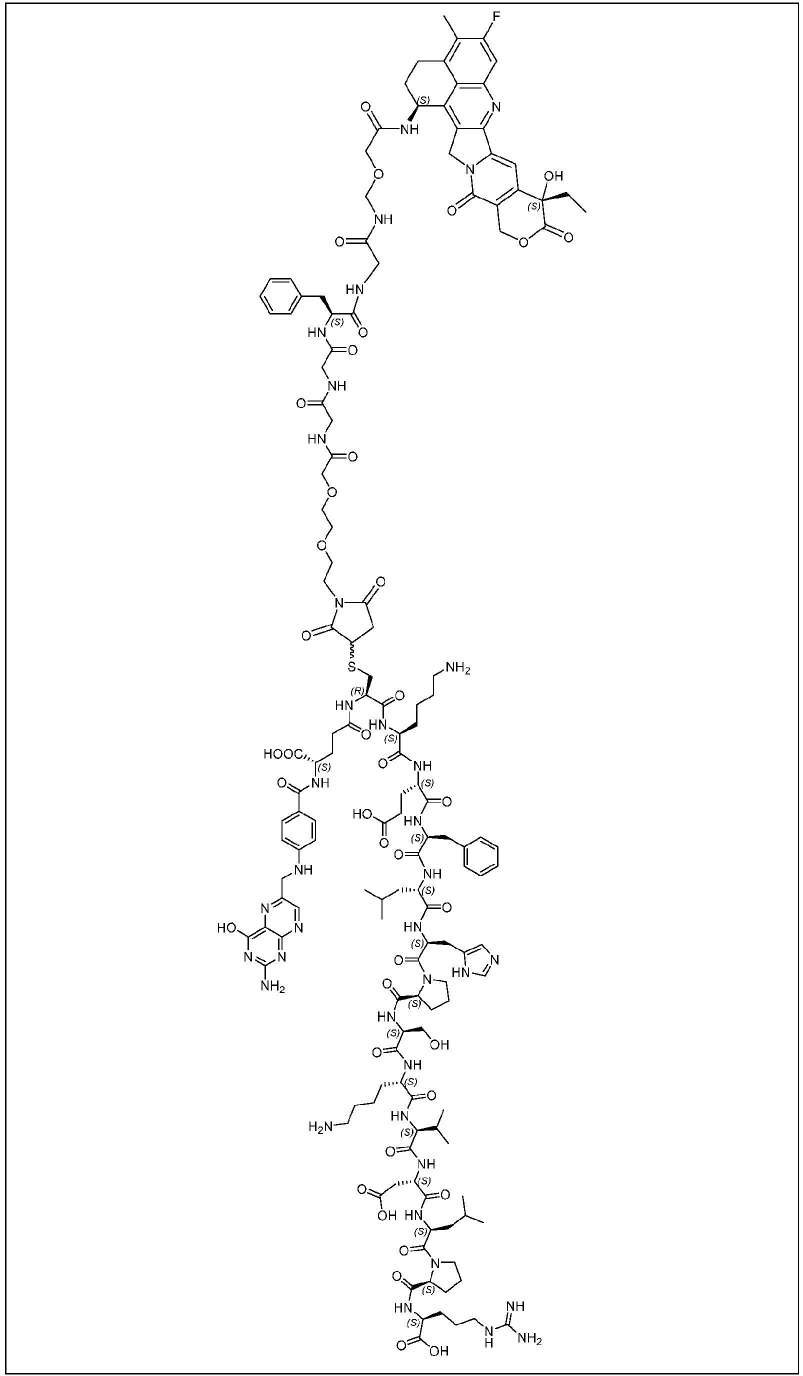

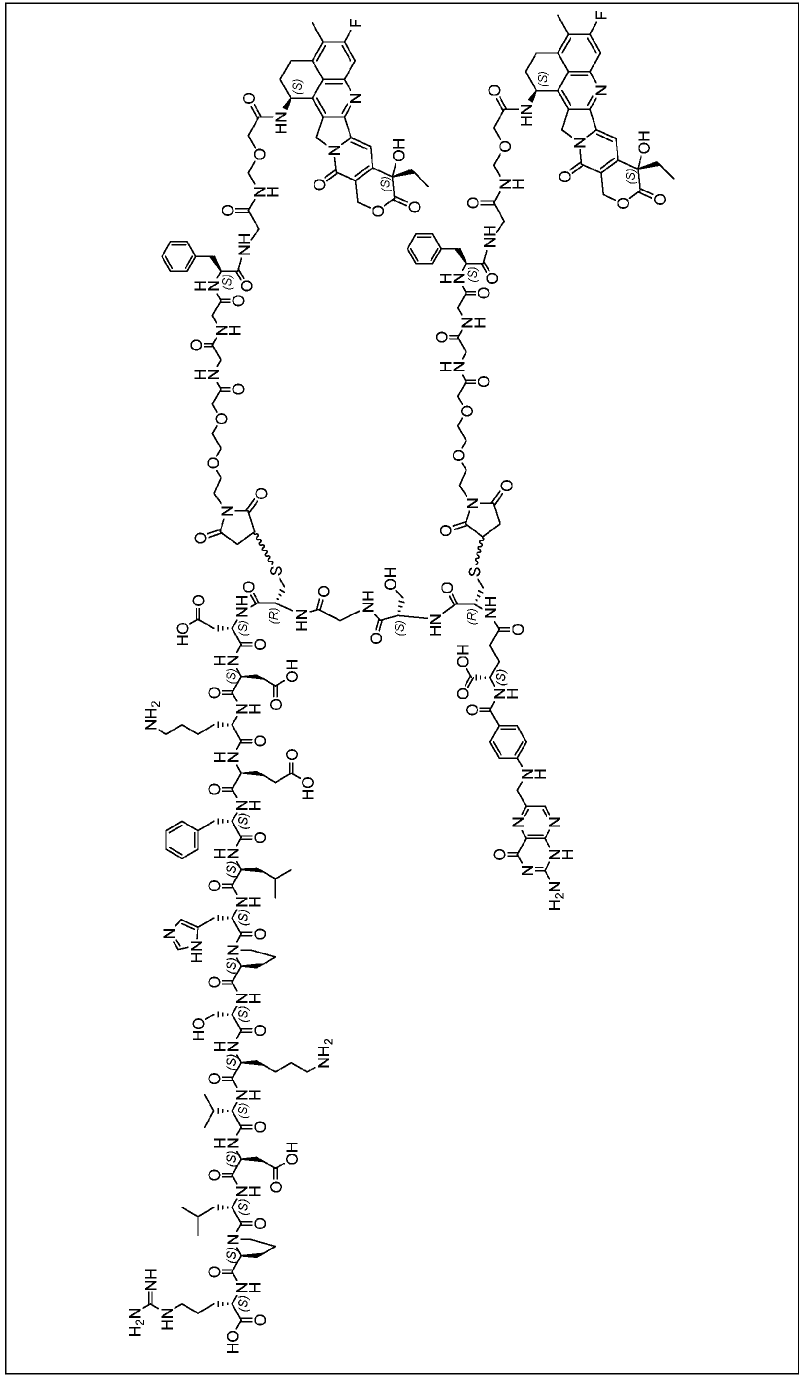

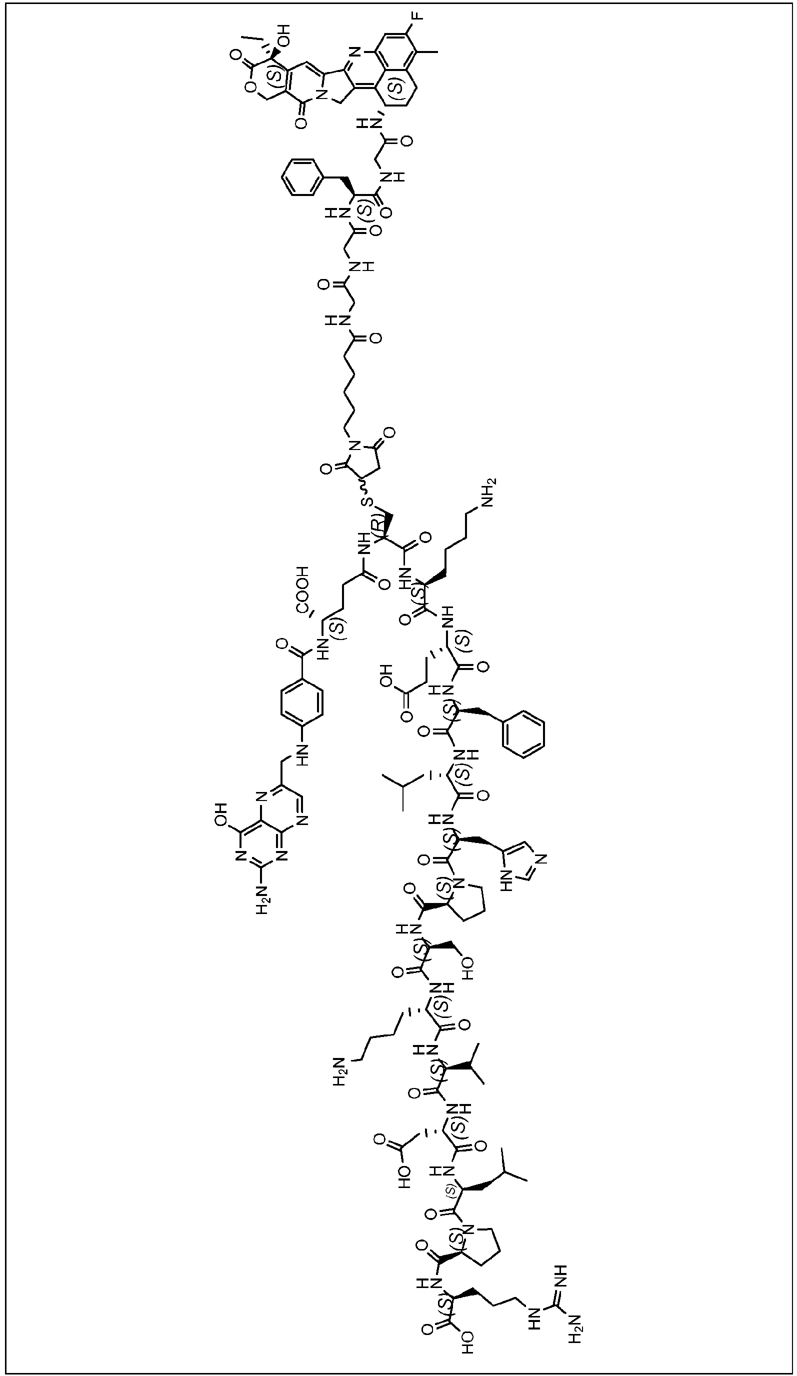

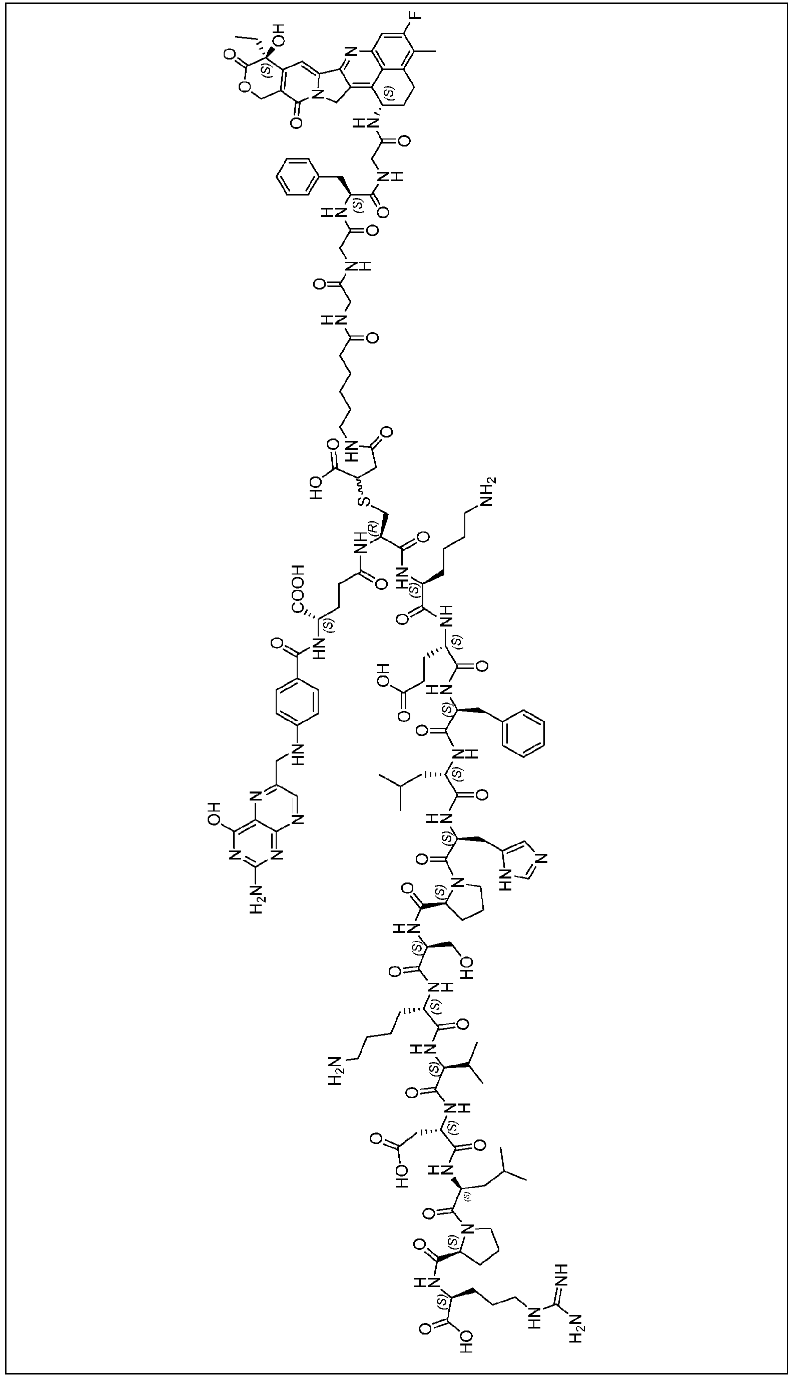

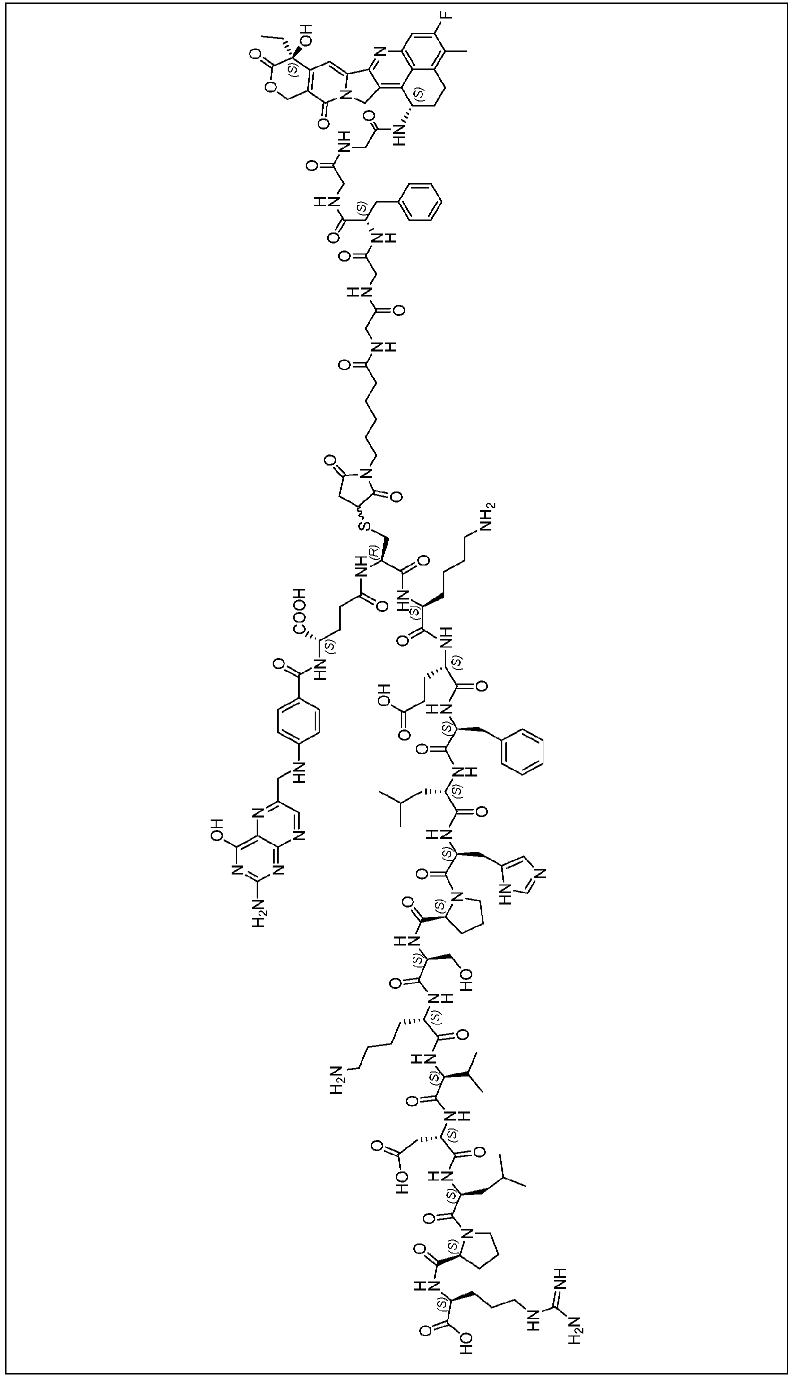

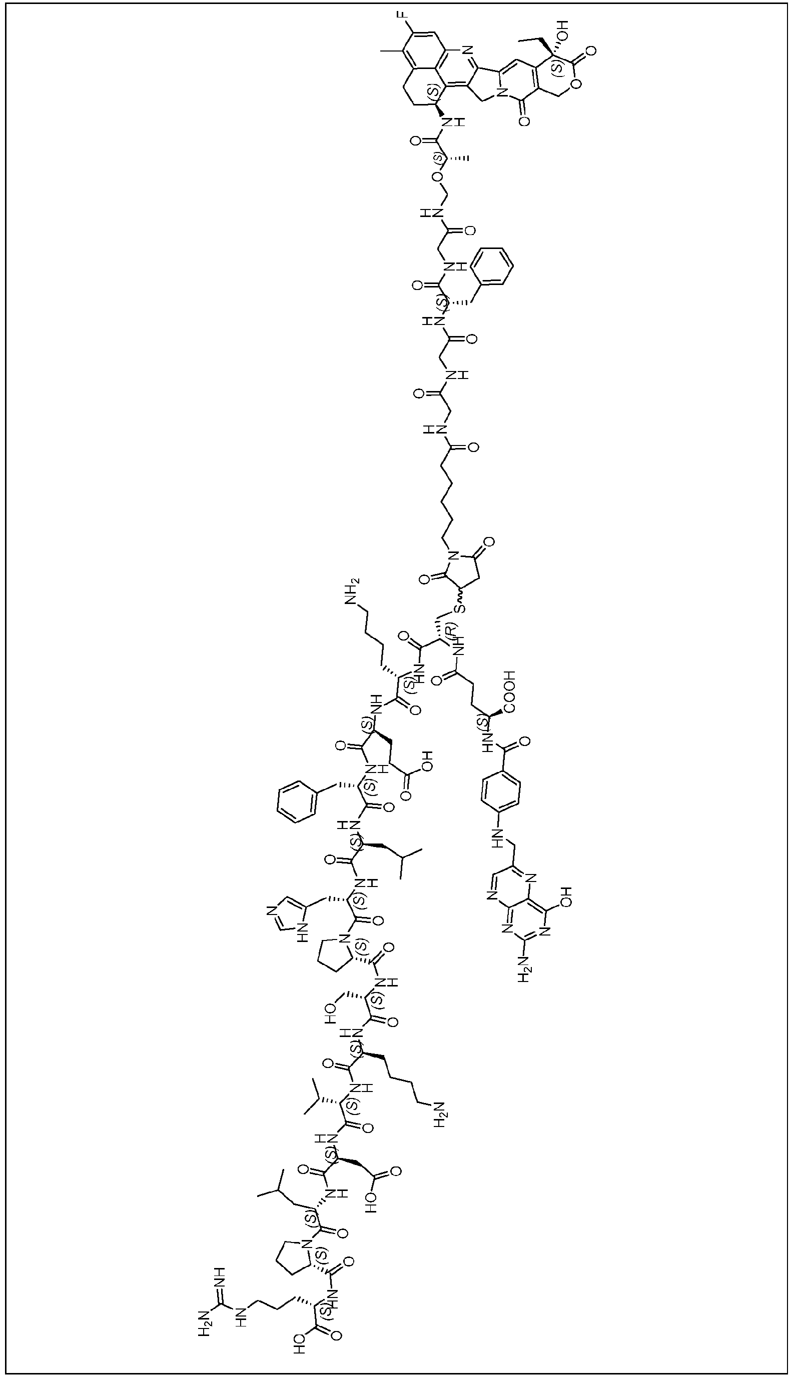

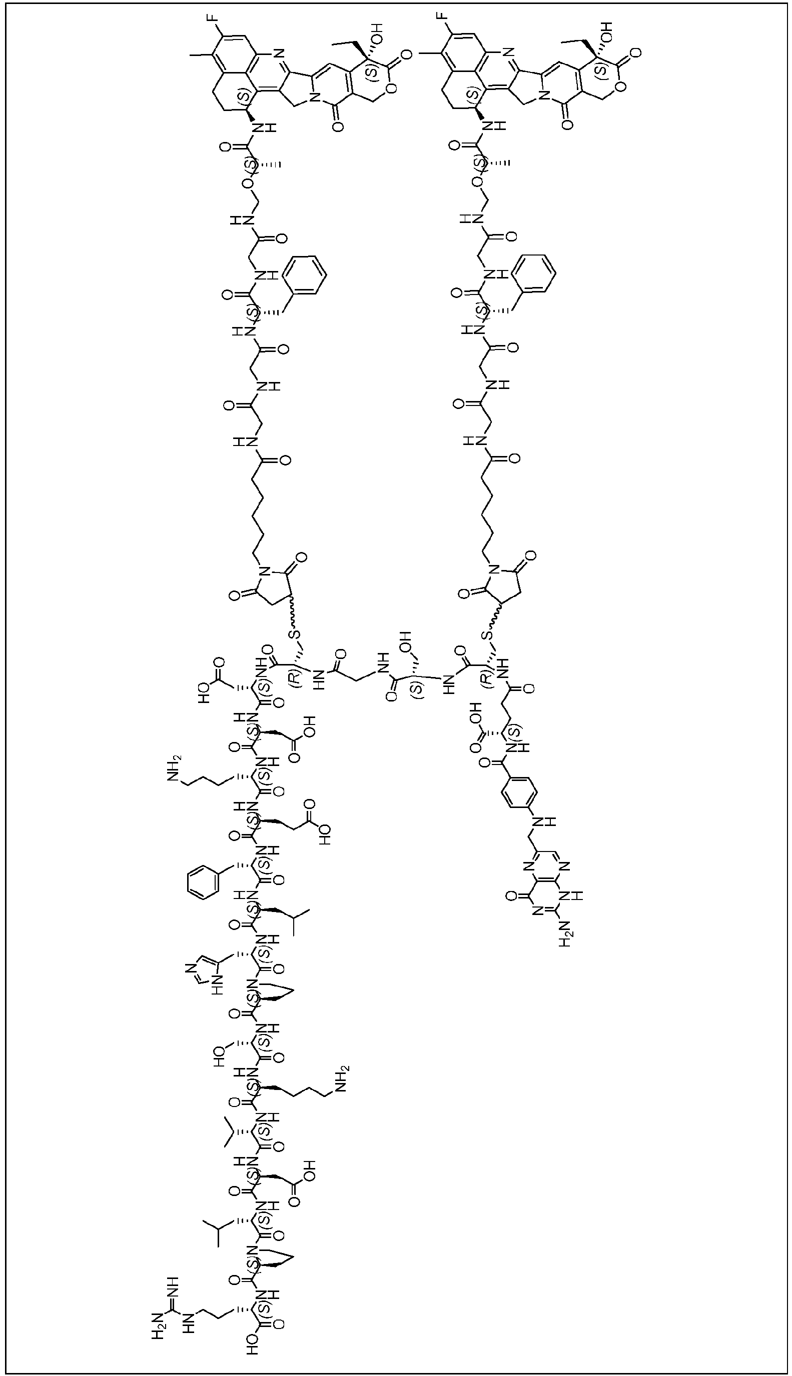

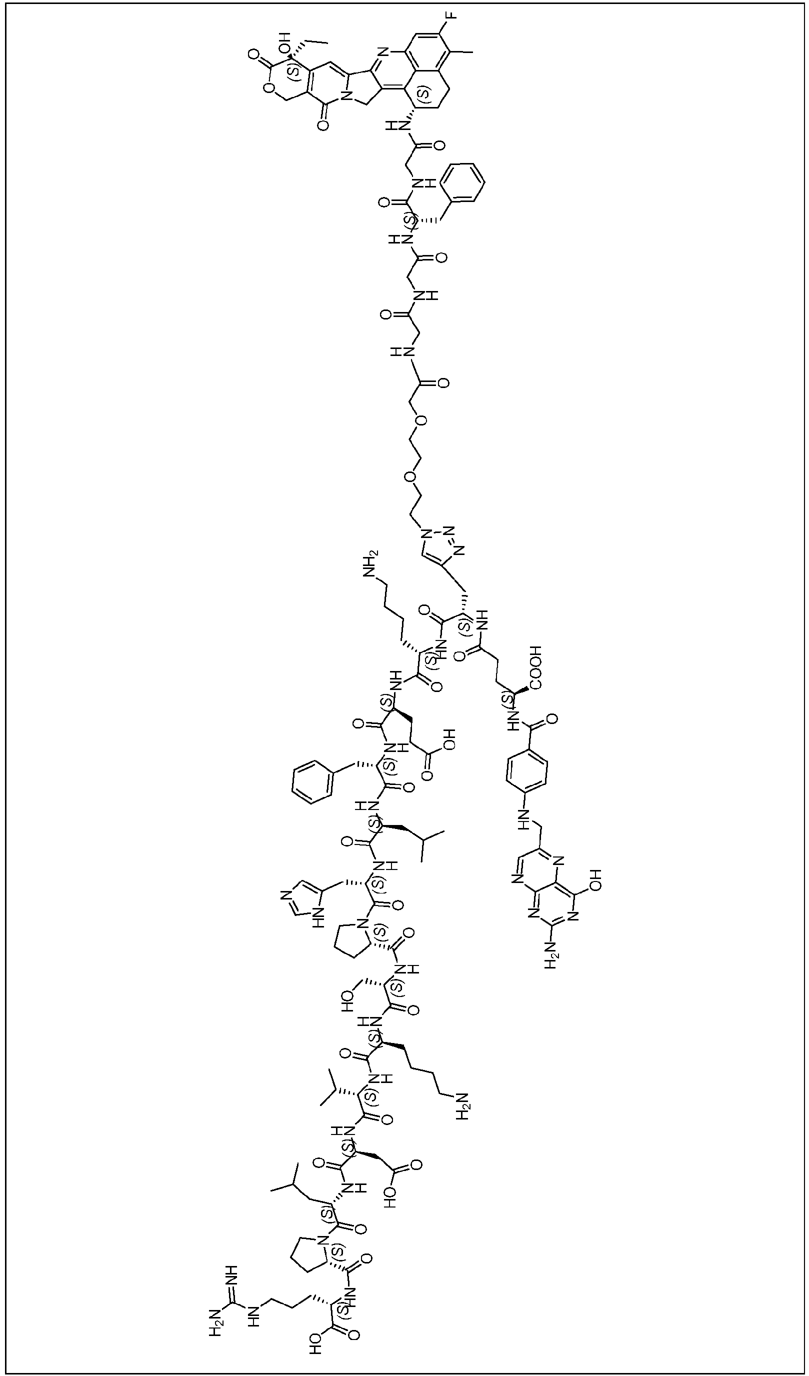

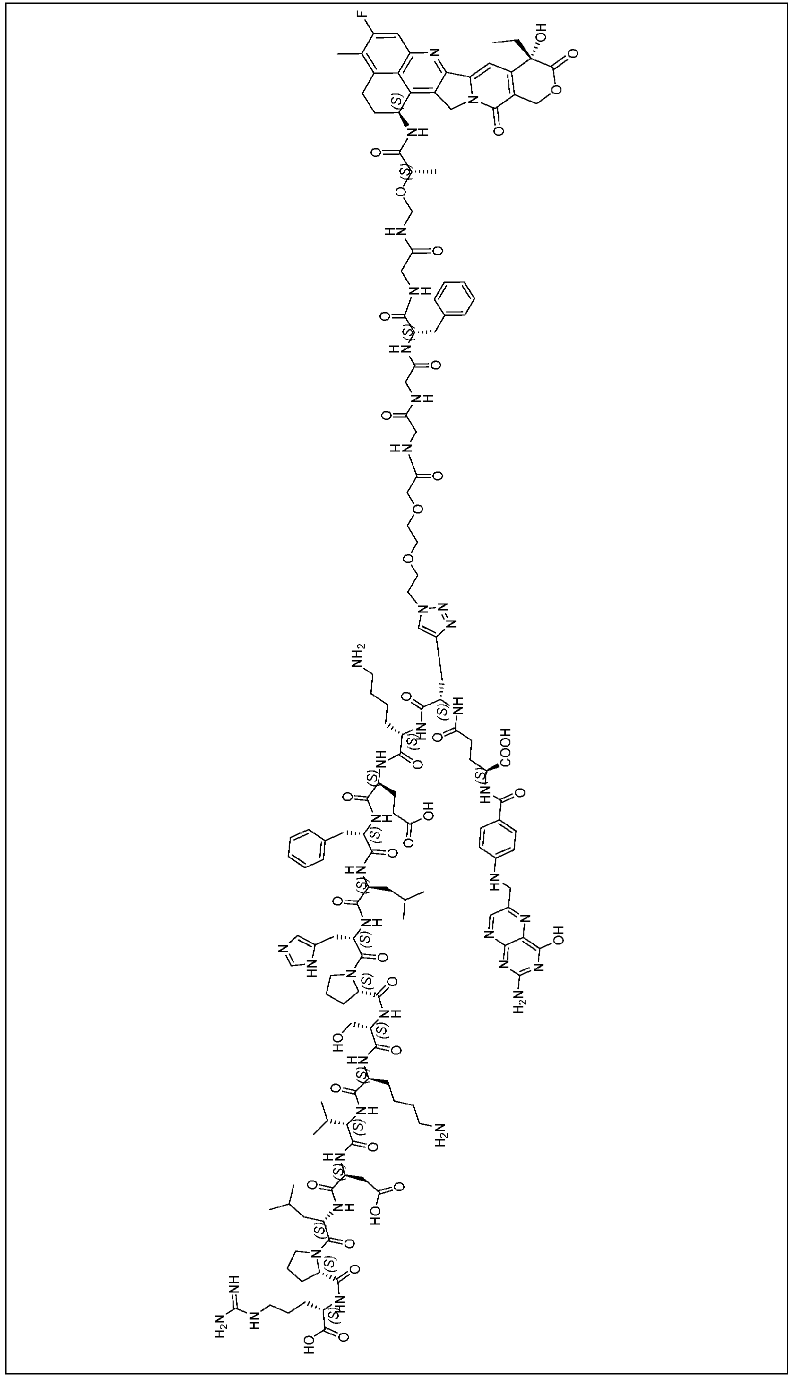

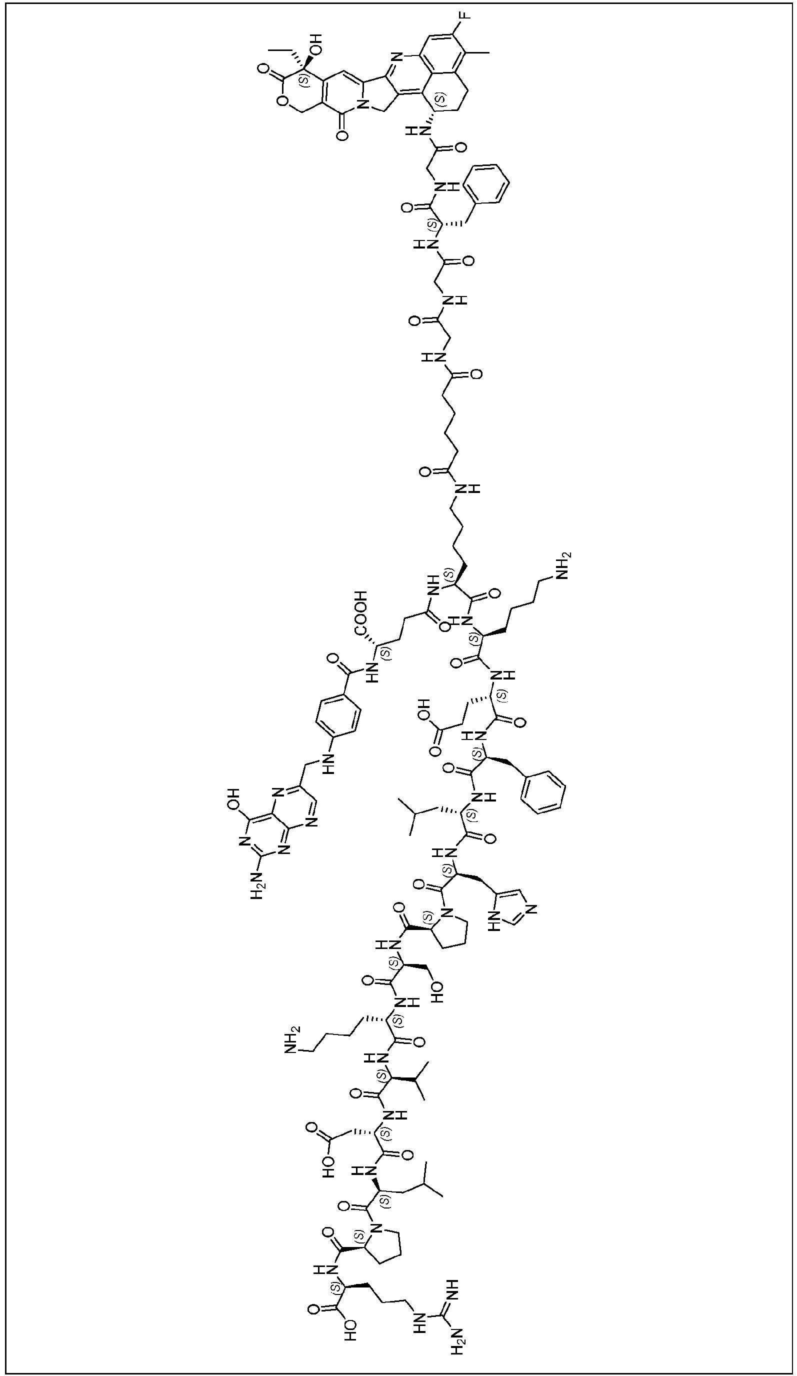

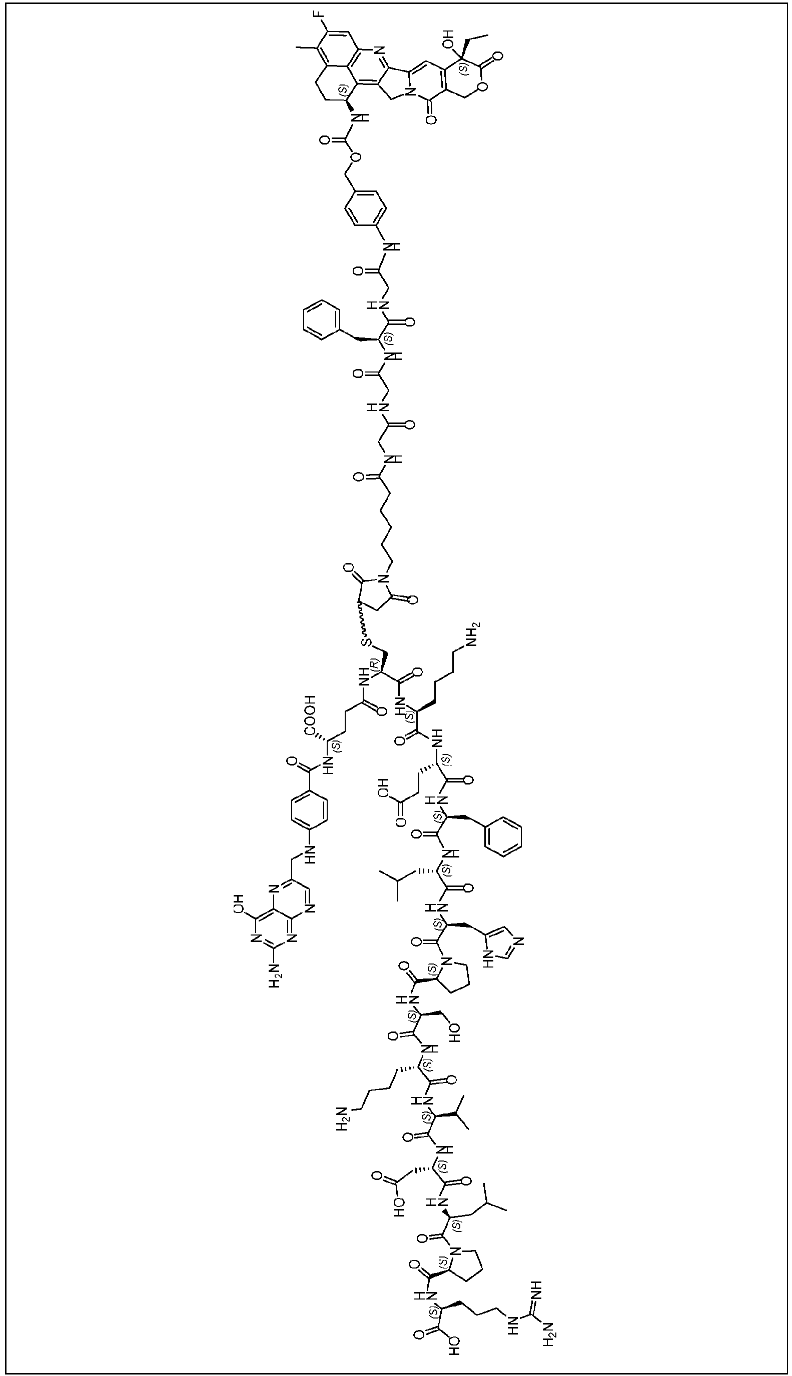

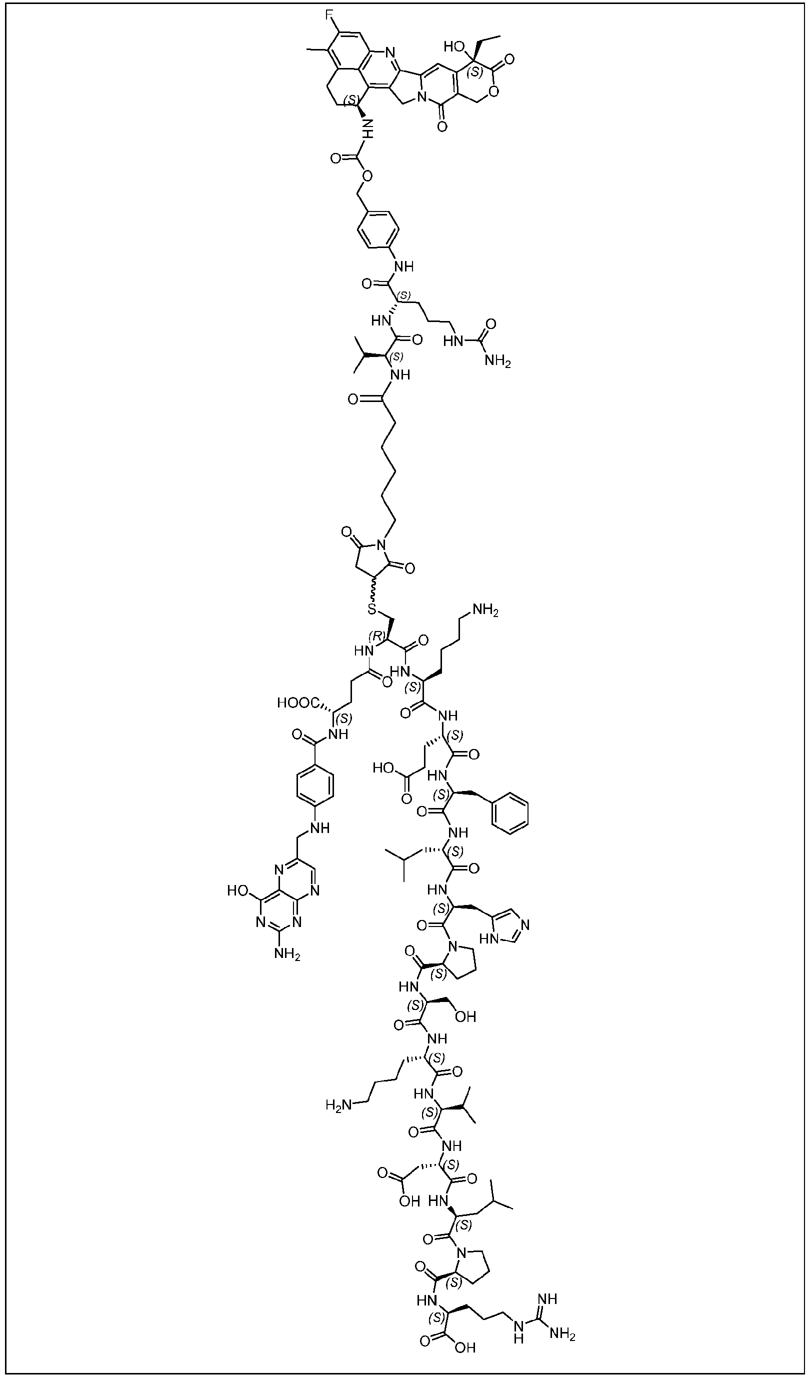

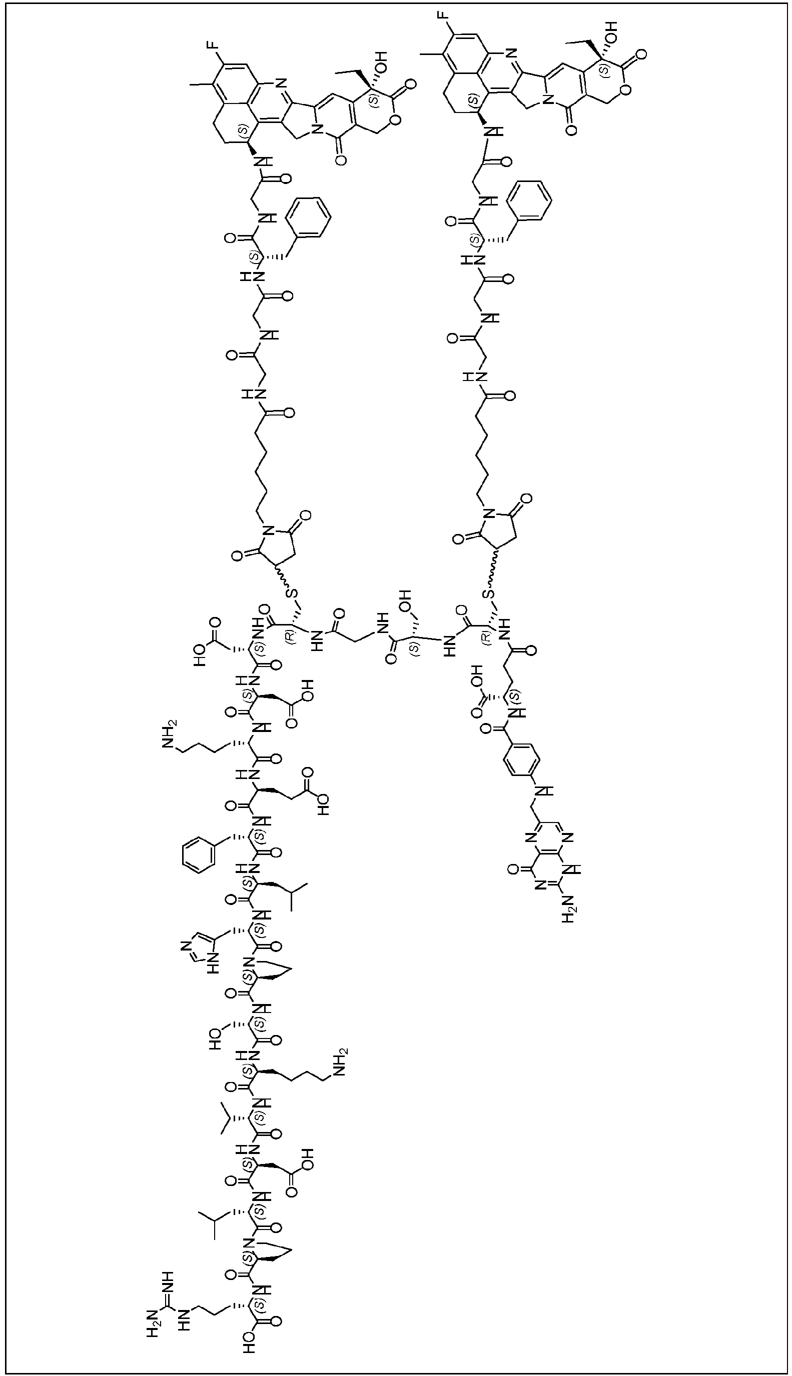

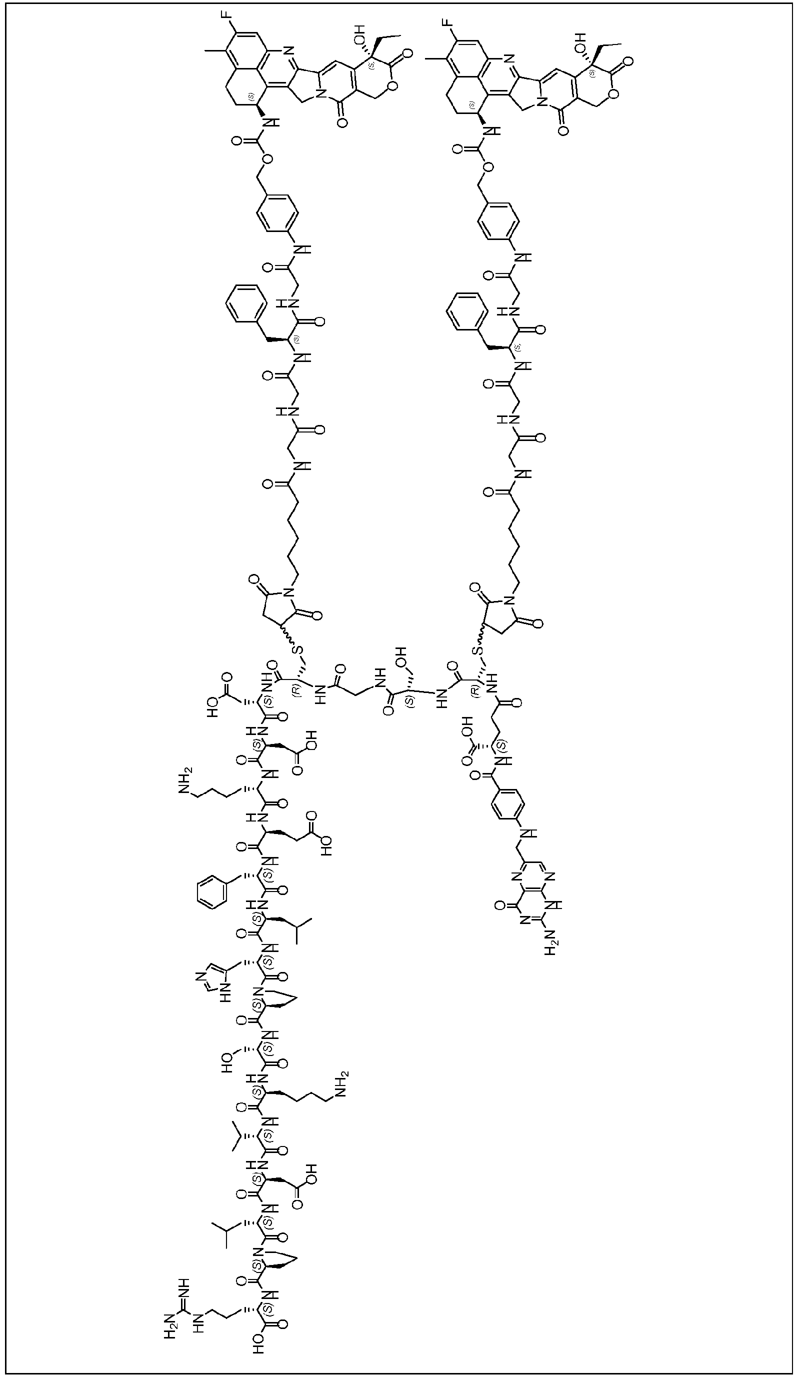

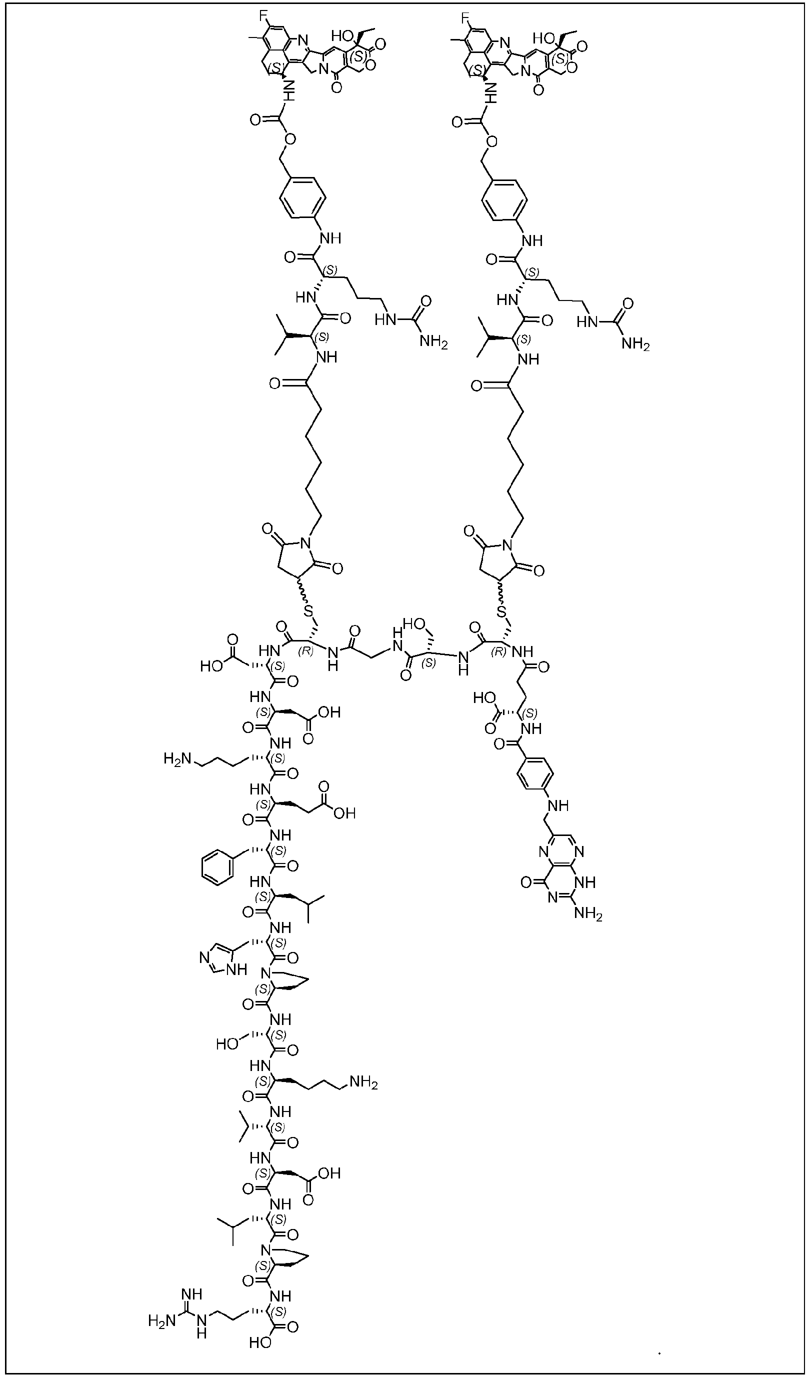

46. A pharmaceutical composition comprising the conjugate compound or the pharmaceutically acceptable salt thereof according to any one of claims 1-45, and a pharmaceutically acceptable carrier.

47. The pharmaceutical composition according to claim 46, wherein, the composition is administered intravenously, subcutaneously, orally, intramuscularly or intraventricularly.

48. A method for delivering a payload to a subject in need thereof, comprising administering to the subject a therapeutically effective amount of the conjugate compound or the pharmaceutically acceptable salt thereof according to any one of claims 1-45, or the pharmaceutical composition according to claim 46 or 47.

49. A method for treating a disease in a subject, comprising administering to the subject a therapeutically effective amount of the conjugate compound or the pharmaceutically acceptable salt thereof according to any one of claims 1-45, or the pharmaceutical composition according to claim 46 or 47.

50. The method according to claim 49, wherein the disease is selected from the group of: a cancer, an immune disease, a metabolic disease, and a neurological disease.

51. The method according to claim 50, wherein the cancer is selected from the group of: pancreatic cancer, biliary tract cancer, liver cancer, breast cancer, thyroid cancer, colorectal cancer, esophagus cancer, lung cancer, kidney cancer, leukemia, ovarian cancer, stomach cancer, uterine cancer, endometrial cancer, colon cancer, testicular cancer, skin cancer, prostate cancer, lymphoma, and multiple myeloma.

52. The method according to claim 50, wherein the immune disease is an autoimmune disease, and preferably, the autoimmune disease is selected from the group of: a connective tissue disease, systemic sclerosis, rheumatoid arthritis, and systemic lupus erythematosus.

53. The method according to claim 50, wherein the metabolic disease is selected from the group of: diabetes, gout, obesity, hypoglycemia, hyperglycemia, and dyslipidemia.

54. The method according to claim 50, wherein the neurological disease is selected from the group of: Alzheimer's disease, Parkinson's disease, Huntington's disease, head injury, multiple sclerosis, vertigo, coma, and epilepsy.

55. A method according to any one of claims 49-54, wherein the method further comprises administering one or more therapeutic agents in combination with the conjugate compound or the pharmaceutically acceptable salt thereof, or the pharmaceutical composition.

56. Use of the conjugate compound or the pharmaceutically acceptable salt thereof according to any one of claims 1-45, or the pharmaceutical composition according to claim 46 or 47 in preparing a medicine for treating a disease in a subject.

57. The use according to claim 56, wherein the disease is selected from the group of: a cancer, an immune disease, a metabolic disease, and a neurological disease.

58. The use according to claim 57, wherein the cancer is selected from the group of: pancreatic cancer, biliary tract cancer, liver cancer, breast cancer, thyroid cancer, colorectal cancer, esophagus cancer, lung cancer, kidney cancer, leukemia, ovarian cancer, stomach cancer, uterine cancer, endometrial cancer, colon cancer, testicular cancer, skin cancer, prostate cancer, lymphoma, and multiple myeloma.

59. The use according to claim 57, wherein the immune disease is an autoimmune disease, and preferably, the autoimmune disease is selected from the group of: a connective tissue disease, systemic sclerosis, rheumatoid arthritis, and systemic lupus erythematosus.

60. The use according to claim 57, wherein the metabolic disease is selected from the group of: diabetes, gout, obesity, hypoglycemia, hyperglycemia, and dyslipidemia.

61. The use according to claim 57, wherein the neurological disease is selected from the group of: Alzheimer's disease, Parkinson's disease, Huntington's disease, head injury, multiple sclerosis, vertigo, coma, and epilepsy.

62. The use according to claim 56, wherein the administration frequency of the conjugate compound or the pharmaceutically acceptable salt thereof or the pharmaceutical composition thereof is once a week, twice a week, three times a week, once every three days, once every two days, once a day, twice a day, or three times a day.

63. The use according to claim 56, wherein the administration frequency of the conjugate compound or the pharmaceutically acceptable salt thereof or the pharmaceutical composition thereof is once a week.

64. The use according to claim 56, wherein the administration frequency of the conjugate compound or the pharmaceutically acceptable salt thereof or the pharmaceutical composition thereof is three times a day.

65. The use according to claim 56, wherein the conjugate compound or the pharmaceutically acceptable salt thereof or the pharmaceutical composition thereof is administered at a dose range of 1-150 mg/kg of the conjugate compound.

66. The use according to claim 56, wherein the conjugate compound or the pharmaceutically acceptable salt thereof or the pharmaceutical composition thereof is administered at a dose of 1 mg/kg, 3 mg/kg, 10 mg/kg, 25 mg/kg, 50 mg/kg, 75 mg/kg, 100 mg/kg or 150 mg/kg of the conjugate compound.

67. The use according to claim 56, wherein the conjugate compound or the pharmaceutically acceptable salt thereof or the pharmaceutical composition thereof is administered at a dose of 50 mg/kg of the conjugate compound.

68. The use according to claim 56, wherein the conjugate compound or the pharmaceutically acceptable salt thereof or the pharmaceutical composition thereof is administered at a dose of 25 mg/kg of the conjugate compound.
